(19) Europäisches Patentamt
European Patent Office
Office européen des brevets

(11) EP 0 938 483 B1

(12) **EUROPEAN PATENT SPECIFICATION**

(45) Date of publication and mention of the grant of the patent:
**26.02.2003 Bulletin 2003/09**

(21) Application number: **97936296.9**

(22) Date of filing: **06.08.1997**

(51) Int Cl.7: **C07D 405/00**, A61K 31/00

(86) International application number:
**PCT/US97/13383**

(87) International publication number:
**WO 98/005292 (12.02.1998 Gazette 1998/06)**

(54) **MUSCARINIC ANTAGONISTS**

MUSCARIN-ANTAGONISTEN

ANTAGONISTES MUSCARINIQUES

(84) Designated Contracting States:
**AT BE CH DE DK ES FI FR GB GR IE IT LI LU NL PT SE**
Designated Extension States:
**LT LV RO**

(30) Priority: **08.08.1996 US 700628**

(43) Date of publication of application:
**01.09.1999 Bulletin 1999/35**

(73) Proprietor: **SCHERING CORPORATION**
**Kenilworth New Jersey 07033 (US)**

(72) Inventors:
- **LOWE, Derek, B.**
  **Kenilworth, NJ 07033 (US)**
- **CHANG, Wei, K.**
  **Livingston, NJ 07039 (US)**
- **KOZLOWSKI, Joseph, A.**
  **Princeton, NJ 08543-7391 (US)**
- **BERGER, Joel, G.**
  **Tucson, AZ 85718 (US)**
- **McQUADE, Robert**
  **Scotch Plains, NJ 07076 (US)**
- **BARNETT, Allen**
  **Pine Brook, NJ 07058 (US)**
- **SHERLOCK, Margaret**
  **Bloomfield, NJ 07003 (US)**
- **TOM, Wing**
  **Cedar Grove, NJ 07009 (US)**
- **DUGAR, Sundeep**
  **Bridgewater, NJ 08807 (US)**
- **CHEN, Lian-Yong**
  **Plainsboro, NJ 08536 (US)**
- **CLADER, John, W.**
  **Cranford, NJ 07016 (US)**
- **CHACKALAMANNIL, Samuel**
  **East Brunswick, NJ 08816 (US)**
- **WANG, Yuguang**
  **North Brunswick, NJ 08902 (US)**
- **McCOMBIE, Stuart, W.**
  **Caldwell, NJ 07006 (US)**
- **TAGAT, Jayaram, R.**
  **Westfield, NJ 07090 (US)**
- **VICE, Susan, F.**
  **Mountainside, NJ 07092 (US)**
- **VACCARO, Wayne, D.**
  **Yardley, PA 19067 (US)**
- **GREEN, Michael, J.**
  **Encinitas, CA 92024 (US)**
- **BROWNE, Margaret, E.**
  **Bloomfield, NJ 07003 (US)**
- **BOYLE, Craig, D.**
  **Bridgewater, NJ 08807 (US)**
- **JOSIEN, Hubert, B.**
  **Jersey City, NJ 07310 (US)**
- **ASBEROM, Theodros**
  **West Orange, NJ 07052 (US)**

(74) Representative: **Ritter, Stephen David et al**
**Mathys & Squire**
**100 Gray's Inn Road**
**London WC1X 8AL (GB)**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 938 483 B1

## Description

BACKGROUND OF THE INVENTION

The present invention relates to di-N-substituted piperazines and 1,4-di-substituted piperidines useful in the treatment of cognitive disorders, pharmaceutical compositions containing the compounds, and to the use of said compounds in combination with acetylcholinesterase inhibitors.

Alzheimer's disease and other cognitive disorders have received much attention lately, yet treatments for these diseases have not been very successful. According to Melchiorre et at. (J. Med. Chem. (1993), 36, 3734-3737), compounds that selectively antagonize M2 muscarinic receptors, especially in relation to M1 muscarinic receptors, should possess activity against cognitive disorders. Baumgold et al. (Eur. J. of Pharmacol., 251, (1994) 315-317) disclose 3-$\alpha$-chloroimperialine as a highly selective m2 muscarinic antagonist.

The present invention is predicated on the discovery of a class of di-N-substituted piperazines and 1,4-di-substituted piperidines, some of which have m2 selectivity even higher than that of 3-$\alpha$-chloroimperialine. Logemann et al (Brit. J. Pharmacol (1961), 17, 286-296) describe certain di-N-substituted piperazines, but these are different from the inventive compounds of the present invention. Furthermore, the compounds of Logemann et al. are not disclosed to have activity against cognitive disorders.

International publication no. W096/26196 discloses benzylpiperidines and piperazines as muscarinic antagonists. However, this document does not disclose or suggest the specific terminal N-substituted piperidyl groups of the present invention.

SUMMARY OF THE INVENTION

The present invention relates to compounds according to the structural formula I,

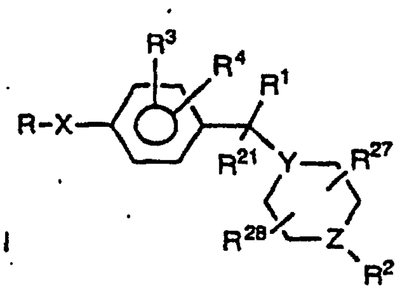

I

including all isomers and pharmaceutically acceptable salts, esters, and solvates thereof,

wherein one of Y and Z is N and the other is N, CH, or C-alkyl;

X is -O-, -S-, -SO-, $-SO_2-$, $-NR^6-$, -CO-, $-CH_2-$, -CS-, $-C(OR^5)_2-$, $-C(SR^5)_2-$, $-CONR^{20}-$, $-C(alkyl)_2-$, -C(H)(alkyl)-, $-NR^{20}-SO_2-$, $-SO_2-NR^{20}-$, $-NR^{20}CO-$, -O-CO-NH-, -NH-CO-O-,

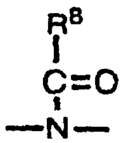 , 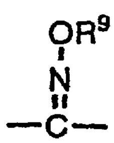 , 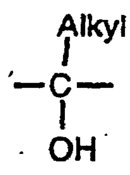 , 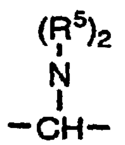 , 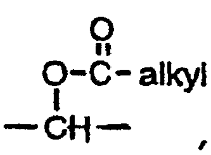 ,

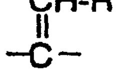 , 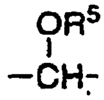 , CH = CH— , —C ≡ C— , 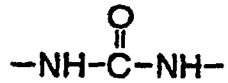 ,

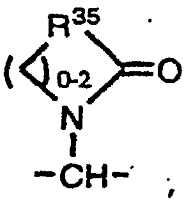 ; 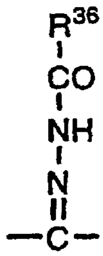 , 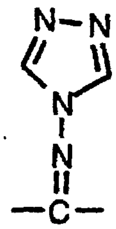 , 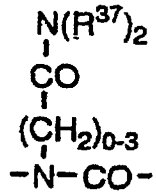 , 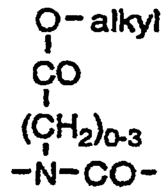 or 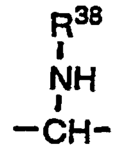 ;

R is

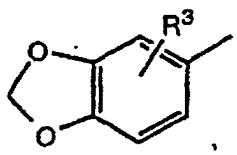 , 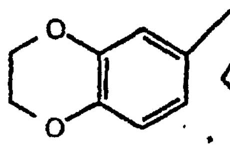 , 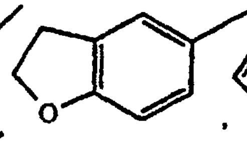 , 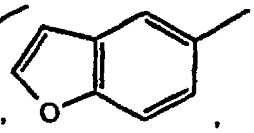 ,

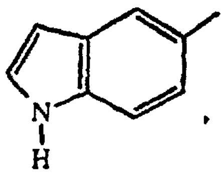 , 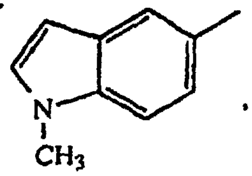 , 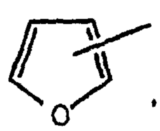 , 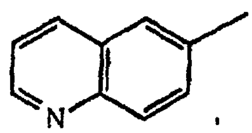 ,

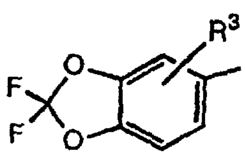 , 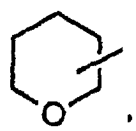 , 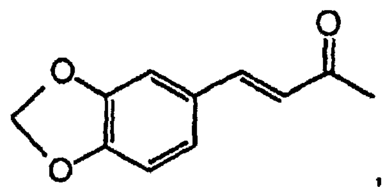 ,

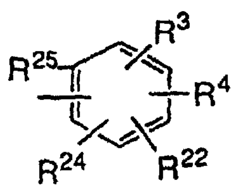 , 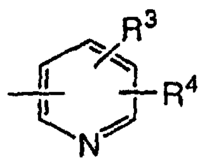 , 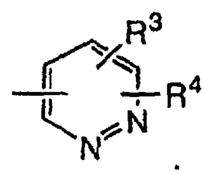 , 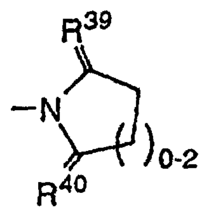 ,

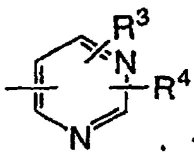 , 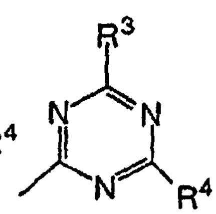 , 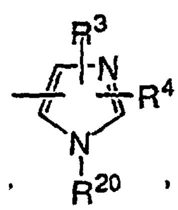 , 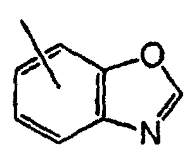 ,

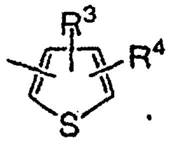, 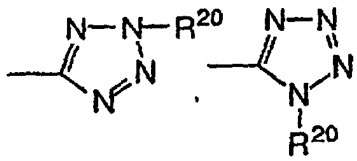, 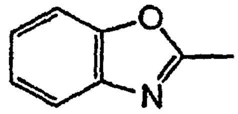,

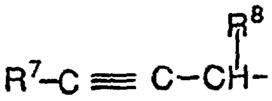, 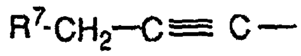, 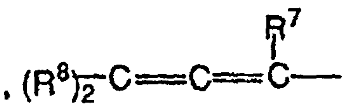,

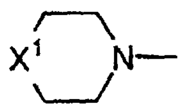

(wherein $X^1$ is $-CH_2-$, -O-, or $-NR^7-$),

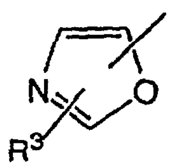, 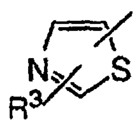, 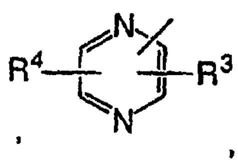, 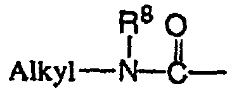,

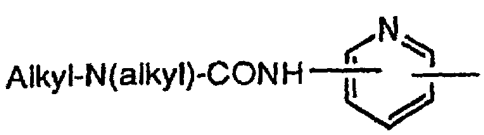,

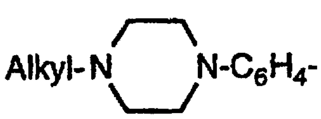, 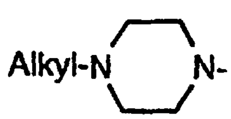, 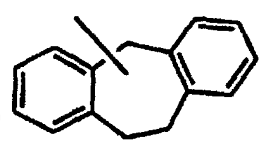,

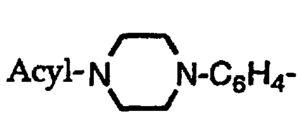, 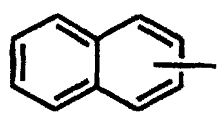,

hydrogen, acyl, alkyl, alkoxy, alkenyl, cycloalkyl, cycloalkyl substituted with one or two groups selected from the group consisting of alkyl and carbonyl, cycloalkenyl, bicycloalkyl, arylalkenyl, benzyl, benzyl substituted with up to three independently selected $R^3$ groups, cycloalkylalkyl, polyhaloacyl, benzyloxyalkyl, hydroxy$C_2$-$C_{20}$alkyl, alkenylcarbonyl, alkylarylsulfonyl, alkoxycarbonylaminoacyl, alkylsulfonyl, or arylsulfonyl, additionally, when X is $-CH_2-$, R may also be -OH; in further addition, when X is not N, R may also be hydroxymethyl, in further addition, R and X may combine to form the group Prot-$(NOAA)_r$-NH- wherein r is an integer of 1 to 4, Prot is a nitrogen protecting group and when r is 1, NOAA is a naturally occuring amino acid or an enantiomer thereof, or when r is 2 to 4, each NOAA is a peptide of an independently selected naturally occuring amino acid or an enantiomer thereof;

$R^1$ and $R^{21}$ are independently selected from the group consisting of H, alkyl, alkenyl, cycloalkyl, cycloalkenyl, bicycloalkyl, alkynyl, cyano, aminoalkyl, alkoxycarbonyl, aminocarbonyl, hydroxyamidino, alkoxycarbonylalkyl, phenyl

alkyl, alkylcarbonlyoxyalkyl,

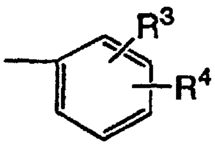,
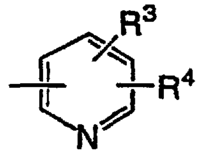,
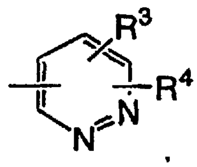,
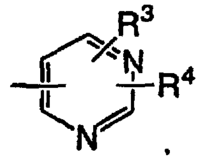,
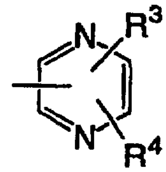,
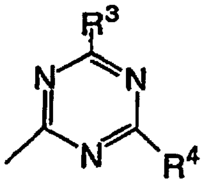,
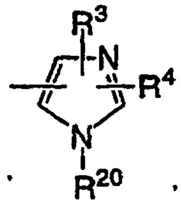,
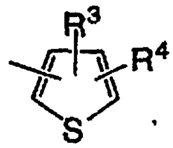,
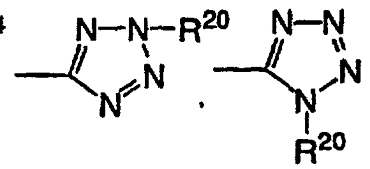,

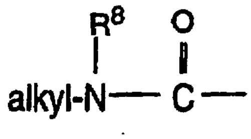,
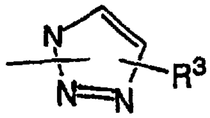,
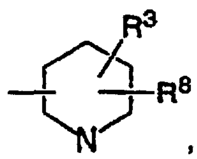,

H, —OH, (provided $R^1$ and $R^{21}$ are both not —OH and Y is not N), formyl, —CO alkyl, —COacyl, —COaryl, and hydroxyalkyl; additionally $R^1$ and $R^{21}$ together may form the group $=CH_2$, $=N-OR^5$, =N-CN, $=N-N(R^5)_2$, =CH-alkyl, alkylene,

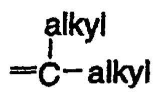

or $=C(halo)_2$; in further addition, $R^1$ and $R^{21}$ together with the carbon atom to which they are attached may form the group

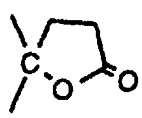 or 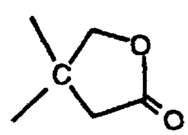

or $R^1$ and $R^{21}$ together with the carbon atom to which they are attached may form a saturated heterocyclic ring containing 3 to 7 carbon atoms, one or more of which may be optionally substituted by alkyl, and one or two groups independently selected from S, O, and $N-R^{20}$;

$R^2$ is

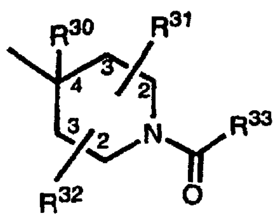 or 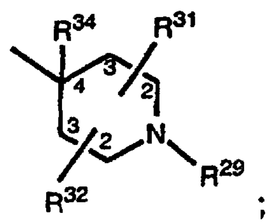;

$R^3$, $R^4$, $R^{22}$, $R^{24}$, and $R^{25}$ are independently selected from the group consisting of alkyl, H, halo, alkoxy, benzyloxy, benzyloxy substituted by nitro or aminoalkyl, haloalkyl, polyhaloalkyl, nitro, cyano, sulfonyl, hydroxy, amino, alkylamino, formyl, alkylthio, polyhaloalkoxy, acyloxy, trialkylsilyl, alkylsulfonyl, arylsulfonyl, acyl, alkoxycarbonyl alkylsulfinyl;

$-OCONH_2$, -OCONH-alkyl, alkylaminoalkyl, dialkylaminoalkyl, -COOH, $-CON(R^{20})_2$, $-OCON(alkyl)_2$, -NHCOO-alkyl, -NHCO-alkyl, phenyl, hydroxyalkyl, or morpholino;

each $R^5$ and $R^6$ is independently selected from the group consisting of H and alkyl, provided that when X is $C(OR^5)_2$ or $C(SR^5)_2$, both $R^5$ groups cannot be H, and in addition, when X is $C(OR^5)_2$ or $C(SR^5)_2$, the two $R^5$ groups in X may be joined to form $-(CR^{20}{}_2)_p-$ wherein p is an integer of 2 to 4;

$R^7$ is independently selected from the group consisting of H, alkyl, arylalkyl, cycloalkyl, aryl and aryl substituted with $R^3$ and $R^4$ as defined herein;

each $R^8$ is independently selected from the group consisting of H, hydroxyalkyl or alkyl, or two $R^8$ groups may be joined to form an alkylene group;

$R^9$ is H, alkyl, aralkyl, or acyl;

$R^{20}$ is H, aryl or alkyl;

$R^{27}$ and $R^{28}$ are independently selected from the group consisting of H, alkyl, hydroxyalkyl, arylalkyl, aminoalkyl, haloalkyl, thioalkyl, alkylthioalkyl, carboxyalkyl, imidazolyalkyl, and indolyalkyl; or $R^{27}$ and $R^{28}$ may combine to form an alkylene group;

$R^{29}$ is H, alkyl, -CO-alkyl, -CO-cycloalkyl, alkoxycarbonyl, aminocarbonyl, aryloxycarbonyl, alkylaminocarbonyl, dialkylaminocarbonyl, alkylsulfonyl, arysulfonyl or $-SO_2-NH-R^{20}$;

$R^{30}$ is H, alkyl, aryl, cycloalkyl, hydroxyalkyl, aminoalkyl, $-COOR^{20}$, $-CON(R^{20})_2$ or cyano;

$R^{31}$ and $R^{32}$ are the same as $R^{30}$ and in addition, two $R^{30}$, $R^{31}$ and $R^{32}$ groups may form the group $-(CH_2)_r-$ (wherein r is 1 to 6), in further addition, $R^{31}$ and $R^{32}$ can also be hydroxy, $-N(R^{20})_2$, -O-acyl, $-N(R^{20})$acyl, $-OCOOR^{20}$, or $-OCON(R^{20})_2$;

$R^{33}$ is aryl or heteroaryl, with the proviso that when $R^{33}$ is heteroaryl, the $CO-R^{33}$ bond is to a carbon atom In the $R^{33}$ group;

$R^{34}$ is alkyl, cycloalkyl or aryl and in addition $R^{34}$ may also be H when $R^1$ and $R^{21}$ together with the carbon atom to which they are attached form a saturated heterocyclic ring containing 3 to 7 carbon atoms and two groups independently selected from S, O, and $N-R^{20}$;

$R^{35}$ is $-CH_2-$, $-NR^{20}-$ or -O-;

$R^{36}$ is $-NH_2$, alkyl or alkoxy;

$R^{37}$ is independently selected from the group consisting of H and alkyl;

$R^{38}$ is $-CO-(CH_2)_{0-5}-OR^5$, $-SO_2$-(alkyl), or

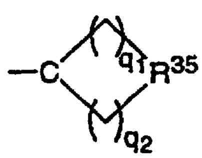

wherein $q_1$ and $q_2$ are independently 1-5, provided that the sum of $q_1$ and $q_2$ is 2-5; and

$R^{39}$ and $R^{40}$ are independently selected from the group consisting of =O and (H,H).

In a preferred group of compounds Y and Z are N.

In another preferred group of compounds Y is CH and Z is N.

In another preferred group of compounds R is

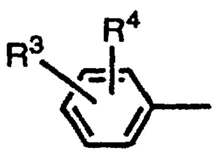
,
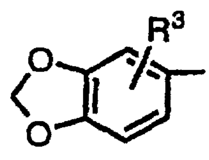
, or
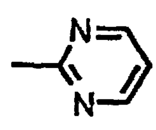

and X is O, SO, $SO_2$, $CH_2$, CH(alkyl), $C(alkyl)_2$, -CH(OH)-, or $-N(R^{20})CO$.

In another preferred group of compounds $R^3$ and $R^4$ are H and either $R^1$ is cycloalkyl, or alkyl, and $R^{21}$ is H or $R^1$ and $R^{21}$ together form =O.

In another preferred group of compounds R is

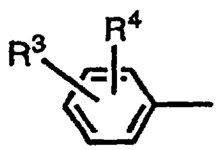, 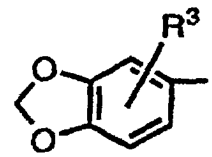, or 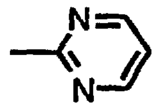.

X is O ,SO, $SO_2$, $CH_2$, CH(alkyl), $C(alkyl)_2$, or $-N(R^{20})CO$; $R^3$ and $R^4$ are H and either $R^1$ is cycloalkyl or alkyl; and $R^{21}$ is H or $R^1$ and $R^{21}$ together form =O or

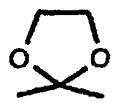.

In another preferred group of compounds at least one of $R^{27}$ and $R^{28}$ is alkyl.

In another preferred group of compound one of $R^{27}$ or $R^{28}$ is methyl and the other is hydrogen.

In another preferred group of compounds R is

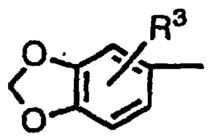 or 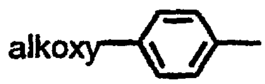.

In another preferred group of compounds X is $SO_2$, $CH_2$, or $-N(CH_3)-CO-$.

In another group of preferred compounds, $R^2$ has the formula

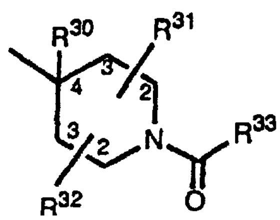

and $R^{30}$ is H or $CH_3$: $R^{31}$ and $R^{32}$ are H: and $R^{33}$ is ortho-substituted aryl or heteroaryl, preferably

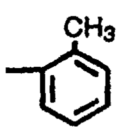. 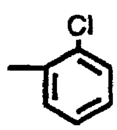. 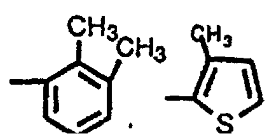, 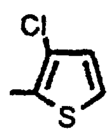 or 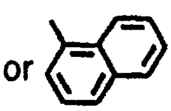

In another group of preferred compounds, $R^2$ has the formula

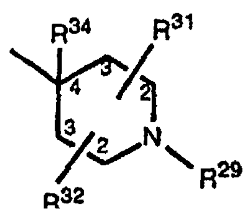

$R^{34}$ is methyl and $R^{31}$ and $R^{32}$ are H.

Preferred specific compounds of this invention are listed hereinafter as compound numbers: **17**, **18**,**25**, **30**, **31**, **32**, **34**, **35**, **36**, **37**, **41**, **43**, **44**, **49**, **53**, **54**, **56**, **57**, **58**, **59**, **80**, **82**, **84**, **85**, **94**, **98**, **100**, **108, 121, 126, 127, 137, 145,**

151, 152, 154, 155, 162, 166, 178, 179, 181, 185, 190, 191, 194, 199, 214, 215, 216, 225, 247, 253, 256, 257, 337, 339, 340, 341, 349, 351, 367, 409, 459, 479, 488, 489, 490, 500, 501, 502, 503, 505, 506, 507, 515, 516, 517, 555, 562.

Another aspect of the invention is a pharmaceutical composition which comprises a compound having structural formula I as defined above in combination with a pharmaceutically acceptable carrier.

Another aspect of the invention is the use of a compound formula I for the preparation of a pharmaceutical composition useful in the treatment of cognitive disorders and neurodegenerative diseases such as Alzheimer's disease.

Yet another aspect of the invention comprises a method for making a pharmaceutical composition comprising mixing a compound of formula I with a pharmaceutically acceptable carrier.

Another aspect of this invention is the use of a compound of formula I in the manufacture of a medicament for treating a cognitive or neurodegenerative disease.

Another aspect of this invention is the use of a compound of formula I in the manufacture of a medicament for treating a cognitive or neurodegenerative disease said medicament comprising an effective amount of a combination of a compound of formula I with an acetylcholinesterase inhibitor.

Another aspect of this invention is a kit comprising in separate containers in a single package pharmaceutical compounds for use in combination to treat cognitive disorders in one container a compound of formula I in a pharmaceutically acceptable carrier and in a second container an acetylcholinesterase inhibitor in a pharmaceutically acceptable carrier, the combined quantities being an effective amount.

## DETAILED DESCRIPTION

Except where stated otherwise the following definitions apply throughout the present specification and claims. These definitions apply regardless of whether a term is used by itself or in combination with other terms. Hence the definition of "alkyl" applies to "alkyl" as well as the "alkyl" portions of "alkoxy", "haloalkyl", etc.

Alkyl represents a straight or branched saturated hydrocarbon chain having 1 to 20 carbon atoms, more preferably 1 to 8 carbon atoms.

Alkenyl represents a straight or branched hydrocarbon chain of from 2 to 15 carbon atoms, more preferably 2 to 12 carbon atoms, having at least one carbon-to-carbon double bond.

Alkynyl represents a straight or branched hydrocarbon chain of from 2 to 10 carbon atoms, more preferably 2 to 8 carbon atoms, having at least one carbon-to-carbon triple bond.

Cycloalkyl represents a saturated carbocyclic ring having 3 to 12 carbon atoms.

Cycloalkenyl represents a carbocyclic ring having from 5 to 8 carbon atoms and at least one carbon-to-carbon double bond in the ring.

Bicycloalkyl represents a saturated bridged carbocyclic ring having 5 to 12 carbon atoms.

Acyl represents a radical of a carboxylic acid having the formula alkyl-CO-, aryl-CO-, aralkyl-CO-, cycloalkyl-CO-, alkylcycloalkyl-CO-, and heteroaryl-CO-.

Halo represents fluoro, chloro, bromo or iodo.

Aryl represents phenyl or naphthyl optionally substituted with 1 to 5 $R^3$ groups.

Heteroaryl represents a cyclic group of 5 or 6 atoms, or a bicyclic group of 9 or 10 atoms, at least one of which is carbon and having at least one O, S, or N atom interrupting a carbocyclic ring having a sufficient number of pi electrons to provide aromatic character. Carbon atoms may optionally be substitiuted by $R^3$ groups. Nitrogen atoms may optionally be substituted $-R^{20}$ or $-COR^{20}$ groups. Preferred heteroaromatic groups are pyridyl, furanyl, benzofuranyl, thienyl, benzothienyl, thiazolyl, thiadiazolyl, imidazolyl, pyrimidinyl, pyrazinyl, pyridazinyl, 1,3,5-triazinyl, and indolyl.

Polyhalo represents substitution of at least 2 halo atoms to the group modified by the term "polyhalo".

Hydroxyamidino represents a group having the formula

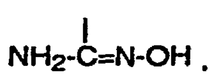

Azabicyclo represents a saturated bridged ring containing from 4 to 8 carbon atoms and at least one nitrogen atom.

Sulfonyl represents a group of the formula $-SO_2-$.

Sulfinyl represents a group of the formula -SO-.

Alkylene represents a group having the formula $-(CH_2)_q$, wherein q is an integer of from 1 to 20.

Naturally occurring amino acid (NOAA) means an acid selected from the group consisting of alanine(ala), arginine (arg), asparagine (asn), aspartic acid (asp), cysteine (cys), glutamine (gln), glutamic acid (glu), glycine (gly),

histadine (his), isoleucine (ile), leucine (leu), lysine (lys), methionine (met), phenylalanine (phe), proline (pro), serine (ser), threonine (thr), tryptophan (trp), tyrosine (tyr), and valine (val).

Nitrogen protecting group (Prot) means a group capable of protecting a nitrogen on a naturally occurring amino acid (or an enantiomer thereof) from reaction. Preferred nitrogen protecting groups are carbobenzyloxy (CBZ), $CH_3OCO(CH_2)_9CO$, and t-butoxycarbonyl (BOC). Of course any operable nitrogen protecting group is included.

When a variable appears more than once in the structural formula, for example $R^5$ when X is $-C(OR^5)_2-$, the identity of each variable appearing more than once may be independently selected from the definition for that variable.

Compounds of this invention may exist in at least two stereo configurations based on the asymmetric carbon to which $R^1$ is attached, provided that $R^1$ and $R^{21}$ are not identical. Further stereoisomerism is present when X is SO, or $C(OR^5)_2$ (when the two $R^5$ groups are not the same) or when $R^{27}$ or $R^{28}$ is not hydrogen. Also within formula I there are numerous other possibilities for stereoisomerism. All possible stereoisomers of formula I are within the scope of the invention.

Compound of formula I can exist in unsolvated as well as solvated forms, including hydrated forms. In general, the solvated forms, with pharmaceutically acceptable solvents such as water, ethanol and the like, are equivalent to the unsolvated forms for purposes of this invention.
A compound of formula I may form pharmaceutically acceptable salts with organic and inorganic acids. Examples of suitable acids for salt formation are hydrochloric, sulfuric, phosphoric, acetic, citric, malonic, salicylic, malic, fumaric, succinic, ascorbic, maleic, methanesulfonic and other mineral and carboxylic acids well known to those skilled in the art. The salts are prepared by contacting the free base forms with a sufficient amount of the desired acid to produce a salt in the conventional manner; The free base forms may be regenerated by treating the salt with a suitable dilute aqueous base solution such as dilute aqueous sodium hydroxide, potassium carbonate, ammonia or sodium bicarbonate. The free base forms differ from their respective salt forms somewhat in certain physical properties, such as solubility in polar solvents, but the salts are otherwise equivalent to their respective free base forms for purposes of the invention.

Compounds in accordance with formula I may be produced by processes known to those skilled in the art as shown by the following reaction steps:

**General Description of Methods:**

Compounds wherein $R^2$ has the formula

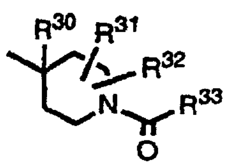

can be made as shown in Scheme 1.

Scheme 1

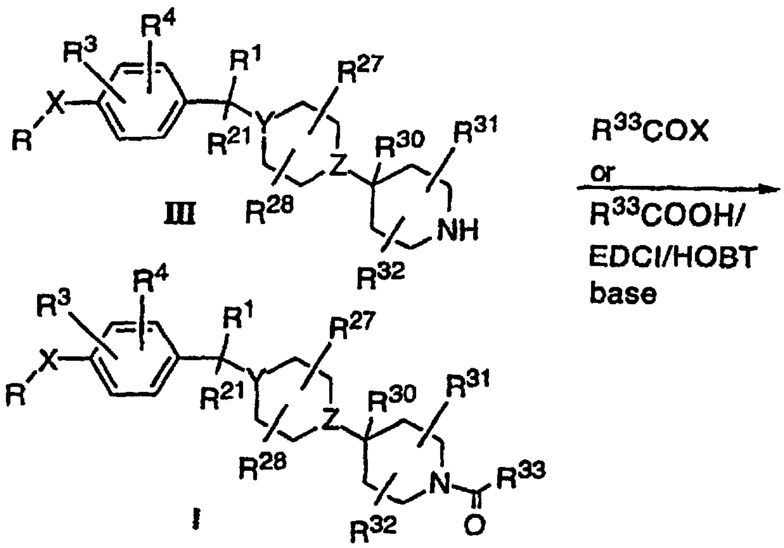


For compounds wherein $R^2$ has the formula

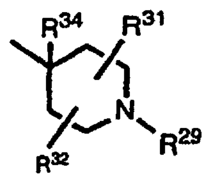
:

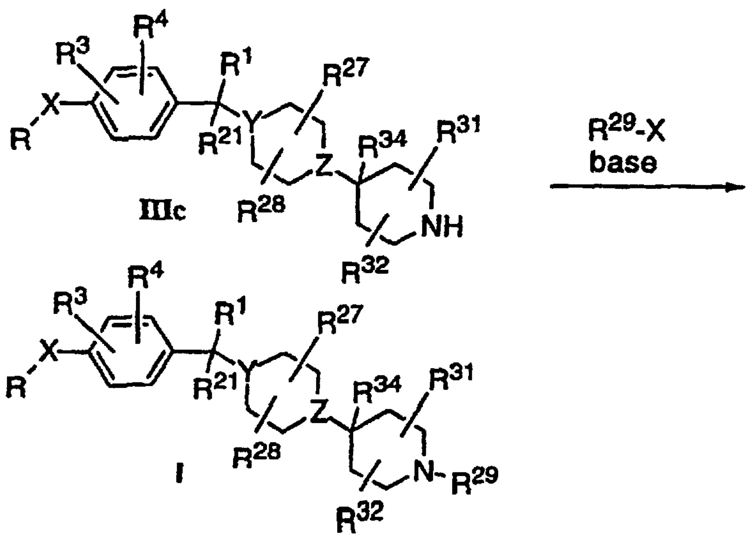


Intermediate **III** is treated with a suitably activated carboxylic acid derivative, $R_{33}$CO-X, where X is a leaving group such as halogen or $OCOCH_3$. Alternatively, a mixture of compound **III** and $R_{33}$COOH can be treated with N-hydroxybenzotriazole and a carbodiimide such as N-ethyl-N-(3-dimethylaminopropyl)-carbodiimide hydrochloride or dicyclohexylcarbodiimide in the presence of a base such as triethylamine and a solvent such as DMF to give **I.** Alternatively, intermediate **IIIc** is treated with a group $R_{29}$-X, where X and $R_{29}$ are as previously defined (except $R_{29}$≠H), in the presence of a base such as triethylamine.

Intermediates **III** can be made via a variety of methods. When X= $CH_2$ or CO, **III** can be made as shown in Scheme 2:

Scheme 2

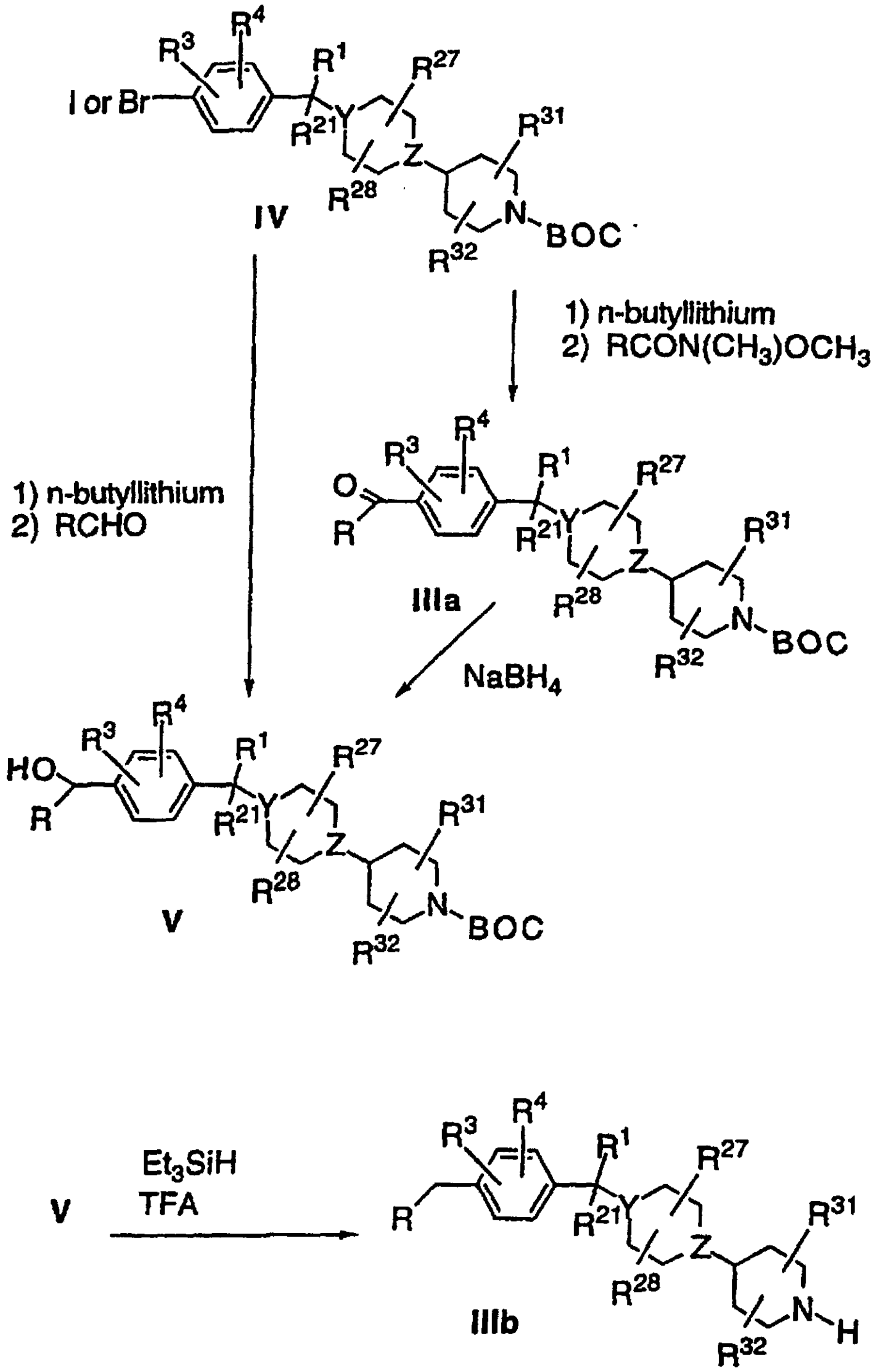


Aryl halide **IV** is treated with an alkyllithium such as n-butyllithium or t-butyllithium followed by reaction with an $RCON(CH_3)OCH_3$ to give compound **IIIa,** which can be deprotected and converted to **I** as shown above. Additionally, **IIIa** can be reduced to the alcohol **V** with a suitable reducing agent such as sodium borohydride. Compound **V** can also be made by treatment of **IV** with an alkyllithium as previously described followed by reaction with RCHO. Compound **V** is converted to compounds **IIIb** by treating **V** with a reducing agent such as triethylsilane in the presence of a strong acid such as trifluoroacetic acid.

Compounds of formula **Ia, Ib,** and **Ic** can be prepared via a related sequence as shown in Scheme 3:

Scheme 3

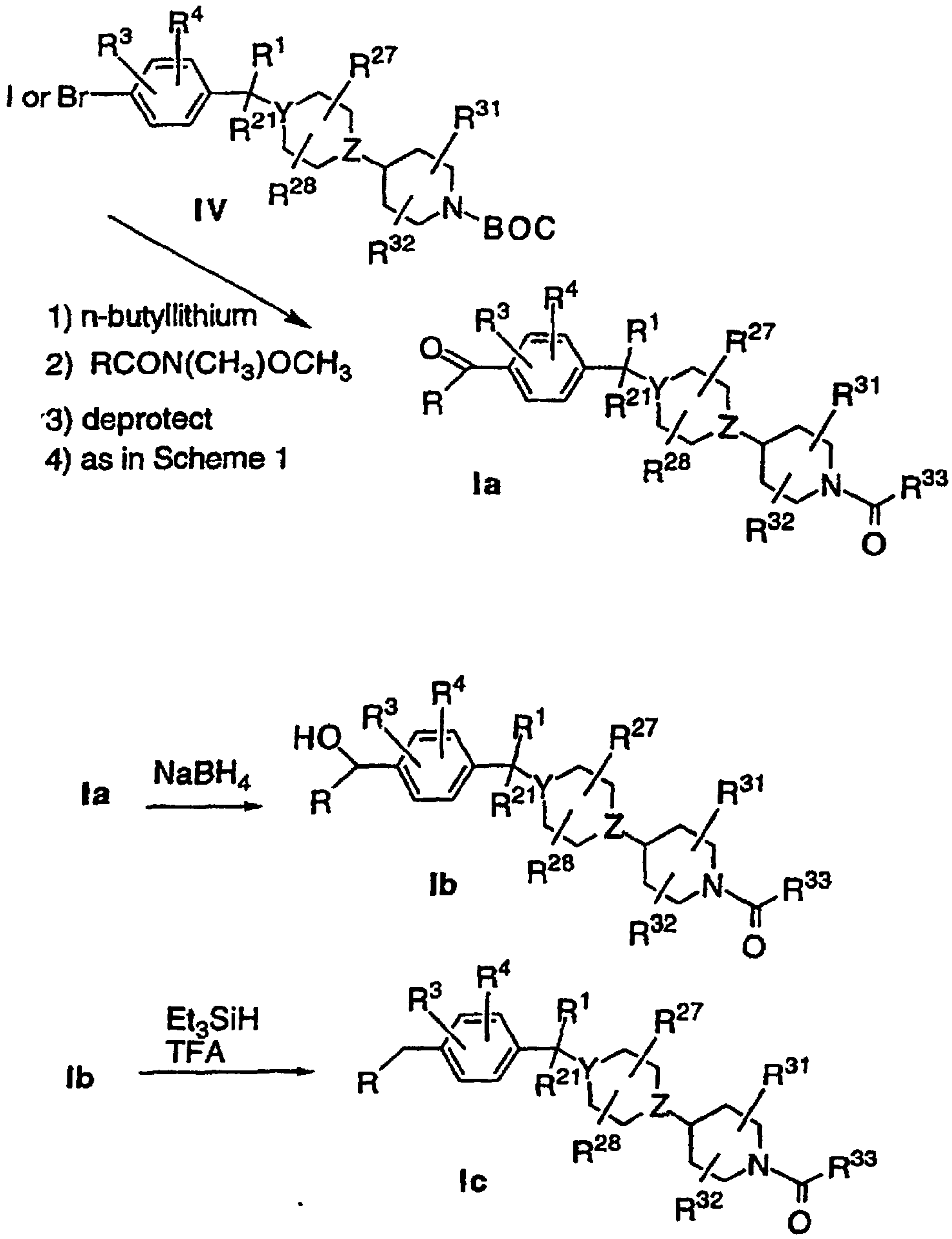


Intermediate **IIIa** is prepared as shown in Scheme 2, deprotected and then converted to **Ia** using methods of Scheme 1. This can then be converted to **Ib** and **Ic** using methods of Scheme 2.

Other compounds of this invention can be prepared by methods similar to those described in Schemes 2-3 wherein the aryl lithium reagent derived from **IV** is reacted with various electrophiles. For instance, compounds where **X** is NHCO or N(alkyl)CO can be prepared as shown in Scheme 4:

Scheme 4

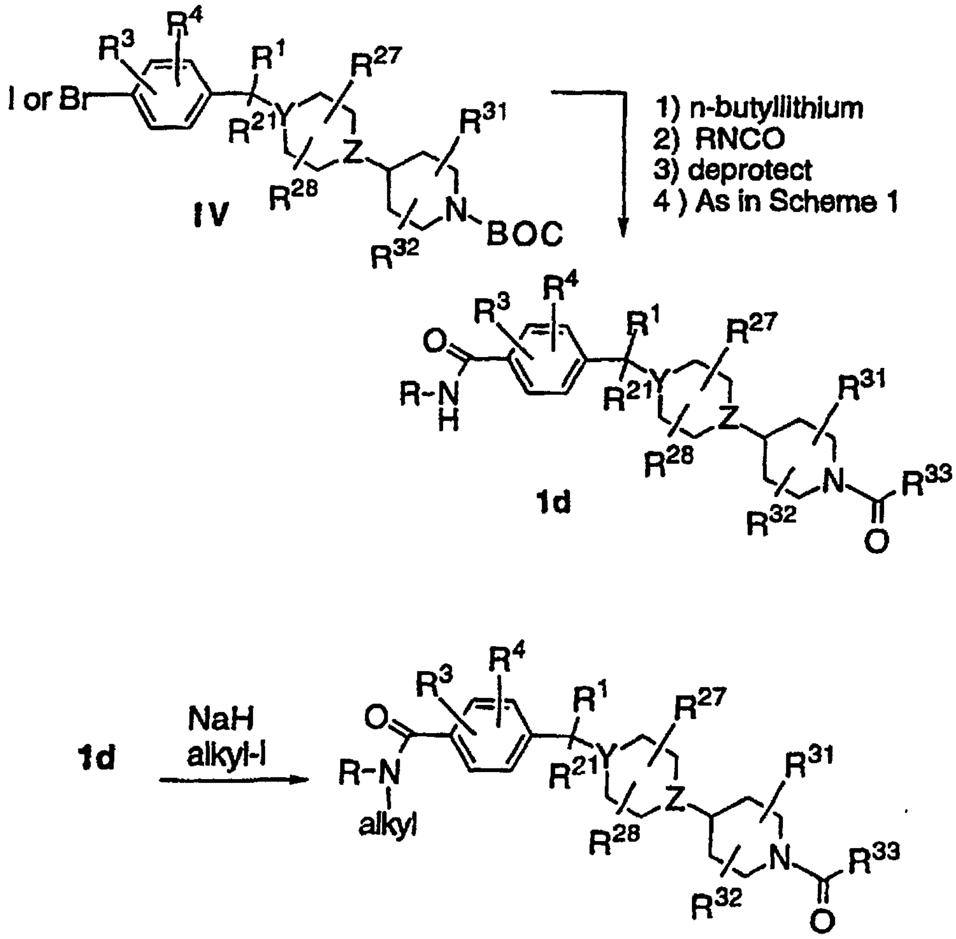


Intermediate **IV** is reacted with an alkyllithium as previously described followed by addition of an isocyanate RNCO. The intermediate is deprotected and converted to compounds of type **1d** as described in Scheme 1. Compounds of type **1d** are converted to compounds **1e** by reacting with an alkyliodide such as methyliodide in the presence of a suitable base such as sodium hydride.

Intermediate **IV** can be prepared via one of the following procedures:

## Scheme 5

**Intermediate IVa:**

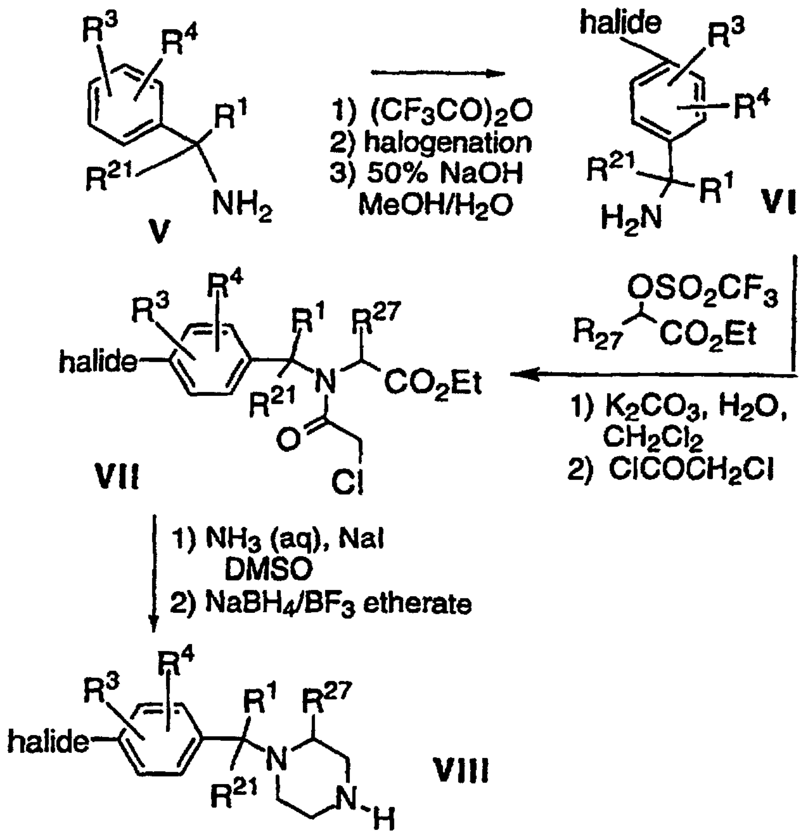


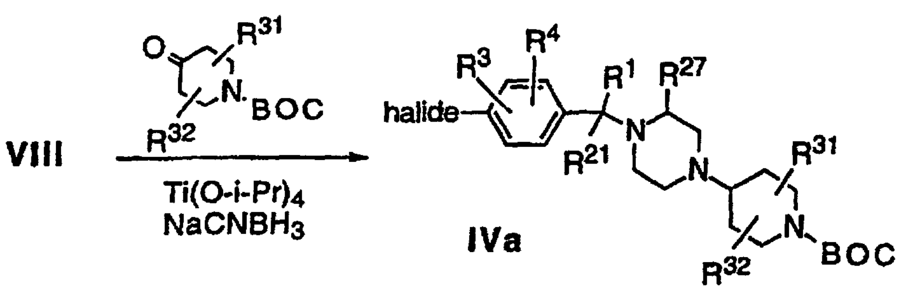


Benzylamine **V** is protected as its trifluoroacetamide, treated with a halogenating agent such as bromine, dibromodimethylhydantoin, or bis(trifluoroacetoxy)iodobenzene, and deprotected with aqueous base to give **VI.** This is treated with a carboxylic ester derivative containing a leaving group such as trifluoromethanesulfonate in the 2-position followed by chloroacetyl chloride to give **VII.** Treatment of **VII** with ammonia followed by reduction with $NaBH_4$/ $BF_3$ etherate gives **VIII.** Treatment of **VIII** with an N-BOC 4-piperidinone derivative preferable in the presence of a Lewis acid such as titanium tetraisopropoxide followed by a reducing agent such as sodium cyanoborohydride affords **IVa.**

## Scheme 6

**Intermediate IVb:**

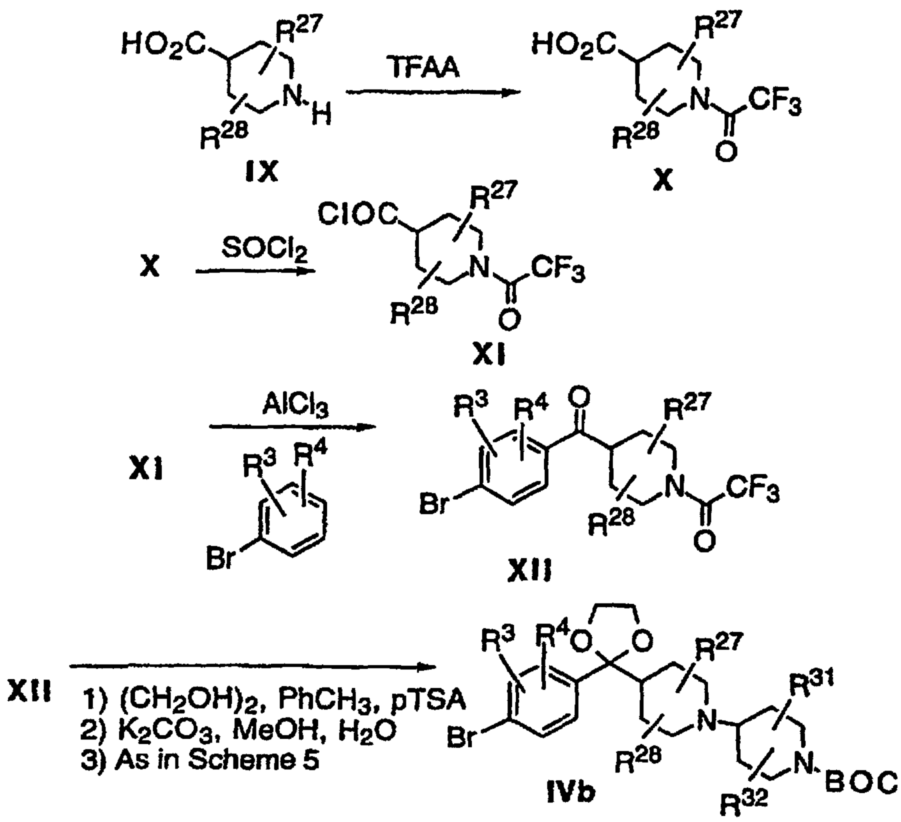


IX X XI XII IVb

A piperidine 4-carboxylic acid derivative **IX** is protected on nitrogen using trifluoroacetic anhydride and converted to the corresponding acid chloride **XI** using thionyl chloride. Intermediate **XI** is reacted with an arylhalide in the presence of a Lewis acid such as aluminum chloride to give **XII.** The carbonyl group of **XII** is protected by treatment with ethylene glycol in the presence of a strong acid such a toluenesulfonic acid. The nitrogen is deprotected using aqueous alcoholic base, and the resulting compound treated with an N-BOC 4-piperidinone derivative as described in Scheme 5 to afford **IVb.**

Compounds of formula **If** (where $R_{30} \neq H$) and **II** are prepared as shown in Scheme 7:

Scheme 7

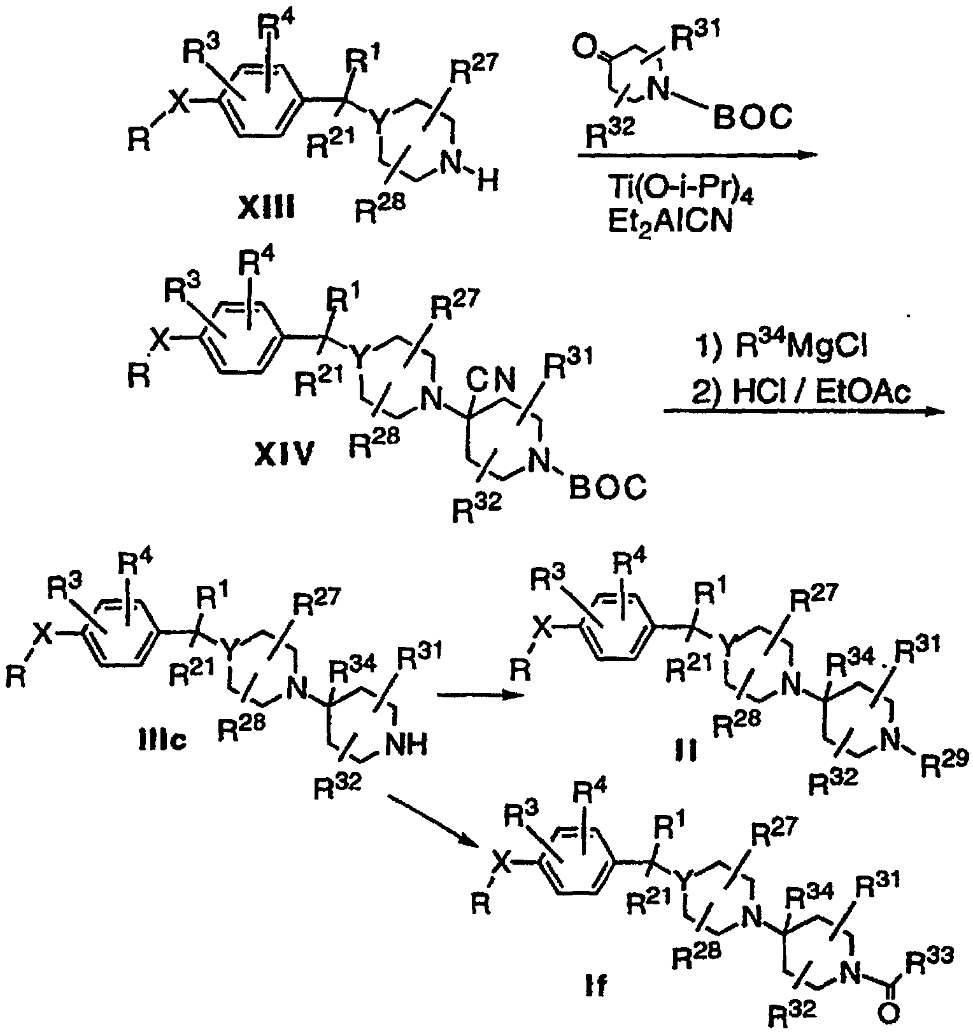


Intermediate **XIII** is treated with an N-BOC 4-piperidinone derivative preferably in the presence of a Lewis acid such as titanium tetraisopropxide followed by treatment with diethylaluminum cyanide to give **XIV.** This is treated with Grignard reagent $R_{34}MgCl$ followed by hydrolysis with aqueous acid to give **IIIc.** Compound **IIIc** can be converted to compounds of type If and **II** using the method shown in Scheme 1.

In addition to methods described above, compounds of formula **Ig** (where **A** is O, S or N-$R^2$O) can be prepared as described in Scheme 8.

Scheme 8

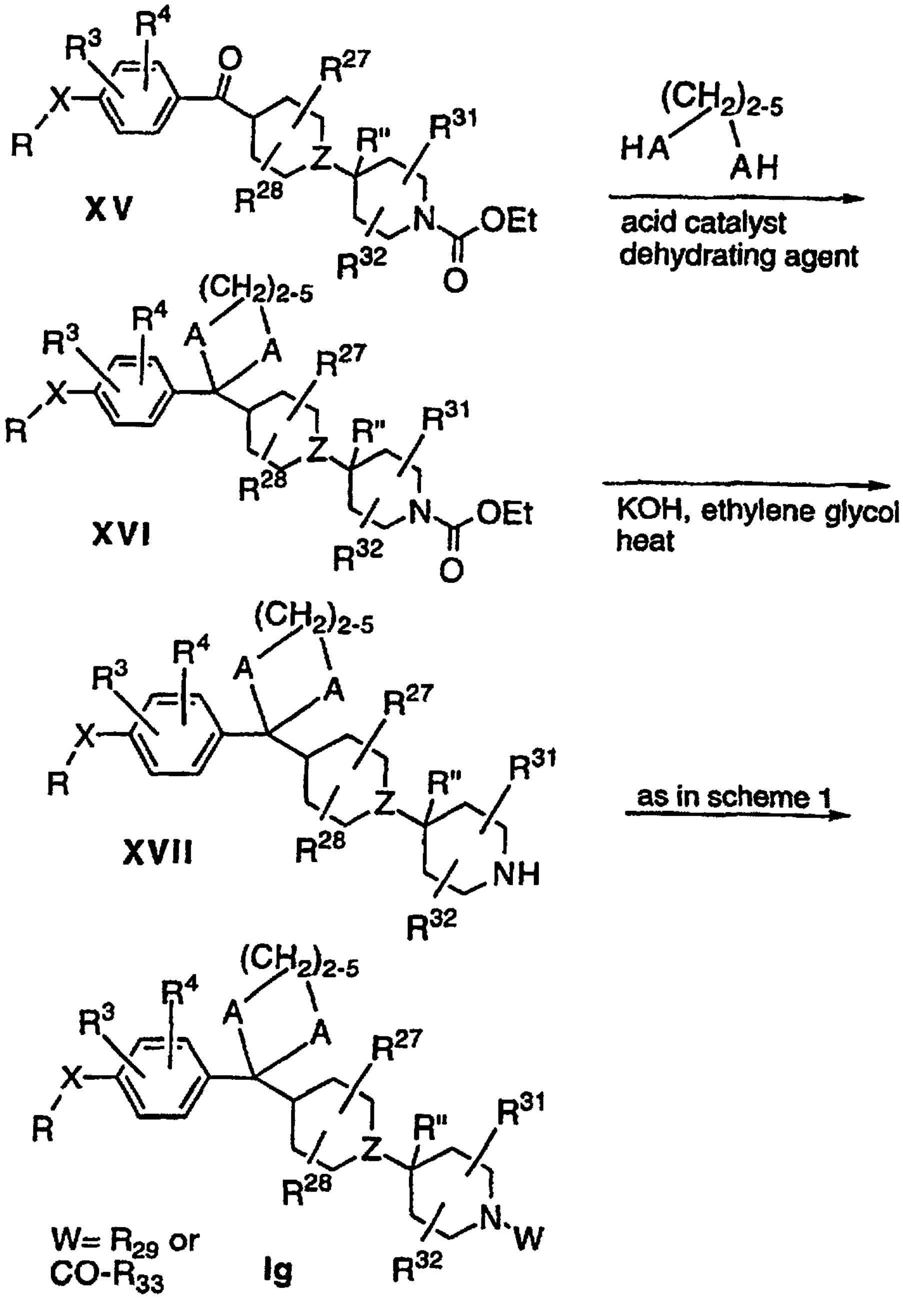


XV

XVI

XVII

W= $R_{29}$ or $CO-R_{33}$

Ig

In the compound HA-$(CH_2)_{2-5}$-AH, each A is independently O, S, or N-$R_{20}$, and each $CH_2$ group may optionally be substituted by one or more alkyl groups. Examples of the compound HA-$(CH_2)_{2-5}$-AH, include

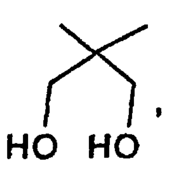 , 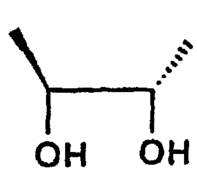 , and 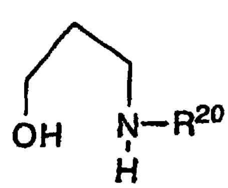

Compound **XV** ( where R" is either $R_{30}$ or $R_{34}$) is prepared as described in schemes 7 and 10. This is treated with HA-$(CH_2)_{2-5}$-AH optionally in the presence of an acid catalyst and a dehydrating agent. When A is O , the acid

catalyst is preferably an organic protic acid such as toluenesulfonic acid and the dehydrating agent is triethyl orthoformate. When A is S, a Lewis acid such as boron trifluoride etherate serves as both acid catalyst and dehydrating agent. The resulting product **XVI** is hydrolyzed with a strong base such as potassium hydroxide to give **XVII.** This is converted to to **Ie via** methods described in scheme 1.

When X= S, SO, or $SO_2$, intermediates **III** are prepared as shown in the following schemes:

Scheme 9

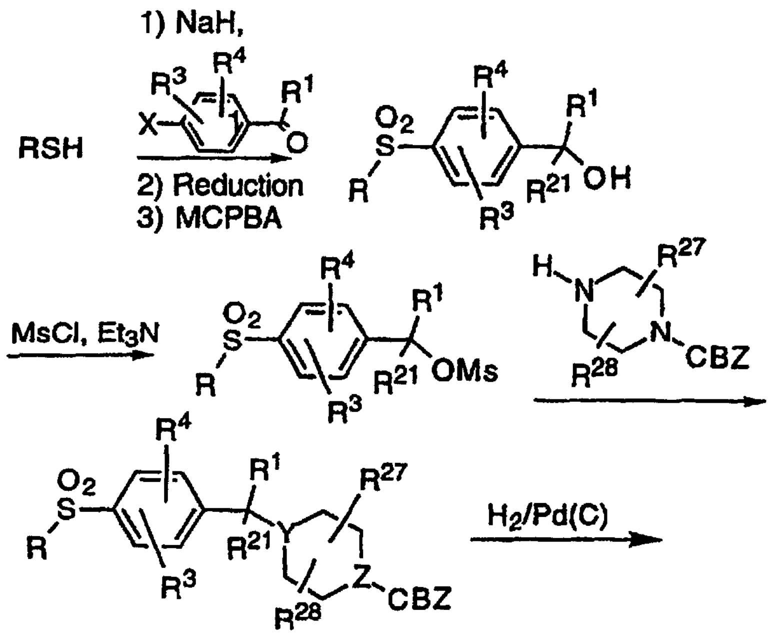

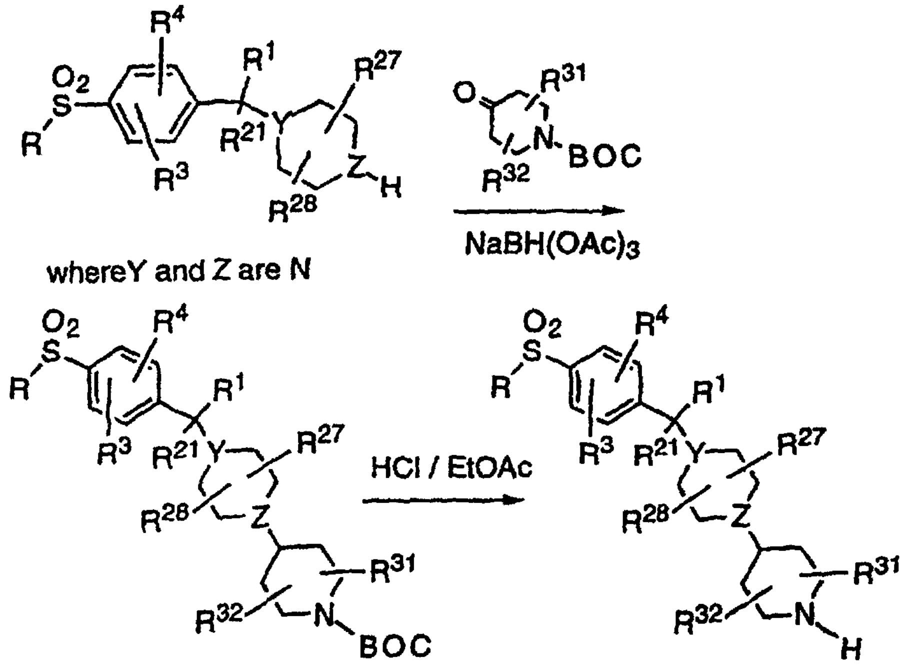


Scheme 10

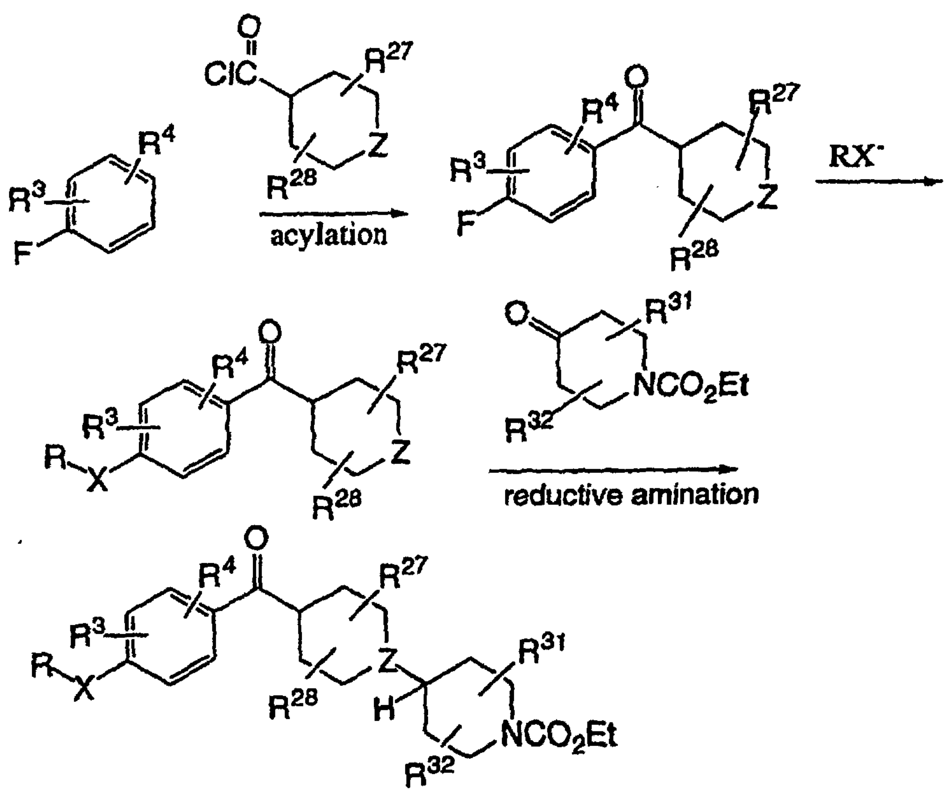


The above reactions may be followed if necessary or desired by one or more of the following steps; (a) removing any protective groups from the compound so produced; (b) converting the compound so-produced to a pharmaceutically acceptable salt, ester and/or solvate; (c) converting a compound in accordance with formula I so produced to another compound in accordance with formula I, and (d) isolating a compound of formula I, including separating stereoisomers of formula I.

Based on the foregoing reaction sequence, those skilled in the art will be able to select starting materials needed to produce any compound in accordance with formula I.

In the above processes it is sometimes desirable and/or necessary to protect certain groups during the reactions. Conventional protecting groups, familiar to those skilled in the art, are operable. After the reaction or reactions, the protecting groups may be removed by standard procedures.

The compounds of formula I exhibit selective m2 and/or m4 muscarinic antagonizing activity, which has been correlated with pharmaceutical activity for treating cognitive disorders such as Alzheimers disease and senile dementia.

The compounds of formula I display pharmacological activity in test procedures designated to indicate m1 and m2 muscarinic antagonist activity. The compounds are non-toxic at pharmaceutically therapeutic doses. Following are descriptions of the test procedures.

<u>MUSCARINIC BINDING ACTIVITY</u>

The compound of interest is tested for its ability to inhibit binding to the cloned human m1, m2, m3, and m4 muscarinic receptor subtypes. The sources of receptors in these studies were membranes from stably transfected CHO cell lines which were expressing each of the receptor subtypes. Following growth, the cells were pelleted and subsequently homogenized using a Polytron in 50 volumes cold 10 mM Na/K phosphate buffer, pH 7.4 (Buffer B). The homgenates were centrifuged at 40,000 x g for 20 minutes at 4°C. The resulting supematants were discarded and the pellets were resuspended in Buffer B at a final concentration of 20 mg wet tissue/ml. These membranes were stored at -80°C until utilized in the binding assays described below.

Binding to the cloned human muscarinic receptors was performed using $^3$H-quinuclidinyl benzilate (QNB) (Watson et al., 1986). Briefly, membranes (approximately 8, 20, and 14 µg of protein assay for the m1, m2, and m4 containing membranes, respectively) were incubated with $^3$H-QNB (final concentration of 100-200 pM) and increasing concentrations of unlabeled drug in a final volume of 2 ml at 25°C for 90 minutes. Non-specific binding was assayed in the presence of 1 µM atropine. The incubations were terminated by vacuum filtration over GF/B glass fiber filters using a Skatron filtration apparatus and the filters were washed with cold 10mM Na/K phosphate butter, pH 7.4. Scintillation cocktail was added to the filters and the vials were incubated overnight. The bound radioligand was quantified in a liquid scintillation counter (50% efficiency). The resulting data were analyzed for $IC_{50}$ values (i.e. the concentration of compound required to inhibit binding by 50%) using the EBDA computer program (McPherson, 1985). Affinity values ($K_i$) were then determined using the following formula (Cheng and Prusoff, 1973);

$$K_i = \frac{IC_{50}}{1+\left[\dfrac{\text{concentration of radioligand}}{\text{affinity } (K_D) \text{ of radioligand}}\right]}$$

Hence, a lower value of $K_i$ indicates greater binding affinity.

To determine the degree of selectivity of a compound for binding the m2 receptor, the $K_i$ value for m1 receptors was divided by the $K_i$ value for m2 receptors. A higher ratio indicates a greater selectivity for binding the m2 muscarinic receptor.

| RESULTS OF THE TESTS | | | |
|---|---|---|---|
| | **Ki (nM)** | | |
| Compound No. | m1 | m2 | m4 |
| <u>**17**</u> | 47.33 | 0.14 | 2.26 |
| <u>**18**</u> | 48.37 | 0.11 | 0.77 |
| <u>**25**</u> | 337.68 | 0.55 | 6.51 |
| <u>**31**</u> | 308.95 | 0.63 | 12.10 |
| <u>**41**</u> | 29.9 | 0.06 | 1.47 |
| <u>**44**</u> | 36.79 | 0.11 | 0.76 |
| <u>**84**</u> | 12.32 | 0.04 | 0.06 |
| <u>**94**</u> | 28.99 | 0.02 | 0.76 |
| <u>**100**</u> | 6497 | 0.12 | 3.38 |
| <u>**554**</u> | 0.81 | 0.01 | 0.04 |
| <u>**590**</u> | 29.33 | 0.03 | 0.80 |

For the compounds appearing in the TABLE OF COMPOUNDS the following range of muscarinic antagonistic activity was observed

m2: 0.01 nM to 106 nM
m1: 0.24 nM to 1900 nM
m4: 0.02 nM to 705 nM

For preparing pharmaceutical compositions from the compounds of formula I, compounds capable of enhancing ACh release, and ACh'ase inhibitors, pharmaceutically acceptable, inert carriers are admixed with the active compounds. The pharmaceutically acceptable carriers may be either solid or liquid. Solid form preparations include powders, tablets, dispersible granules, capsules, cachets and suppositories. A solid carrier can be one or more substances which may also act as dilutents, flavoring agents, solubilizers, lubricants, suspending agents, binders or tablet disintegrating agents; it may also be an encapsulating material.

Liquid form preparations include solutions, suspensions and emulsions. As an example may be mentioned water or water-propylene glycol solutions for parenteral injection.

Also included are solid form preparations which are intended to be converted, shortly before use, to liquid form preparations for either oral or parentertal administration. Such liquid forms include solutions, suspensions and emulsions. These particular solid form preparations are most conveniently provided in unit dose form and as such are used to provide a single liquid dosage unit.

The invention also contemplates alternative delivery systems including, but not necessarily limited to, transdermal delivery. The transdermal compositions can take the form of creams, lotions and/or emulsions and can be included in a transdermal patch of the matrix or reservoir type as are conventional in the art for this purpose.

Preferably, the pharmaceutical preparation is in unit dosage form. In such form, the preparation is subdivided into unit doses containing appropriate quantities of the active components. The unit dosage form can be a packaged preparation, the package containing discrete quantities of preparation such as packeted tablets, capsules and powders in vials or ampules. The unit dosage form can also be a capsule, cachet or tablet itself, or it may be the appropriate number of any of these in a packaged form.

The quantity of active compound in a unit dose preparation may be varied or adjusted from 1 mg to 100 mg according to the particular application and the potency of the active ingredient and the intended treatment. This would correspond to a dose of about 0.001 to about 20 mg/kg which may be divided over 1 to 3 administrations per day. The composition may, if desired, also contain other therapeutic agents.

The dosages may be varied depending on the requirement of the patient, the severity of the condition being treating and the particular compound being employed. Determination of the proper dosage for a particular situation is within the skill of those in the medical art. For convenience, the total daily dosage may be divided and administered in portions throughout the day or by means providing continuous delivery. When a compound of formula I or a compound capable of enhancing ACh release is used in combination with an acetylcholinesterase inhibitor to treat cognitive disorders these two active components may be co-administered simultaneously or sequentially, or a single pharmaceutical composition comprising a compound of formula or a compound capable of enhancing ACh release and an acetylcholinesterase inhibitor in a pharmaceutically acceptable carrier can be administered. The components of the combination can be administered individually or together in any conventional oral or parenteral dosage form such as capsule, tablet, powder, cachet, suspension, solution, suppository, nasal spray, etc. The dosage of the acetylcholinesterase inhibitor may range from 0.001 to 100 mg/kg body weight.

In the following examples intermediate numerals have normal type and are enclosed by parenthesis, e.g., (5). Product compounds are in bold type and underlined, e.g., **<u>5</u>**

**Example 1: Synthesis of Compound No. <u>4</u>**

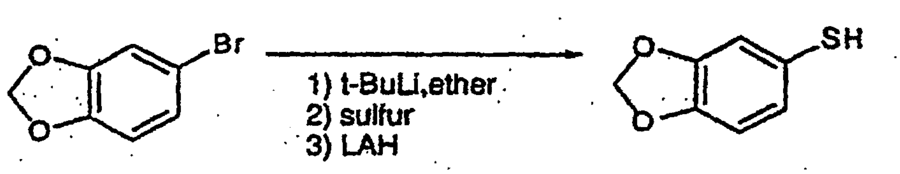


An ether (650 ml) solution of the bromide (15.5 g, 0.07 mol) was cooled in a dry ice acetone bath where t-BuLi (100 ml, 1.7 M) was added dropwise. After stirring for 3h, the sulfur powder (4.9 g, 0.15 mol) was added and

stirring continued for an additional hour. The temperature was warmed to room temperature and stirring continued for 16h. After quenching with water, ether ($Et_2O$) was added and the organic phase was washed with 5% HCl, water, 10% $Na_2CO_3$ and then brine. The solution was concentrated and purified by chromatography with 5% ethyl acetate (EtQAc) / hexane ($R_f$ = 0.7/ $CH_2Cl_2$: hexane 1:1) with 33 g yellow solid collected.

The sample was dissolved in THF (50 mL) and cooled in an ice water bath where it was added to a mixture of LAH (0.91 g, 0.02 mol) and 20 mL THF. Stirring was continued for 10 minutes, then warmed to room temperature and stirred for 1h. The reaction was diluted with EtOAc and quenched with water. 10% HCl was added and the organic layer separated, washed with water and brine. After concentration and drying under vacuum. 6.6 a of a yellow oil was collected.

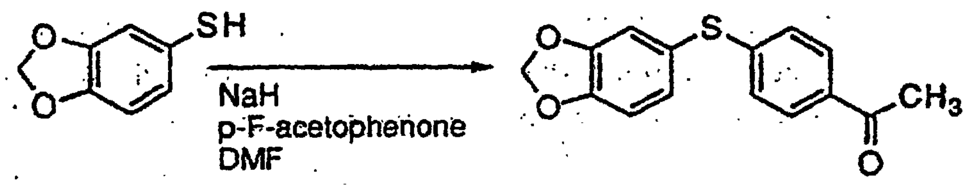


The thiol (19 g, 124 mmol) was dissolved in DMF (180 ml) and cooled in an ice bath where NaH (4.9 g, 60% in oil) was added in portions. Stirring was continued for 1h, then warmed to room temperature and stirred for an additional 1h. The 4-fluoroacetophenone (18.7 g, 136 mmol) was added dropwise and then stirred at 70°C for 3h. The reaction was concentrated on a rotovap, diluted with EtOAc, and washed with water and brine. The solution was evaporated on a rotovap and the residue chromatographed on silica gel (20% EtOAc/hexane) to collect 24.5g (73%) of a pale yellow solid.

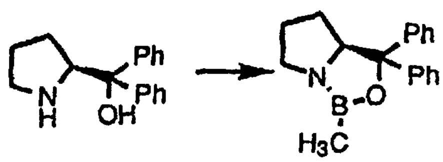

To a solution of the alcohol (10.1 g,40 mmol) in toluene (100mL) was slowly added the trimethyl boroxine (3.3 g, 27 mmol). After 4h, the volatiles were removed by distillation (oil bath temp=140-150°C). Cooling to room temperature was followed by addition of toluene (50mL), and removal of the volatiles by distillation (repeat again). The residue was placed under high vacuum and the flask heated to 150°C. After drying under vacuum overnight the off white solid was dissolved in THF (100ml) and used without further purification.

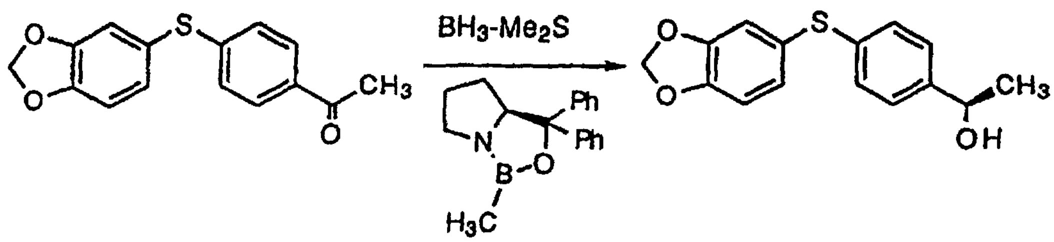


The ketone (36 g, 133 mmol) was dissolved in THF (200 ml), followed by addition of the catalyst (8.8 g in 80 ml THF). $BH_3(CH_3)_2S$ (42 ml, 2mol/L in THF) was added dropwise, and the reaction stirred for 30min. $CH_3OH$ (200mL) was added and the solution concentrated. The residue was dissolved in EtOAc and washed with water and brine. The solution was dried over $Na_2SO_4$, filtered and concentrated to give 40.3 g of a pink oil.

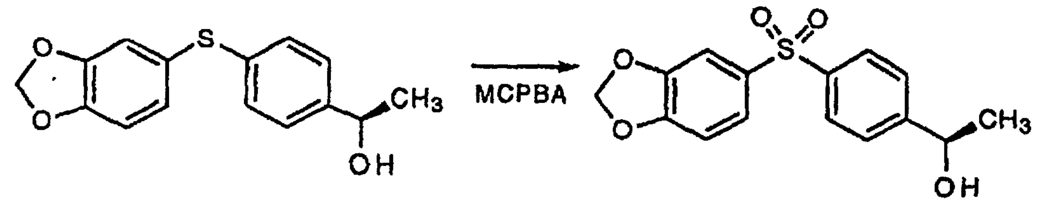


A $CH_2Cl_2$ (600 ml) solution of the sulfide (40.3g, 147mmol) was cooled in an ice water bath where MCPBA (79.7 g, 70%) was added in portions. After stirring for 1h the temperature was warmed to room temperature and stirred for 4h. After diluting with $CH_2Cl_2$, the reaction was washed with 10%$Na_2CO_3$, water, and brine. The solution *was* dried over, $Na_2SO_4$, filtered and concentrated to collect 42.5g of an off white solid.

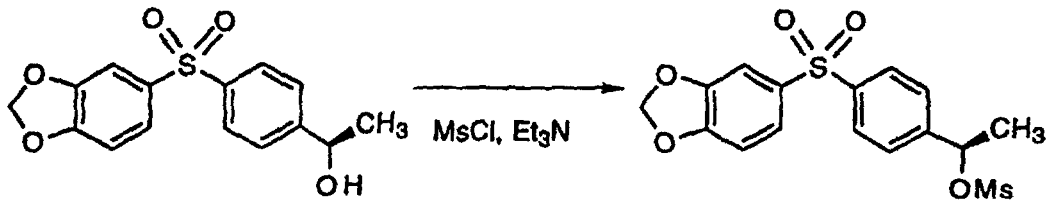


A $CH_2Cl_2$ (250 ml) solution of the alcohol (21.4 g, 70 mmol) and triethyl amine ($Et_3N$) (19.5 ml) was cooled in an ice water bath where methanesulfonyl chloride (6.5 ml) were added dropwise. After 1h the reaction was diluted with $CH_2Cl_2$ and washed with 2% HCl, water, saturated $NaHCO_3$, water, brine, and dried over $Na_2SO_4$. Filtration and concentration gave 27g of an oil which was used without further purification.

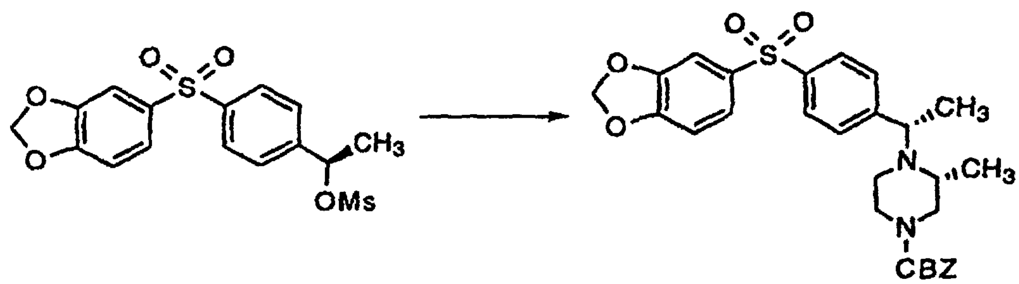

A mixture of the mesylate (made from 21.4 g alcohol), piperazine (18 g, 0.07 mol), and 2,2,6,6-tetramethyl piperidine (11.8 g, 0.084 mol) was stirred in $CH_3CN$ (200 ml) at reflux overnight. After cooling to room temperature the $CH_3CN$ was removed on a roto vap and replaced with EtOAc. The solution was washed with 10% $Na_2CO_3$, and water. After concentration the residue was chromatographed on silica gel using 1:3 EtOAc/hexane. 21.9g (60%) of a gum was collected.

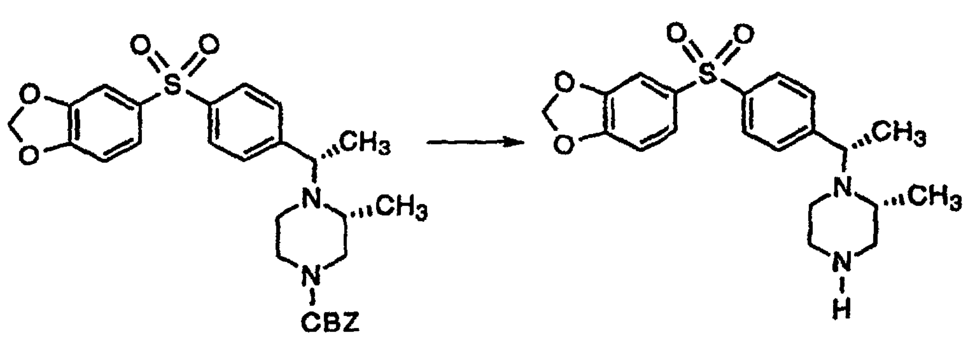

A mixture of the CBZ amine (10.3g, 19.6 mmol), 100 ml water, $CH_3OH$ (100 ml), and 100 ml conc. HCl was heated in an oil bath at 100°C for 5h. The mixture was cooled in an ice bath and 50% NaOH was added until the pH=9.

EtOAc was added, followed by separation and washing with water. The organic solution was concentrated and the residue chromatographed on silica gel ($CH_2Cl_2$:$CH_3OH$ 50:1, saturated with $NH_4OH$ solution). The product was collected in 86% as a heavy oil.

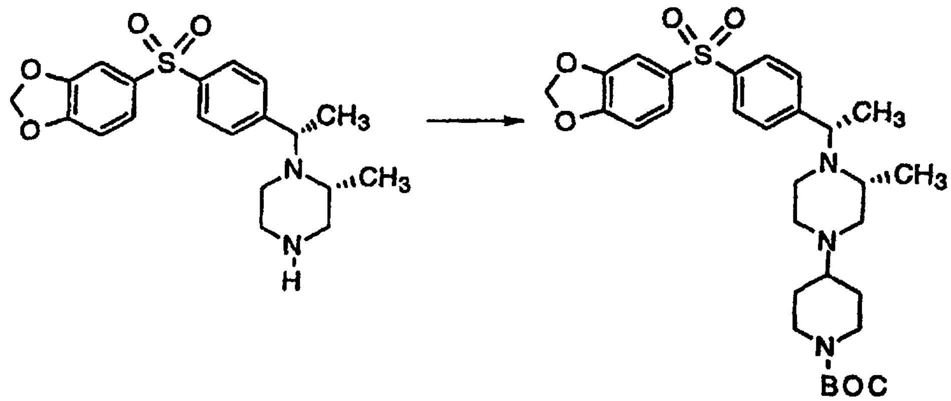

The amine (1.0 g, 2.5 mmol), N-BOC piperidinone (0.5 g, 2.5 mmol), and HOAc (0.15 ml) were dissolved in $CH_2Cl_2$ (20 ml). NaBH $(OAc)_3$
(0.55 g, 3.8 mmol) was added in several portions and the solution stirred overnight. $CH_2Cl_2$ was added and washed with saturated $NaHCO_3$, water and brine. The solution was dried over $Na_2SO_4$, filtered and concentrated. The residue was chromatographed using EtOAc. A colorless gum (0.96g) was obtained in 65% yield.

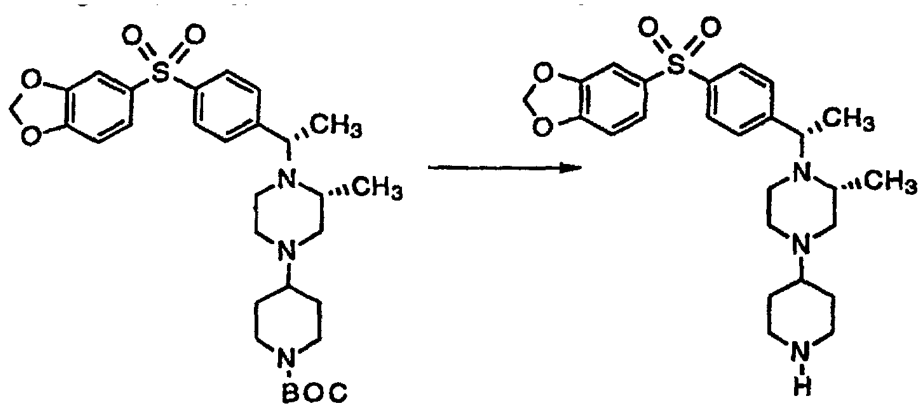

The N-BOC compound (0.87 g, 1.5 mmol) was dissolved in EtOAc (20 ml), and 6N HCl (3.5 ml) was added with stirring. After 2h, $CH_2Cl_2$ was added and the solution was washed with saturated $NaHCO_3$, and dried over $Na_2SO_4$. After filtration the solution was concentrated and dried under vacuum to give a white powder (0.6g, 85%).

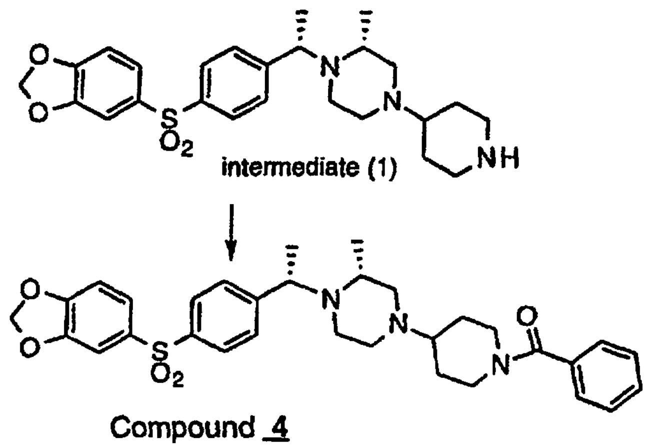

intermediate (1)

Compound 4

Stir for 20h. at RT a solution of intermediate (1) (0.025 g), N-hydroxybenzotriazole (HOBT) (0.01 g), benzoic acid (0.0125 g), $Et_3N$ (0.01 mL) and N-ethyl-N-(3-dimethylaminopropyl)-carbodiimide hydrochloride (EDC) (0.025 g) in DMF (0.3 ml). Dilute with EtOAc, wash with water and with $NaHCO_3$ solution, dry over $MgSO_4$ and filter through a small plug of silica gel, washing with EtOAc. Evaporate to obtain the free base form of the title compound. Dissolve this in $CH_2Cl_2$ (0.2 mL) and add to a solution of HCl in dioxane (4M; 0.1 mL) in $Et_2O$ (1.5 mL). Stir for 5 min., centrifuge, wash by suspension and centrifugation 3x with $Et_2O$ and dry at RT in a stream of $N_2$, then at high vacuum to obtain the dihydrochloride. Mp: 215-225°C, with decomposition; Mass spectrum: MH+ = 574.

**Example 2: Synthesis of Compound No. 17**

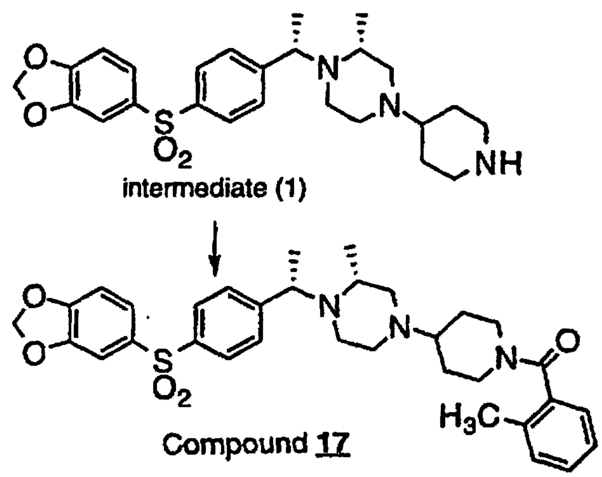

intermediate (1)

Compound 17

Stir for 20h. a mixture of intermediate (1) from example 1, (0.05 g), $CH_2Cl_2$ (1.5 ml), o-toluoyl chloride (0.1 ml) and 1N aq. NaOH (2 ml). Separate the organic phase, dry and evaporate. Purify by preparative tlc, eluting with 5% $CH_3OH$-$CH_2Cl_2$. Extract the major band with 50% $CH_3OH$-$CH_2Cl_2$ and evaporate. Precipitate, wash and dry the hydrochloride (yield = 0.065 g) in the manner described in the foregoing preparation. Mp: 182-190°C with decomposition; Mass spectrum: MH+ = 588.

**Example 3: Synthesis of Compound No. 28**

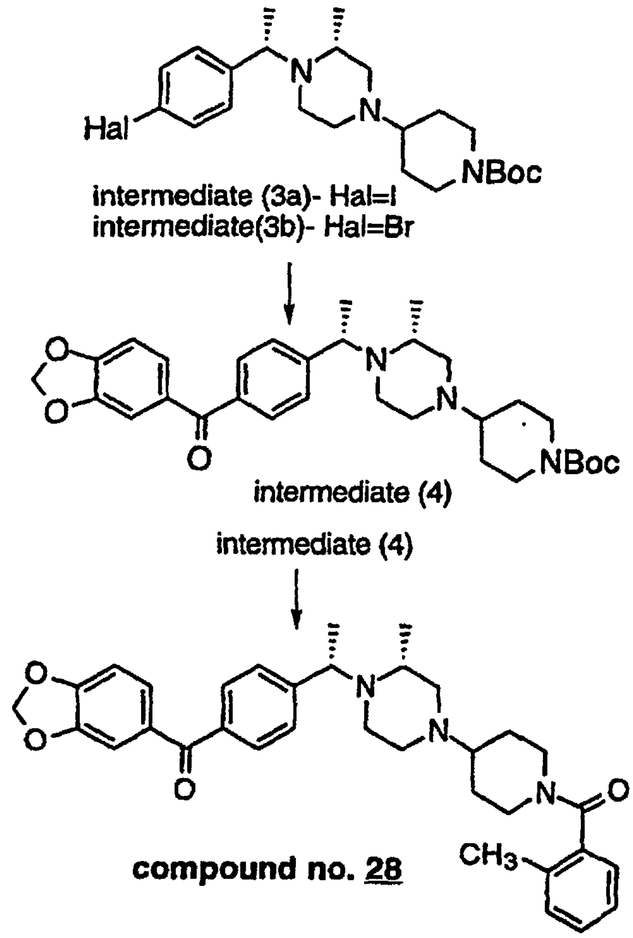


This synthesis can be carried out using either intermediate (3a) or (3b) produced as shown in examples 10 and 11. To a solution of (3b) (1.86 g) in dry THF (30 ml) at -70°C under $N_2$ add n-BuLi (2.5 M in hexanes; 1.6 ml), stir for 10 min. and add a solution of N-piperonoyl-N,O-dimethyl-hydroxylamine (0.83 g) in THF (1 ml). Stir without cooling for 2h., add EtOAc, wash with water, dry ($MgSO_4$) and evaporate. Chromatograph on silica gel with EtOAc-$CH_2Cl_2$ to obtain the major component intermediate (4) as a thick oil (1.21 g). Stir a solution of (4) (0.4 g) in EtOAc (24 ml) and conc. HCl (4.5 ml) at RT for 3h. Dilute with EtOAc, and wash with excess 1N NaOH solution. Extract with EtOAc, wash with saturated brine, dry and evaporate to obtain the NH compound. Dissolve this in $CH_2Cl_2$ (10 ml), and stir for 20h. at RT with o-toluoyl chloride (0.15 g) and NaOH (0.8 g) in $H_2O$ (10 ml). Extract with $CH_2Cl_2$, dry ($MgSO_4$), evaporate and chromatograph on silica gel with 4% $CH_3OH$-$CH_2Cl_2$. Evaporate the pure fractions to obtain the free base as a foam (0.33 g) Dissolve a small portion this in $CH_2Cl_2$ and precipitate the hydrochloride salt as described in previous preparations. M.p.: 200-210°C, with decomposition; Mass spectrum: MH+ = 554

**Example 4: Synthesis of Compound No. 30**

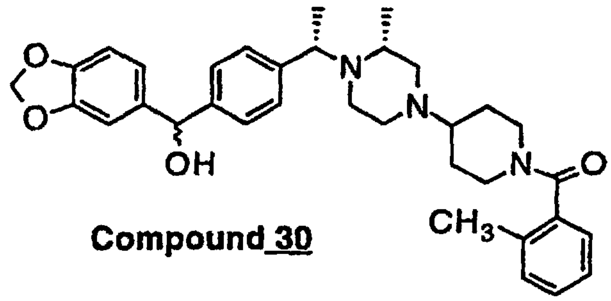

Compound 30

Stir a solution of the product of example 3 (0.31 g), ethanol (15 ml) and sodium borohydride (0.042 g) at RT for 3h. Evaporate, add water, extract with EtOAc, dry, evaporate and chromatograph on silica gel with 10% $CH_3OH$-$CH_2Cl_2$ to obtain the product (0.28 g) as a white foam, a mixture of diastereoisomers. Mp: 85-95°C; Mass spectrum: MH+ = 556.

**Example 5: Synthesis of Compound No. 31**

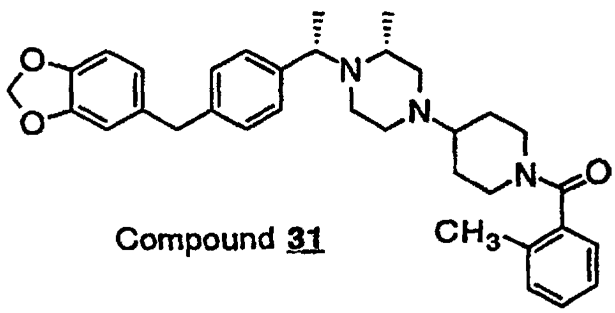

Compound 31

Stir a solution of the product of example 4 (0.1 g) in $CH_2Cl_2$ (3 ml) and add triethylsilane (0.3 mL) and trifluoroacetic acid (0.12 mL). Stir at RT for 72h. Add excess 1 N NaOH solution, extract with EtOAc, dry, evaporate and purify by preparative tic on silica plates, eluting with 5% $CH_3OH$-$CH_2Cl_2$. Remove and extract the major band, evaporate the eluate, and convert to the hydrochloride in the usual manner, to obtain a white powder (0.08 g). Mp: 210-215°C, with decomposition; Mass spectrum: MH+ = 540.

**Example 6: Preparation of Compound No. 24**

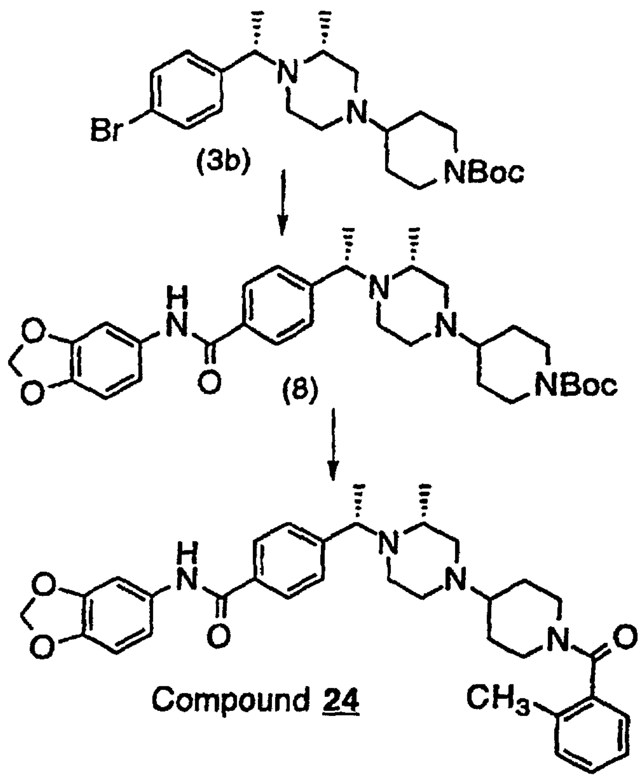

(3b)

(8)

Compound 24

To a solution of (3b) (Example 10) (1.38 g) in dry THF (20 ml) at -70°C under dry $N_2$ add n-BuLi (2.5M in hexanes; 1.7 ml). Stir for 5 min., and add a solution of 3,4-methylenedioxyphenyl isocyanate (crude product, prepared by heating the corresponding acyl azide in toluene) (0.68 g) in THF (5 ml). Stir for 30 min without cooling, and work up in EtOAc-aq.$NaHCO_3$, dry ($MgSO_4$) and evaporate. Isolate the desired compound (Rf = 0.4 in 5% $CH_3OH$-$CH_2Cl_2$) by column chromatography on silica gel, and evaporate to a pale brown foam (0.54 g).

Convert a small portion to the HCl salt in the previously described manner.
Mp: 240-250°C, with decomposition; Mass spectrum: MH+ = 551.

Stir a mixture of free base (8) (0.5 g), EtOAc (5 mL) and conc. HCl (3 ml) at RT for 2h. Add excess aq.NaOH, extract with $CH_2Cl_2$, dry and evaporate to a foam (0.35 g). Dissolve 0.12 g of this in $CH_2Cl_2$ (4 ml) and stir for 2h. at RT with 1N-NaOH (5 ml) and o-toluoyl chloride (0.05 mL). Separate and evaporate the organic phase, and isolate the product by preparative tic on silica with 5% $CH_3OH$-$CH_2Cl_2$. Convert a small portion to the hydrochloride in the usual way. Mp: 220-230°C, with decomposition; Mass spectrum: MH+=569.

**Example 7: Synthesis of Compound No. 25**

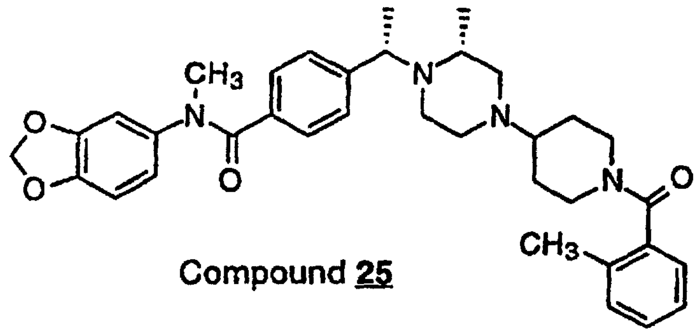

Compound 25

Stir a solution of the product of example 6 (0.08 g) in DMF (1 ml) and add NaH (0.044 g), then after 10 min. add $CH_3I$ and stir for 30 min. Work up in EtOAc-$H_2O$, dry, evaporate, purify by preparative tic and convert to the HCl salt as in the foregoing preparation. Mp: 190-200°C, with decomposition; Mass spectrum: MH+ = 583.

**Example 8: Synthesis of Compound No. 83 and 84**

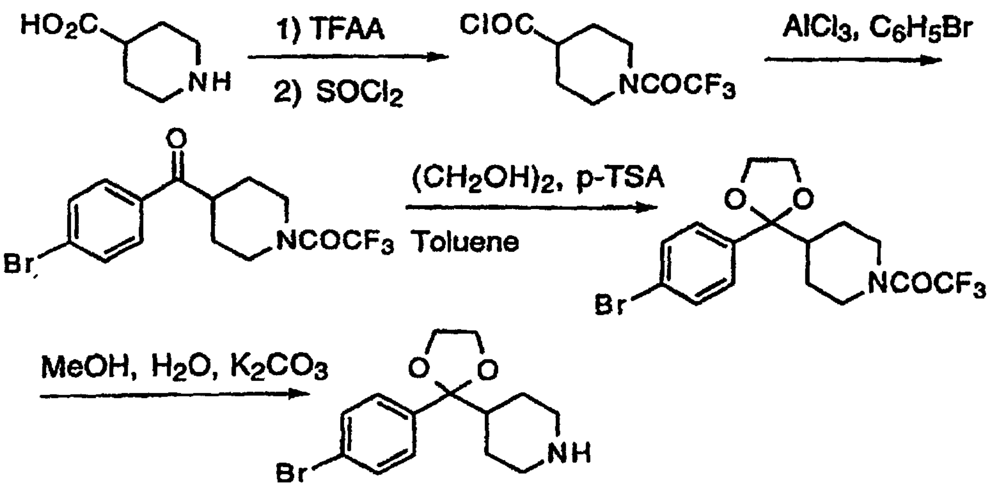


(10)

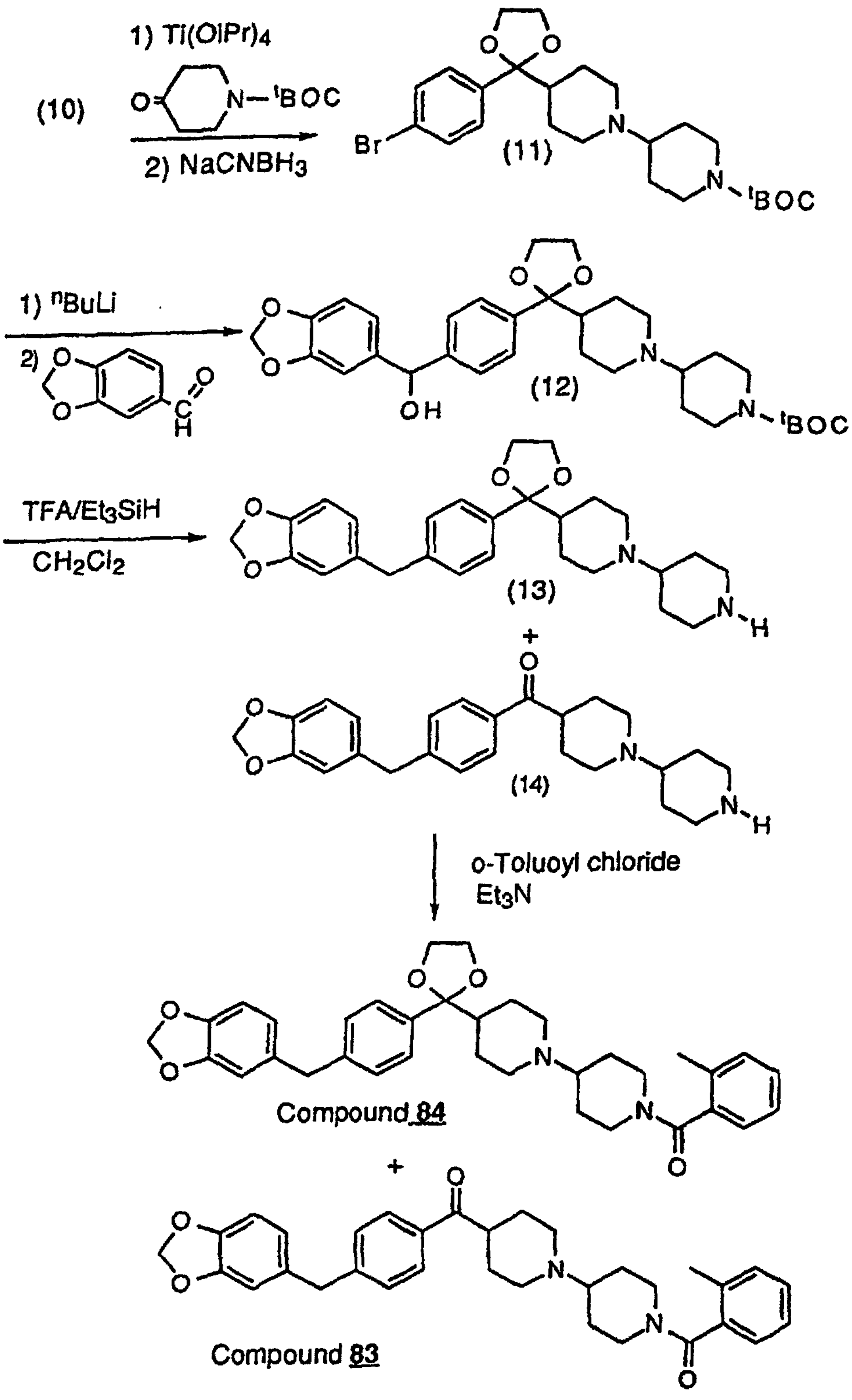

(10) (11) (12) (13) (14)

Compound 84

Compound 83

300 ml of trifluoroacetic anhydride (TFAA) was added to 96 g of isonipecotic acid at 0°C. After the addition, the reaction mixture was heated at reflux for 4h. Excess TFAA was removed under vacuo and the reaction mixture taken up in EtOAc and washed with water and concentrated to give 160 g of the amide. 50 g of this amide was treated with 300 ml $SOCl_2$ and the reaction mixture heated at reflux overnight. At the end of this time excess $SOCl_2$ was removed under vacuo to give 54 g of the acid chloride.

11g $AlCl_3$ was added slowly to a solution of 10 g of the above acid chloride in 40 ml of bromobenzene at ambient temperature and the reaction mixture heated at reflux for 4 h. It was then cooled and poured into a mixture of

conc. HCl and ice and product extracted with EtOAc. The organic layer was separated and washed with water, half saturated $NaHCO_3$ solution and concentrated to give 16.21 g of the ketone.

16.21 g of the ketone was dissolved in 200 ml toluene containing 25 ml ethylene glycol and 0.5 g p-toluenesulfonic acid. The reaction mixture was heated at reflux with azeotropic removal of water until no further water was collected. The reaction mixture was concentrated to give 17.4 g of the ketal.

17.4 g of the crude ketal was dissolved in 100 ml of $CH_3OH$ and to this was added 25 ml of water and 12 g of $K_2CO_3$ and the reaction mixture stirred at ambient temperature overnight. The reaction mixture was diluted with water and extracted with EtOAc. The organic layer was separated and washed with water and brine and concentrated to give 12.55 g of (10).

A mixture of 6.5 g of (10), 4.15 g of N-BOC-4-piperidone and 10 ml of $Ti(OiPr)_4$ was stirred at ambient temperature overnight. The reaction mixture was cooled to 0°C and 3.2 g of $NaCNBH_3$ dissolved in 40 mL of $CH_3OH$ was added. The reaction mixture was then allowed to warm to ambient temperature and stirred for 1 h., diluted with 100 ml of EtOAc and quenched with a mixture of 60 ml water/20 ml of conc. $NH_4OH$. The mixture was stirred for 1 h. and filtered though celite. The aqueous layer was extracted with 3x100 ml of EtOAc. The combined organic layers were dried over $MgSO_4$, filtered and concentrated. The crude was purified by flash chromatography using 100% EtOAc as eluent. To give 6.8 g, 66% of (11).

To a stirred solution of (11) in 25 mL of THF at -78°C was added 1.7 ml (1.6 M in Hex.) of $^{n}$BuLi. The mixture was stirred at -78°C for 10 min., then 700 mg of piperonal dissolved in 5 ml of THF was added. The mixture was allowed to reach ambient temperature and stirred for 2 h. The mixture was quenched with 30 ml of water and extracted with 3x50 ml of EtOAc. The combined organic layers were dried over $MgSO_4$, filtered and concentrated. The crude was purified by silica gel flash chromatography using 10%$Et_3N$-ether to give 475 mg, 41.5% of (12).

To a stirred solution of 475 mg of (12) and 1 ml of $Et_3SiH$ in 10 ml of $CH_2Cl_2$ was added 3 ml of TFA. The mixture was stirred at ambient temperature for 2 h. The solvent was evaporated and the residue was taken up in 20 ml of $CH_2Cl_2$, washed with 20 ml of 10% NaOH. The aqueous layer was extracted with 3x30 ml of $CH_2Cl_2$, the combined organic layers were dried over $MgSO_4$, filtered and concentrated to give a mixture of (13) & (14) which was used without purification. (350 mg, 90%).

350 mg of the crude mixture of (13) and (14) and 1 ml of $Et_3N$ in 10 ml of $CH_2Cl_2$ was treated with 0.5 ml (1.2 eq.) of o-toluoyl chloride for 3 h. The mixture was then concentrated and the residue purified by silica gel prep. TLC using 4% $Et_3N$-$Et_2O$ to give compounds **83** and **84.** The HCl salts of compounds **83** and **84** were prepared by dissolving the free base in EtOAc and adding HCl-ether solution. After evaporating the solvent, the salts were collected as powder.

| | |
|---|---|
| Compound No. **83** | (50 mg, 54%) $(M+H)^+$ Cal: 525.2753; Found: 525.2746 |
| Compound No. **84** | (190 mg, 50%) $(M+H)^+$ Cal: 569.3015; Found: 569.3022 |

**Example 9: Synthesis of Compound No. 85**

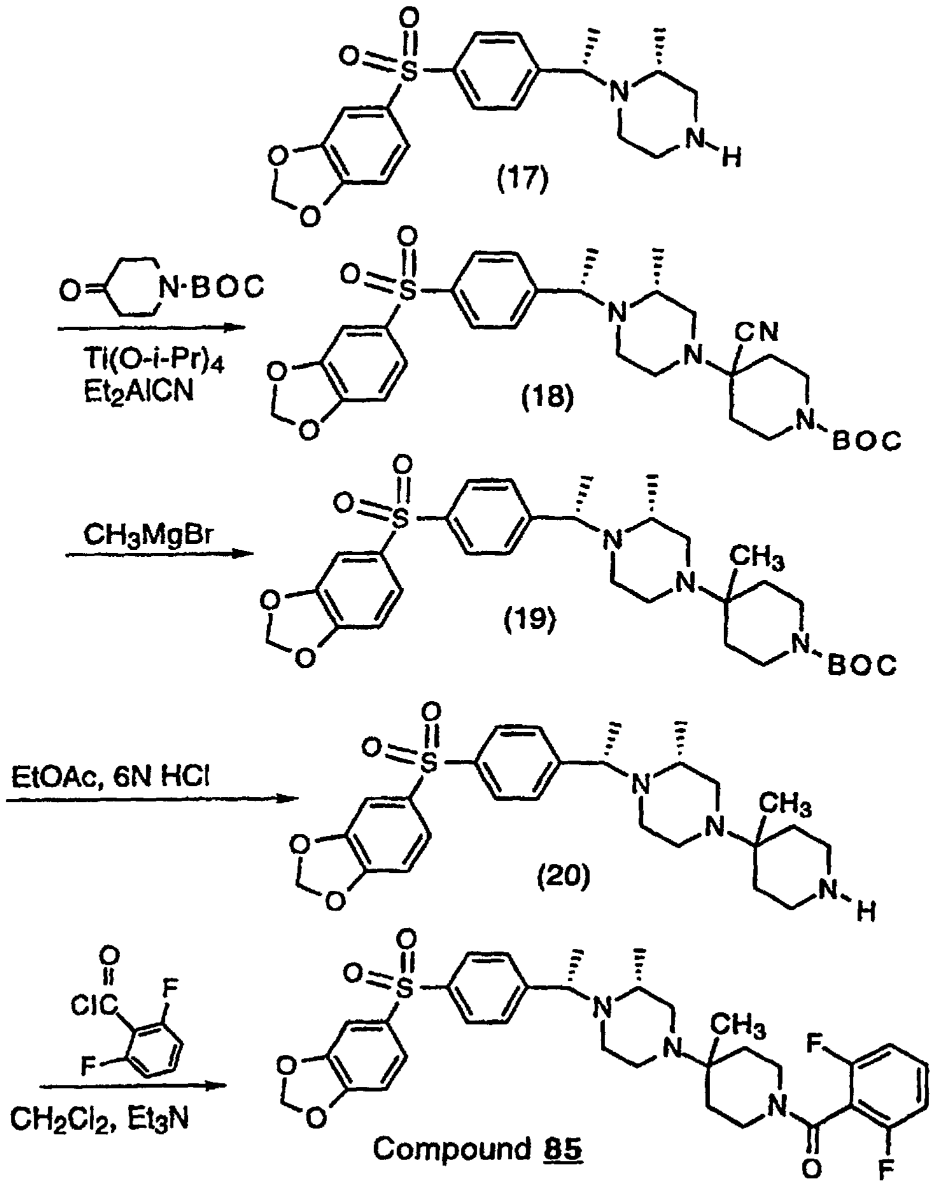

(17)
(18)
(19)
(20)
Compound 85

The piperazine (17) (1.1 g, 2.8 mmol), prepared as described in example 1, is dissolved in $CH_2Cl_2$ (10 ml) followed by addition of the N-BOC piperidinone (0.85 g, 4.25 mmol) and $Ti(OiPr)_4$ (0.8 g, 2.8 mmol). The mixture is stirred overnight at room temperature. A 1 molar toluene solution of diethylaluminum cyanide (6.5 ml, 5.6 mmol) is added, via syringe, and the mixture stirred for an additional 20h. The mixture is then diluted with EtOAc and washed with 10% $Na_2CO_3$, water and brine. The organic phase is concentrated in vacuo and the residue chromatographed through a silica gel column (1:1 EtOAc/hexane). The product (18) is collected as a colorless oil (0.93 g, 55%).

The cyano intermediate (18) (0.13 g, 0.19 mmol) is dissolved in THF (4 ml) followed by the addition of $CH_3MgBr$ (0.64 ml, 3.0 M, 1.9 mmol), via syringe. After stirring for 10 min the temperature is raised to 60°C where stirring is continued for 2.5h. Cooling to room temperature is followed by quenching with water (1 ml) and then saturated $NaHCO_3$ (20 ml). After 10 min EtOAc (50 ml) is added, stirred vigorously, and separated. The organic layer is washed with brine and dried over $Na_2SO_4$. Filtration and concentration with under vacuum gives a viscous oil which is purified on silica gel prep TLC plates (100% EtOAc). The intermediate (19) is collected in 60% yield.

The N-BOC piperidine (19) (0.27 g, 0.4 mmol) is dissolved in EtOAc (10 ml) and 1.5 ml of a 6N HCl solution added with vigorous stirring. After 1.5h saturated $NaHCO_3$ is added until the pH was basic. $CH_2Cl_2$ (25 ml) is added and the layers separated, and the aqueous phase extracted with $CH_2Cl_2$. The organic layer is dried over $Na_2SO_4$,

filtered and concentrated to collect 0.22 g, 95%, of (20) as a thick oil which solidifies under vacuum.

The amine (20) (0.025 g, 0.044 mmol) is dissolved in $CH_2Cl_2$ (1 ml) followed by the addition of $Et_3N$ (11 ul, 0.08 mmol) and the 2,6-difiuoro-benzoyl chloride (0.014 g, 0.08 mmol). After stirring at room temperature overnight, saturated $NaHCO_3$ and $CH_2Cl_2$ are added. The layers are separated and the organic phase dried over $Na_2SO_4$. The solvent is removed and the residue dissolved in EtOAc. The solution was placed on a preparative TLC plate and eluted with EtOAc (100%). Compound **<u>85</u>** is collected as a clear oil (0.026 g), yield = 95%. This is converted to the dihydochloride salt as follows: The free amine (26 mg) is dissolved in 1 ml EtOAc and stirred while HCl /$Et_2O$ is added until the pH persists at 2. The precipitate is transferred to a centrifuge tube and spun in a centrifuge. The supematant was removed and replaced with $E_2O$ (gentle stirring with a spatula), and the sample spun again. The process is repeated 3X. After removal of the solvent, the solid is transferred to a vial where it is dried under vacuum. 15 mg of a white solid was collected (m.p. = 242-245 dec).

**Example 10: Synthesis of Intermediate (3a) from example 3**

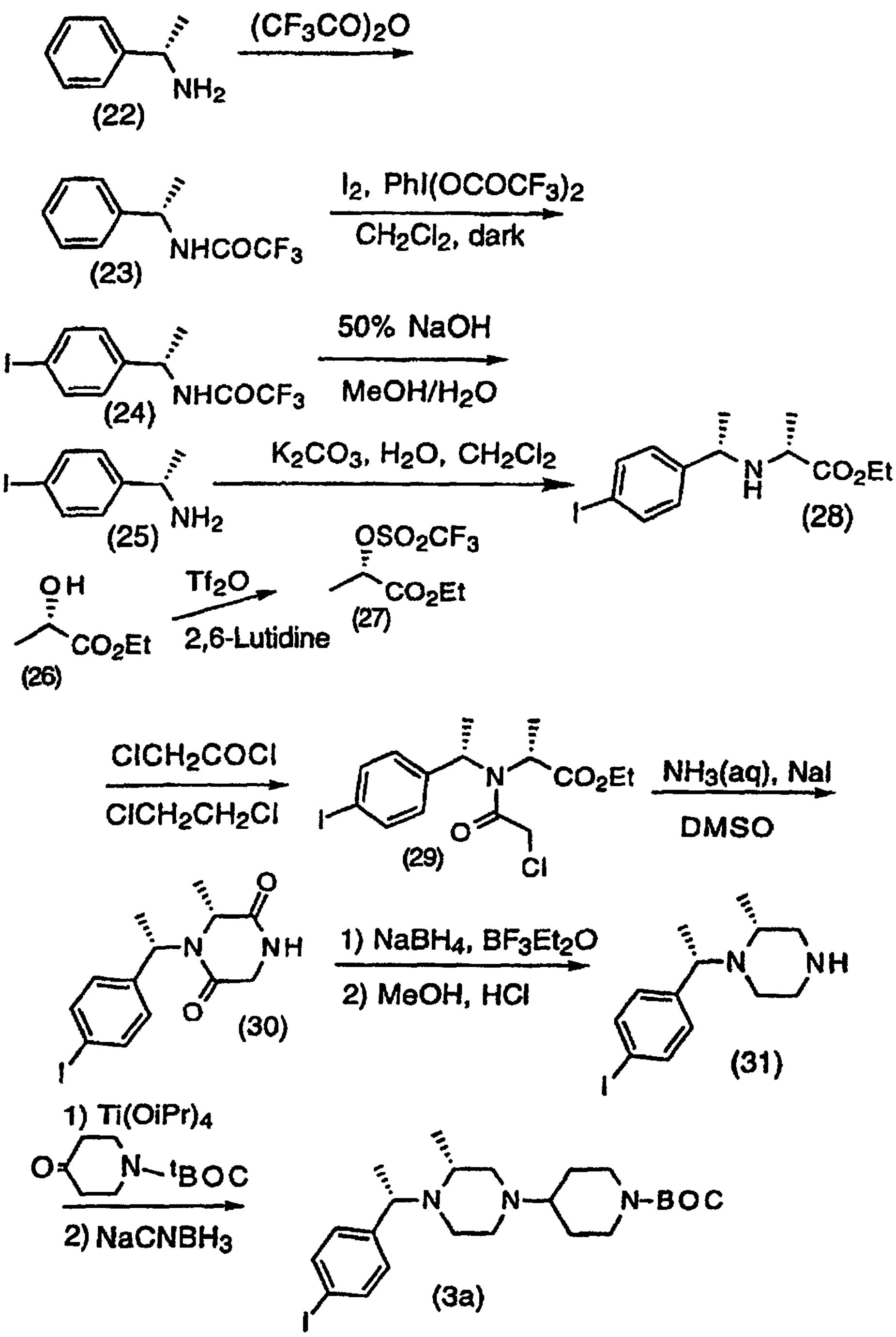


**Step 1**

TFAA (150 ml, 1.06 mol) in $CH_2Cl_2$ (500 ml) was cooled to 0°C. A solution of (S)-α-methylbenzylamine ( 22) (100 g, 0.83 mol) in $CH_2Cl_2$ (200 ml) was added slowly over 30 min. The cooling bath was removed and the mixture was stirred for ~2h, transferred to a separatory funnel, washed with water, dried over anhydrous $MgSO_4$, filtered, and concentrated to give 189 g (~100%) of (23) as a white solid

**Step 2**

Iodine (41 g, 162 mmol) was added to a solution of (23) (65.4 g, 301 mmol) in $CH_2Cl_2$ (500 ml) at room temperature. [Bis(trifluoroacetoxy)iodo]-benzene (75 g, 174 mmol) was added. The mixture was stirred for 30 min, a mild exotherm occurred (30°C), then kept in the dark overnight. The mixture was added slowly to a stirred mixture of $NaHCO_3$ (50 g), sodium bisulfite (10g) and water (600 ml), washing in with $CH_2Cl_2$. The mixture was stirred for 15 min, filtered and the filter cake was well washed with $CH_2Cl_2$ until TLC (100% $CH_2Cl_2$, Rf=0.75) shows no product in the washings. (Note: the initial solid contains considerable product which is moderately soluble in $CH_2Cl_2$). The filtrate was dried over anhydrous $MgSO_4$, filtered through a 1" pad of flash silica gel, washed with 2% $Et_2O/CH_2Cl_2$. The filtrate was evaporated to a slurry, $Et_2O$ (~75 ml) and hexanes (-700 ml) were added and the mixture was stirred for 30 min. The resulting solid was collected, washed well with hexanes, air dried and then dried under vacuum to give 63.05 g (61%) of (24) as a white solid. Filtrates were evaporated to an oil and some crystals, added hexanes (500 ml), stirred at room temperature for 1h, collected and additional 4.6 g (4%) of (24)

**Step 3**

(24) (10g, 29.15 mmol) was dissolved in $CH_3OH$ (150 ml) and water (15 ml). 50% NaOH (30g) was added and the mixture was stirred at room temperature overnight (20 h). TLC (50% EtOAc/hex.) indicated consumption of starting material. Most of $CH_3OH$ was removed in vacuo, the residue was dissolved in water and $CH_2Cl_2$, transferred to a separatory funnel and extracted with $CH_2Cl_2$. The extracts were combined, dried over anhydrous $Na_2CO_3$, filtered and concentrated to give 6.7 g (93%) of (25) as a white solid.

**Step 4**

Triflic anhydride (10.6 ml, 62.98 mmol) was added to a 0°C solution of (S) ethyl lactate (26) (7.0 ml, 61.75 mmol) in $CH_2Cl_2$ (100 ml) at 0°C. 2,6-Lutidine (7.55 ml, 64.83 ml) was added and the mixture was stirred for 1h. The mixture was transferred to a separatory funnel, washed with 0.5 M HCl and brine, dried over anhydrous $Na_2SO_4$, concentrated almost to dryness, filtered through a plug of silica eluting with $CH_2Cl_2$, conc. to provide 10.8 g (70%) (27) as a reddish oil.

**Step 5**

(25) (6.7 g, 27.11 mmol) was added to a mixture of $CH_2Cl_2$ (90 ml), water (90 ml) and $K_2CO_3$ (5.5 g, 39.6 mmol). Ethyl (S) lactate triflate (27) (7.5 g, 29.82 mmol) was added and the mixture was stirred overnight (20 h). TLC (50% EtOAc/hex.) indicated consumption of starting material. Ammonia (conc.) (30 ml) was added, stirred 15 min, transferred to a separatory funnel, washed with water, dried over anhydrous $Na_2SO_4$, filtered and concentrated to give 10.3 g (~110%) of (28).

**Step 6**

$ClCH_2OCl$ (12 ml, 150 mmol) was added to a solution of (28) (9.37g, 27.11) in dichloroethane (80 ml). The mixture was refluxed for 2h. TLC (20% EtOAc/hex.) indicated consumption of starting material. The solution was cooled to room temperature, diluted with $CH_2Cl_2$, water (200 ml) followed by $K_2CO_3$ (~15g) added in small portions. The mixture was stirred for an additional 15 min., transferred to a separatory funnel, washed with water, dried over anhydrous $Na_2SO_4$, filtered and concentrated to give 11.53g (~110%) of (29).

**Step 7**

(29) (27.11 mmol) was dissolved in dimethyl sulfoxide (DMSO) (120 ml). Concentrated aqueous ammonia (24 ml) and NaI (13 g) were added. $CH_3OH$ (10 ml) was used in the NaI. The mixture was stirred over the weekend (60h, overnight is sufficient). TLC (50% EtOAc/hex.) indicated consumption of starting material. Water (500 ml) was added, the mixture was stirred for 30 min and the resulting precipitate was collected via vacuum filtration. The precipitate was well washed with water and dried under vacuum to afford 8.2g (84%) of (30).

**Step 8**

(30) (8.2 g, 22.9 mmol) was added to a mixture of $NaBH_4$ (8.66 g, 228.9 mmol) in dimethoxyethane (250 ml). Boron trifluoride etherate (16.9 ml, 137.3 mmol) was added and the resulting mixture was refluxed under nitrogen for

3h. TLC (5% $CH_3OH/CH_2Cl_2$) indicated consumption of starting material. The mixture was cooled to 0°C, $CH_3OH$ (60 ml) was added slowly and the resulting mixture was stirred for 20 min. Concentrated HCl (10 ml) was added slowly and the resulting mixture was refluxed for 1h. TLC (5% $CH_3OH/CH_2Cl_2$) indicated formation of a more polar product consistent with an amine. The mixture was cooled to room temperature and concentrated almost to dryness. The residue was partitioned between 2N NaOH and $CH_2Cl_2$, transferred to a separatory funnel and extracted with $CH_2Cl_2$. The extracts were combined, washed with water, dried over anhydrous $Na_2SO_4$ and concentrated to provide 6.5 g (87%) of (31) as an oil.

**Step 9:**

Intermediate (31) was converted to 3a using using procedures described in example 8.

**Example 11: Synthesis of Intermediate (3b) from example 3**

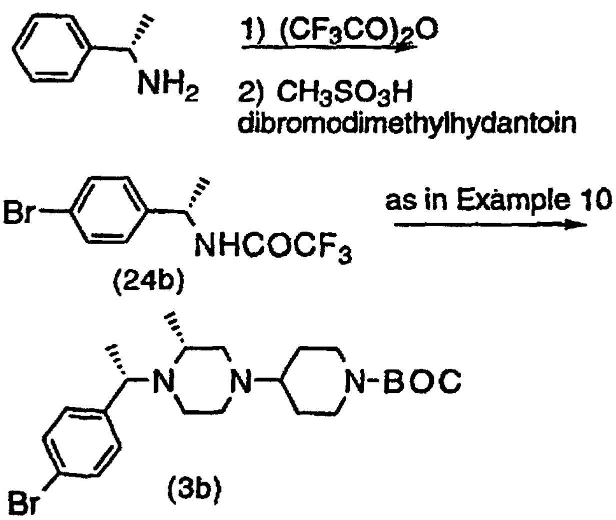

(24b)
(3b)

A solution of TFAA (0.4 mol) in 300 ml cold $CH_2Cl_2$ is treated with 0.3 mol (S)-$\alpha$-methylbenzylamine in 100 ml $CH_2Cl_2$. The resulting mixture is stirred with ice cooling for 30 minutes and at room temperature for 1.5 hours. The solution is again cooled in an ice bath and 80 ml $CH_3SO_3H$ is added followed by 45 grams dibromodimethylhydantoin. Stirring is continued until the mixture is homogeneous, then the mixture is allowed to stand overnight in the dark. A solution of 20 g $NaHSO_3$ in 300 ml ice water is added. The organic layer is separated and washed twice with water and dried over $MgSO_4$. The solution is filtered through a 4" pad of flash-grade silica gel, eluting with 400 ml $CH_2Cl_2$, and evalporated to a solid. The solid is suspended in 200 ml $Et_2O$ and 1l hexane is added. After stirring 30 minutes, the solids are collected, washed with hexane, and dried to give 35.7 grams of intermediate (24b). This compound is converted to (3b) using the same sequence described in scheme 9.

**Example 12: Synthesis of Compound No. 53**

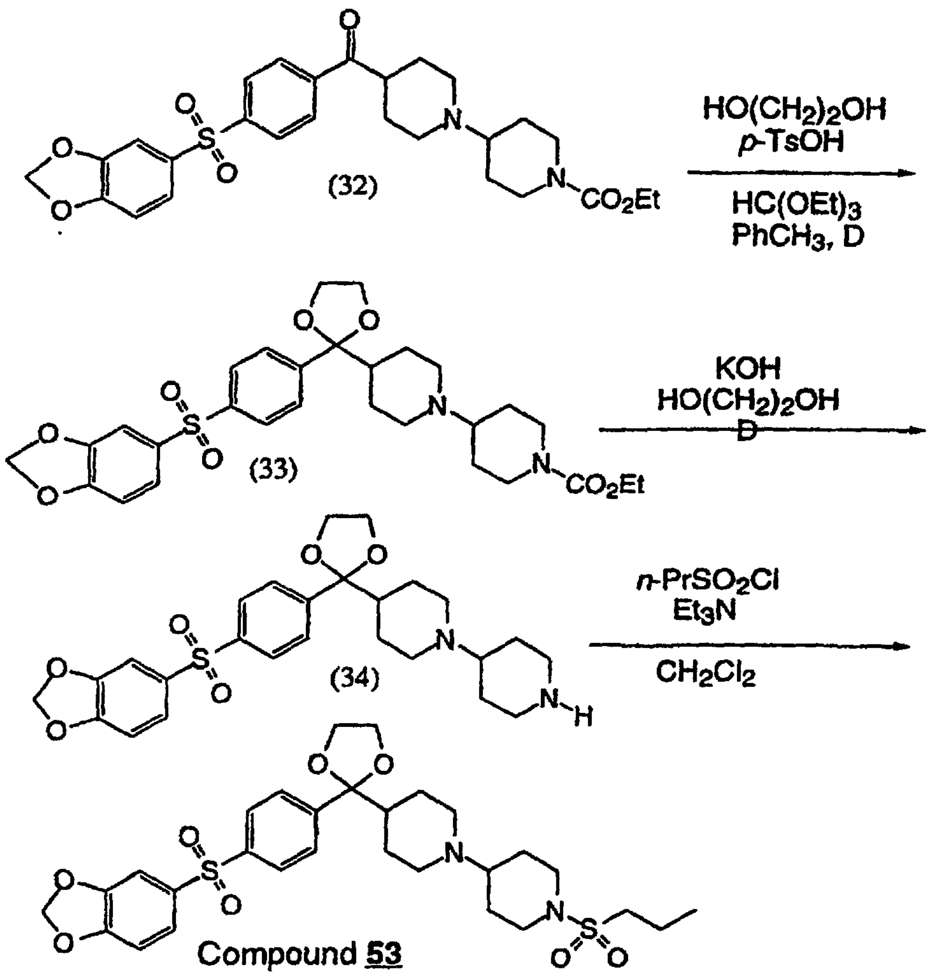


To a stirred solution of (32) prepared via the procedure described for intermediate (7) of example 14, but using N-ethyoxycarbonyl-4-piperidinone instead of N-tBOC-4-piperidinone (1.80 g, 3.41 mmol) in toluene (34 ml) was added dry ethylene glycol (1.33 ml, 23.8 mmol), triethylorthoformate (1.70 ml, 10.2 mmol) and *p*-toluenesulfonic acid monohydrate (0.97 g, 5.12 mmol). The reaction was heated overnight at 55°C under nitrogen. The mixture was diluted with $CH_2Cl_2$, washed sequentially with 1 N NaOH and brine, dried over $Na_2SO_4$, filtered and concentrated in vacuo. The resulting product (33) (2.40 g) was used without further purification.

The crude ketal (33) (0.83 g, ~1.45 mmol) was dissolved in ethylene glycol (44 ml) and crushed KOH (2.90 g, 51.6 mmol) was added to the rigorously stirred solution. The reaction was heated for 24 h at 100°C. After cooling to room temperature, the mixture was diluted with EtOAc followed by the addition of 1N NaOH. The aqueous portion was extracted with EtOAc and the combined organics washed several times with 1N NaOH. The crude solution was dried over $Na_2SO_4$, filtered, and concentrated to yield 0.31 g of (34) which was used without further purification.

To a solution of (34) (31.4 mg, 62.8 μmol) in $CH_2Cl_2$ (0.60 ml) was added *n*-propylsulfonyl chloride (20 μL, 178 μmol) and $Et_3N$ (30 μL, 215 μmol). The reaction was stirred for 2 h at room temperature under nitrogen. The mixture was concentrated under reduced pressure and purified by PTLC (10/90 $CH_3OH/CH_2Cl_2$), yielding (53) (12.3 mg, 32% over 3 steps). LRMS(FAB): (M+H)=606

**Example 13: Synthesis of Compound No. 22**

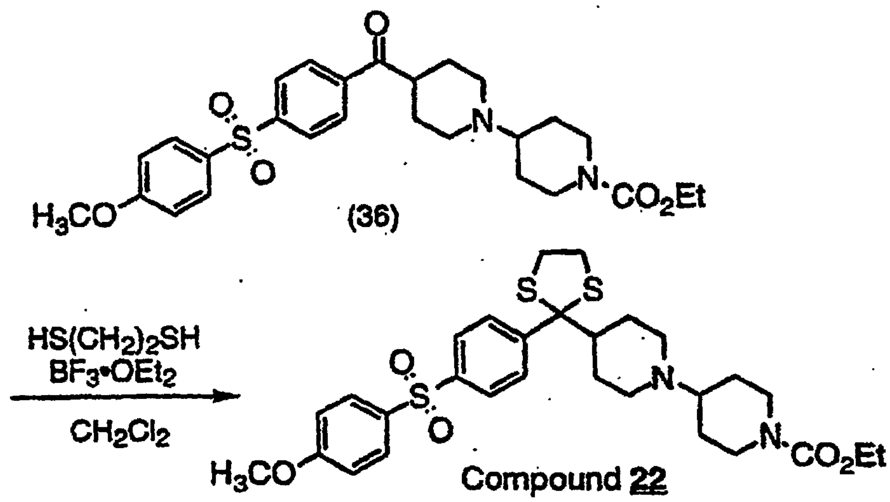


(36)

Compound 22

To a solution of (36) prepared via the procedure described far intermediate (7) of example 14, substituting 4-mercaptoanisole for 4-mercaptomethylenedioxybenzene and N-ethyoxycarbonyl-4-piperidinone for N-tBOC-4-piperidinone (29.5 mg, 58.9 μmol) in $CH_2Cl_2$ (0.24 ml) was added ethanedithiol (7.4 μL, 88 μmol) and $BF_3$•$OEt_2$ (11 μL, 88 μmol). The reaction was stirred overnight at room temperature under nitrogen, then diluted with $CH_2Cl_2$ and washed with 10% NaOH. The crude mixture was dried over $Na_2SO_4$, filtered, and concentrated under reduced pressure. Purification by PTLC (10/90 $CH_3OH/CH_2Cl_2$) yielded **37** (17 mg, 50% yield). LRMS (FAB): (M+H)=591.

**Example 14: Synthesis of Compound 43**

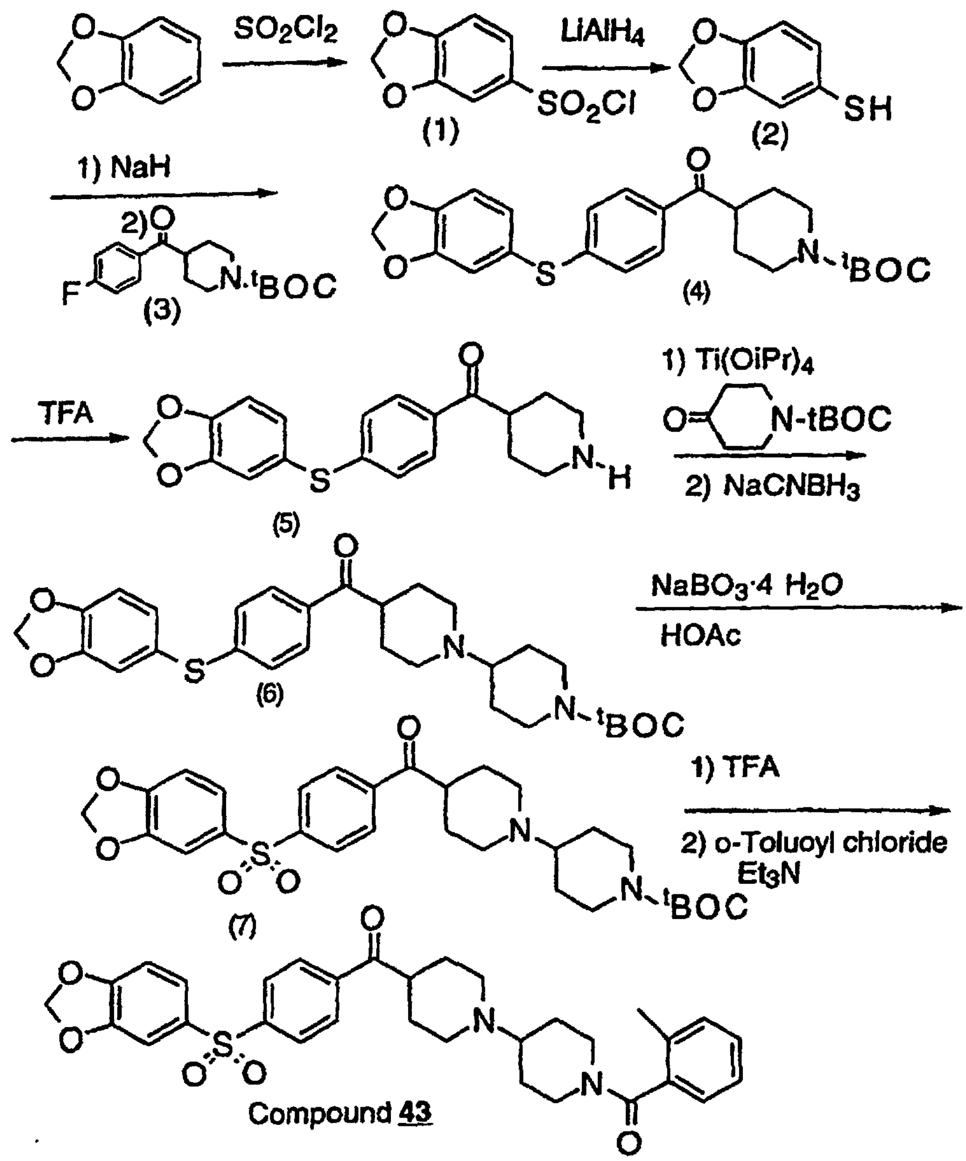


To a stirred solution of 41 ml of anhydrous DMF was added 43 ml of $SO_2Cl_2$ at 0-10°C followed by slow addition of 61 grams of methylenedioxy-benzene. After the addition was completed, the mixture was heated at 80°C for 10 min., and then 5-10 min. at 110°C. The reaction mixture turned to dark brown. The mixture was cooled to 40°C and was poured into a mixture composed of 200 ml $H_2O$/200g crushed ice/300 ml $CHCl_3$. Two layers were separated, the organic layer was dried over $MgSO_4$, filtered, and concentrated to give a crude product (1) which was triturated with 100 ml of hexanes. (60.6 g, 53%)

To a stirred suspension of 16 g LAH in 500 ml of THF at 0°C, was added dropwise 60.6 g of (1) dissolved in 100 ml of THF. The temperature was allowed to reach RT and the mixture was stirred at this temperature for 2 h.. The reaction mixture was then quenched carefully with 1N HCl at 0°C to pH=1, diluted with 1 L EtOAc. The organic phase was washed with 500 ml of water, dried over $MgSO_4$, filtered and concentrated to give a crude which was used directly in the next step without purification. (29 g, 68%)

28 g of the crude thiol (2) dissolved in 20 ml of DMF was added to a stirred suspension of 8 g of NaH(60%) in 80 ml of DMF. The mixture was stirred at RT for 1 h., ketone (3) dissolved in 150 ml of DMF was added at once. The mixture was heated at 70°C over night. The mixture was diluted with 400 ml of $CH_2Cl_2$ and then quenched with 300 ml of water. The organic phase was washed with 3x200 ml of water, dried with $MgSO_4$, filtered and concentrated to

give crude (4) which was purified by flash chromatography using EtOAc/Hex./$CH_2Cl_2$=1/4.5/4.5 as eluents. (35 g, 53%)

To a stirred solution of 6 g of (4) in in 100 ml of $CH_2Cl_2$ was added 10 ml of TFA. The mixture was stirred at RT for 1 h. The solvent was eliminated under reduced pressure and the residue was taken up in 100 ml of $CH_2Cl_2$ and extracted with 3x60 ml of $CH_2Cl_2$. The combined organic phase was chloride, quenched with 50 ml of 10% NaOH. The aqueous phase was dried over $MgSO_4$, filtered and concentrated to give crude (5), used without purification. (4.6 g, 100%)

A mixture of 3.4 g of (5) 2.4 g of N-BOC-4-piperidone and 5.9 ml of $Ti(OiPr)_4$ was stirred at RT over night. 1.6 g of $NaCNBH_3$ dissolved in 40 ml of $CH_3OH$ was added at 0°C. The mixture was stirred at RT for 1 h. diluted with 100 ml of EtOAc, quenched with a mixture of 60 ml water/20 ml of $NH_4OH$. The mixture was stirred at RT for 1 h. and filtered though celite. The aqueous phase was extracted with 3x100 ml of EtOAc. The combined organic phases were dried over $MgSO_4$, filtered and concentrated. The crude (6) was purified by flash chromatography using 100% EtOAc as eluent. (3.8 g, 73%)

To a solution of 1.32 g of (6) in 15 ml of HOAc was added 1.41 g of $NaBO_3•4H_2O$, and the mixture was stirred at RT for 3 days. The solvent was evaporated and the residue was quenched with 20 ml of 10% NaOH, then extracted with 3x50 ml of EtOAc. The combined organic extract was dried over $MgSO_4$, filtered and concentrated. The crude (7) was purified by silica gel prep TLC using 20%EtOH-EtOAc. (620 mg, 44%)

620 mg of (7) in 15 ml of $CH_2Cl_2$ and 5 ml of TFA was stirred at RT for 3 h. The solvent was evaporated and residue was treated with 10% NaOH, then extracted with EtOAc. The organic extract was dried over $MgSO_4$, filtered and concentrated to give crude product (510 mg, 100%). 0.052 mmol of the resultant crude free amine was reacted with 1.2 eq. of o-toluoyl chloride and 1.3 eq. of $Et_3N$ in 3 ml of $CH_2Cl_2$ for 3 h. The mixture was concentrated and the residue was purified by silica gel preparative TLC using 20% EtOH-EtOAc. (18 mg, 50%) $(M+H)^+$ Cald: 575.2216; Found: 575.2223.

The HCl salts were prepared by dissolving **43** in EtOAc and adding HCl-ether solution. After evaporating the solvent, the salts were collected as powder.

**Example 15: Preparation of Compound No. 82**

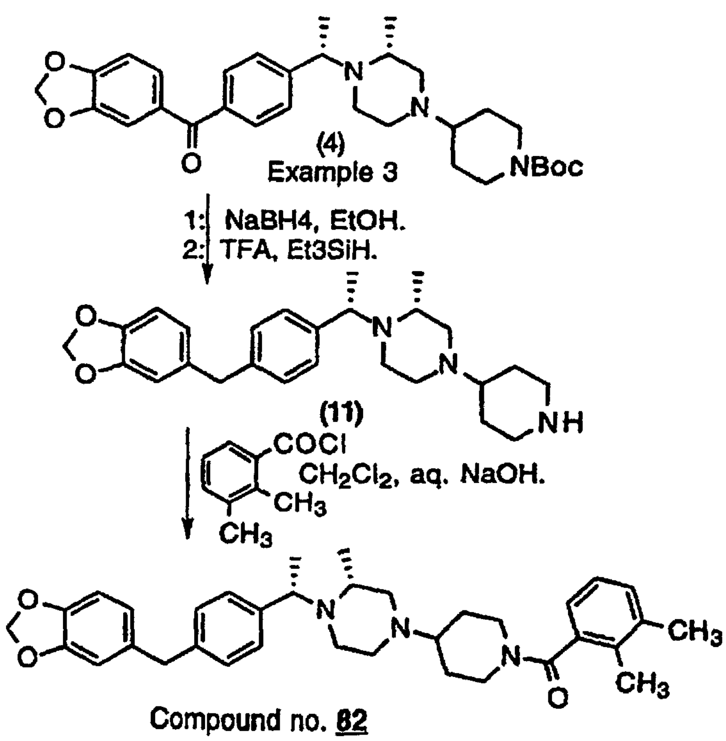


Compound no. 82

Stir a solution of compound (4) from Example 3 (0.5 g) and $NaBH_4$ (0.2 g) in ethanol (3 ml) at RT for 3 h. Add water, extract with $CH_2Cl_2$, dry over $MgSO_4$ and evaporate. To the residue in $CH_2Cl_2$ (5 ml) add triethylsilane (3 ml) and TFA (3 ml), relux for 3 h., evaporate, partition with $CH_2Cl_2$ and 1N aq. NaOH, dry and evaporate to obtain (11) as

a thick oil, used as such in subsequent acylation experiments.

Stir a mixture of (11) (0.03 g), $CH_2Cl_2$ (2 ml) and 1N aq. NaOH (2 ml) and add 2,3-dimethylbenzoyl chloride (0.07 g). Stir at RT for 18 h., extract with $CH_2Cl_2$, dry and evaporate. Dissolve the residue in 1:1 $CH_2Cl_2$:acetone and filter through a small plug of silica gel, washing with the same solvent mixture. Evaporate, dissolve the residue in a small volume of $CH_2Cl_2$ and precipitate the dihydrochloride by adding to excess HCl in $Et_2O$ as described in earlier preparations Wash with $Et_2O$ and dry to obtain the product compound No. <u>**82**</u> as an off-white powder (0.036 g). Mp: 207-212°C, with decomposition; MS: MH+ = 554.

**Example 16: Preparation of Compound No. <u>94</u>**

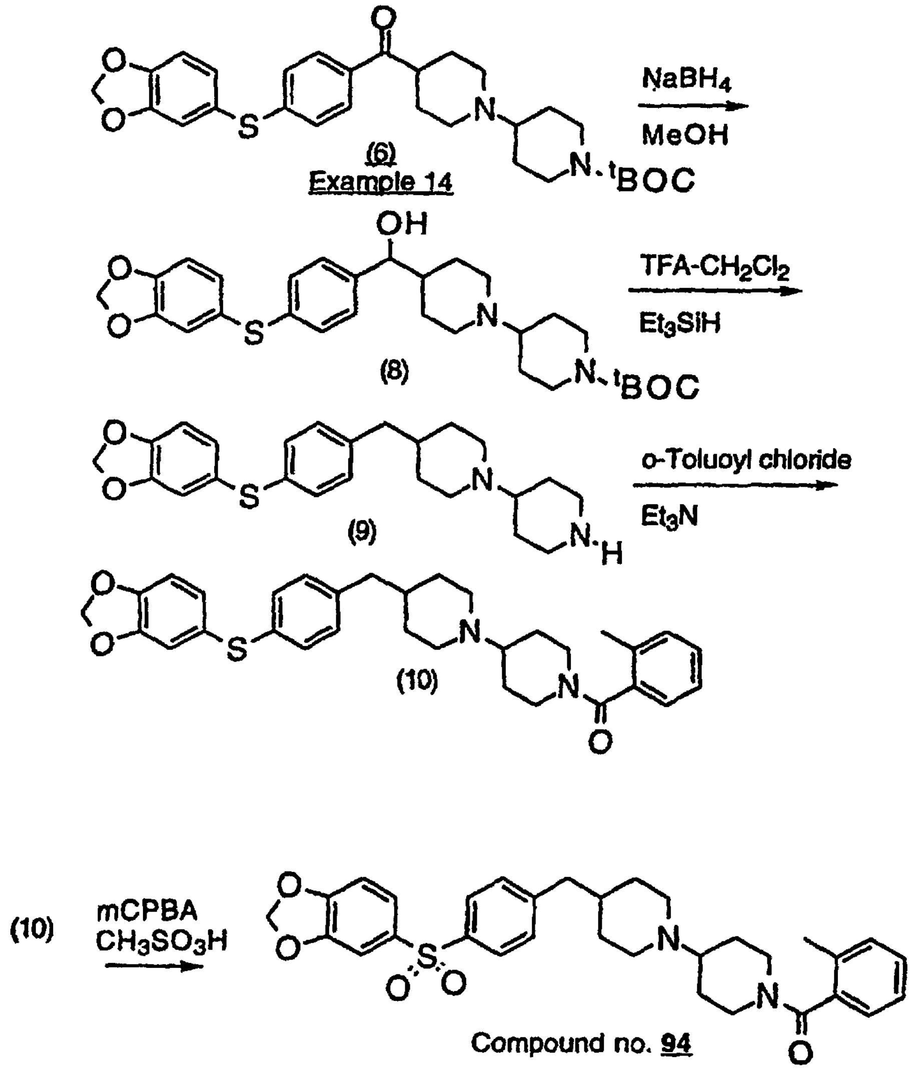


(6) Example 14

(8)

(9)

(10)

(10)

Compound no. 94

To a stirred solution of 899 mg (1.72 mmol) of (4), prepared as described in Example 14, in 15 ml of anhydrous $CH_3OH$ is added by portion 130 mg of $NaBH_4$. The mixture is stirred at room temperature for 30 min. and then quenched with 15 ml of 10% NaOH solution. The aqueous layer is extracted with 4x20 ml of EtOAc. The combined organic layers are dried over $Na_2SO_4$, filtered and concentrated to give 859 mg (95%) of (8).

To a stirred solution of 850 mg of crude (8) in 5 ml of $CH_2Cl_2$ is added 563 mg (3 eq.) of triethylsilane, followed by 2 ml of TFA. The mixture is stirred at room temperature for 3 hrs. and then is concentrated. The residue is taken up in 15 ml of $CH_2Cl_2$ and 15 ml of 10% NaOH. The aqueous layer is extracted with 3x15 ml of $CH_2Cl_2$. The combined organic layers are dried over $MgSO_4$, filtered and concentrated to give 600 mg (92%) of crude (9).

To a stirred solution of 600 mg of (9) and 455 mg of $Et_3N$ in 5 ml of $CH_2Cl_2$ is added 464 mg of o-toluoyl chloride. The mixture is stirred at room temperature for 2 hrs. The mixture is quenched with 20 ml of water, extracted

with 3x20 ml of EtOAc. The combined organic layers are dried over $MgSO_4$, filtered and concentrated to give a crude. Purification on silica gel prep TLC affords 555 mg (70%) of (10).

To a stirred solution of 43 mg of (10) and 0.5 ml of $CH_3SO_3H$ (0.5 M $CH_2Cl_2$) in 5 ml of $CH_2Cl_2$ is added at 0°C, 58 mg (57-86%) of mCPBA. The mixture is stirred at 0°C for 1 h. and is quenched with 15 ml of 10% $NaHCO_3$. The aqueous layer was extracted with 3x15 ml of EtOAc. The combined organic layers are dried over $MgSO_4$, filtered and concentrated to give a crude product. Purification on silica gel prep. TLC affords 36 mg (80%) of free base of compound number **94.** The HCl salt is prepared by dissolving the free base in EtOAc and adding HCl-ether solution. After evaporating the solvent, the salts are collected as powder. $(M+H)^+$ Cal: 561.2423; Found: 561.2416.

Using appropriate starting materials in the above procedures, with modifications well known in the art, the compounds exemplified in the following table are prepared:

## TABLE OF COMPOUNDS

| NO. | STRUCTURE | PHYSICAL DATA |
|---|---|---|
| 1 | 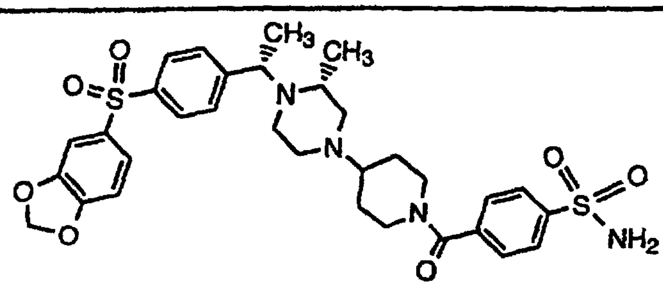 | - |
| 2 | 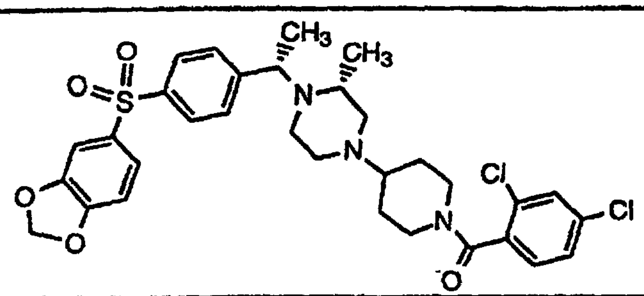 | - |
| 3 | 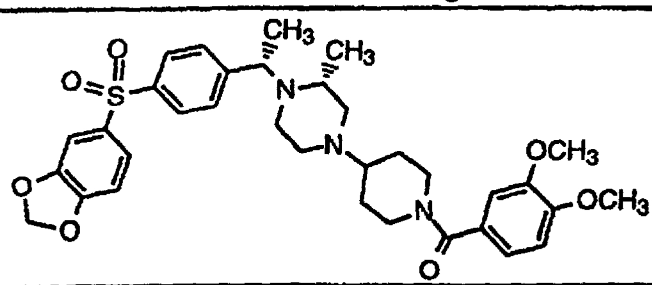 | - |
| 4 | 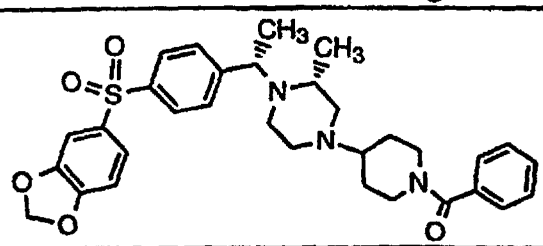 | mp=215-225°C (dec)<br>MS MH+=574 |
| 5 | 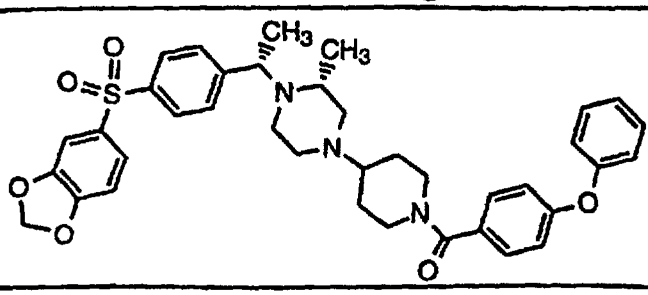 | - |

| | | |
|---|---|---|
| 6 | 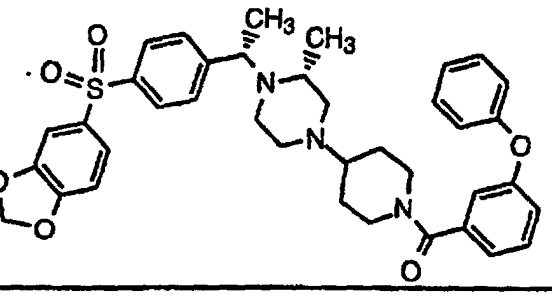 | - |
| 7 | 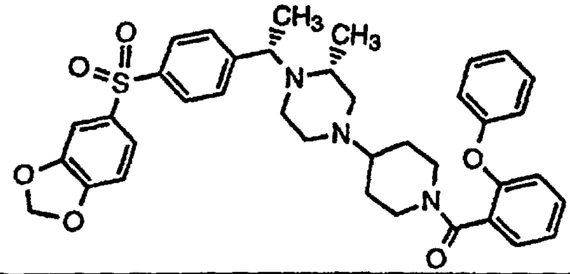 | - |
| 8 | 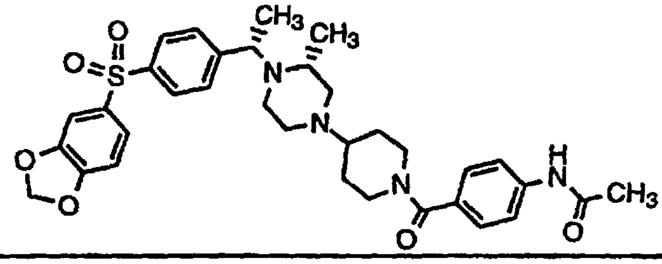 | - |
| 9 | 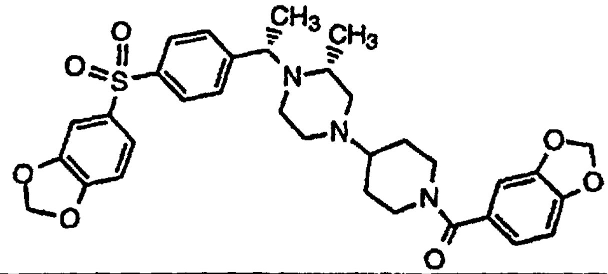 | - |
| 10 | 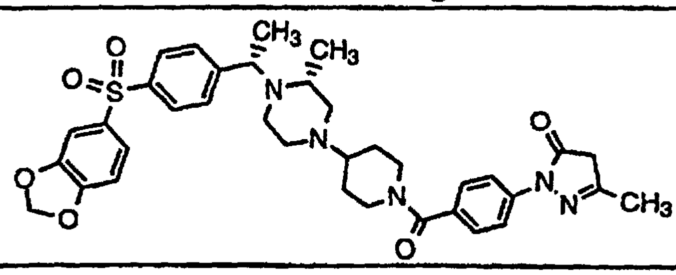 | - |
| 11 | 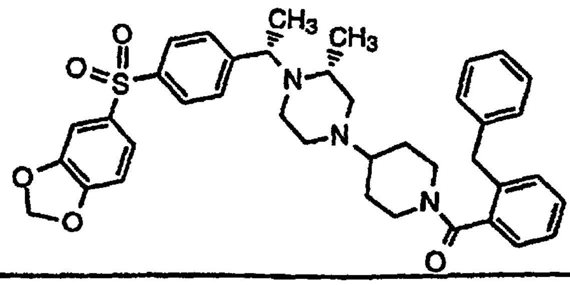 | - |

| | | |
|---|---|---|
| 12 | 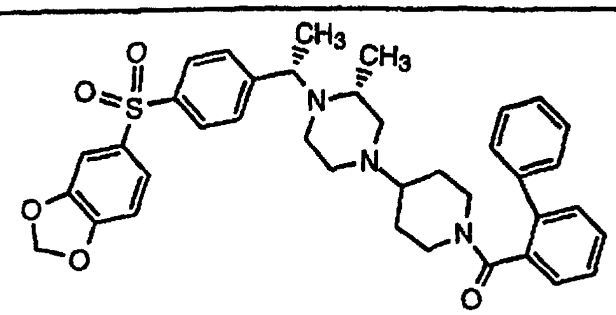 | - |
| 13 | 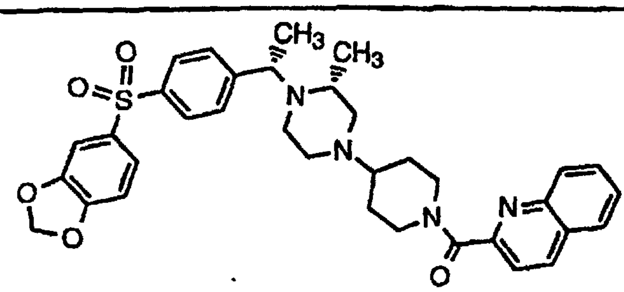 | - |
| 14 | 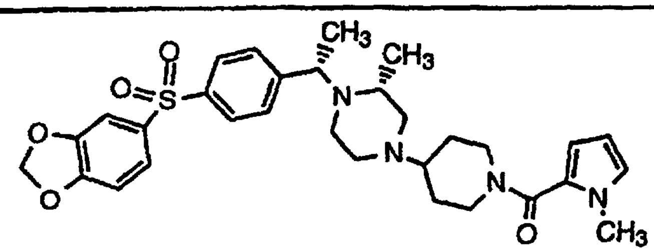 | - |
| 15 | 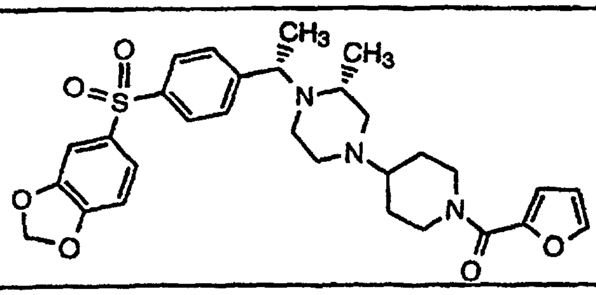 | - |
| 16 | 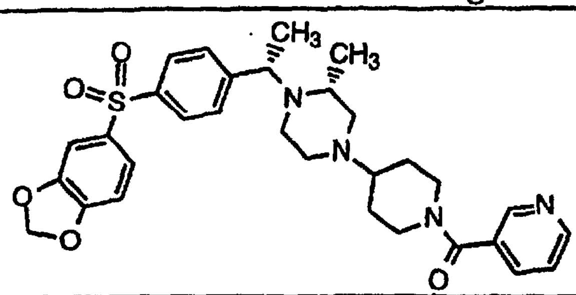 | - |
| 17 | 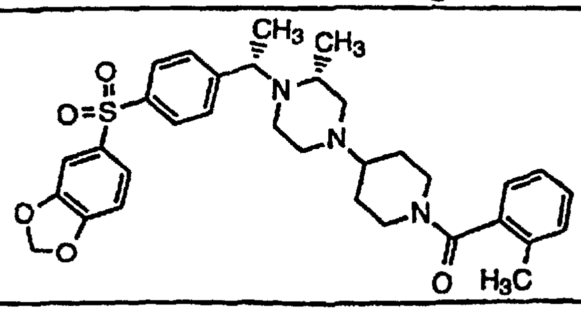 | mp=182-190 oC (dec) MS MH+=588 |

| | | |
|---|---|---|
| 18 | 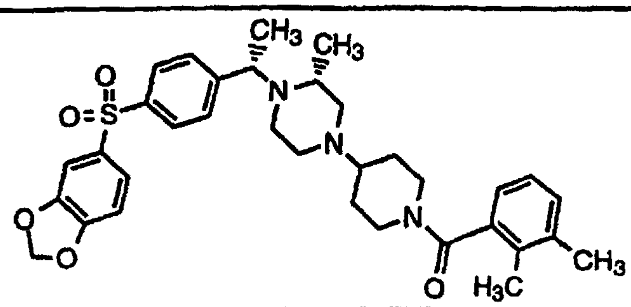 | - |
| 19 | 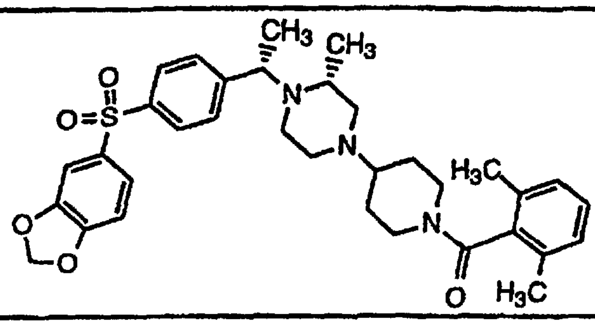 | - |
| 20 | 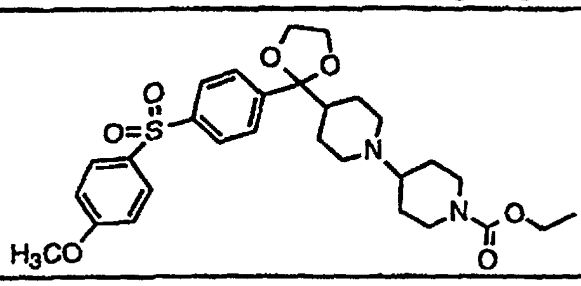 | - |
| 21 | 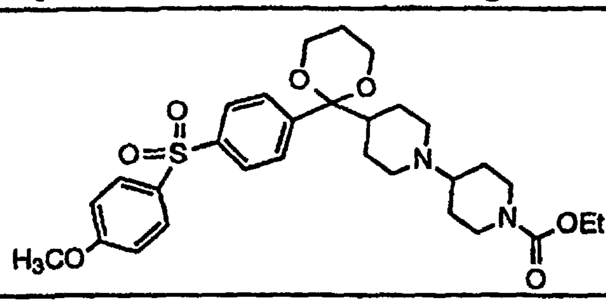 | - |
| 22 | 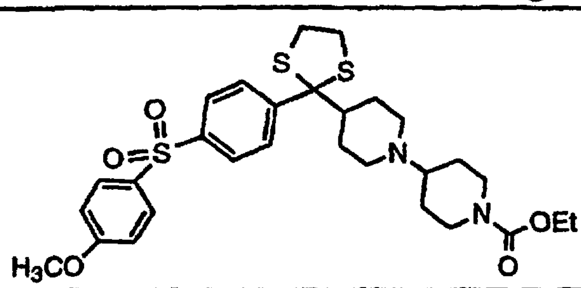 | LRMS (FAB) Calc. for $C_{29}H_{38}O_5N_2S_3$ (M+H): 591 Found: 591 |
| 23 | 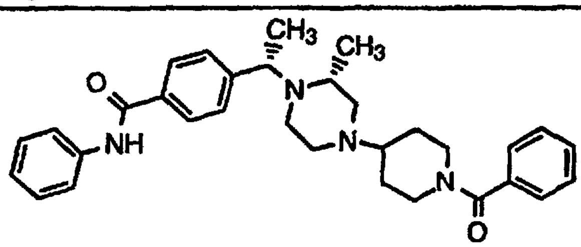 | - |

| | | |
|---|---|---|
| **24** | 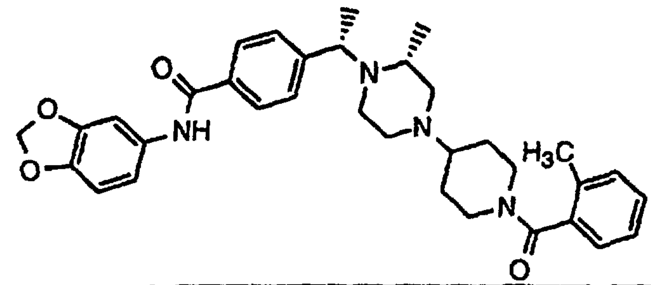 | mp=220-230°C (dec) MS MH+=569 |
| **25** | 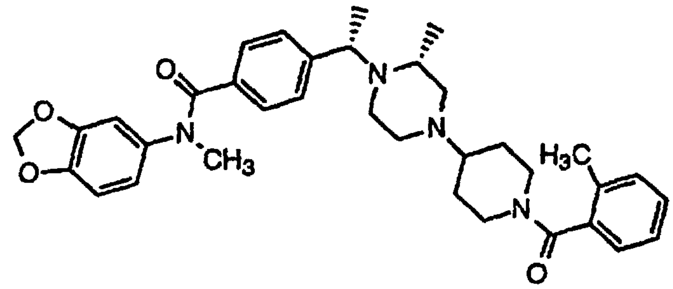 | mp=190-200°C (dec) MS MH+ = 583 |
| **26** | 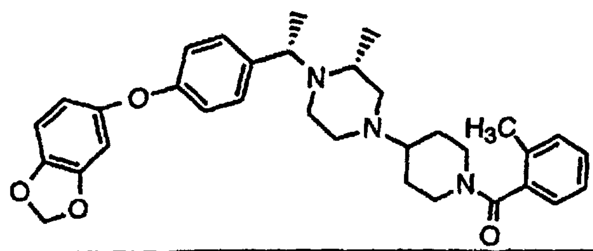 | - |
| **27** | 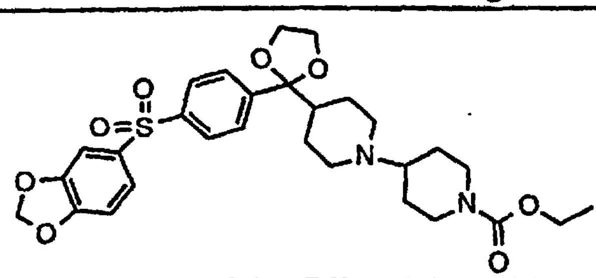 | HRMS Calcd: 573.2278 Found: 573.2271 |
| **28** | 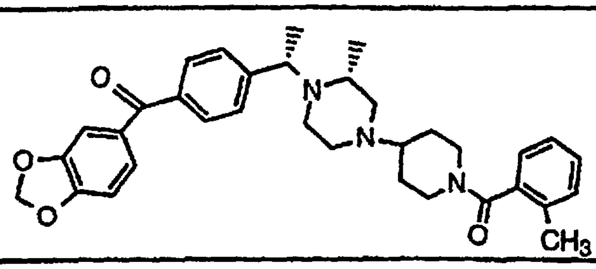 | mp = 200-210°C (dec) MS MH+ = 554 |
| **29** | 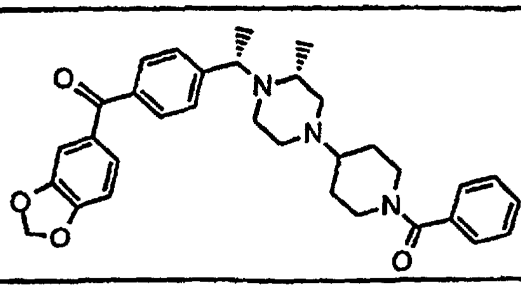 | - |

| | | |
|---|---|---|
| **30** | 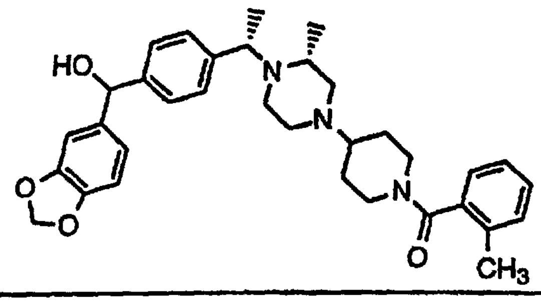 | mp = 85-95°C (dec) MS MH+ = 556 |
| **31** | 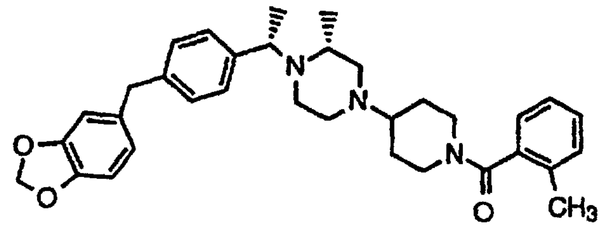 | mp= 210-215°C (dec) MS MH+=540 |
| **32** | 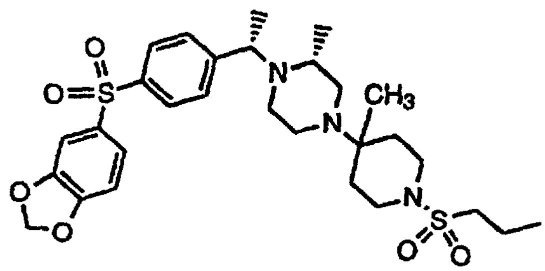 | mp=235-237°C (dec) |
| **33** | 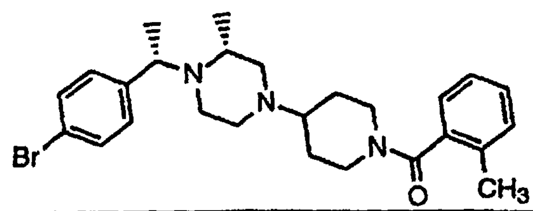 | - |
| **34** | 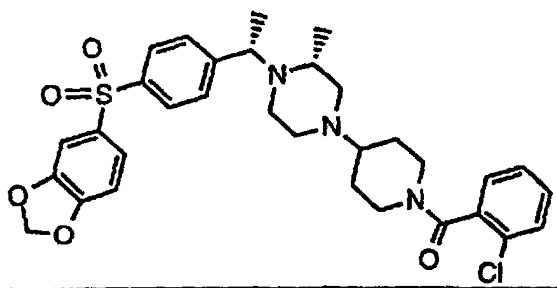 | mp=185-200°C (dec) MH+ 608/610 |
| **35** | 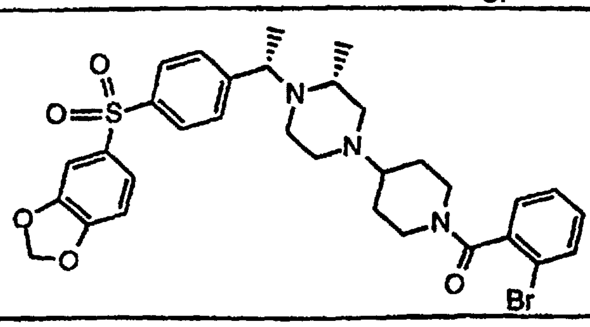 | mp=178-190°C (dec) MS MH+ = 652/654 |

| | | |
|---|---|---|
| **36** | 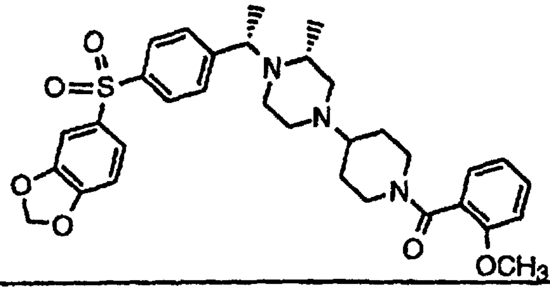 | mp=180-190°C (dec) MH+= 604 |
| **37** | 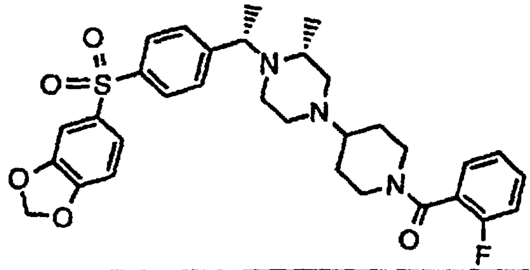 | mp=180-195°C (dec) MH+=593 |
| **38** | 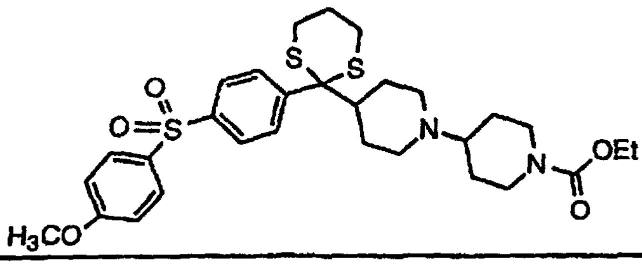 | - |
| **39** | 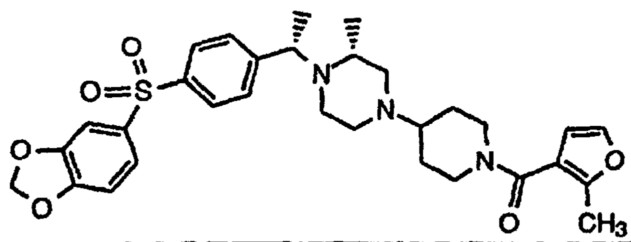 | mp= 190-205°C (dec) MS MH+=578 |
| **40** | 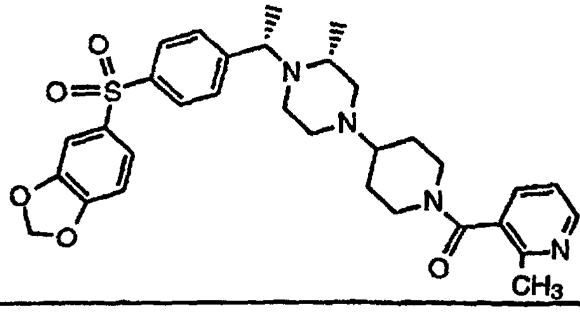 | - |
| **41** | 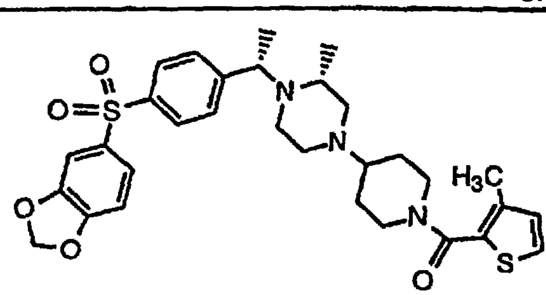 | mp = 180-290°C (dec) MS MH+=594 |

| | | |
|---|---|---|
| **42** | 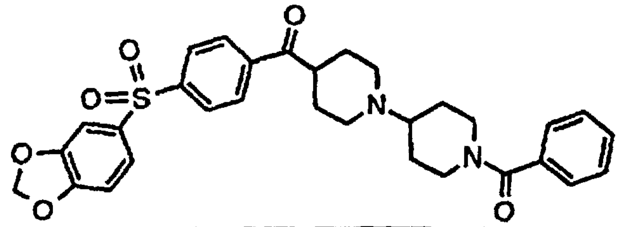 | - |
| **43** | 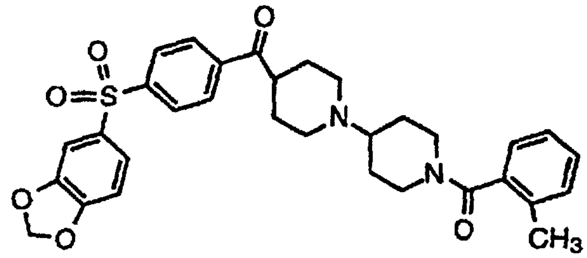 | HRMS<br>Calcd 575.2216<br>Found<br>575.2223 |
| **44** | 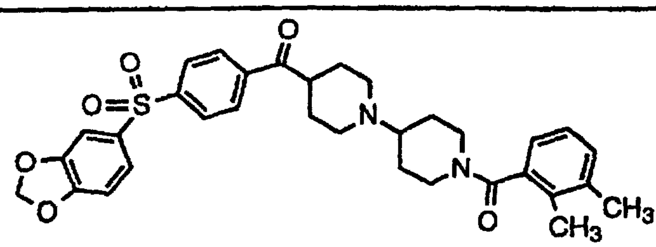 | HRMS<br>Calcd 589.2372<br>Found<br>589.2379 |
| **45** | 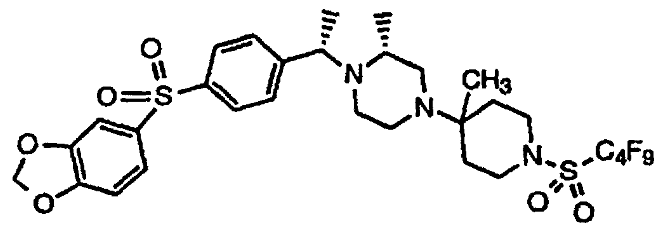 | mp=255-257°C<br>(dec) |
| **46** | 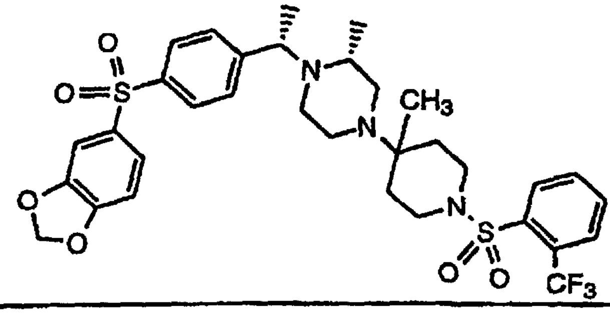 | FAB MH+=694 |
| **47** | 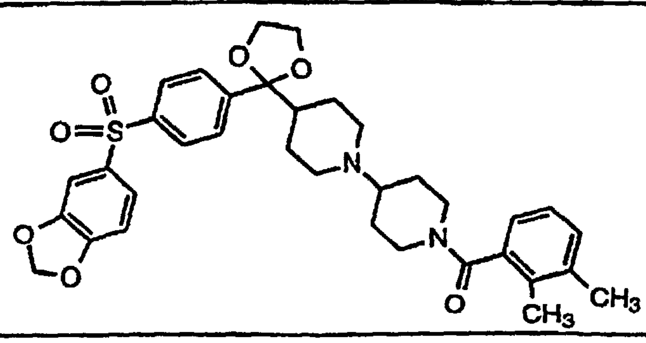 | - |

| | | |
|---|---|---|
| 48 | 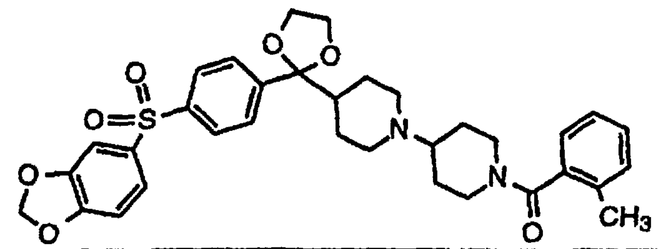 | - |
| 49 | 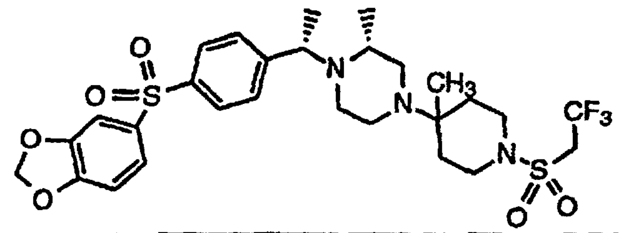 | FAB MH+=632 |
| 50 | 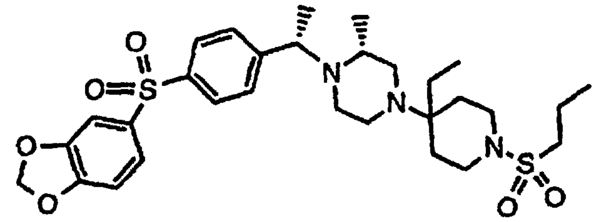 | mp=195-200°C (dec) |
| 51 | 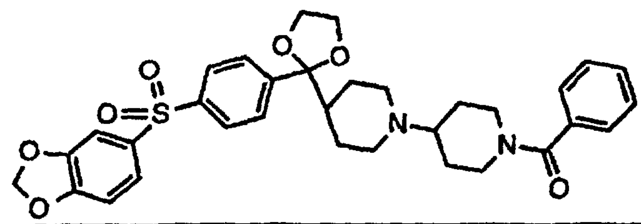 | - |
| 52 | 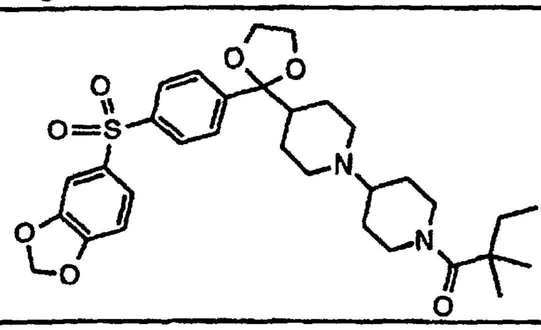 | - |
| 53 | 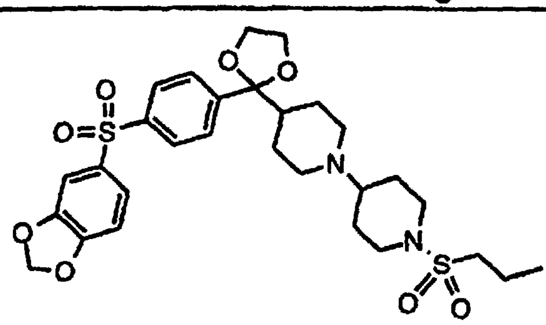 | LRMS (FAB) Calc. for $C_{29}H_{38}O_8N_2S_2$ (M+H): 606 Found: 606 |

| | | |
|---|---|---|
| 54 | 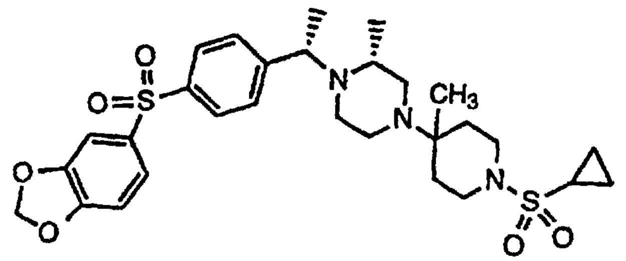 | FAB (NBA-G/TG-DMSO): 590 (M+), 574, 406, 389 |
| 55 | 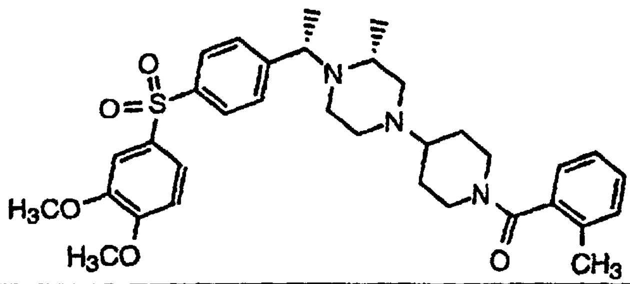 | HRMS calcd 594.2672 found 594.2668 |
| 56 | 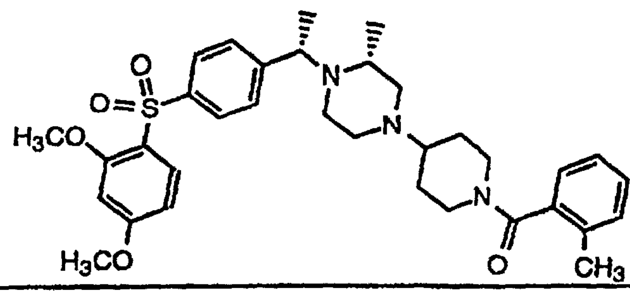 | HRMS calcd 642.2686 found 642.2685 |
| 57 | 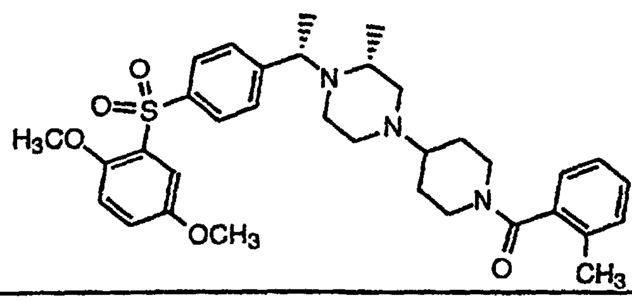 | HRMS calcd 606.3002 found 606.2987 |
| 58 | 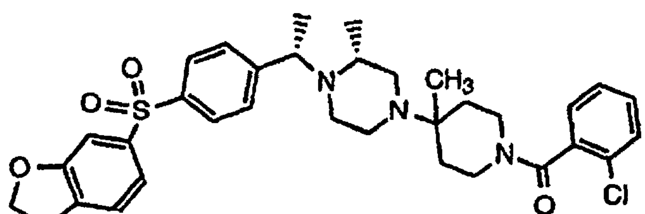 | mp=218-220°C (dec) |
| 59 | 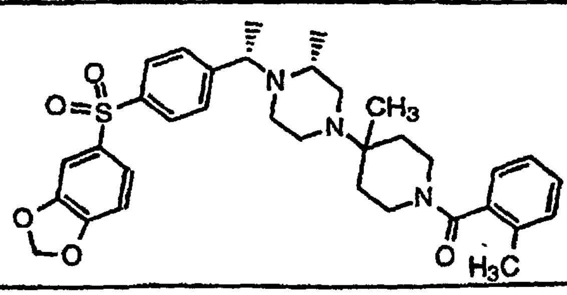 | mp=203-205°C (dec) |

| | | |
|---|---|---|
| **60** | 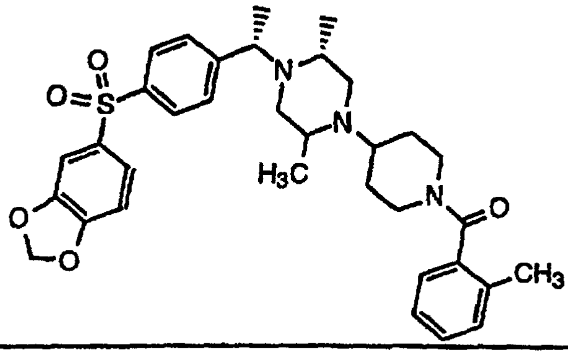 | - |
| **61** | 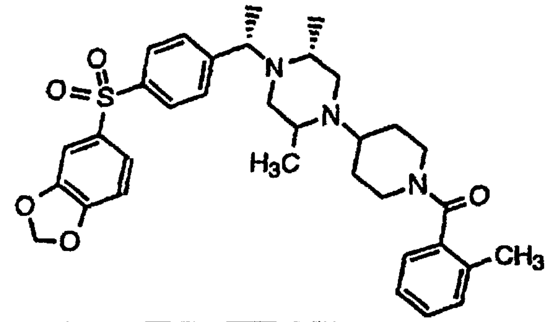 | - |
| **62** | 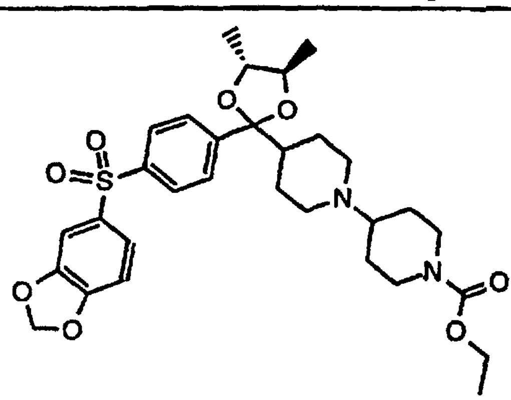 | LRMS (FAB) Calc. for $C_{31}H_{40}O_8N_2S$ (M+H): 601 Found: 601 |
| **63** | 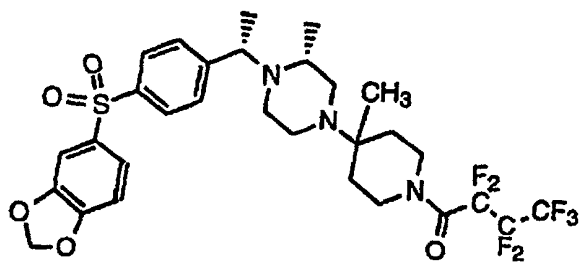 | FAB MH+=682 |
| **64** | 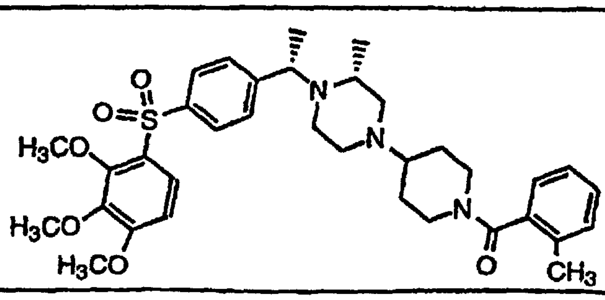 | HRMS calcd 636.3107 found 636.3119 |

| | | |
|---|---|---|
| **65** | 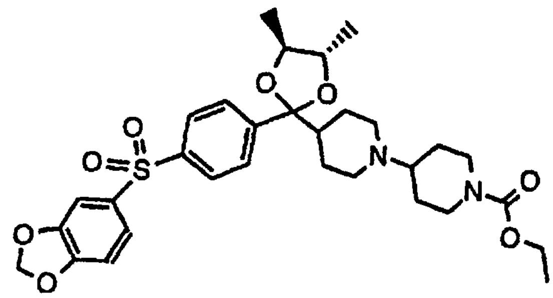 | - |
| **66** | 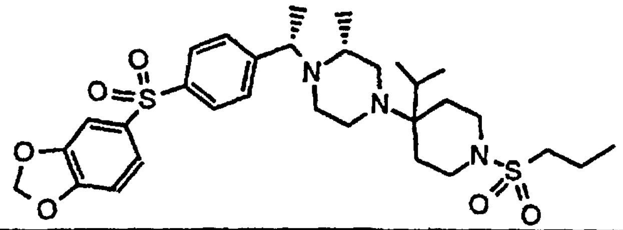 | mp=157-160°C (dec) |
| **67** | 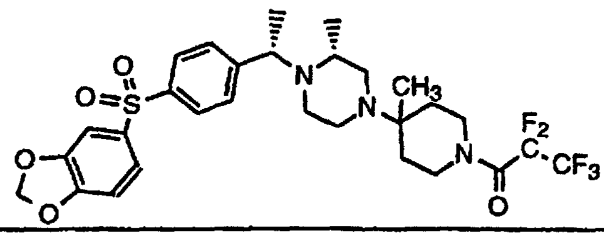 | FAB MH+=632 |
| **68** | 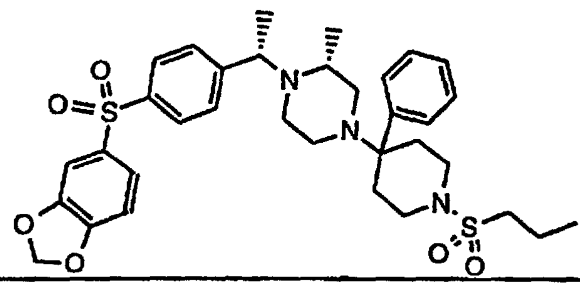 | mp=172-175°C (dec) |
| **69** | 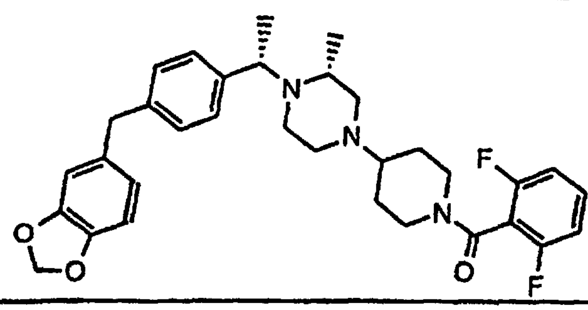 | |
| **70** | 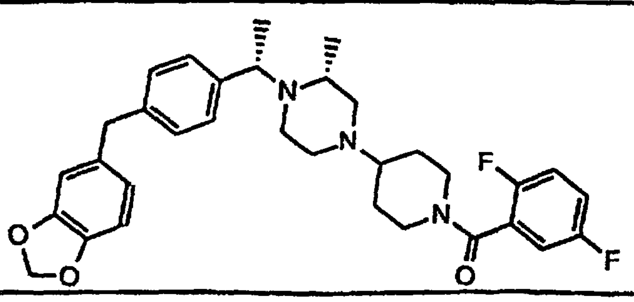 | |

| | | |
|---|---|---|
| 71 | 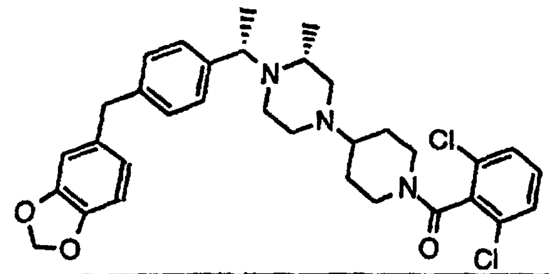 | |
| 72 | 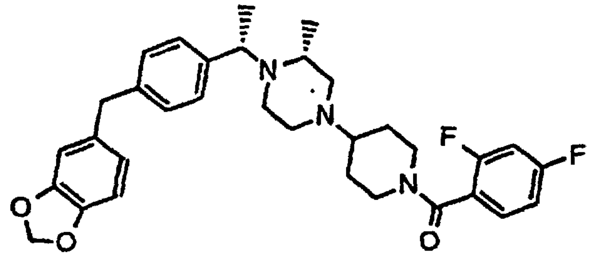 | |
| 73 | 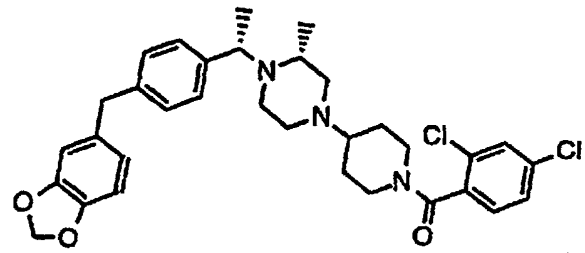 | |
| 74 | 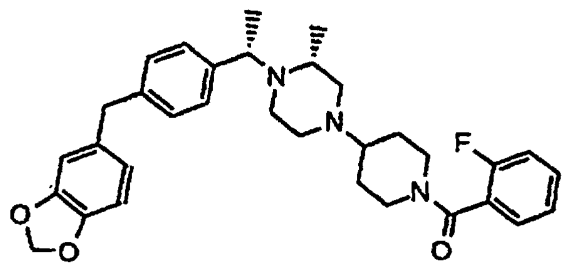 | |
| 75 | 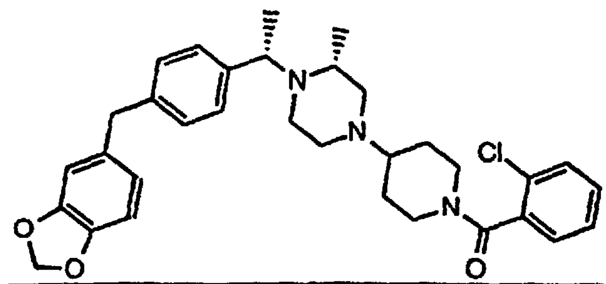 | |
| 76 | 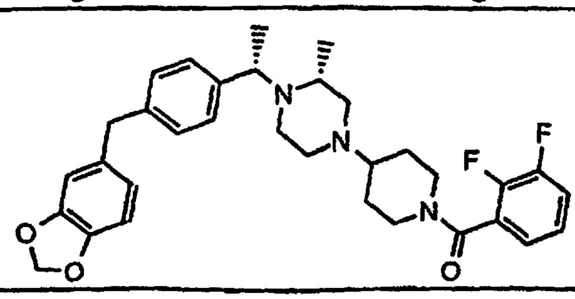 | |

| | | |
|---|---|---|
| 77 | 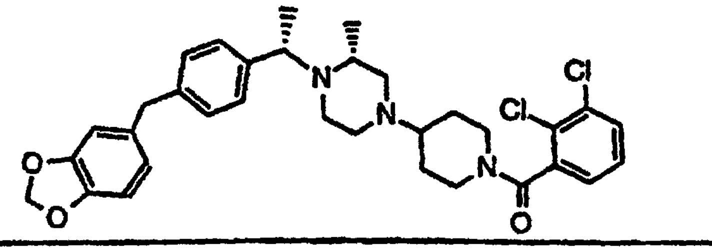 | |
| 78 | 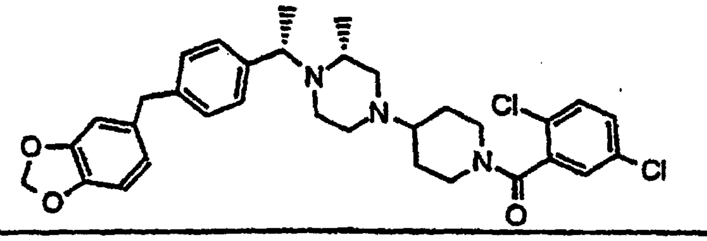 | |
| 79 | 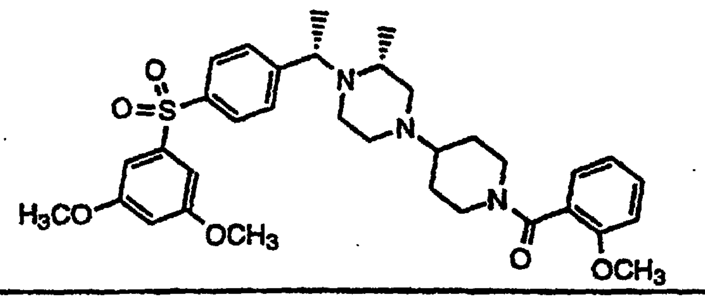 | HRMS<br>calcd 622.2951<br>found 622.2932 |
| 80 | 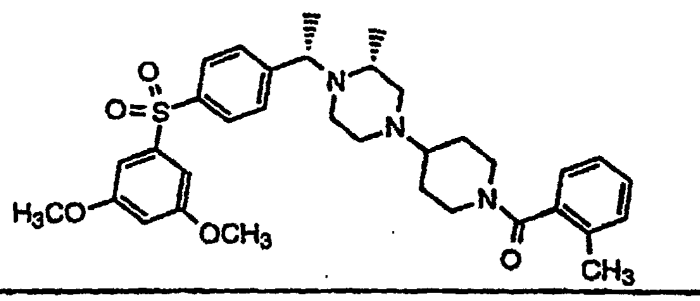 | HRMS<br>calcd 606.3002<br>found 606.2999 |
| 81 | 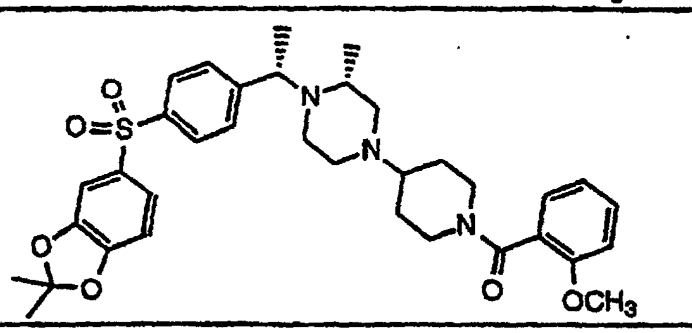 | HRMS<br>calcd 634.2952<br>found 634.2962 |
| 82 | 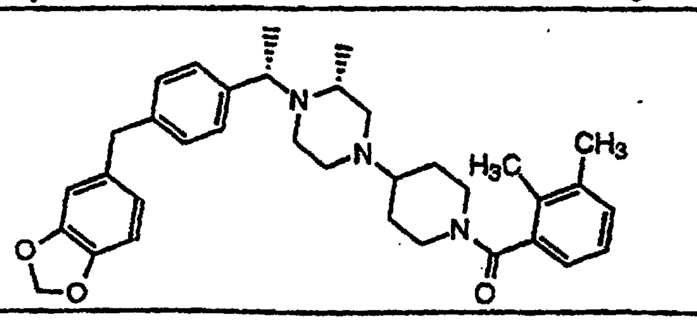 | mp: 207-212 °C<br>(dec)<br>Mass spectrum:<br>MH+ = 554 |

| | | |
|---|---|---|
| 83 | 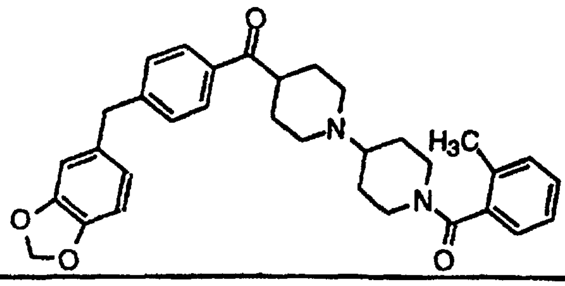 | - |
| 84 | 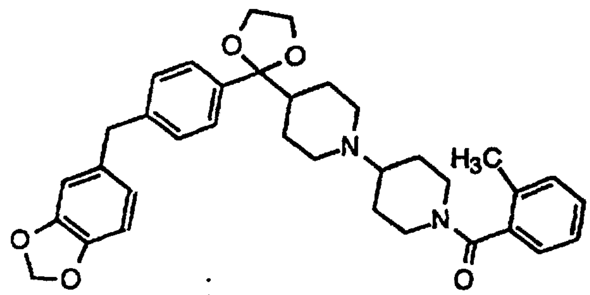 | HRMS Calcd 579.3015 Found 569.3022 |
| 85 | 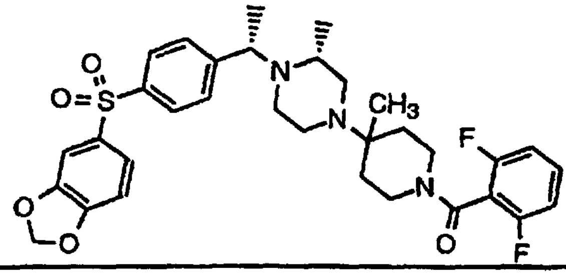 | mp=242-245 (dec) |
| 86 | 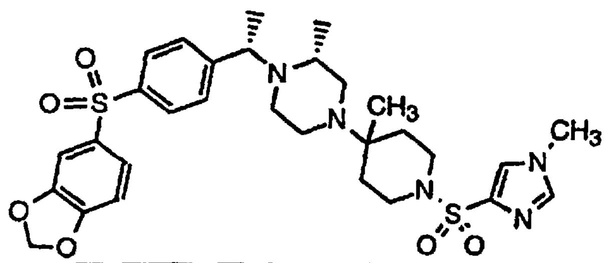 | mp=206-209 (dec) |
| 87 | 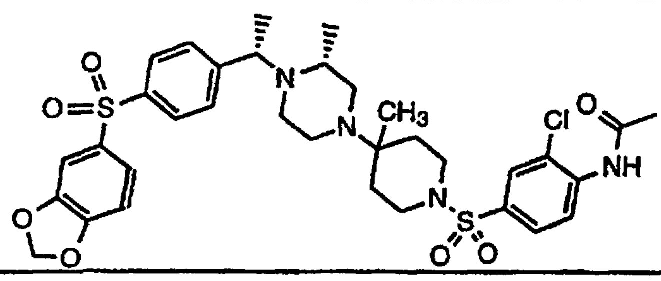 | mp=237-239 (dec) |
| 88 | 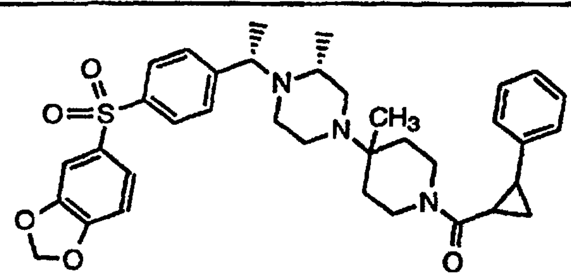 | FAB (SIMS): 630 (M+), 391, 329, 307, 289 |

| | | |
|---|---|---|
| **89** | 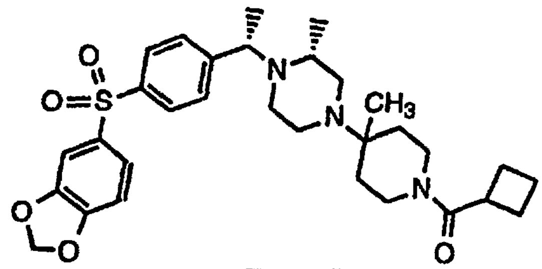 | FAB (SIMS): 568 (M+), 391, 307, 232 |
| **90** | 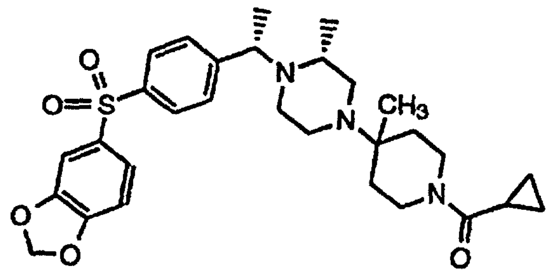 | FAB (NBA-G/TG-DMSO): 554 (M+), 538, 389, 289 |
| **91** | 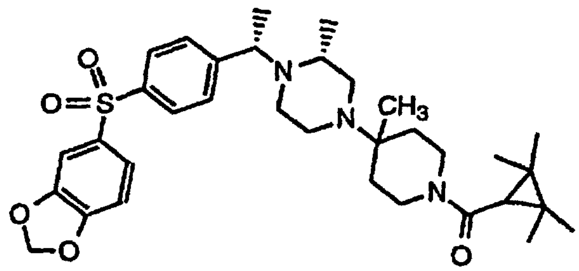 | FAB (NBA-G/TG-DMSO): 610 (M+), 594, 490, 426, 322 |
| **92** | 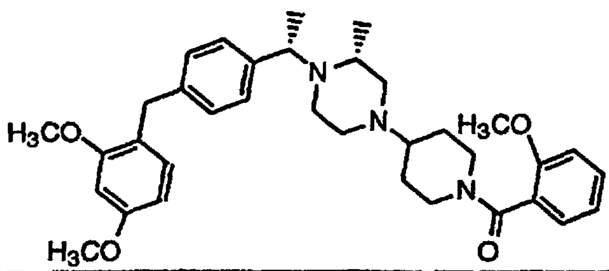 | HRMS calcd 572.3488 found 572.3491 |
| **93** | 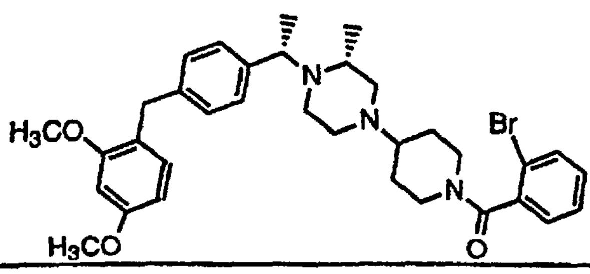 | HRMS calcd 620.2488 found 620.2478 |
| **94** | 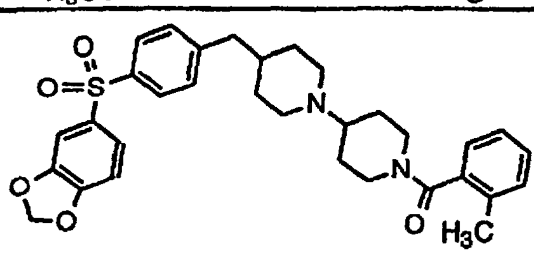 | HRMS Calcd 561.2323 Found 561.2423 |

| | | |
|---|---|---|
| **95** | 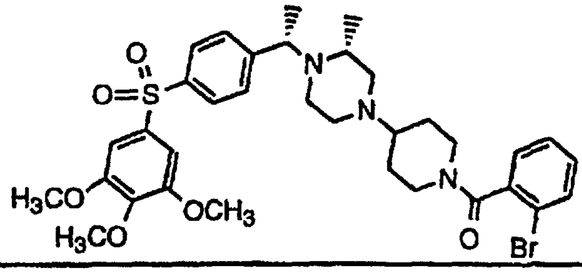 | HRMS<br>calcd 700.2056<br>found 700.2044 |
| **96** | 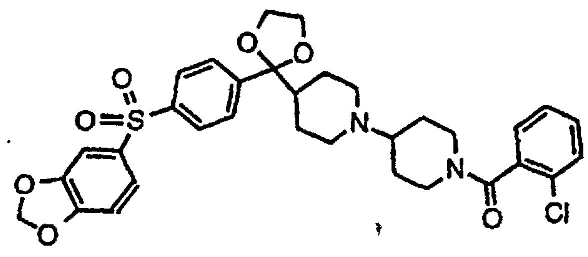 | LRMS (FAB)<br>Calc. for<br>$C_{33}H_{35}O_7N_2SCl$<br>(M+H): 639<br>Found: 639 |
| **97** | 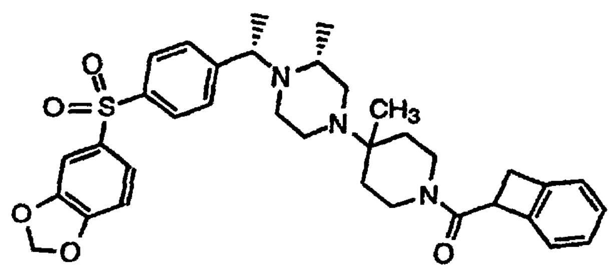 | FAB (NBA-G/TG-DMSO): 616 (M+), 432, 389, 328 |
| **98** | 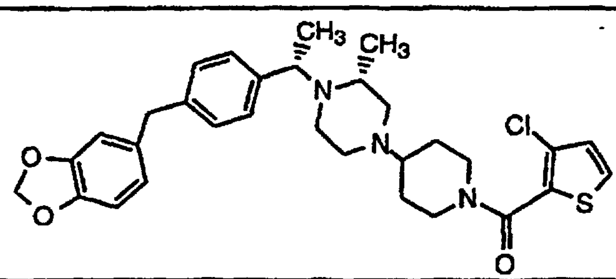 | - |
| **99** | 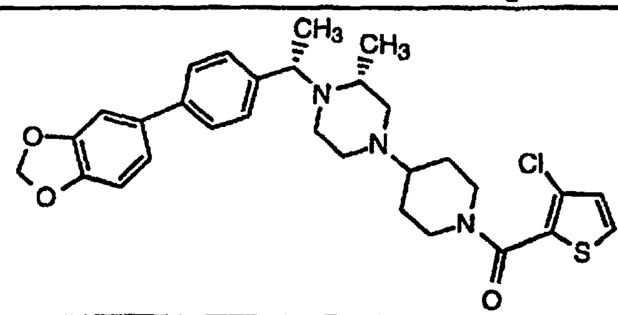 | - |
| **100** | 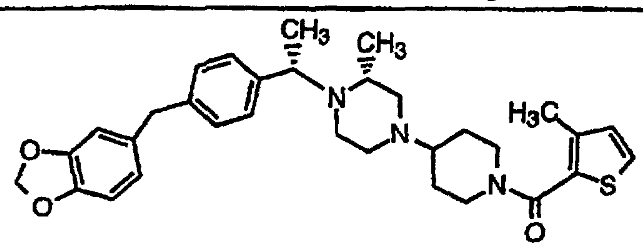 | mp: 210-220°C (dec)<br>Mass spectrum:<br>MH+ = 546. |

| | | |
|---|---|---|
| **101** | 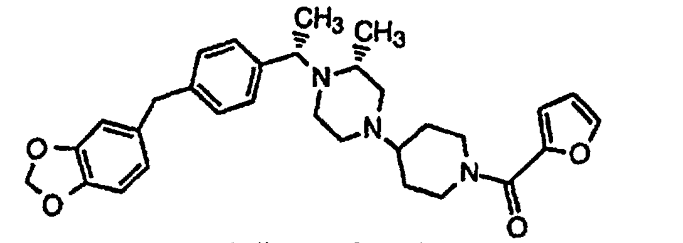 | - |
| **102** | 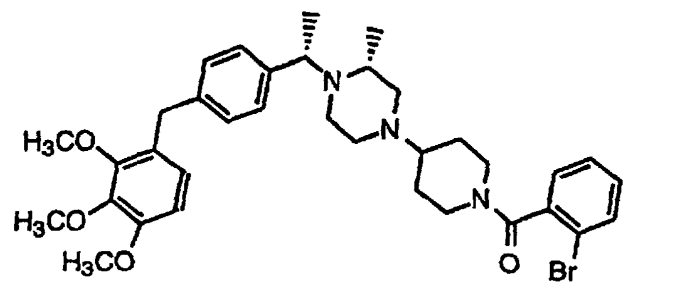 | HRMS<br>calcd 650.2593<br>found 650.2588 |
| **103** | 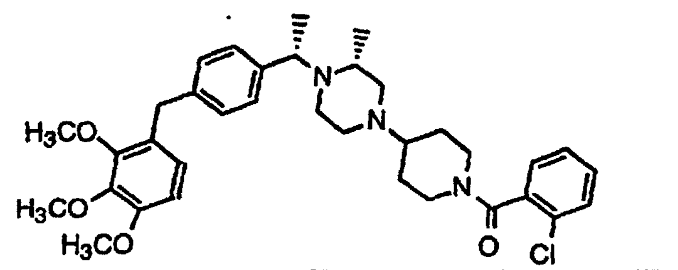 | HRMS<br>calcd 606.3099<br>found 606.3084 |
| **104** | 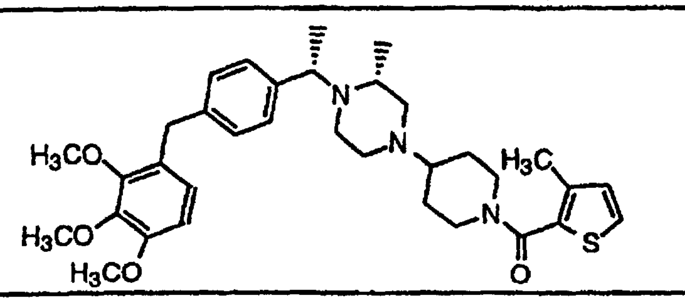 | HRMS<br>calcd 592.3209<br>found 592.3215 |
| **105** | 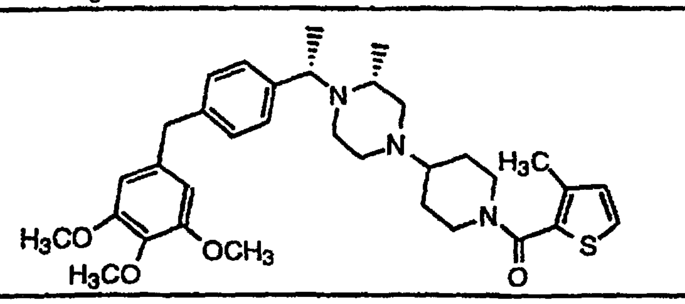 | HRMS<br>calcd 592.3209<br>found 592.3215 |
| **106** | 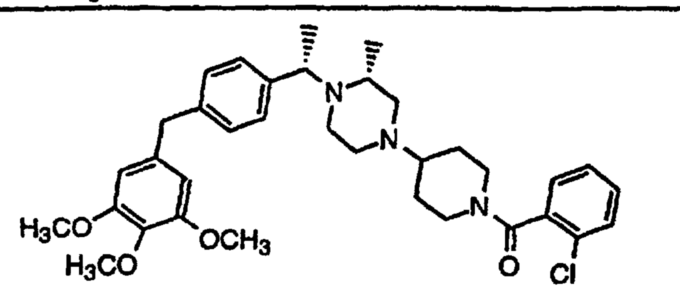 | HRMS<br>calcd 606.3099<br>found 606.3090 |

| | | |
|---|---|---|
| 107 | 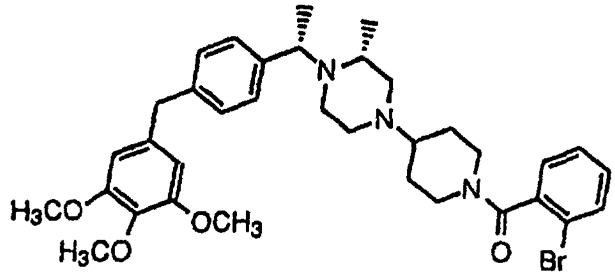 | HRMS calcd 650.2593 found 650.2579 |
| 108 | 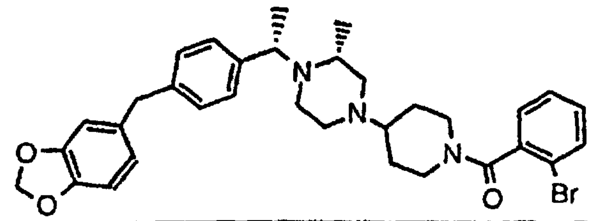 | - |
| 109 | 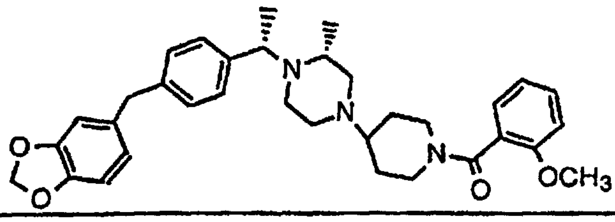 | - |
| 110 | 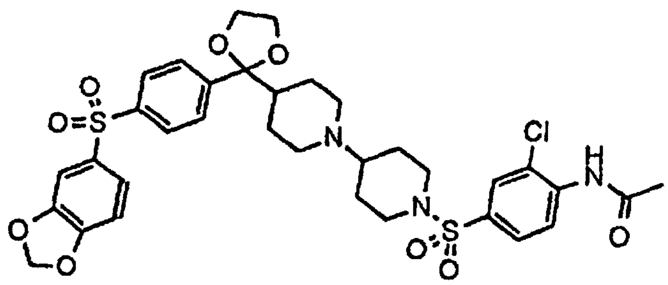 | LRMS(FAB): M+H = 732 |
| 111 | 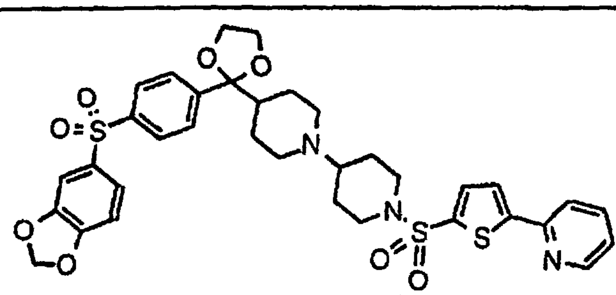 | LRMS(FAB): M+H = 724 |
| 112 | 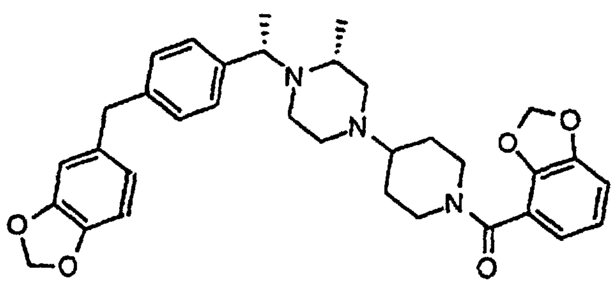 | mp: 190-195 °C (dec) Mass spectrum: MH+ = 546. |

| | | |
|---|---|---|
| 113 | 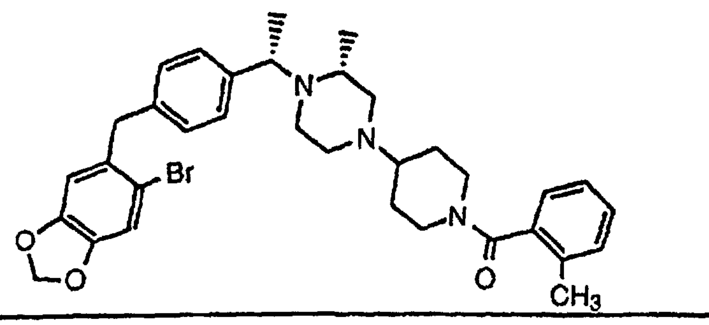 | MH+ = 618, 620 |
| 114 | 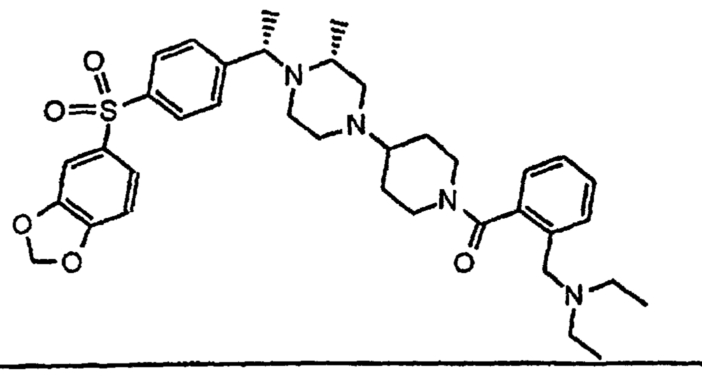 | mp=191°C (dec)<br>MH+ = 611 |
| 115 | 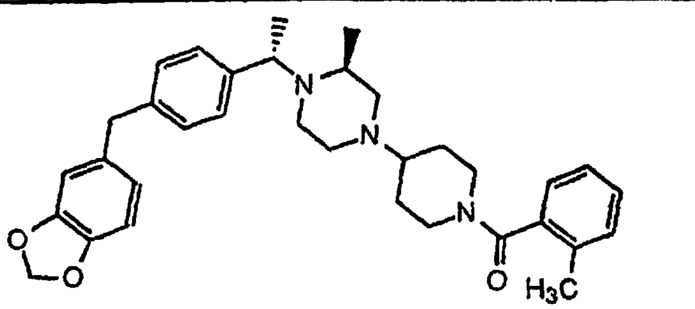 | MH+ = 540 |

**Example 17**

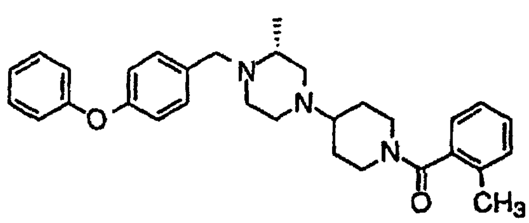

116

Step 1:

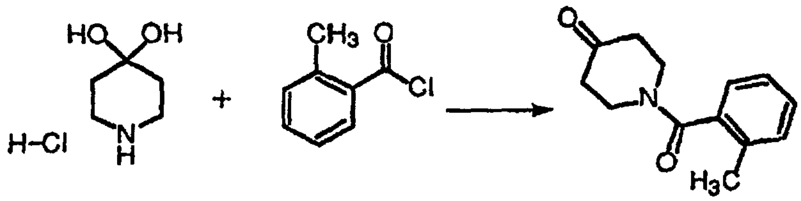

To a mixture of piperidone monohydratehydrochloride (27.5 g, 0.18 mol), $K_2CO_3$ (50g), 350 ml $CH_2Cl_2$, 250 ml water, cooled in an ice water bath, was added dropwise (over 1 h) a solution of o-toluoyl chloride (26 g, 0.17 mol) in 25 ml $CH_2Cl_2$. The mixture was warmed to room temperature and stirred overnight. The organic phase was separated, washed with water, dried over $Na_2SO_4$, filtered and concentrated on a rotarty evaporator. The crude product was purified on a silica column using hexane: EtOAc (4:1). Yield =93%

Step 2:

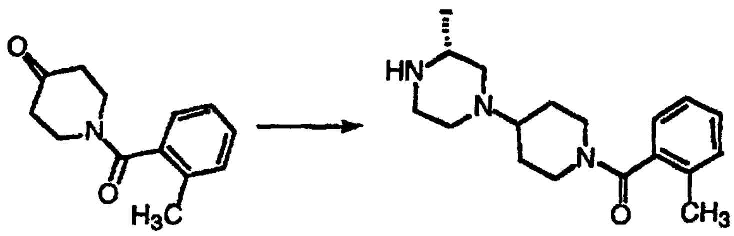

To a solution of 2-(R)-methylpiperazine (6.1 g, 61 mmol) in $CH_2Cl_2$ (250 ml) was added acetic acid (3.6ml), N-o-toluoylpiperidone (13.3 g, 61 mmol), and NaBH(OAc)3 (15.5 g, 73 mmol). The mixture was stirred overnight followed by dilution with $CH_2Cl_2$, washing with 10% $Na_2CO_3$ and water. Concentration of the organic phase was followed by purification on a silica column ($CH_2Cl_2$:$CH_3OH$ = 20:1, sat'd with aq. $NH_3$). Yield = 50%

Step 3:

To NaBH(OAc)3 (0.042 g, 0.2 mmol) was added a solution of the product of Step 2 (0.03 g, 0.1 mmol), acetic acid (0.006 ml), and 4-phenoxy-benzaldehyde (0.1 mmol). The mixture was stirred overnight followed by dilution with $CH_2Cl_2$ (10 ml), washing with 10% $Na_2CO_3$, and drying over $Na_2SO_4$. Concentration of the organic phase was followed by purification on a silica column (EtOAc). The hydrochloride. was prepared by dissolving the product in a minimum amount of EtOAc, followed by addition of anhydrous HCl. The precipitate was collected on a centrifuge and washed with $Et_2O$ (3x), followed by drying under vacuum. Yield = 66%. M.p. 217-219°C(dec).

**Example 18**

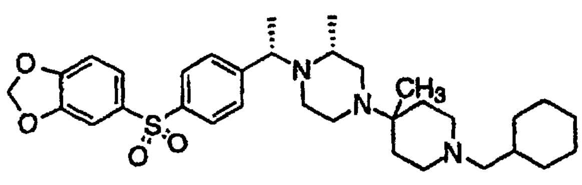

117

To NaBH(OAc)3 (0.026 g, 0.12 mmol) was added $CH_2Cl_2$ (1 ml), solution of cyclohexanecarboxaldehyde

(0.125 ml, 1M), acetic acid (0.004 ml), and intermediate (20) of Example 9 (0.03 g, 0.06 mmol). The mixture was stirred overnight followed by dilution with $CH_2Cl_2$ (10 ml), washing with 10% $Na_2CO_3$ and drying over $Na_2SO_4$. Concentration of the organic phase was followed by purification on a silica column (EtOAc). The hydrochloride was prepared as described in Example 17. Yield=67%. M.p. 208-209°C (dec).

**Example 19**

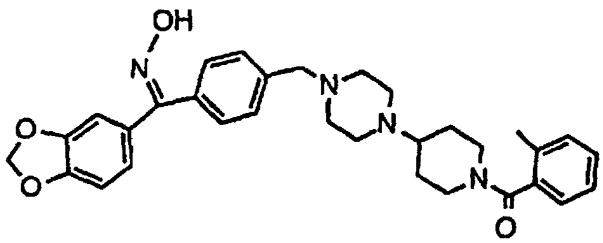

**118**

160 mg. of compound **28** were taken up in 4 ml ethanol with 45 mg of $H_2NOH•HCl$. 2 ml of pyridine were added and the mixture was heated to reflux for 5 hours. The solvent was removed *in vacuo*, and the reaction was diluted with $CH_2Cl_2$ and extracted with aqueous $NaHCO_3$. The organic layers were dried over $MgSO_4$, evaporated, and the residue purified by column chromatography in a gradient of $CH_3OH$/EtOAc. This gave 130 mg of product as a mixture of *syn* and *anti* isomers.
FAB (MH+) = 541.

**Examples 20A and 20B**

**20A:**

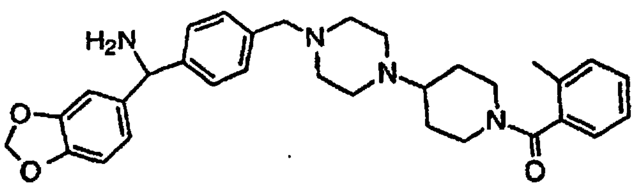

**119**

1.1 g of the product of Example 19 were taken up in 15 ml $CH_2Cl_2$ with 2 eq. of $Et_3N$, and cooled to -78°C under $N_2$. 440 mg (1.1 eq.) of chlorodiphenylphosphine were taken up in 2 ml of $CH_2Cl_2$ and added slowly to the reaction at this temperature. The reaction was stirred for 2 h in the cold and allowed to come to room temperature overnight. The solvent was removed *in vacuo*, and the crude material was taken up in 20 ml THF. 100 mg (excess) $NaBH_4$ was added and the mixture was stirred for 16 h at room temperature. The solvent was removed *in vacuo*, and the crude product was dissolved in a mixture of 10 ml of 6N HCl and 30 ml of $CH_3OH$. This mixture was stirred overnight at room temperature. The $CH_3OH$ was evaporated from the mixture, and the reaction was taken to pH 9 with the addition of aqueous NaOH. This solution was extracted with $CH_2Cl_2$ and the organic layers washed with water, dried over $MgSO_4$ and evaporated to a crude oily product. This was purified by column chromatography in a gradient of $CH_3OH$/EtOAc to give 356 mg of amine product. MS (FAB): 527.5 (M+1), 510.5

**20B:**

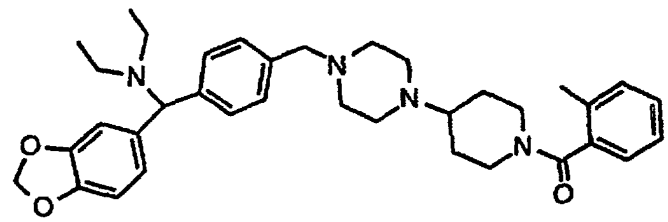

**120**

25 mg of the product of Step 20A were taken up in 2 ml $CH_2Cl_2$ with 90 mg of sodium triacetoxyborohydride. 9 microliters of acetaldehyde were added, and the mixture was stirred at room temperature for 1.5 hours. The reaction was diluted with addtiional solvent and extracted with aqueous $NaHCO_3$. The organic layers were dried over $MgSO_4$, evaporated, and purified by preparative thin-layer chromatography in 5% $CH_3OH$/EtOAc. This gave 12 mg of the desired product as an oil. FAB (M+) = 583.

**Example 21**

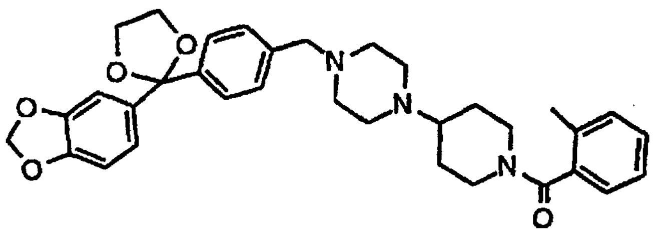

**121**

44 mg. of **28** were taken up in 20 ml of dry toluene with 2 ml of ethylene glycol and excess p-toluenesulfonic acid. The reaction was heated to reflux with a Dean-Stark water trap attached for 24 hours. NMR analysis showed partial reaction. Additional glycol and acid were added, and the reaction was heated an additional 24 hours. The mixture was extracted with aqueous $NaHCO_3$, evaporated, and purified by column chromatography in a gradient of $CH_3OH$/EtOAc to give 15 mg of product, which still contains small amounts of the starting ketone.
MS (electrospray): 598 (M+1), 302.

**Example 22**

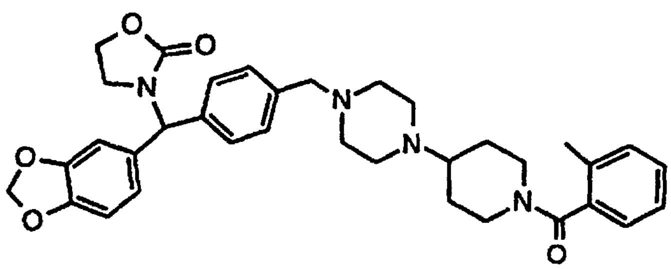

**122**

24 mg of compound **30** were taken up in 1 ml of $CF_3CO_2H$. An excess of 2-oxazotidinone was added and the mixture was stirred for 72 h at room temperature. Excess saturated $NaHCO_3$ solution was added, and the mixture was extracted with EtOAc. This was dried over $Na_2SO_4$, evaporated, and the residue purified by column chromatography

in 5% $CH_3OH$/EtOAc to give approx. 15 mg of product as an oil.
MS (electrospray): 625.2 (M+1), 302.

**Example 23**

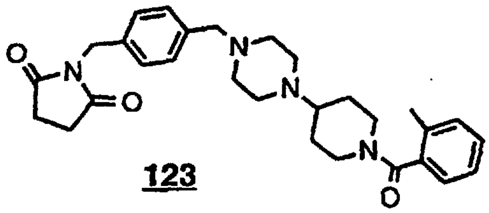

123

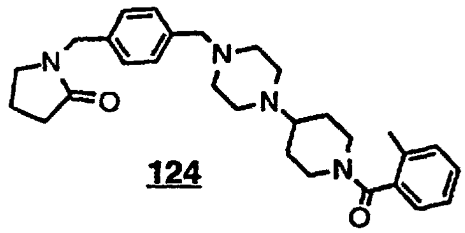

124

Step 1:

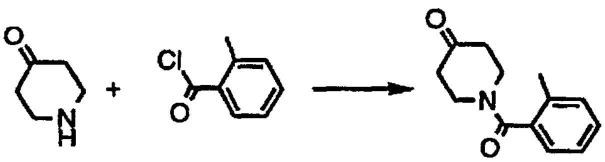

11 g of piperidinone hydrochloride were stirred in 100 ml $CH_2Cl_2$. 100 ml of 20% aqueous $K_2CO_3$ solution were added with vigorous stirring, followed by 8.8 ml of ortho-toluyl chloride. The mixture was stirred overnight at room temperature. The organic layer was separated and the aqueous layer was washed with additional solvent. The combined $CH_2Cl_2$ layers were dried over $Na_2SO_4$, evaporated, and purified by column chromatography in a gradient of hexane/EtOAc to give the product as an oil that solidified on standing.

Step 2:

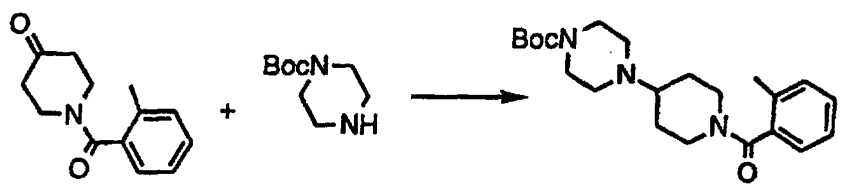

5.55 g of the product of Step 1 were combined with 4.75 g (1 eq.) of N-(t-butoxycarbonyl)piperazine in 100 ml of $CH_2Cl_2$. Sodium triacetoxyborohydride (5.4 g) was added and the reaction was stirred at room temperature for 72 hours. An additional gram of sodium triacetoxyborohydride was added, along with 2 ml of acetic acid, and the mixture was stirred an additional 24 hours. 25 ml of $CH_3OH$ was added and the mixture was evaporated. The crude material was dissolved in $CH_2Cl_2$ and extracted with water. The organic layer was dried over $Na_2SO_4$, evaporated, and the residue purified by column chromatography in a gradient of EtOAc/hexane to give 6.7 grams of product.

Step 3:

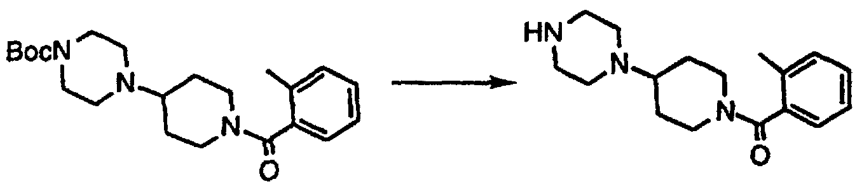

6.7 g of the product of Step 2 were taken up in 100 ml $CH_2Cl_2$, and 20 ml of $CF_3CO_2H$ were added slowly at room temperature. The reaction was stirred for 24 hours. 20 ml of water were added and the reaction was brought to pH 10 by the slow addition of solid NaOH. The organic layers were separated and the aqueous layers washed with additional $CH_2Cl_2$. The organic layers were dried over $Na_2SO_4$ and evaporated to give 5.1 g of product as a solid, which was used directly.

Step 4:

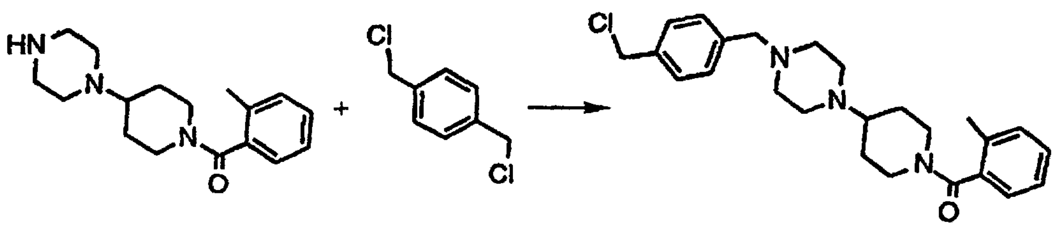

3.9 g of the product of Step 3 were taken up in 200 ml THF with 7.15 g (3 eq.) of 1,4-(bis)-chloromethylbenzene and 2 ml (1 eq.) of $Et_3N$. The mixture was heated to reflux for 7 hours and cooled to room temperature. 300 ml of $Et_2O$ were added and the precipitated $Et_3N$•HCl was removed by filtration. 34 ml of 1M HCl in $Et_2O$ (2.1 eq.) was added in portions, with stirring, and the precipitate was filtered off and dried in vacuo to give 5.6 g of the dihydrochloride salt of the product, slightly contaminated with $Et_3N$•HCl.

**Compound 123:**

65 mg of the product of Step 4 (as a mixture containing 60% $Et_3N$•HCl) were taken up in 2 ml dry DMF with excess $K_2CO_3$. The mixture was stirred for 1 h at room temperature until the solid starting material had dissolved. In a separate flask, excess succinimide was reacted with NaH in 1 ml dry DMF for 30 min. at room temperature. The DMF solution of the free amine was removed from the $K_2CO_3$ and added to this anion, and the mixture was stirred for 16 h at room temperature. Water was added, and the mixture was extracted twice with EtOAc. The organic layers were washed with water, dried over $Na_2SO_4$ and evaporated. This gave the product in sufficient purity to form its HCl salt directly. MS (electrospray): 489.1 (M+1), 276.

**Compound 124:**

In a manner similar to that described for compound 123, but using pyrrolidinone in place of succinimide and potassium hexamethyldisilazide (KHMDS) (in toluene) in place of NaH, compound **124** was prepared in sufficient purity to form its HCl salt directly.
MS (electrospray): 475 (M+1), 408.

**Example 24:**

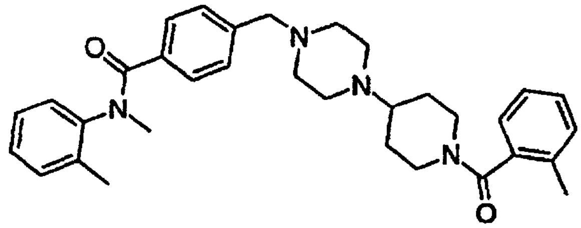

125

Step 1:

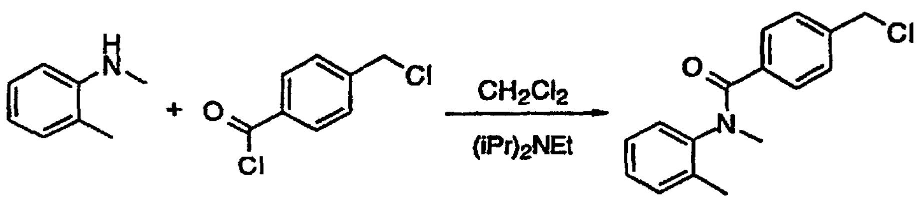


0.8 ml (6.35 mmol) of N-methyl-o-toluidine, 1.0 g (5.29 mmol) of 4-chtoromethyl(benzoyl chloride) and 1.4 ml (7.93 mmol) of $(iPr)_2NEt$ were taken up in $CH_2Cl_2$ (20 ml) at 0°C. The solution was warmed to r.t., stirred at r.t. for 17 h, then was diluted with $CH_2Cl_2$ and washed with 1M HCl(aq.). The aqueous layer was washed with $CH_2Cl_2$. The combined $CH_2Cl_2$ layers were dried over $NaSO_4$, filtered and concentrated to give the desired benzyl chloride as a light brown oil (1.55 g, quant.).

Step 2:

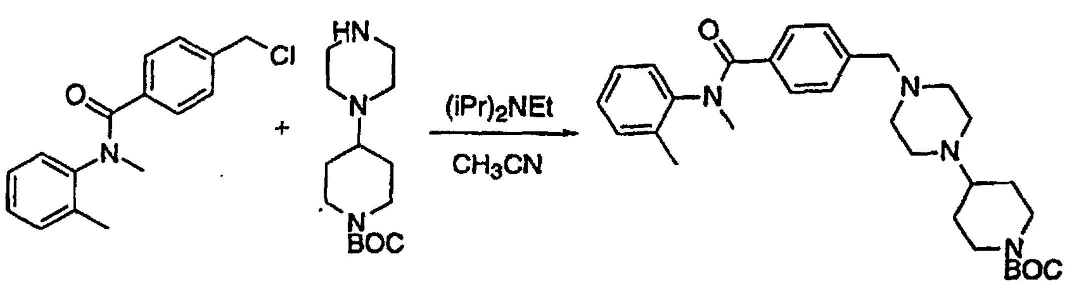


The product of Step 1 (1.45 g, 5.29 mmol), piperazine-piperidine Boc compound (1.42g, 5.29 mmol), and $(iPr)_2NEt$ (1.84 ml, 10.6 mmol) were taken up in $CH_3CN$ (25 ml). The solution was heated at reflux for 2.75 h. The solution was partitioned between $CH_2Cl_2$ and 1 N NaOH (aq.). The aqueous layer was extracted with $CH_2Cl_2$. The combined $CH_2Cl_2$ layers were dried over $Na_2SO_4$. Filtration and concentration gave gave a tan foam (2.7 g). Purification via flash chromatography(15/1 $CH_2Cl_2$/MeOH, $SiO_2$) gave 2.26 g (84 %) of a colorless foam.

Step 3:

The BOC-protected product of Step 2 (2.26 g, 4.47 mmol) was taken up in $CH_2Cl_2$ (20 ml) and cooled to 0°C. TFA (5 ml) was added, and the solution was warmed to r.t. The solution was atirred at r.t. for 20 h. The solution was concentrated and the residue triturated with $CH_2Cl_2/Et_2O$ to give a white solid. The solid was washed with $Et_2O$ and

dried, which gave 4.1 g (99 %) of the TFA salt of the amine.

Step 4:

Using a procedure similar to that described in Example 1, treat the product of Step 3 to obtain **125.** M.p. = 235 - 240°C. HRMS (FAB) calc'd for $C_{33}H_{41}N_4O_2$ (MH): 525.3230. Found: 525.3239.

**Example 25**

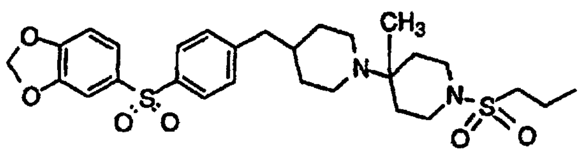

**126**

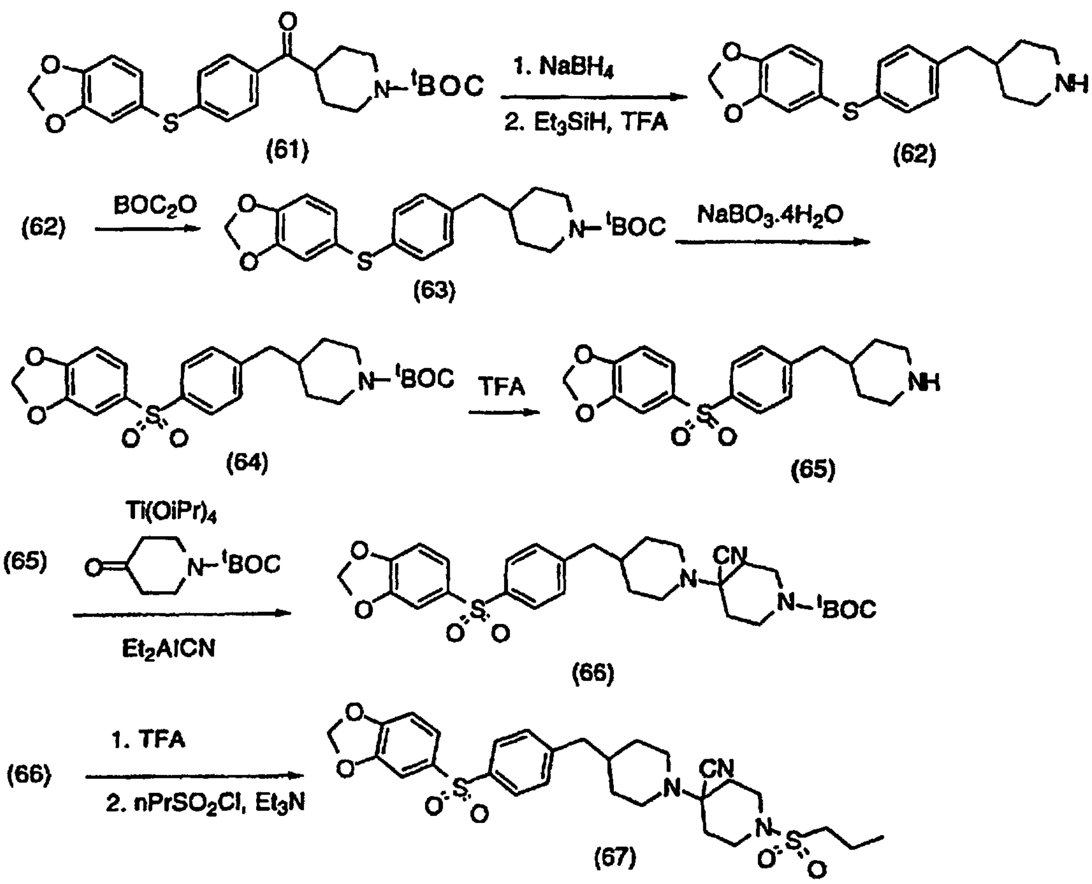


Step 1: To a stirred solution of 10.0 g (22.6 mmol) of (61) in 50 ml of anhydrous $CH_3OH$ was added $NaBH_4$ in portions at 0°C. The mixture was stirred at rt for 30 min and then quenched with water. The aqueous layer was extracted with (4x30 ml) EtOAc. The combined layers were dried over $Na_2SO_4$, filtered and concentrated to give 10.0 g (99%) of an alcohol which was used directly. To a stirred solution of the alcohol 10.0 g (22.5 mmol) in $CH_2Cl_2$ (100 ml) was added 16 ml of $Et_3SiH$, followed by 40 ml of TFA. The mixture was stirred at rt for 3 h and then quenched with 1N NaOH. The aqueous layer was extracted with $CH_2Cl_2$ (100 ml). The combined organic layers were dried over $Na_2SO_4$ and concentrated to yield 6.0 g (82%) of (62) as a colorless oil.

Step 2: To a stirred solution of (62) (5.0 g, 15.29 mmol) in $Et_2O$ (200 ml) and 40 ml of 10% NaOH was added $BOC_2O$. The mixture was then stirred at rt for 24 h. The layers were separated and the organic layer washed with water, dried ($Na_2SO_4$), filtered, concentrated and chromatographed to yield 5.7 g (87%) of (63) as colorless oil.

Step 3: To a stirred solution of (63) (5.7 g, 13.34 mmol) in acetic acid was added $NaBO_3$•$4H_2O$ and stirred for 24 h at rt. The reaction mixture was then quenched with 25% NaOH. The organic layer was then extracted with EtOAc

(3x30ml). The combined organic layers were dried ($Na_2SO_4$), filtered, concentrated and chromatographed to yield 2.8 g (46%) or (64).

Step 4: To a stirred solution of (64) (1.5 g, 3.26 mmol) in $CH_2Cl_2$ (35 ml) was added TFA (2 ml). The mixture was stirred at rt for 1 h and concentrated. The residue was then diluted with $CH_2Cl_2$ and washed with 10% NaOH. The extracts were dried ($Na_2SO_4$), filtered and concentrated to yield 0.983 g (84%) of (65).

Step 5: To a stirred solution of (65) (0.983 g, 2.73 mmol) and t-butoxycarbonyl piperidine (0.571 g, 2.87 mmol) in $CH_2Cl_2$ (3 ml) was added $Ti(iOPr)_4$ (1 ml). The reaction mixture was stirred at rt for 24 h and to the resulting solution was added 1.0 M toluene solution of $Et_2AlCN$ (8.7 ml). The reaction was the stirred at rt for 2 h, diluted with EtOAc (10 ml) and quenched with water (3 ml). The resulting slurry was then filtered through celite, concentrated and chromatographed to yield 1.1 g (74%) of (66).

Step 6: To a stirred solution of (66) (0.225 g, 0.398 mmol) in $CH_2Cl_2$ was added TFA (1 ml). The mixture was stirred at rt for 1 h and concentrated. The residue was then diluted with $CH_2Cl_2$ and washed with 10% NaOH. The extracts were dried ($Na_2SO_4$), filtered and concentrated to yield crude amine which was used directly in the next step without purification 0.164 g (89%).

To a stirred solution of crude amine (0.053 g, 0.115 mmol) in $CH_2Cl_2$ (1 ml) was added $Et_3N$ (0.100 ml) followed by n-$PrSO_2Cl$ (0.05 ml) and stirred at rt for 1 h. The reaction was quenched with 2N NaOH and the organic layer was extracted with $CH_2Cl_2$. The combined organic layers were dried ($NaSO_4$), filtered, concentrated and chromatographed to afford 0.025 g (50%) of (67).

Step 7: To a stirred solution of (67) (0.018 g, 0031 mmol) in THF (2 ml) was added 3.0 M solution of MeMgBr in $Et_2O$ dropwise at rt. The reaction was the stirred for 2 h at rt and quenched with $H_2O$. The aqueous layer was then extracted with EtOAc, dried ($Na_2SO_4$), filtered, concentrated and chromatographed to yield 0.010 g (60%) of **126**. LRMS: calc'd: 562; found ($M+H^+$) 563.

**Example 26**

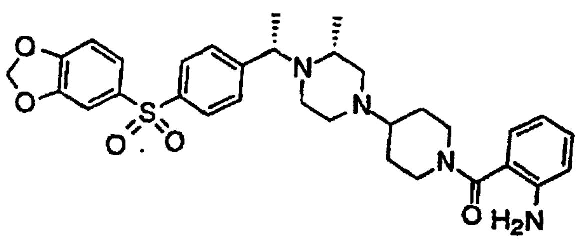

**127**

Intermediate (1) (0.029 g) was dissolved in pyridine (0.1 ml) and isatoic anhydride (0.044 g) was added. The reaction solution was stirred for 48 h at room temperature then diluted with EtOAc (10 ml), washed with water, dried over $MgSO_4$, filtered and evaporated to give a solid. The acetone-soluble portion of this solid was purified by thin layer chromatography (silica gel adsorbent; 95:5 EtOAc:$Et_3N$ eluant) to give a light colored foam (0.029 g) in 65% yield. MP (of hydrochloride):
decomp >205°C

**Example 27**

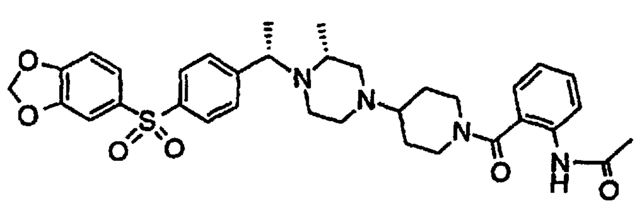

**128**

To a solution of **127** (0.096 g) and $CH_2Cl_2$ (0.32 ml) was added pyridine (0.01ml), acetic anhydride (0.02 ml) and N, N-dimethyl aminopyridine (4.8 mg). The resulting solution was stirred for 1.5 h, water (0.4 ml) was added and the mixture was extracted with $CH_2Cl_2$ (3 X 1 ml). The organic extract was dried over $MgSO_4$, filtered and evaporated

to give a solid residue which was purified by thin layer chromatography (silica gel adsorbent; 9:1 $CH_2Cl_2$:$CH_3OH$ eluant) to give light colored foam (0.036 g) in 37% yield. MP (of hydrochloride): decomp >181°C.

**Example 29**

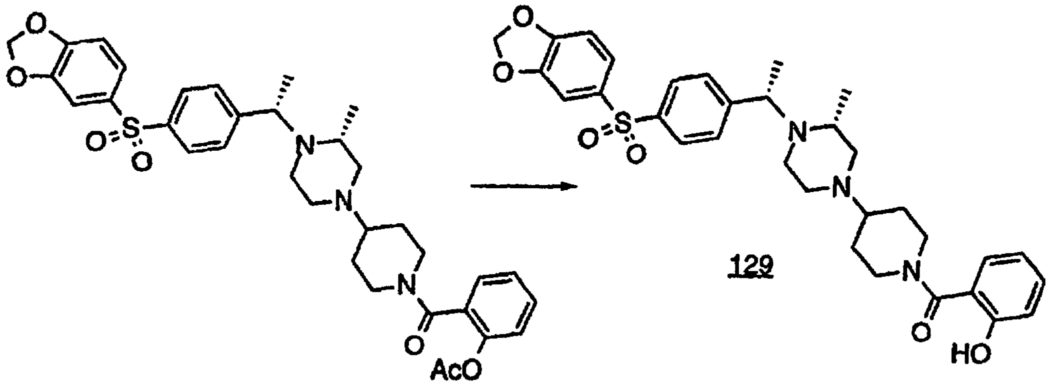

129

To a solution of the acetate shown above (prepared as described in Example 1) (0.071 g), and $CH_3OH$ (0.32 ml) was added $Et_3N$ in three portions, over a 3 h period (0.06 ml total). The resulting solution was stirred 3 days at room temperature, water was added and the solution was extracted with $CH_2Cl_2$. The organic extracts were dried over $MgSO_4$, filtered and evaporated to give the product (0.075 g) in 100% yield. M.p. (of hydrochloride): decomp >130°C.

**Example 29**

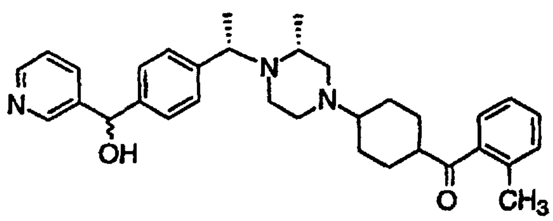

130

Step 1:

(3b)

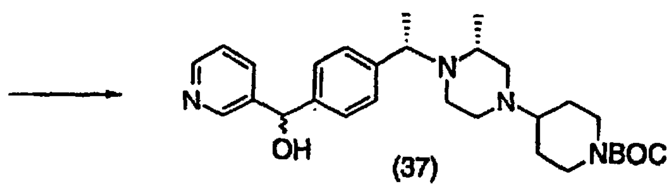

(37)

To a cooled (-78°C) solution of intermediate (3b) (0.41 g; 0.88 mmol) and THF (4.2 ml) was added nBuLi (0.34 ml of a 2.7 M solution in heptane) and the resulting solution was stirred for 3 min. Pyridine-3-uocarboxaldehyde was added as a solution in THF (0.17 ml in 2 ml, respectively) dropwise, over 1.5 min. The solution was stirred for 10 min at low temperature, 40 min at room temperature, poured into water, extracted with EtOAc, washed with brine and dried over $MgSO_4$. After filtration and evaporation, the resulting crude yellow oil was chromatographed on silica gel (77 g), eluting with 76:19:5 EtOAc: hexanes:$Et_3N$. Evaporation of the appropriate fractions gave, as a diastereomeric mixture of carbinols, (37) as a clear oil (0.28 g) in 64% yield. HRMS: $MH^+$: $C_{29}H4_3N_4O_3$: calc'd: 495.3335; found: 495.3319

Step 2:

Treat the product of Step 1 with o-toluoyl chloride using a procedure similar to that described in Example 3 to obtain the desired compound **130.** MP (of hydrochloride): 174-177°C.

**Examples 30A and 30B**

30A:

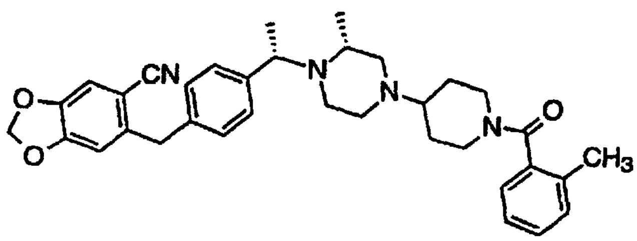

**131**

Steps 1 to 4:

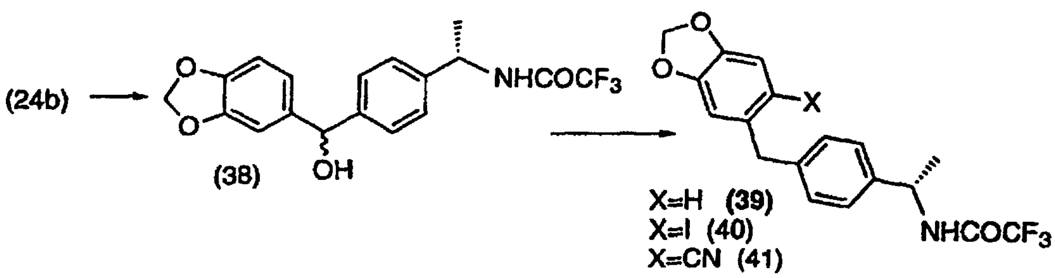

Step 1:

To a cooled (-78°C) solution of (24b) (17.9 g) and THF (170 ml) was added $CH_3Li$ (43.2 ml of a 1.4 M solution in hexane) and the resulting solution was stirred for 10 min. N-BuLi (24.0 ml of a 2.5 M solution in hexane) was added and the resulting solution was stirred for 15 min. Piperonal was added as a solution in THF (9.6 g in 30 ml, respectively) dropwise and the cooling bath was removed. The solution was stirred for 15 min at room temperature, poured into dilute HCl and $Et_2O$ and the $Et_2O$ extracts were dried over $MgSO_4$. After filtration and evaporation, the resulting crude yellow oil was chromatographed on silica gel, eluting with 1:1 hexane:$CH_2Cl_2$ then 0→20 % $Et_2O$:$CH_2Cl_2$. The fractions containing the desired product were combined and evaporated to give as a mixture of carbinols (38) (16.1 g) in 75% yield.

Step 2:

To a solution of (38) (15.8 g), $CH_2Cl_2$ (300 ml) and $Et_3SiH$ (25 ml) was added TFA (25 ml). After 15 min at room temperature, the solution was washed with water, aqueous $NaHCO_3$, dried over $MgSO_4$ and filtered through a pad (20g) of silica, washing through the product with $CH_2Cl_2$. Evaporation and trituration with hexane (200 ml) gave, after filtration and drying, a white solid, (39) (13.9 g) in 92% yield. M.p.: 99-101°C.

Step 3:

To a solution of (39) (2.23 g, 6.37 mmol), $CH_2Cl_2$ (23 ml) and iodine (1.84 g) was added, in one portion, silver trifluoroacetate (1.60 g). The resulting mixture was stirred for 1.5 h at room temperature, stored at 5°C for 14 h, diluted with $CH_2Cl_2$ (126 ml) and filtered. The resulting $CH_2Cl_2$ extract was poured onto a column of silica gel (63 g) and the

product washed through with $CH_2Cl_2$. Any fractions containing a light pink color were washed with 10% $Na_2S_2O_3$ and dried, then combined with the other fractions and evaporated, to give (40) (2.86 g) as a white solid in 94% yield. M.p.: 125-127°C.

Step 4:

(40) (1.50 g, 3.14 mmol) was dissolved in N,N-dimethylacetamide (10 ml) and CuCN (0.94 g) and NaI (0.14 g) were added. The resulting mixture was degassed and heated at 110°C for 8 h. The mixture was cooled, diluted with $CH_2Cl_2$, filtered and the resulting solids were washed with $CH_2Cl_2$. The combined $CH_2Cl_2$ extracts were washed with 1:1 $NH_4OH$:$H_2O$ (3X), water and brine, dried over $MgSO_4$, filtered, evaporated and applied to a silica gel column (55 g). After elution with hexanes (100 ml) and 4:1 hexanes:EtOAc, the desired fractions were evaporated to give a waxy white solid, (41), (1.10 g) in 94% yield.
M.p.: 114-116°C.

Steps 5 and 6:

(41)

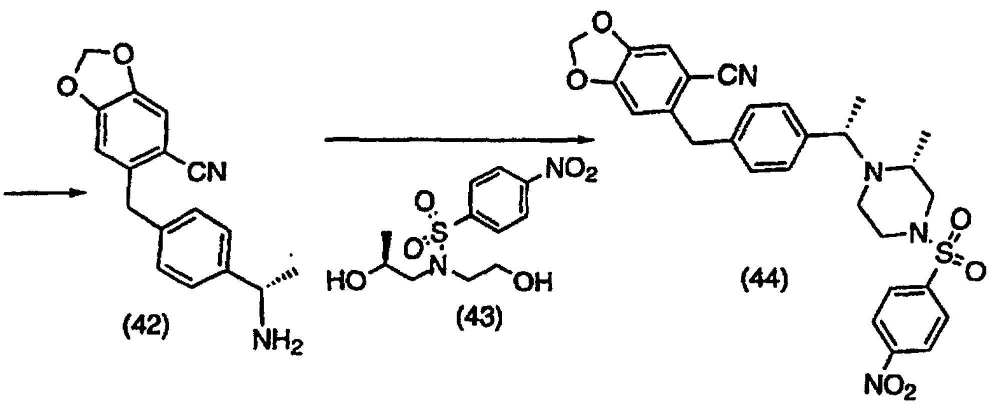

(42) (43) (44)

Step 5:

Treat (41) using a similar procedure to that described in Example 10, Step 3, to give the amine (42) as a white solid in 70% yield.
M.p.: 55-58°C

Step 6:

To a cooled (0°C) solution of diol (43) (0.88g), $CH_2Cl_2$ (29 ml) and Et3N (1.21 ml) was added $CH_3SO_2Cl$ (0.46 ml). The resulting solution was stirred at 0°C for 15 min and at room temperature for 1 h. The solution was poured into a mixture of $CH_2Cl_2$ and ice water, the $CH_2Cl_2$ layer was removed and washed with water, brine and dried over $Na_2SO_4$. After filtration and careful evaporation of the solvent, $CH_3CN$ (2.30 ml) and $iPr_2EtN$ (1.20 ml) and (42) (0.64 g) were added and the mixture was heated at 80°C for 12 h. The resulting solution was diluted with EtOAc, washed with 2 N NaOH, water and brine, dried over $MgSO_4$, filtered and evaporated to give a gold foam which was applied to a silica gel (91 g) column and eluted with 3:1 hexane:EtOAc, then 2:1 hexane:EtOAc. The desired fractions were combined and evaporated to give (44) (1.09 g) as a gold foam in 87% yield. HRMS:$MH^+$: $C_{28}H_{29}O_6N_4S$: calc'd: 549.1808; measured: 549.1803.

Step 7:

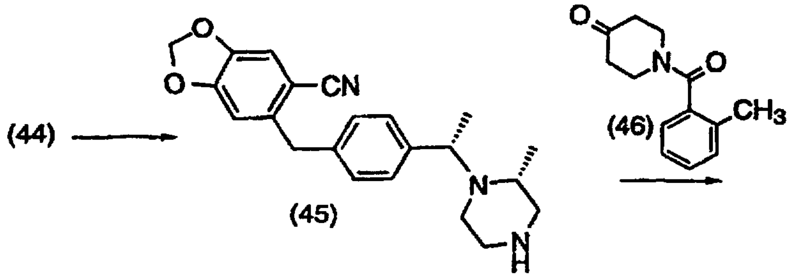

(44) (45) (46) **131**

Part A: To a cooled (0°C) mixture of piperidone hydrate hydrochloride (27.5 g), $CH_2Cl_2$ (350 ml), $K_2CO_3$ (50 g) and water (250 ml) was added dropwise a solution of o-toluoyl chloride (22 ml) and $CH_2Cl_2$ (25 ml). After the addition was complete, the cooling bath was removed and the mixture was stirred for 24 h, the $CH_2Cl_2$ layer was removed, washed with water, dried over $MgSO_4$, filtered and evaporated to give a clear oil. The oil was chromatographed on silica gel (320 g), eluting with 4:1 hexane:EtOAc and the desired fractions were combined and evaporated. The resulting clear oil crystallized on standing to give a white solid, (46), (35.7 g) in 92% yield.

Part B: To a solution of (44) (0.21 g) and DMF (0.75 ml) was added PhSH (0.047 ml) and $K_2CO_3$ (0.15 g) and the resulting mixture was stirred for 28 h. The reaction mixture was diluted with EtOAc and water and 2 N NaOH (1.0 ml) were added. The aqueous layer was extracted with EtOAc (three portions), the EtOAc extracts were combined and washed with brine, dried *over* $MgSO_4$, filtered and evaporated to give 0.55 g of an oil (45) which was taken up in $CH_2Cl_2$ (0.55 ml) and acetic acid (9 µl). To this solution was added (46) (0.04 g) and $NaB(OAc)_3H$ (0.048 g). After stirring for 6 h, the reaction solution was partitioned between $CH_2Cl_2$ and 2 N NaOH. The $CH_2Cl_2$ layer was washed with water and brine, dried over $MgSO_4$, filtered, evaporated and applied to a thin layer silica plate. After elution with 2:1 $CH_2Cl_2$:acetone, the desired product, **131**, was collected as a foam (0.72 g) in 85% yield.

M.p. (hydrochloride): 165-167°C.

**30B:**

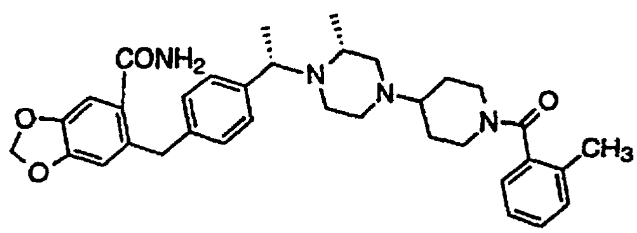

**132**

To a solution of **131** (0.029 g) and THF (0.5 ml) was added $nBu_4NOH$ (0.085 ml) and 30% $H_2O_2$ (0.017 ml). More $nBu_4NOH$ and $H_2O_2$ were added as described above after 3 h and 48 h then the reaction was stirred 3 h more after the last addition. The reaction was diluted with EtOAc, the aqueous layer was removed, extracted with EtOAc (3X) and the EtOAc extracts were combined, washed with water and brine, dried over $MgSO_4$, filtered and evaporated to give a white crystalline solid which was further purified by chromatography (silica gel; 100 mg; 95:5 EtOAc:$Et_3N$ eluant). The desired product was collected and evaporated to give a white solid, **132**, (10.5 mg) in 35% yield.

M.p. (of hydrochloride): decomp >164°C.

**Example 31**

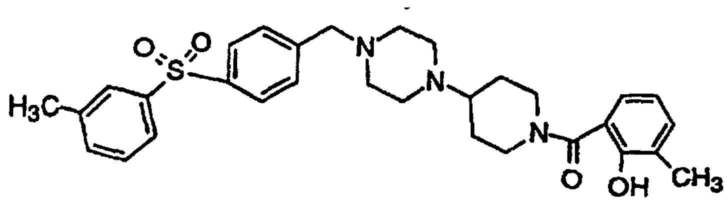

133

Part A:

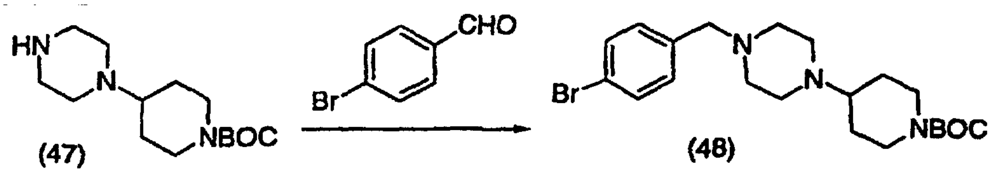

To a solution of piperazine (13 g), N-BOC 4-piperidone (10 g), $CH_2Cl_2$ (200 ml) and acetic acid (2.0 ml) was added $NaB(OAc)_3H$ (20 g) in four portions over 2 h. The mixture was stirred for 22 h, diluted with $CH_2Cl_2$ and added slowly to 1 N NaOH (250 ml). The aqueous layer was extracted with $CH_2Cl_2$ (3X), the $CH_2Cl_2$ extracts were combined, washed with water and dried over $MgSO_4$. The solution was filtered, concentrated and purified by silica gel chromatography (eluant: 8:1 $CH_2Cl_2$ :$CH_3OH$, then 10:5:1 $CH_2Cl_2$ :$CH_3OH$: $NH_3$(aq)). After evaporation of the solvent, (47) was collected as a white solid (7.18 g) in 53 % yield.

To a solution of p-bromo benzaldehyde (5.25 g), $CH_2Cl_2$ (100 ml) and (47) (8.72 g) was added $NaB(OAc)_3H$ (8.05 g) in 8 portions over 40 min. After stirring for 19 h, $CH_2Cl_2$ (20 ml) and $NaB(OAc)_3H$ (1.05 g) were added, the reaction mixture was stirred for 2 h, diluted with $CH_2Cl_2$, poured into 2 N NaOH and the aqueous layer was extracted with $CH_2Cl_2$. The $CH_2Cl_2$ extracts were washed with water and brine, dried over $MgSO_4$, filtered, evaporated and chromatographed on silica gel (250 g), eluting with 76:19:5 hexane:EtOAc:$Et_3N$. The desired fractions were combined and evaporated to give (48) as a white solid (8.48 g) in 69 % yield. M.p.: 87-90°C.

Part B:

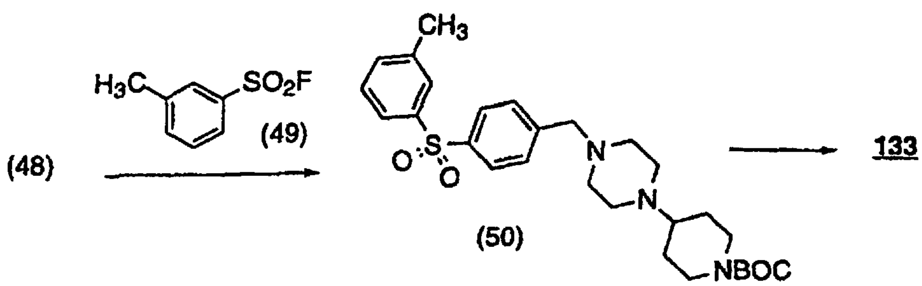

To a solution of m-toluene sulfonyl chloride (13.2 g) and acetone (107 ml) was added a solution of KF (11 g) and water (87 ml), slowly dropwise. The resulting solution was diluted with EtOAc and the organic layer was removed. The aqueous layer was extracted with EtOAc, combined with the initial EtOAc extract, dried over $MgSO_4$, filtered and evaporated to give (49), (11.7 g) in 97% yield, which was used directly without further purification.

(50) was prepared using a procedure similar to Example 29, except (49) was used in place of 3-pyridine carboxaldehyde. Using procedures described in Example 1 (or Example 2), (50) was converted to **133.** M.p. (hydrochloride): decomp >227°C.

**Example 32**

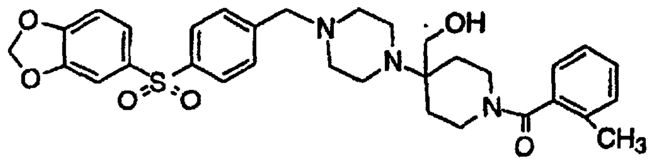

**134**

Part A:

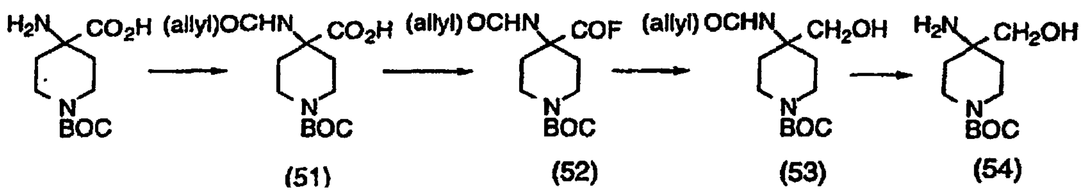

The amino acid (0.21 g), DME (15 ml) and 1 M NaOH (3 ml) were mixed and allyl chloroformate (0.2 ml) was added. The solution was stirred for 15 h, cooled to 0°C, acidified with 1 M HCl and extracted with EtOAc. The EtOAc extracts were washed with brine and dried over $Na_2SO_4$, filtered and concentrated to give (51) as a colorless foam, which was used directly in the next procedure.

Cyanuric fluoride (0.41 g), (51) (0.50 g), pyridine (0.15 ml) and $CH_2Cl_2$ (15 ml) were mixed at 0°C. The mixture was stirred at 0°C for 3.75 h, diluted with $CH_2Cl_2$ and poured into cold water. The aqueous layer was extracted with $CH_2Cl_2$ and the combined $CH_2Cl_2$ extracts were dried over $MgSO_4$, filtered and concentrated to give crude (52) (0.50 g) as a thick oil, which was used directly in the next procedure.

The acid fluoride (52) (0.31 g) and $CH_2Cl_2$ (10 ml) were mixed and $NaBH_4$ (72 mg) was added followed by $CH_3OH$ (0.9 ml), dropwise over 10 min. The reaction was stirred for 20 min at room temperature, poured into a separatory funnel containing 1 M HCl and EtOAc and the aqueous layer extracted with EtOAc. The organic extracts were combined, washed with brine, dried over $MgSO_4$, filtered and concentrated to give (53) as an oil (0.30 g) in 51 % yield.

Palladium acetate (4 mg), 3,3',3''-phosphinidynetris(benzene sulfonic acid) sodium salt (20 mg), Et2NH (1.3 g) and (53) (0.28 g) were mixed with $CH_3CN$ (3 ml) and water (3 ml) and stirred for 3 h. The reaction mixture was co-evaporated with toluene (6 ml) to obtain (54), which was used directly in the next part.

Part B:

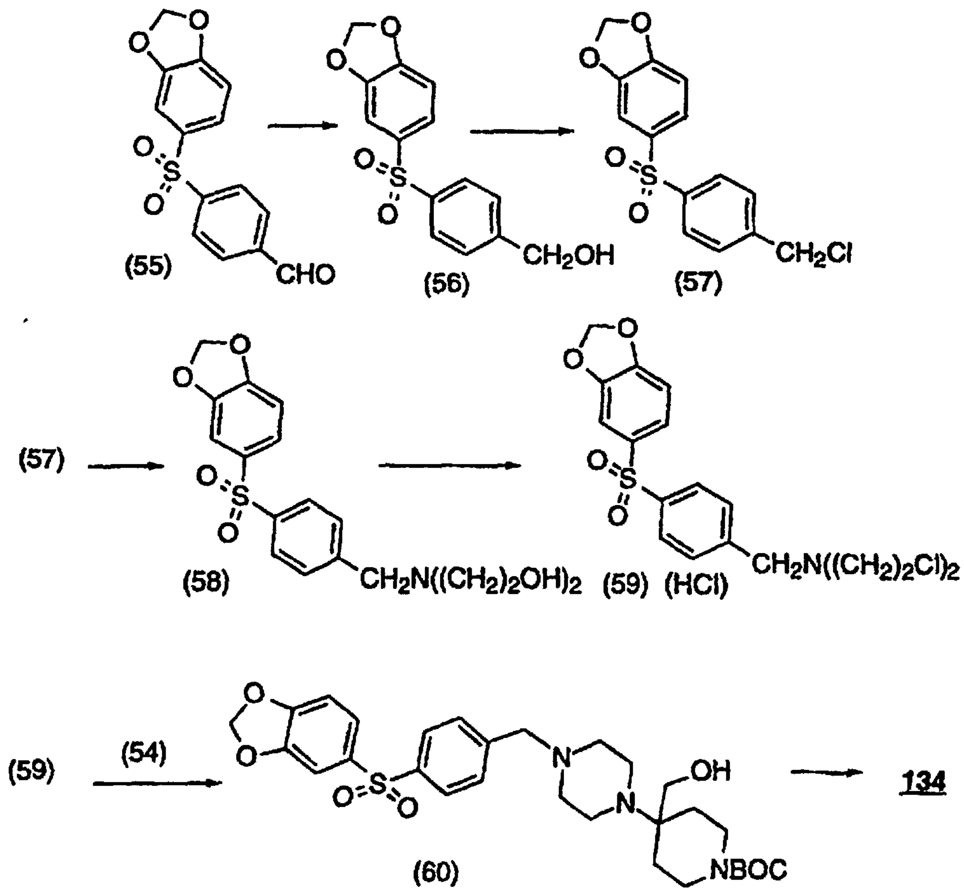

(55) (56) (57)

(57) (58) (59) (HCl)

(59) (60) 134

(55) (0.50 g) (prepared using the procedure of Example 1, using p-fluorobenzaldehyde) was taken up in $CH_3OH$ (20 ml) and $CH_2Cl_2$ (10 ml), cooled to 0°C and $NaBH_4$ (80 mg) was added. After stirring for 2.5 h at room temperature, the solution was partitioned between EtOAc and 1 M HCl. The EtOAc layer was removed, the aqueous layer was extracted with EtOAc, the organic extracts were combined, washed with brine, dried over $MgSO_4$, filtered and evaporated to give (56) (0.52 g) as a colorless oil. A solution of $CH_2Cl_2$, $SOCl_2$ (0.13 ml) and DMF (0.033 ml) was stirred for 4 h at room temperature and concentrated. The residue was co-evaporated with toluene (2x 20 ml) to give a yellow solid (57) (0.20 g) which was dissolved in $CH_3CN$ (10 ml) and diethanolamine (0.24 g). After heating at reflux for 2 h, the reaction solution was partitioned between water and EtOAc, the EtOAc layer was removed, the aqueous layer was extracted with EtOAc, the organic extracts were combined, washed with brine, dried over $Na_2SO_4$, filtered and evaporated to give the product (58) (0.26 g) as a colorless oil. This product was dissolved in 1,2-dichloroethane (5 ml), $SOCl_2$ (0.16 ml) was added and the resulting mixture was heated at reflux for 3 h. The volatile materials were removed in vacuo to give the product (59) as a thick gum, which solidified on storage at 5°C. This material (0.17 g) was mixed with (54) (0.10 g), NaI (0.20 g), $CH_3CN$ (10 ml) and diisopropyl ethyl amine (0.45 ml) and the resulting mixture was heated at reflux for 3.5 h. The reaction solution was partitioned between water and EtOAc, the EtOAc layer was removed, the aqueous layer was extracted with EtOAc, the organic extracts were combined, washed with brine, dried over $Na_2SO_4$, filtered and evaporated to give a brown oil. Purification on silica gel, eluting with 10:1 $CH_2Cl_2$ : CH3OH, gave (60) (77 mg) as an oil. HRMS:$MH^+$:$C_{29}H_4ON_3O_7S$ calc'd: 574.2587; measured: 574.2580.

(60) (77 mg) was dissolved in 1 M HCl in $CH_3OH$ (5 ml), stirred for 24 h, concentrated and the residue was taken up in $CH_3OH$ and triturated with $Et_2O$ to give a white solid (59 mg). Using the procedure of Example 1, (60) was converted to **134.** HRMS (FAB) calc'd for $C_{32}H_{38}N_3O_6S$ ($MH^+$): 592.2481; found: 592.2467; m.p. 187-197°C.

**Example 33**

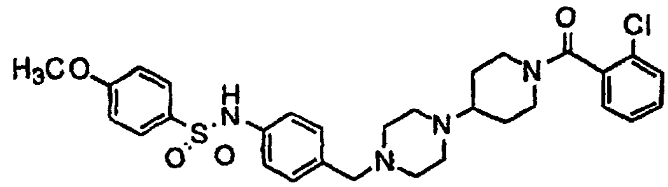

**135**

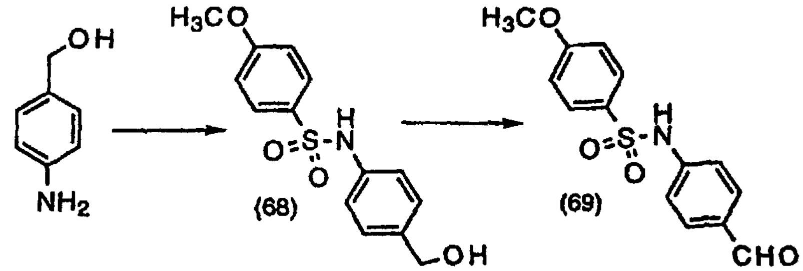

(68) (69)

(69)

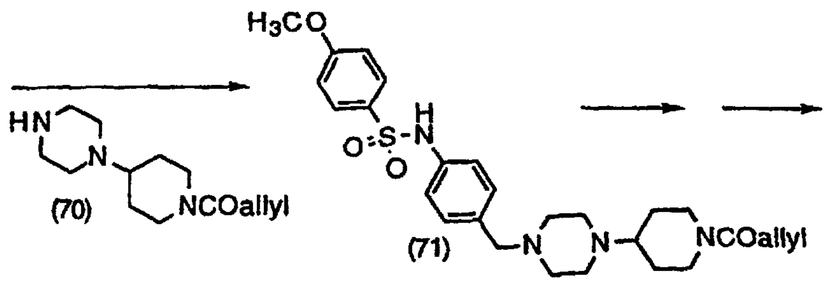

(70) (71)

**135**

Step 1: To a cooled (-78°C) mixture of 4-amino benzyl alcohol (0.50 g), diisopropyl ethyl amine (1.41 ml) and $CH_2Cl_2$ (40 ml) was added TMSCI (0.52 ml). The resulting slurry was stirred at -78°C for 15 min and at 0°C for 1 h. The resulting solution was cooled to -78°C, p-methoxyphenyl sulfonyl chloride (0.84 g) was added, the cooling bath was removed and the reaction was stirred at room temperature for 15 h. The reaction solution was diluted with $CH_2Cl_2$ and water was added. The aqueous layer was extracted with $CH_2Cl_2$, the combined organic extracts were dried over $MgSO_4$, filtered and evaporated to give a yellow oil, which was taken up in $CH_3OH$ (20 ml) and $K_2CO_3$ (1.5 g) was added. The resulting mixture was stirred for 1 h., filtered through a plug of Celite, concentrated, taken up in EtOAc and washed with water. The aqueous layer was extracted with EtOAc, the combined organic extracts were dried over $MgSO_4$, filtered and evaporated to give a yellow oil (0.78 g), which was used without further purification in the next step.

Step 2: (68) (0.47 g) and $MnO_2$ (3.0 g) were mixed with $CH_2Cl_2$ (20 ml) and stirred for 20 h. The solution was filtered through Celite and concentrated to give 0.30 g of a thick oil, (69), which was used directly in the next step.

Step 3: Use a procedure similar to the second portion of Part A in Example 31, with intermediates (69) and (70) as the reactants. Crude (71) (0.30 g) was used directly in the next step.

Step 4: Treat (71) (148 mg) using a procedure similar to that in the last portion of Part A of Example 32 to obtain the corresponding brown crude amine, which was used directly in the next step.

Step 4: Using the procedure of in Example 1, treat (71) to obtain **135.** M.p. (with decompostion) 190-200°C.

**Example 34**

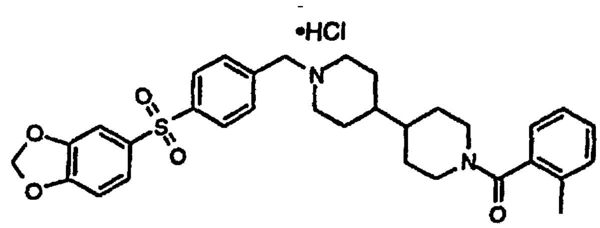

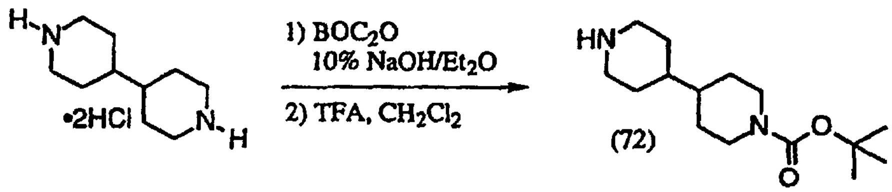


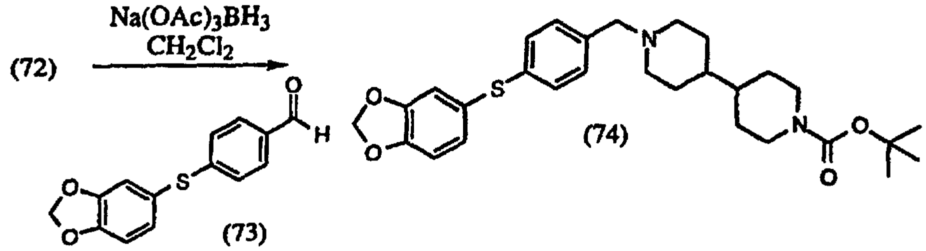


1) MCPBA, $MeSO_3H$
2) TFA, $CH_2Cl_2$
3) *o*-MePhCOCl, $Et_3N$
4) HCl, $Et_2O$

(74) **136**

Step 1: To a stirred 0°C solution of 4,4'-bispiperidine hydrochloride (2.53 g. 10.5 mmol) in a 1:1 solution of $Et_2O$/ 10% aq. NaOH (26 ml) was added a 2.5 M solution of di-*tert*-butyldicarbonate in $Et_2O$ (10 ml, 23.1 mmol) over 30 min with vigorous stirring. After stirring at room temperature for 2 h, the mixture was poured into a separatory funnel, extracted with $Et_2O$, washed with brine, dried over $Na_2SO_4$, filtered and concentrated in vacuo. The resulting product (3.00 g) was used without further purification.

The crude bispiperidine (2.74 g, 7.44 mmol) was dissolved in $CH_2Cl_2$ (50 ml) and TFA (0.57 ml, 7.44 mmol) was added to the solution. The reaction stirred at room temperature and monitored by TLC. After two more equivalents of TFA were added, the reaction was approximately a 1:2:1 mixture of starting material, (72), and 4,4'-bispiperidine. The mixture was diluted with $CH_2Cl_2$, washed with 1N NaOH, dried over $Na_2SO_4$, filtered, and concentrated. Purification by flash chromatography (10/90 $CH_3OH/CH_2Cl_2$, then 5/10/85 $NH_4OH/CH_3OH/CH_2Cl_2$) yielded 1.00 g of (72) (53% yield).

Step 2: To a solution of (73) (480 mg, 1.85 mmol) in $CH_2Cl_2$ (7.4 ml) was added (72) (520 mg, 2.04 mmol) and sodium triacetoxyborohydride (590 mg, 2.78 mmol). The reaction was stirred for 12 h at room temperature under $N_2$. The mixture was quenched with $NaHCO_3$ (sat), extracted with EtOAc, dried over $Na_2SO_4$, filtered, and concentrated under reduced pressure, yielding (74) (880 mg, 94% yield).

Step 3: To a solution of (74) (880 mg, 1.72 mmol) in $CH_2Cl_2$ (8.6 ml) was added $CH_3SO_3H$ (0.17 ml, 2.58 mmol) in $CH_2Cl_2$ (4.7 ml). After cooling to 0°C with stirring, 60% MCPBA (1.04 g, 3.61 mmol) in $CH_2Cl_2$ was added dropwise. The reaction was stirred for 1 h at room temperature under $N_2$. The mixture was washed with $Na_2S_2O_3$ (sat), 1 N NaOH, and $H_2O$, dried over $Na2SO_4$, filtered, and concentrated to yield 0.93 g of the sulfone (100% yield).

The sulfone (0.93 g, 1.71 mmol) was dissolved in $CH_2Cl_2$ (11.4 ml) and TFA (2.6 ml, 34.2 mmol) was added. The reaction was stirred for 2 h at room temperature under $N_2$. The mixture was diluted with $CH_2Cl_2$, washed with 1N NaOH, filtered, and concentrated under reduced pressure, yielding the free amine (0.45 g, 59% yield).

To a solution of the amine (23.6 mg, 53.3 μmol) in $CH_2Cl_2$ (0.53 ml) was added *o*-toluoyl chloride (8.3 μL, 64

μmol) and $Et_3N$ (11 μL, 80 μmol). The reaction was stirred for 2 h at room temperature under $N_2$. The mixture was concentrated under reduced pressure and purified by PTLC (10/90 $CH_3OH/CH_2Cl_2$), concentrated in the presence of 1N $HCl/Et_2O$, yielding **136** (15.8 mg, 50% over three steps). LRMS calc'd: 560; found (M+H): 561.

**Example 35**

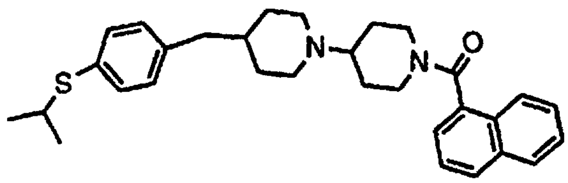

**137**

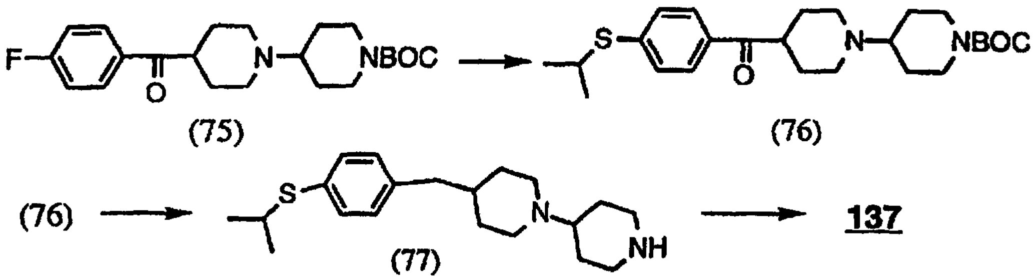

(75) (76)

(76) (77) **137**

NaH (0.59 g, 14.6 mmol, 60% purity) was suspended in DMF (10 ml) followed by addition of 2-propanethiol (1 ml, 11.2 mmol) at 0°C under $N_2$. The reaction mixture was stirred at room temperature for 5 min. (75) (2.94 g, 7.5 mmol) in DMF (10 ml) was added dropwise. The reaction mixture was heated with stirring for 2 hrs. The reaction mixture was then diluted with 1N NaOH (40 ml) and extracted with $Et_2O$ (3x 80 ml). The organic extract was dried with $NaHCO_3$. Removal of solvent and recrystallization with $Et_2O$ afforded (76) (1.08 g, 32%).

(76) (1.08 g, 2.4 mmol) was dissolved in $CH_3OH$ (10 ml) followed by addition of $NaBH_4$ (0.14 g, 3.6 mmol). The reaction mixture was stirred at room temperature for 1 hr. The reaction mixture was diluted with $CH_2Cl_2$ (200 ml) followed by washing with 1N NaOH (50 ml). The organic solution was dried with $NaHCO_3$ and concentrated to give solid residue which was then dissolved in $CH_2Cl_2$ (10 ml). To this solution was added $Et_3SiH$ (1.5 ml) followed by TFA (3 ml). The reaction mixture was stirred at room temperature for 1hr. The reaction mixture was diluted with $CH_2Cl_2$ (100 ml), washed with 1N NaOH (100 ml), and dried with $NaHCO_3$. Removal of solvent afforded pure (77) (0.7 g, 88%).

(77) (49 mg, 0.15 mmol) was dissolved in a solution of $CH_2Cl_2$ (3 ml) and $Et_3N$ (0.3 ml) followed by addition of 1-naphthoyl chloride (0.5 ml). The reaction mixture was stirred for 30 min and separated by PTLC to afford **137** (53 mg, 74%) as free base. HRMS: calc'd: (M+1): 487.2783; found 487.2798.

**Example 36**

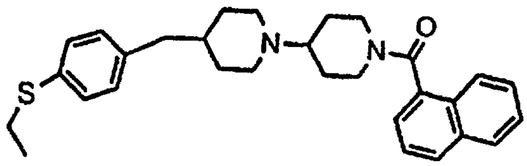

**138**

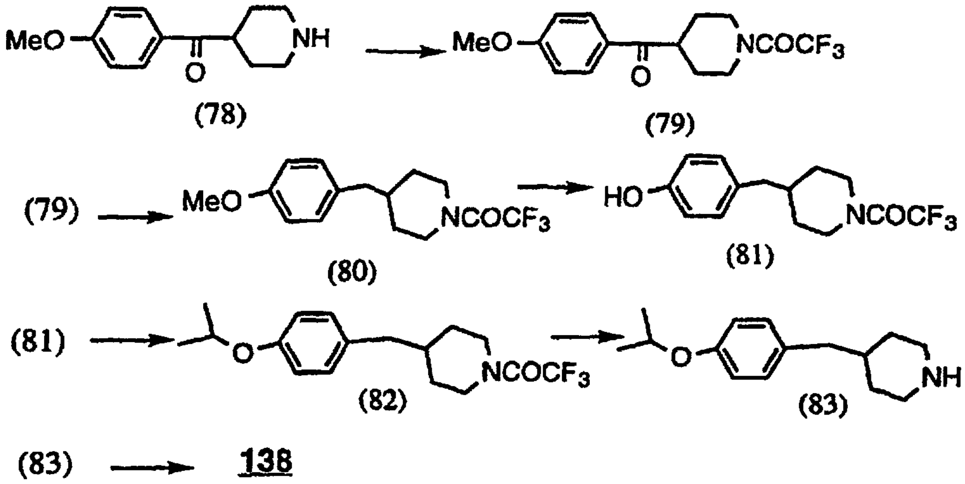

(78) (79)

(79) (80) (81)

(81) (82) (83)

(83) 138

(78) (1 g, 3.6 mmol) was dissolved in a solution of $CH_2Cl_2$ (20 ml) and $Et_3N$ (1.5 ml) followed by addition of $(CF_3CO)_2O$ (0.82 ml, 5.7 mmol). The reaction mixture was stirred at room temperature overnight. The reaction mixture was diluted with $CH_2Cl_2$ (150 ml), washed with 1N HCl (50 ml) followed by 1N NaOH (50 ml), and dried with $MgSO_4$. Removal of solvent afforded crude (79).

(79) was dissolved in $CH_3OH$ (25 ml) followed by addition of $NaBH_4$ (0.2 g, 5.3 mmol). The reaction mixture was stirred at room temperature for 1 hr. The reaction mixture was diluted with $CH_2Cl_2$ (100 ml) followed by washing with 1N NaOH (50 ml). The organic solution was dried with $MgSO_4$ and concentrated to give a residue which was then dissolved in $CH_2Cl_2$ (30 ml). To this solution was added $Et_3SiH$ (3 ml) followed by TFA (10 ml). The reaction mixture was stirred at room temperature for 4 hrs. The reaction mixture was diluted with $CH_2Cl_2$ (200 ml), washed with 1N NaOH (100 ml), and dried with $MgSO_4$. Removed solvent to afford crude (80).

(80) was dissolved in $CH_2Cl_2$ (5 ml) followed by addition of 1M $BBr_3/CH_2Cl_2$ (8.6 ml) at 0°C. The reaction mixture was stirred at 0°C for 1 hr, diluted with $CH_2Cl_2$ (100 ml), washed with 10% $NaHCO_3$, and dried with $MgSO_4$. Removed solvent followed by column chromatography (20% EtOAc/hexane) to afford (81) (0.725 g, 58% from (78)).

(81) (0.35 g, 1.2 mmol), 2-propanol (0.084 g, 1.4 mmol) and $PPh_3$ (0.38 g, 1.4 mmol) were dissolved in THF (5 ml) under $N_2$. The reaction mixture was cooled to 0°C followed by addition of DEAD (0.27 ml, 1.mmol). The reaction mixture was stirred at room temperature overnight and separated by PTLC without workup to afford (82) (0.3 g, 75 %).

(82) (0.3 g, 0.9 mmol) was dissolved in a solution of $CH_3OH$/water (5:1) (10 ml) followed by addition of $K_2CO_3$ (0.5 g, 3.6 mmol). The reaction mixture was stirred at room temperature for 1h. Standard aqueous workup afforded (83) (0.203 g, 96%).

(83) was converted to **138** following the procedures described in Example 35. HRMS: calc'd: (M+1): 471.3012; found 471.3009.

**Example 37**

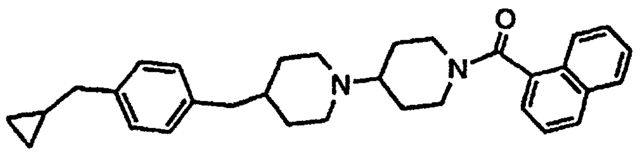

139

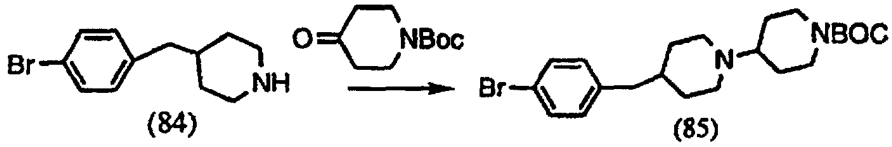

(84) (85)

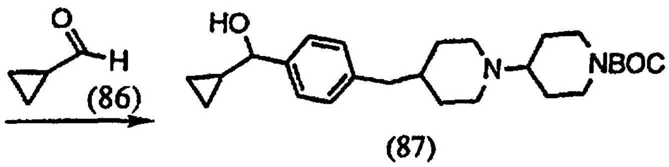

(85) (86) (87)

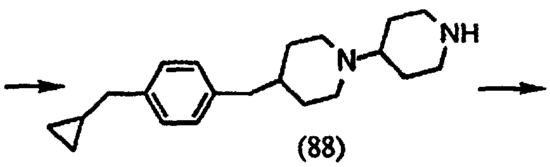

(87) 139 (88)

(84) (0.85 g, 3.4 mmol) and N-BOC-4-piperidone (1g, 5.0 mmol) were dissolved to 1,2-dichloroethane (50 ml) followed by addition of $Na(OAc)_3BH$ (2.2 g, 10.1 mmol). The reaction mixture was stirred at room temperature for two days. The reaction mixture was diluted with $CH_2Cl_2$ (200 ml), washed with 1N NaOH (100 ml) and dried with $NaHCO_3$. Column chromatography (5% $CH_3OH/CH_2Cl_2$) afforded (85) (1.27 g, 87%).

(85) (0.2 g, 0.46 mmol) was dissolved in THF (8 ml) under $N_2$ and cooled down to -78°C followed by addition of 2.5 M n-BuLi (0.3 ml, 0.73 mmol). The reaction mixture was stirred at -78°C for 45 min followed by addition of a solution of (86) (0.06 g, 0.83 mmol) in THF (1 ml). The reaction mixture was stirred at -78°C for 30 min, diluted with $CH_2Cl_2$ (100ml), washed with water (50 ml), and dried with $NaHCO_3$. The organic solution was concentrated and column chromatography (5% $CH_3OH/CH_2Cl_2$) afforded (87) (0.135 g, 69%).

To a mixture of (87) (0.135 g, 0.32 mmol) and $Et_3SiH$ (1ml) was added 20% $TFA/CH_2Cl_2$ (5 ml). The reaction mixture was stirred at room temperature for 1h and concentrated to give (88) (0.09 g, 91%).

(88) was treated in a manner similar to that decribed in Example 35 to obtain **139.** HRMS: calc'd: (M+1): 467.3062; found 467.3066.

**Examples 38A and 38B**

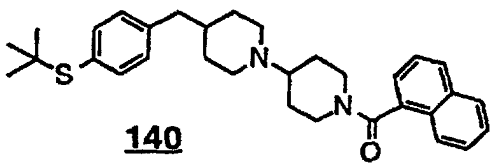

140

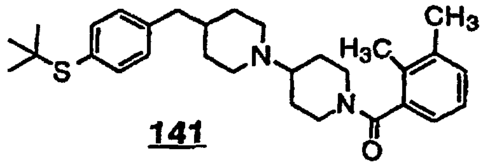

141

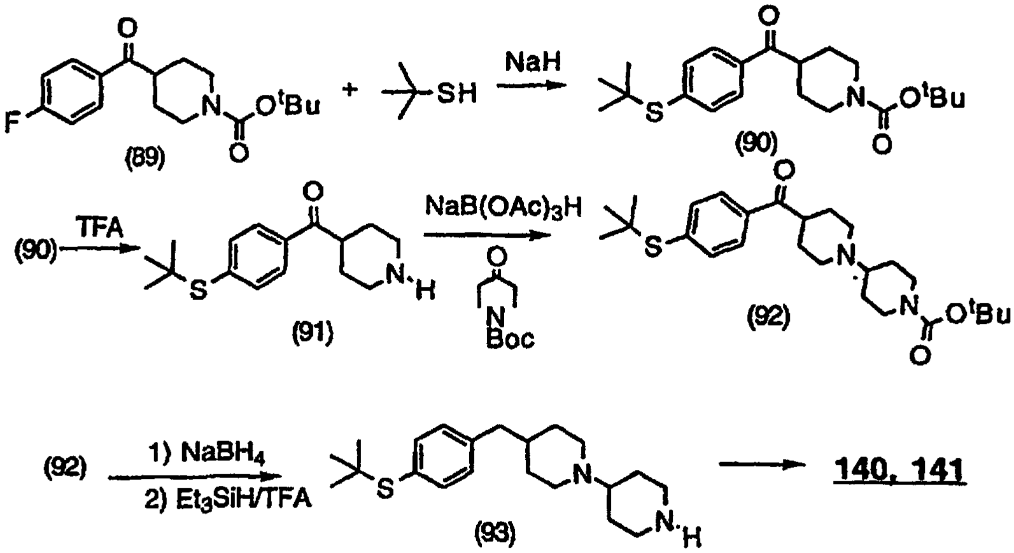


(89) (90) (91) (92) (93) 140, 141

To a suspension of 500 mg of NaH (60% in mineral oil) in 60 ml of DMF was added slowly 1.0 g of tert-butyl thiol. The mixture was stirred at room temperature for 30 min. To this mixture, 3.1 g (10 mmol) of ketone (89) was added dropwise. The mixture was heated at 70°C overnight. After cooling down to room temperature, the mixture was quenched with 100 ml of water. The aqueous phase was extracted with 3X100 ml of $CH_2Cl_2$. The combined organic phases were dried over $MgSO_4$, filtered and concentrated. The crude (90) was purified by column chromatography on silica gel using 5% EtOAc-Hexanes (2.65 g, 69%).

To a solution of 2.65 g of (90) in 40 ml of $CH_2Cl_2$ was added 10 ml of TFA. The mixture was stirred at room temperature for 2 hours and then was concentrated. The residue was taken up in 100 ml of $CH_2Cl_2$, washed with 10% NaOH. The organic layer was dried over $Na_2SO_4$, filtered and concentrated to give a crude (91) (1.8 g, 92%).

A mixture of (91) (1.8 g), N-Boc-4-piperidone (1.5 g, 1.1 eq.), $NaB(OAc)_3H$ (2.8 g, 2 eq.) and 0.5 ml HOAc in 40 ml of 1,2-dichloroethane was stirred at room temperature over night. The mixture was quenched with 10% NaOH, extracted with $CH_2Cl_2$. The organic layer was dried over $Na_2SO_4$, filtered and concentrated. The crude (92) was purified by column chromotography on silica gel using 20% EtOH/EtOAc (2.7 g, 90.3%).

To a solution of (92) (2.5 g) in 100 ml of $CH_3OH$ was added 500 mg (2 eq.) of $NaBH_4$. The mixture was stirred at room temperature for 1 hour and then was concentrated to dryness. The residue was taken up in 70 ml of 10% NaOH and extracted with 3X70 ml of $CH_2Cl_2$. The combined organic phases were dried over $MgSO_4$, filtered and concentrated (1.8 g crude). This crude was dissolved in 40 ml of $CH_2Cl_2$ and 1 ml of $Et_3SiH$. To this mixture was added 20 ml of TFA and stirred at room temperature for 1 hour. The mixture was concentrated to dryness and the residue was washed with cold hexanes. The residue was taken up in 100 ml of $CH_2Cl_2$ and washed with 10% NaOH. The combined organic phases were dried over $MgSO_4$, filtered and concentrated to give a crude (93) (810 mg, 43%).

(93) and 1.3 ml (4 eq.) of $Et_3N$ were dissolved in 40 ml of 1,2-dichloroethane and this solution was used for parallel synthesis to react with different acid chlorides. Typical yield of the coupling reaction is 80%. The Hcl salts were prepared by adding $HCl \cdot OEt_2$ to the free base. **140:** LRMS calc'd: 500; found 501; **141:** LRMS calc'd: 478; found 479.

**Example 39**

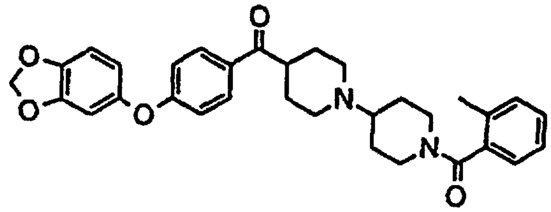

142

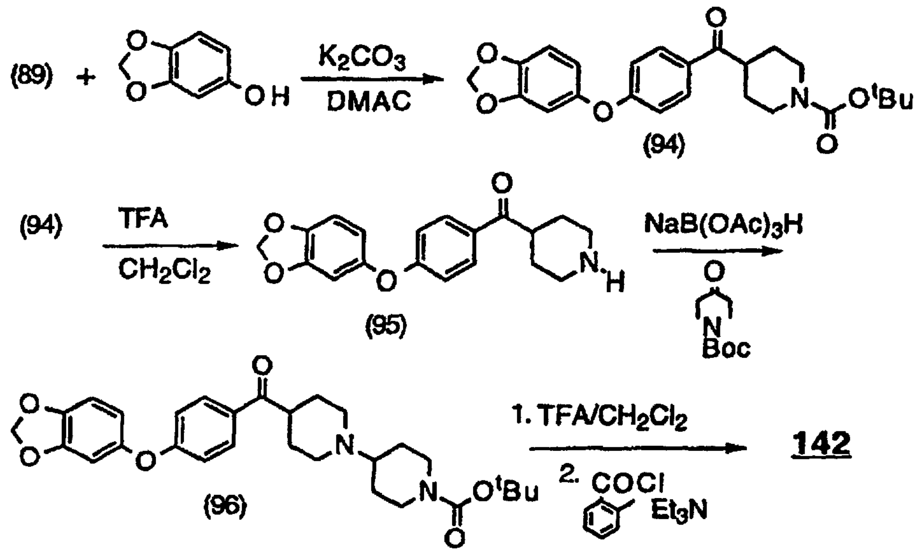


A mixture of (89) (7.9 g, 25.6 mmol), sesamol (1.1 g, 1 eq.) and $K_2CO_3$ (1.5 g) in 40 ml of dimethylacetamide was heated at 150°C over night. The mixture was allowed to cool to room temperature and then was quenched with 200 ml of water. The aqueous phase was extracted with 3X150 ml of $CH_2Cl_2$. The combined organic phases were dried over $MgSO_4$, filtered and concentrated. The crude (94) was purified by column chromatography on silica gel using 20% EtOAc-Hexanes (2.8 g, 26%).

To a solution of (94) (2.8 g) dissolved in 50 ml of $CH_2Cl_2$ was added 5 ml of TFA. The mixture was stirred at room temperature for 1 h and was then concentrated to dryness. The residue was taken up in 150 ml of $CH_2Cl_2$, washed with 100 ml of 10% NaOH. The aqueous layer was extracted with 3X100 ml of $CH_2Cl_2$. The combined organic phases were dried over $MgSO_4$, filtered and concentrated to give crude (95) as a solid (1.7 g, 79%).

The crude (95) (1.7 g, 5.2 mmol) was treated with N-BOC-4-piperidone, NaB(OAc)3H and acetic acid (1.3 g, 4 eq.) in a manner similar to that described in Example 38 to obtain crude (96) (2.3 g, 87%).

The BOC-group was removed to give a crude (97) (1.7 g, 93%), which was reacted with 2-toluoyl chloride as described in Example 14, Step 8, followed by purification by TLC on silica gel using 20% EtOH-EtOAc to obtain **142** (197 mg, 90%). The hydrochloride salt was prepared by adding HCl•Et2O to the free base. LRMS calc'd: 526; found: 527.

**Examples 40A and 40B**

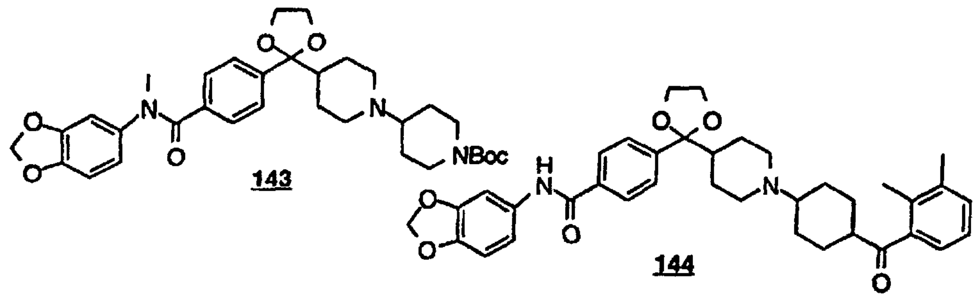
143
144

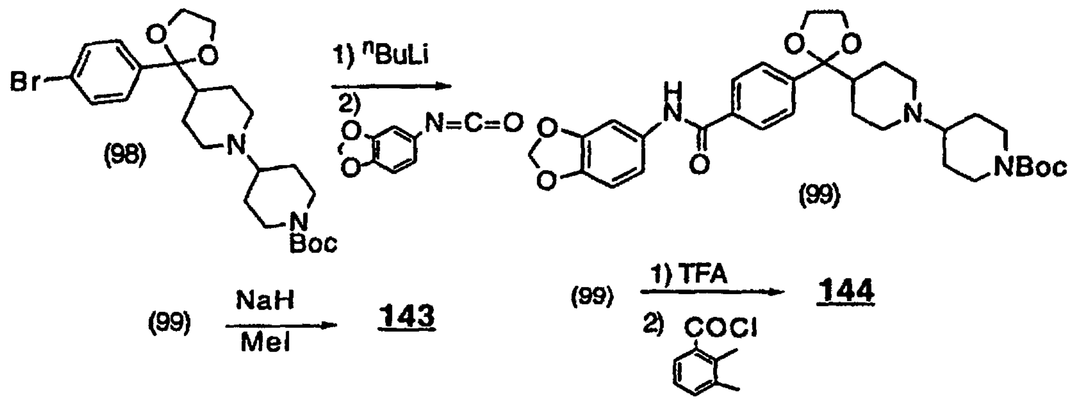


To a stirred solution of 500 mg of (98) in 10 ml of THF was added 0.6 ml of BuLi (2 M in hexane) at -78°C. The mixture was stirred at this temperature for 15 mins. and then a solution of 179 mg of the isocyanate in 15 ml of THF was added. The mixture was stirred at -78°C for 1 hour. The mixture was quenched with water, extracted with EtOAc. The organic phase was dried ($Na_2SO_4$), filtered and concentrated. The crude (99) was purified by prep.TLC on silica gel using 25%EtOH-EtOAc (388 mg).

To a stirred solution of 328 mg of (99) in 50 ml of THF was added 41 mg (60% in mineral oil) NaH, followed by 85 mg of $CH_3I$. The mixture was stirred at room temperature for 1 hour and then was purified by prep. TLC on silica gel using 25%EtOH-EtOAc to obtain 141 mg of **143.** LRMS calc'd: 593; found: 594.

To 92 mg of (99) in 20 ml of $CH_2Cl_2$ was added 5 ml of TFA. The solution was stirred at RT for 1 hour, quenched with 10% NaOH, extracted with $CH_2Cl_2$. The organic layer was dried ($K_2CO_3$), filtered and concentrated (66 mg crude). This crude was dissolved in 20 ml of $CH_2Cl_2$, added 0.25 ml of Et3N followed by 0.25 ml (1M in $CH_2Cl_2$) of 2,3-dimethylbenzoyl chloride. The mixture was stirred at RT for 1 hour and was purified by prep. TLC using 25%EtOH-EtOAc to obtain 81 mg **144.** LRMS calc'd: 611; found: 612.

**Example 41**

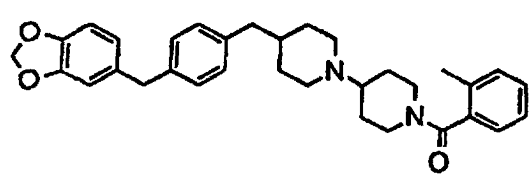

**145**

To a stirred solution of **83** (255 mg) in 50 ml of $CH_3OH$ was added 186 mg of $NaBH_4$, the mixture was stirred at room temperature for 30 min. and was then quenched water. The mixture was extracted with EtOAc and the organic layer was dried over $Na_2SO_4$, filtered and concentrated to give a yellowish oil (286 mg). The crude was dissolved in 10 ml of $CH_2Cl_2$ and 2ml of $Et_3SiH$. To this mixture, 2ml of TFA was added and the mixture was stirred at room temperature for 5 hours. The mixture was neutralized with 3N NaOH, the organic layer was dried over $Na_2SO_4$, filtered

and concentrated. The crude was purified by prep. TLC on silica gel using 25%EtOH-EtOAc (20 mg, 10%). LRMS: calc'd: 510; found: 511.

**Example 42**

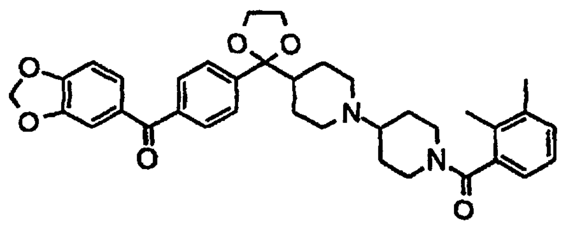

**146**

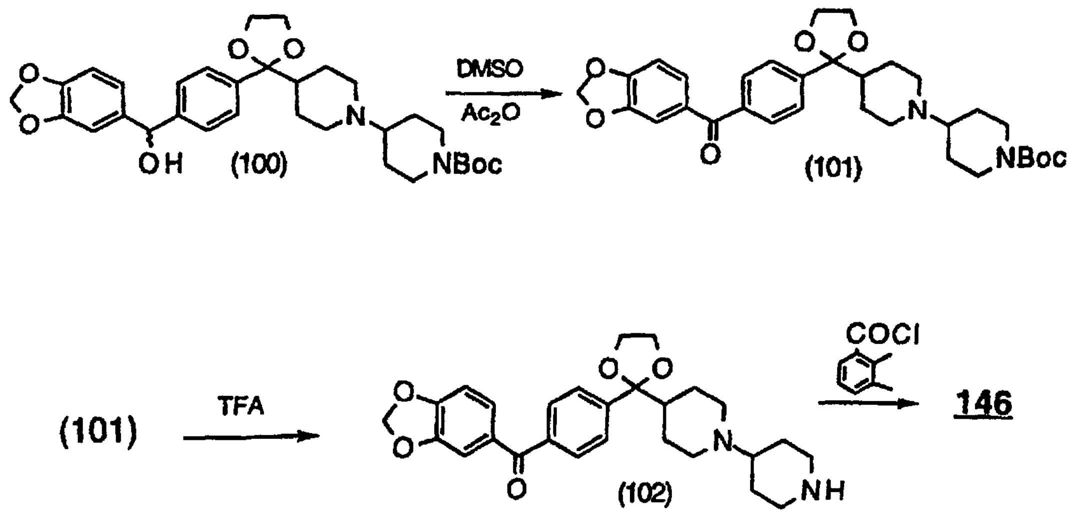


To a stirred solution of (100) in 10 ml of DMSO was added 10 ml of $Ac_2O$, the mixture was stirred at room temperature for 2 h, quenched with water and extracted with $CH_2Cl_2$. The organic phase was dried ($Na_2SO_4$), filtered and concentrated. The crude (101) was purified by prep. TLC on silica gel using 25%EtOH-EtOAc (110 mg). (101) was dissolved in 40 ml of $CH_2Cl_2$, and 5 ml of TFA was added. The mixture was stirred at room temperature for 30 min., concentrated to dryness. The residue was taken up in 100 ml of $CH_2Cl_2$ and was washed with 10% NaOH. The organic phase was dried (Na2SO$_4$), filtered and concentrated to give a crude (102) (108 mg).

To a stirred solution of 28 mg of (102) and 0.1 ml of $Et_3N$ in 3 ml of $CH_2Cl_2$ was added 0.25 ml of 2,3-dimethylbenzoyl chloride (1.0 M solution in $CH_2Cl_2$). The mixture was stirred at room temperature for 1.5 h. The crude was purified by prep. TLC on silica gel using 25%EtOH-EtOAc to obtain **146** (25 mg). LRMS calc'd: 596; found: 597.

**Example 43**

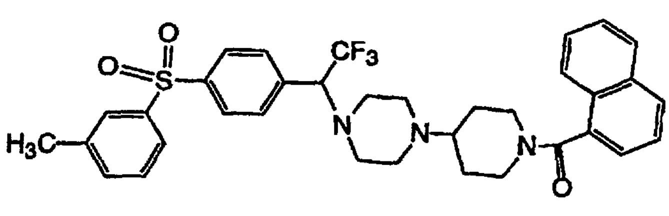

**147**

Step1:

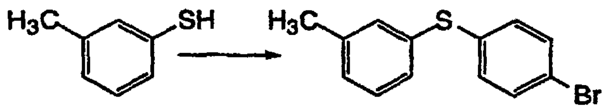

(103)

m-Thiocresol (5.0 g), 1-bromo-4-iodobenzene (10.5 g), CuI (7.7 g) and $K_2CO_3$ (20.0 g) in anhyd. DMF (100 ml) was heated at 130°C under $N_2$ overnight. The mixture was diluted with EtOAc and 10% $NH_4OH/NH_4Cl$ and stirred, open to air, for 2 hours. The layers were separated, the organic phase was washed with the buffer solution 2x, followed by a water wash (3x). The organics were dried over sodium sulfate, filtered and concentrated to a dark brown oil. The crude was flash chromatographed (silica gel) eluted with hexane to give a pale yellow oil which solidified on standing under vacuum (10.9g of (103)).

Step 2:

(103)

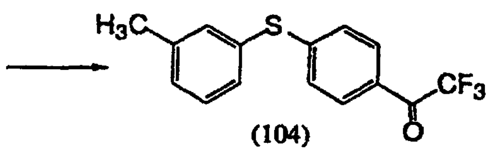

(104)

To a cold (-78°C) solution of (103) (2.0g) in anhyd. THF (50 ml) was slowly added n-BuLi (3.2 ml, 7.92 mmol) and the mixture was stirred at -78°C for 15 min. Ethyltriflouroacetate (1.53 g, neat) was added in one portion and this mixture was stirred at -78°C to room temp over 3 h. The mixture was poured into water, THF was removed in vacuo and the aqueous residue was extracted with EtOAc. The organics were washed with brine, dried over anhyd. $Na_2SO_4$ and concentrated to 1.97 g of a yellow oil, **2** (104)

Step 3:

(104)

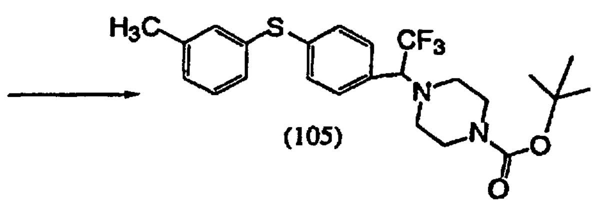

(105)

To a solution of crude (104) (1.9g) and t-butyl -1-piperazine-carboxylate (3.6 g) in dry dichloroethane (20 ml) was added titanium IV chloride (6.4 ml, 6.4 mmol) in portions. This was stirred at room temp. for 2 days. The reaction was cooled to 0°C and $NaBH_3CN$ (1.2 g) in $CH_3OH$ (20 ml) was added. The mixture was stirred overnight at room temp, then filtered through a pad of celite. The layers were separated and the organic was washed with water and brine, then dried over $Na_2SO_4$, filtered and concentrated to a pale red viscous oil. The crude was purified by column chromatography eluted with hexane (neat) to 10% EtOAc/hexane to give 1.7g of viscous pale yellow oil, (105).

Step 4:

(105) 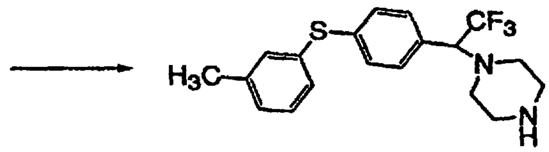 (106)

To a solution of (105) (1.72 g) in $CH_2Cl_2$ (25 ml) was added TFA (20 ml). he reaction was stirred for 1 hour, cooled to 0°C and neutralized with $NaHCO_3$. The aqueous phase was saturated with NaCl crystals and extracted with $CH_2Cl_2$. The organics were dried over $Na_2SO_4$, filtered and concentrated to 1.32g of viscous pale yellow oil, (106).

Step 5:

(106) 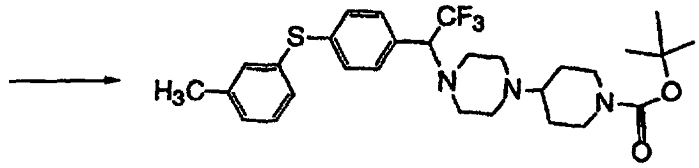 (107)

A solution of (106) (1.32g) and 1-t-butyloxycarbonyl-4-piperidone (858 mg) in dichloroethane (15 ml) was treated with $NaBH(OAc)_3$ (1.1 g) followed by HOAc (0.42 ml, 7.4 mmol). The mixture was stirred at room temp under $N_2$ for 4 days. The reaction was diluted with EtOAc, washed with 1N NaOH (2x), water, and brine. The organics were dried over $Na_2SO_4$, filtered and concentrated. The crude was purified by column chromatography eluted with 25% EtOAc/hexane to give 1.1g of viscous clear oil (107).

Step 6:

(107) 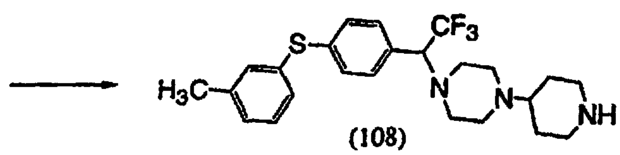 (108)

A solution of (107) (1.0 g) in anhyd. $CH_2Cl_2$ (10 ml) was treated with TFA (10 ml) and stirred at room temp, under $N_2$ for 1 hour. The mixture was then cooled to 0°C, neutralized with saturated $NaHCO_3$ and extracted with $CH_2Cl_2$. The organics were dried over $Na_2SO_4$, filtered and concentrated to 949 mg of pale yellow foam, (108).

Step 7:

(108) 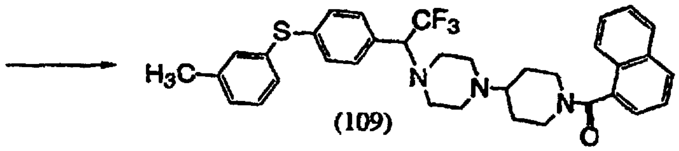 (109)

To a solution of (108) (100 g) in anhyd. $CH_2Cl_2$ (1.0 ml) containing $Et_3N$ (38 µl, 0.27 mmol) was added 1-naphthoyl chloride (41 µl,0.27 mmol). This solution was stirred overnight at room temp. The crude was purified by directly applying to a Preparative TLC plate (1x2000 micron) eluted with 5% $Et_3N$/EtOAc to give 86.4 mg of (109).

Step 8:

To a solution of (109) (63.3mg) in anhyd. $CH_2Cl_2$ (4.0 ml) was added 0.5M $CH_3SO_2H$ solution in $CH_2Cl_2$ (1.2 ml. 0.6 mmol). After stirring for 0.5 h, 30% $H_2O_2$ (31µl, 0.6 mmol) was added and stirred overnight at room temp. The mixture was diluted with $CH_2Cl_2$, neutralized with saturated $NaHCO_3$. The layers were separated, dried over $Na_2SO_4$ and applied to a Preparative TLC plate (2000 micron) eluted with 5% $Et_3N$/EtOAc. Isolated 19.4 mg of **147.** HRMS calc'd: 636.2508; found: 636.2509.

**Example 44**

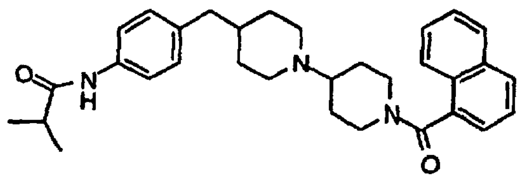

148

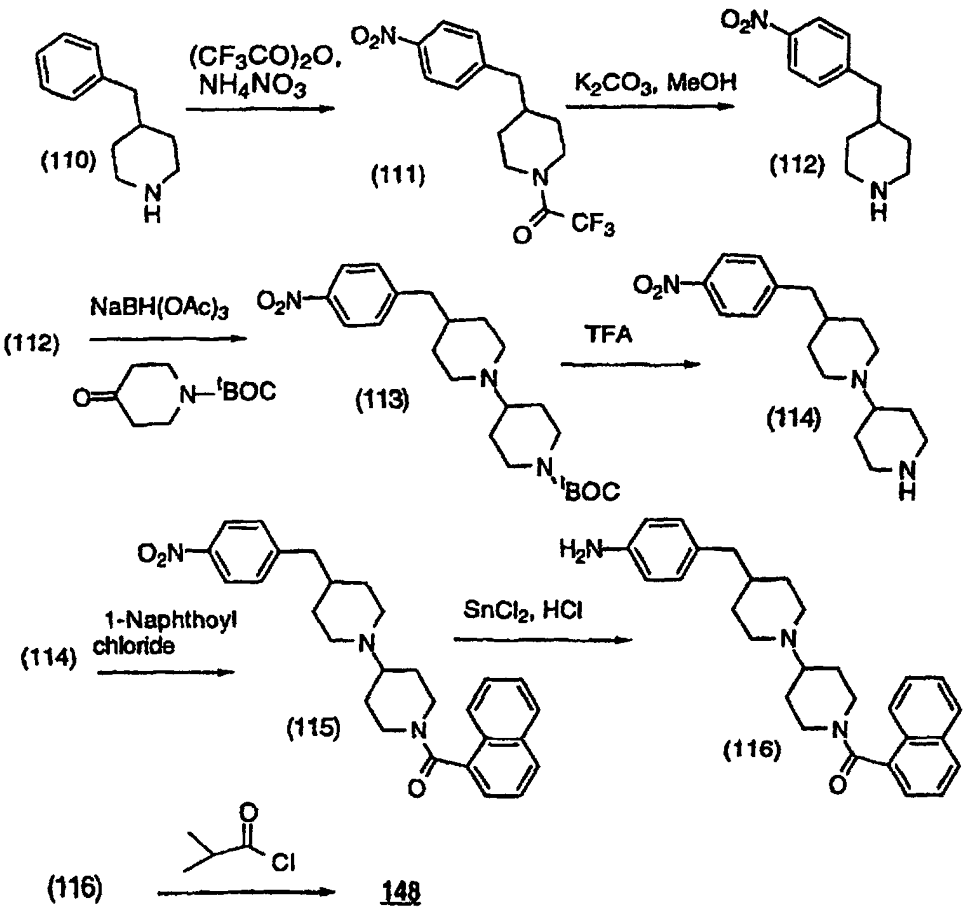


To a stirred solution of (110) (9.3 g, 53.2 mmol) and $NH_4NO_3$ (4.4 g, 55.8 mmol) was added trifluroacetic anhydride (40 ml) at -15°C. The solvent was evaporated under vacum at rt. The resulting oil was then diluted with water and the compound was extracted with $CH_2Cl_2$ (2x50 ml). The combined organic layers were washed sequentially with dilute $NaHCO_3$ and brine, dried ($Na_2SO_4$), concentrated and chromatographed to afford (111) (3.8 g, 43%) as colorless oil.

To a stirred solution of (111) (3.8 g, 12.0 mmol) in $CH_3OH$:$H_2O$ (5:1) 100 ml was added $K_2CO_3$ (3.3 g, 24.0 mmol) and stirred for 3 h at rt. The reaction mixture was concentrated and poured into 2N NaOH and extracted with $CH_2Cl_2$ (2x50 ml). The combined layers were dried ($Na_2SO_4$), filtered, concentrated to yield (112) (1.79 g, 70%) as a colorless oil.

(112) was treated with N-BOC-4-piperidone, then extracted as described in Example 38 to yield (113) (0.700g, 20%) as a colorless oil.

The BOC group on (113) was removed with TFA to obtain the amine (114), and (114) was reacted with 1-naphthoyl chloride as described in Example 14, step 8, to obtain (115) (0.448 g, 73%) as a colorless oil.

To a solution of (115) (0.170 g, 0.345 mmol) in $CH_3OH$ (5 ml) was added $SnCl_2$ (0.262 g, 1.38 mmol) and concentrated HCl (1 ml) and the reaction was heated to reflux for 12 h. The reaction mixture was then cooled to rt and poured into 10% NaOH and extracted with $CH_2Cl_2$ (2x10 ml). The extracts were dried ($Na_2SO_4$), filtered, and concen-

trated to yield (116) (0.150 g, 100%).

To a solution of (116) (0.02 g, 0.047 mmol) in $CH_2Cl_2$ (1 ml) was added $Et_3N$ (0.013 ml, 0.094 mmol) followed by isobutyryl chloride (0.010 ml, 0.07 mmol) and the mixture stirred at rt for 2 h. The reaction mixture was then poured into sat'd aq $NaHCO_3$ and extracted with $CH_2Cl_2$ (2x5 ml). The extracts were dried ($Na_2SO_4$), filtered, concentrated and chromatographed to yield **148** (0.014 g, 65%). LRMS: calc'd: 497; found ($M+H^+$): 498.

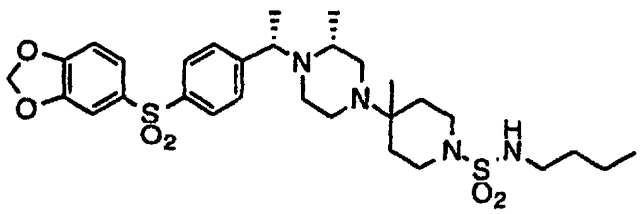

**149**

**Example 45**

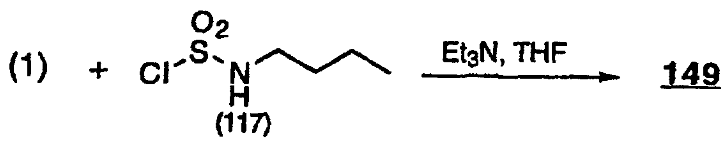


To a solution of (1) (20.0 mg, 0.042 mmol) in anhydrous THF (0.4 ml) at 0°C were successively added *n*-butyl sulfamoyl chloride (117) (literature prep.) (14.4 mg, 0.084 mmol) and $Et_3N$ (11.8 µl, 0.084 mmol) and the resulting mixture was stirred 14 h at room temperature. After evaporation of the solvent, the residue was treated with saturated aquous $NaHCO_3$ solution (10 ml), extracted with $CH_2Cl_2$ (3 x 5 ml), dried over $Na_2SO_4$ and evaporated. Purification of the crude by preparative silica gel chromatography (eluent $CH_2Cl_2/CH_3OH/NH_3$ 96:4:1) provided **149** (17.3 mg, 66%) as an oil: MH+ = 621 (hydrochloride salt).

**Example 46**

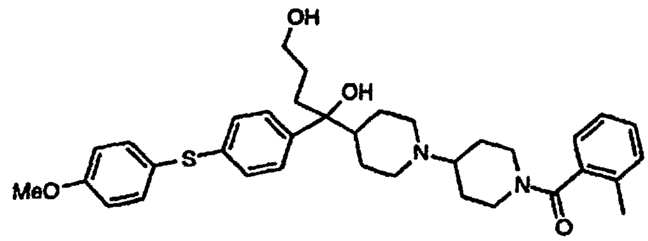

**150**

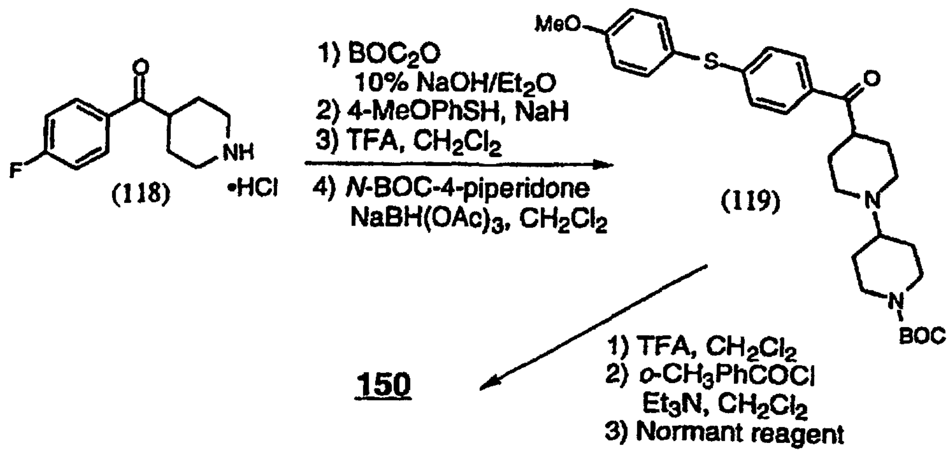


To a stirred 0°C solution of (118) (50.0 g, 205 mmol) in a 1:1 solution of $Et_2O$/10% aq. NaOH (500 ml) was added a 2.5 M solution of di-*tert*-butyldicarbonate in $Et_2O$ (100 ml, 246 mmol) over 30 min with vigorous stirring. After stirring at room temperature for 2 h, the mixture was extracted with $Et_2O$, washed with brine, dried over $Na_2SO_4$, filtered and concentrated in vacuo. The resulting product (73.9 g) was used without further purification.

3,4-Methylenedioxythiophenol (19.1 g, 124 mmol) was added dropwise to a stirred 0°C suspension of NaH (5.46 g of a 60% dispersion, 136 mmol) in DMF (75 ml). After stirring 30 min at 0°C, (118) (31.8 g, 103 mmol) in DMF (75 ml) was added and the solution warmed to room temperature overnight. The reaction was quenched with water and extracted with EtOAc. The organics were washed with brine, dried over $MgSO_4$, filtered, and concentrated in vacuo. The crude product was recrystallized from EtOAc/hexanes, with the first two crops yielding 15.8 g pure sulfide (35% yield).

The sulfide was treated with TFA to obtain the free amine, then reacted with N-BOC-4-piperidone as described in Example 38 to obtain (119) (8.20 g, 69% yield).

The BOC-group was removed and the resultant amine was reacted with 2-toluoyl chloride in a manner similar to that described in Example 14, Step 8. The mixture was concentrated under reduced pressure, yielding the aryl amide (0.65 g, 100% yield).

A solution of the aryl amide (0.21 g, 0.39 mmol) in THF (0.70 ml) was cooled to 0°C under $N_2$. A solution of Normant reagent (~0.5 M) in THF was added in 1 ml portions until the reaction was complete by TLC (10/90 $CH_3OH$/$CH_2Cl_2$). The reaction was quenched with $NH_4Cl$ (sat), diluted with $CH_2Cl_2$, and 1 N NaOH was added. The aqueous layer was extracted with $CH_2Cl_2$, and the combined organics were dried over $K_2CO_3$, filtered, and concentrated to yield **150** (0.17 g, 71% yield). LRMS calc'd: 602; found (M+H): 603.

**Example 47**

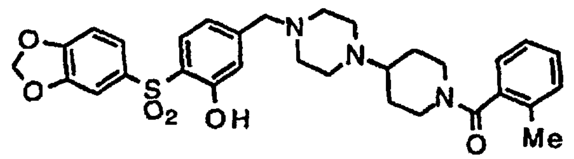

**485**

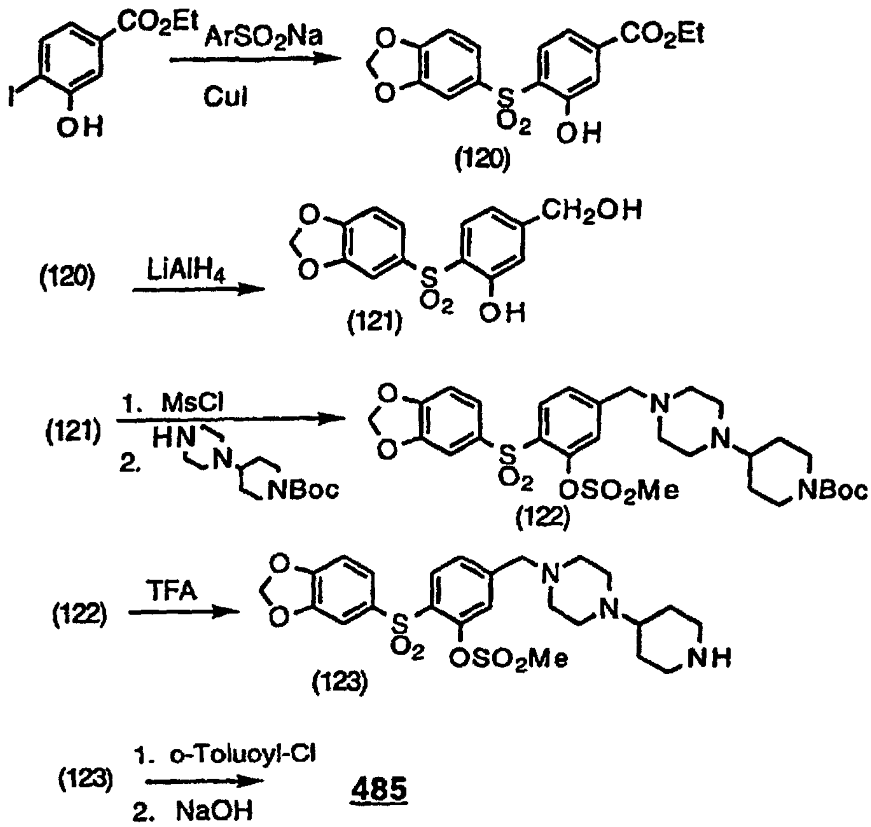


(120) (121) (122) (123) 485

Step 1: Ethyl 3-hydroxy-4-iodobenzoate (2.92 g), sodium 3,4-methylenedioxybenzenesulfinate (3.2 g), copper (I) iodide (2.8 g) and DMF (20 ml) were heated under $N_2$ for 20 h, added to 20% aqueous NaI (200 ml), extracted with EtOAc, dried and evaporated. The residue was flash-chromatographed on silica, eluting with hexanes-EtOAc, and pure fractions evaporated to give the product (120) as a solid (0.81 g), mp 133-135°C.

Step 2 (120) (0.40 g) in THF (5 ml) was added to a stirred, ice-cooled mixture of $LiAlH_4$ (0.08 g) and THF (10 ml). After 0.5 h, the mixture was treated with $H_2O$ then 1N HCl, extracted with EtOAc, dried over $MgSO_4$ and evaporated to give (121), which was used in the next step.

Step 3: (121) in $CH_2Cl_2$ (20 ml) and $Et_3N$ (1.0 ml) was stirred with ice cooling and $CH_3SO_2Cl$ (0.21 ml) added. After 0.5 h, the mixture was washed with 1N HCl, and then $NaHCO_3$ solution, dried and evaporated. This residue was stirred with diisopropylethylamine (0.3 ml) and the piperazine derivative (0.5 g) in DMF (3 ml) for 20 h at RT, worked up in water-EtOAc, dried, evaporated and chromatographed on flash silica, eluting with 1 to 3% $CH_3OH$ in $CH_2Cl_2$. Pure fractions were evaporated to give the product (122) as a white foam (0.60 g). Mass spectrum: MH+ = 638.

Step 4: (122) (0.43 g) was stirred for 45 min at RT in $CH_2Cl_2$ (2 ml) and 95% TFA (5 ml), evaproated, worked up in EtOAc - aq, $NaHCO_3$, dried, and evaporated to give (123) as a white foam (0.27 g). Mass spectrum: MH+ = 538.

Step 5: (123) (0.07 g) and o-toluoyl chloride (0.06 g) were stirred for 45 min at RT in $CH_2Cl_2$ (5 ml) and saturated $NaHCO_3$ (5 ml). The mixture was extracted with $CH_2Cl_2$ and the organics were evaporated. The residue was sitrred

for 0.5 h in a 5% solution of NaOH in 95% $CH_3OH$ (4 ml), diluted with $H_2O$ and $CH_2Cl_2$, and treted with stirring with small portions of solid $CO_2$ until the pH of the aq. phase was 7-8. The organic phase was dried and evaporated, and the residue dissolved in $CH_2Cl_2$ (2 ml) and added to $Et_2O$ (15 ml) containing 4M HCl-dioxan (0.4 ml). The precipitate was centrifuged, the solid washed twice with $Et_2O$, and dried at RT in $N_2$ to give the hydrochloride of **485** as a white powder (0.051 g). Mass spectrum: MH+ = 607.

**Example 48**

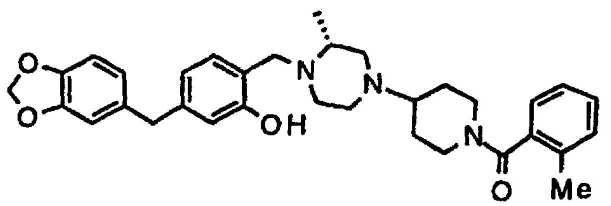

**300**

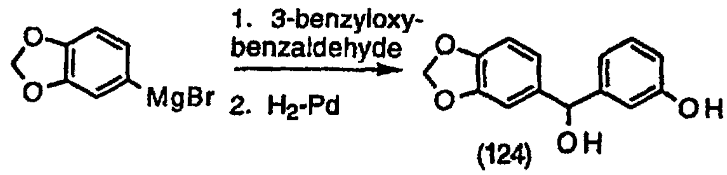


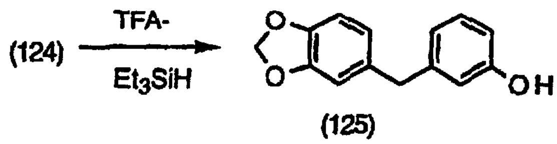


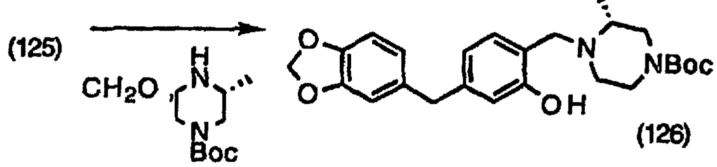


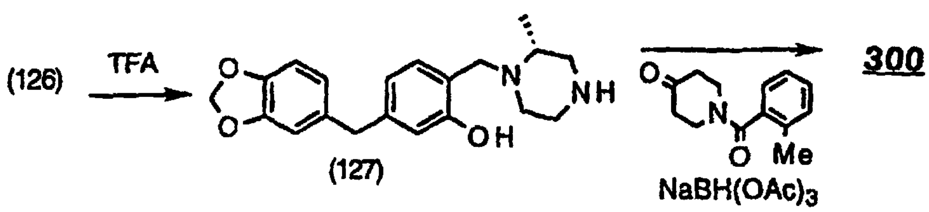


Step 1: The Grignard reagent prepared by reacting 3,4-methylene-dioxy bromobenzene (20 g) and Mg (3 g) in THF (60 ml) was cooled in ice and 3-benzyloxybenzaldehyde (16 g) in THF (60 ml) was added slowly. After 15 min, the mixture was added to a stirred mixture of ice and HCl (75 ml of 2N), extracted with $Et_2O$, washed with $NaHCO_3$ solution, dried and evaporated. The residue was extracted by stirring followed by decanting with hexanes (2 x 100 ml) and the residue was dried at high vacuum to a brown oil.

This material was shaken in $H_2$ (60 psi) at RT for 4 h in EtOAc (150 ml), HOAc (1.5 ml) and 20% palladium hydroxide on carbon (1.5 g). After filtration and evaporation, the residue was triturated with 1:1 $Et_2O$-hexanes (2 x 50 ml) and dried at RT to give (124) as a white solid (8.2 g). Mp 139-142°C.

Step 2: (124) (8.1 g) was stirred with $Et_3SiH$ (20 ml) in $CH_2Cl_2$ (200 ml) and TFA (10 ml) was added dropwise. After 0.5 h. the solution was washed twice with $H_2O$ and evaporated. The residue in $CH_2Cl_2$ (100 ml) was treated for 15 min. with 1M tetrabutylammonium fluoride in THF (50 ml), washed with 1N $H_2SO_4$, dried, evaporated and chromatographed on flash silica, eluting with 0 to 2% $Et_2O$ in $CH_2Cl_2$. Pure fractions on evaporation gave (125) as a thick oil (6.28 g). Mass spectrum: MH+ = 229.

Step 3: A mixture of (125) (2.3 g), 4-t-butoxycarbonyl-2-(R)-methylpiperazine (2.4 g), ethanol (40 ml), 37% formalin (2.5 ml) and **4M** HCl-dioxan (1.25 ml) was refluxed for 24 h, worked up In $CH_2Cl_2$: aq. $NaHCO_3$, dried, evaporated and the residue was chromatographed on flash silica, eluting with 5 to 30% $Et_2O$-hexanes. Pure fractions were evaporated to give (126) as a white foam (2.6 g). Mass spectrum: MH+ = 441.
Step 4: A solution of (126) (1.7 g) in $CH_2Cl_2$ (20 ml), $H_2O$ (0.5 ml) and TFA (5 ml) was stirred at RT for 1 h., diluted with $CH_2Cl_2$ and $H_2O$, and treated with small portions of $K_2CO_3$ until the pH of the aqueous phase remained at 8-9. The organic phase was dried and evaporated to give (127) as a white foam (1.2 g). Mass spectrum: MH+=341.
Step 5: A mixture of (127) (1.12 g), 1-(o-toluoyl)-4-piperidinone (0.8 g), $CH_2Cl_2$ (25 ml) and $NaBH(OAc)_3$ (1.5 g) was stirred at RT for 20 h, washed with excess aq. $NaHCO_3$, dried and evaporated. The residue was dissolved in $CH_3OH$ containing 4M HCl-dioxan (2 ml), evaporated, co-evaporated with $CH_3OH$ (100 ml), and this residue dissolved in $CH_3OH$ (1 ml) and $CH_2Cl_2$ (15 ml) and added to stirred $Et_2O$ (100 ml) containing 4M HCl-dioxan (1 ml). The precipitate was filtered, washed with $Et_2O$ and dried to give the hydrochloride of 300 as a white powder (1.76 g). Mass spectrum: MH+ = 542.

**Example 49**

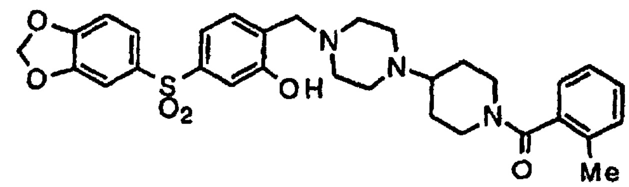

210

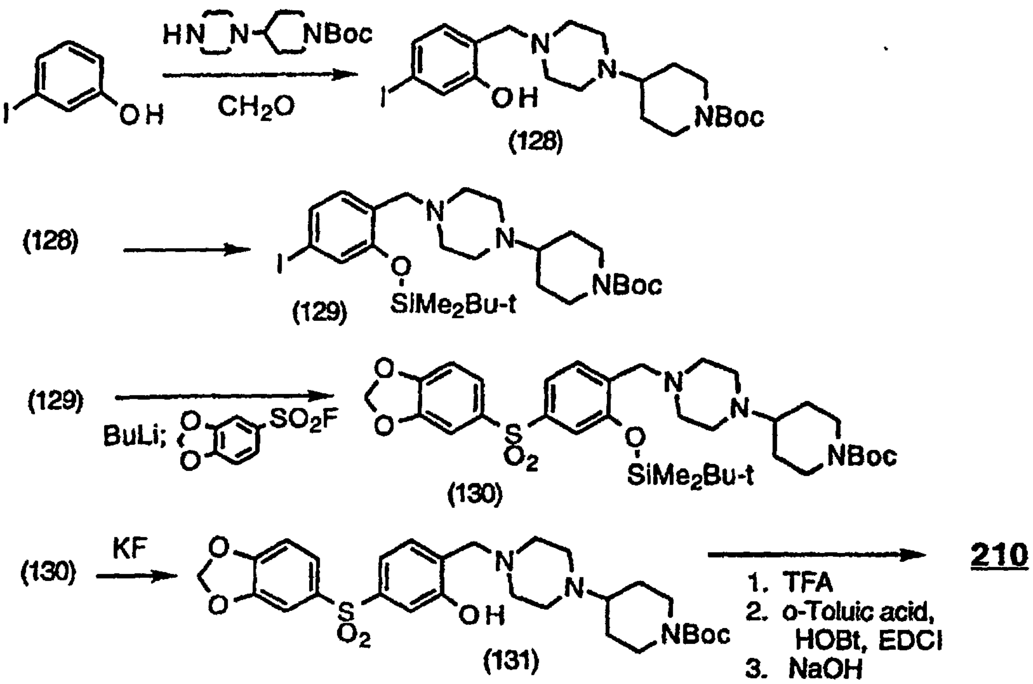


Step 1: A mixture of 3-iodophenol (1.76 g), the piperazine (1.35 g), 37% formalin (1.25 ml) and ethanol (15 ml) was refluxed for 1 h, then additional formalin (0.75 ml) was added and heating continued for 10h. The reaction was cooled, worked up in $CH_2Cl_2$-$H_2O$, dried and evaporated. The major product was isolated by flash chromatography on silica, eluting with 0 to 1.5% $CH_3OH$ in EtOAc.
Evaporation of pure fractions gave a yellow foam, (128) (1.31 9). Mass spectrum: MH+ = 502.
Step 2: (128) (1.25 g) and t-butylchlorodimethylsilane (0.8 g) with $Et_3N$ (0.8 ml) amd dimethylaminopyridine (0.03 g) was stirred in $CH_2Cl_2$ (30 ml) for 40h, evaporated, and chromatographed on flash silica in EtOAc to obtain the product (129) as a white foam (1.48 g). Mass spectrum; MH+ = 602.
Step 3: A solution of (129) (1.42 g) in dry THF (25 ml) at -70°C was treated with n-BuLi in hexanes (2M; 1.4 ml). Immediately, a solution of 3,4-methylenedioxybenzenesulfonyl fluoride (0.65 g) in THF (1 ml) was added, and the

mixure stirred to RT over 0.5h. The reaction was partitioned in $H_2O$ - EtOAc, dried and evaporated, and the residue chromatographed on flash silica in hexanes-EtOAc. Pure fractions were evaporated to give the product (130) as a pale yellow foam (0.76 g). Mass spectrum: MH+ = 674.

Step 4: (130) (0.55 g) was stirred for 20 h in $CH_3OH$ (15 ml) containing HOAc (0.1 g) and KF (1.0 g), partitoned in $H_2O$-$CH_2Cl_2$, dried and evaporated. Flash chromatography on silica with EtOAc gave the pure product (131) as a pale yellow foam (0.41 g). Mass spectrum: MH+ = 560.

Step 5: A mixture of (131) (0.37 g), $CH_2Cl_2$ (3 ml). $H_2O$ (0.05 ml) and TFA (2 ml) was kept at RT for 2 h, evaporated, and partitioned in $H_2O$-$CH_2Cl_2$ with sufficient $NaHCO_3$ to maintain the aq. phase at pH 8-9. The $CH_2Cl_2$ extracts were dried and evaporated, and this residue in DMF (15 ml) stirred 20 h at RT with o-toluic acid (0.2 g), 1-hydroxybenzotriazole (0.15 g) and EDCI (0.4 g). The mixture was partitioned in EtOAc and aq. $NaHCO_3$, dried and evaporated. The major component was isolated by ptlc on silica plates with 3% $CH_3OH$-$CH_2Cl_2$ to give a white foam (0.38 g) which was mainly the N,O-di-toluoyl compound. 0.3 g of his material was stirred for 24 h at RT in $CH_3OH$ (7 ml) and $H_2O$ (0.8 ml) with NaOH (0.5 g). The solution was stirred with $H_2O$-$CH_2Cl_2$ and neutralised to pH 7-8 with solid $CO_2$. The organic phase was dried and evaporated, and the hydrochloride of **210** precipitated as described in earlier examples, and dried at RT in vacuum to give a white solid (0.22 g). Mass spectrum: MH+ = 578.

**Example 50**

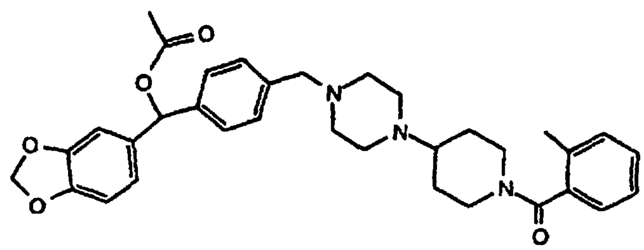

**520**

27 mg of **30** were taken up in 1.5 mL of glacial HOAc. The solution was stirred for 7 h at 40°C. Excess saturated $NaHCO_3$ solution was added, and the mixture was extracted with EtOAc. This was dried over $Na_2SO_4$, evaporated, and the residue purified by column chromatography in 10% $CH_3OH$/EtOAc, to give approx. 17 mg of **520** as an oil. MS (FAB): 598.4 (M+1), 538.5.

**Example 51**

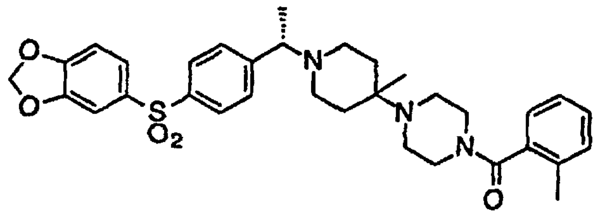

. 2 HCl

**587**

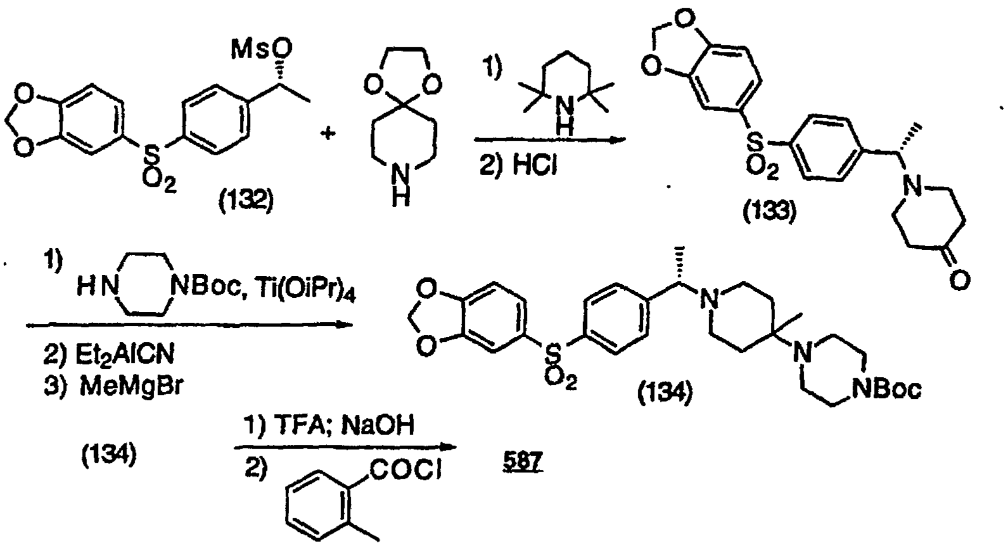


To a solution of (132) (2.31 g, 6.00 mmol) in anhydrous $CH_3CN$ (30 ml) were successively added 4-piperidone ethylene ketal (0.77 ml, 6.00 mmol) and 2,2,6,6-tetramethyl piperidine (1.22 ml, 7.20 mmol), and the mixture was heated 14 h at 60°C. After evaporation of the solvent, the residue was taken up in saturated aqueous $NaHCO_3$ (100 ml), extracted with $CH_2Cl_2$ (3 x 50 ml), dried over $Na_2SO_4$, and evaporated. The oil thus obtained (2.89 g) was refluxed with 6 N HCl (40 ml) for 7 h then evaporated. Workup as above and purification by flash chromatography ($SiO_2$, $CH_2Cl_2$/$CH_3OH$/$NH_3$ 96:4:1) afforded (133) (1.26 g, 49%).

To a solution of (133) (470 mg, 1.21 mmol) and *N*-Boc piperazine (255 mg, 1.27 mmol) in anhydrous $CH_2Cl_2$ (1 ml) was added titanium (IV) isopropoxide (447 ml, 1.21 mmol), and the mixture was stirred 3 days at room temperature. The solution was diluted with $CH_2Cl_2$ (1 ml), $Et_2AlCN$ 1 N in toluene (3.65 ml) was added at 0°C and the resulting mixture was allowed to warm to room temperature and stirred 2 h. Upon addition of Celite and water (100 ml), the final suspension was filtered over Celite, dried over $Na_2SO_4$ and evaporated. The crude thus obtained (655 mg,-93%) was dissolved in anhydrous THF (7 ml), treated at 0°C with $CH_3MgBr$ 3 N in $Et_2O$ (3.8 ml, 11.3 mmol) and the solution was allowed to warm to room temperature then heated 3 h at 55°C. After cooling, Celite (2 g) and $CH_2Cl_2$ (10 ml) were added and the suspension was slowly poured into ice. The resulting suspension was filtered over Celite and worked up as before to provide (134) (375 mg, 58%), after flash chromatography ($SiO_2$, eluent $CH_2Cl_2$/$CH_3OH$/$NH_3$ 80:20:1).

Removal of the Boc group with TFA gave, after basic workup with 1 N NaOH and extraction with $CH_2Cl_2$, 260 mg of intermediate. This (20 mg, 0.042 mmol) was subjected to *N*-acylation with *o*-toluoyl chloride to provide, after conversion to the hydrochlorid salt, **587** (15 mg) as a white solid: MH+ = 590.2.

Using the appropriate starting materials in the procedures described above or modifications of those procedures well known to those skilled in the art, the compounds shown in the following tables are prepared.

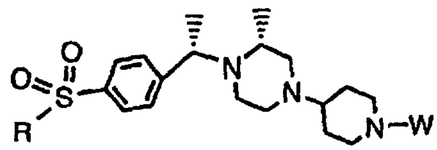

| No. | R | W | Physical Data |
|---|---|---|---|
| 151 | 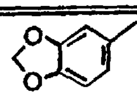 | 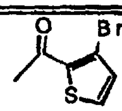 | m.p. = 198-206°C |
| 152 | 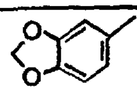 | 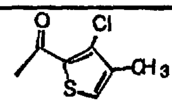 | m.p. = 186-192°C |
| 153 | 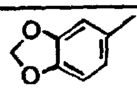 | 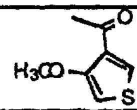 | (M-15) = 596 |
| 154 | 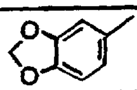 | 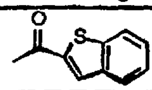 | m.p. = 210-15°C |
| 155 | 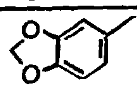 | 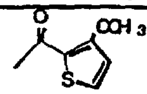 | MH+ = 612 |
| 156 | 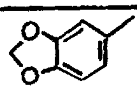 | 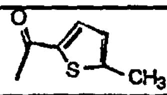 | MH+ = 596 |
| 157 | 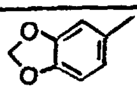 | 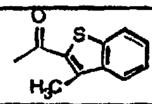 | MH+ = 660 |
| 158 | 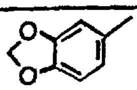 | 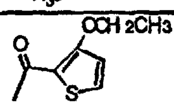 | MH+ = 626 |
| 159 | 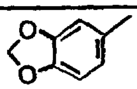 | 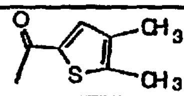 | MH+ = 610 |
| 160 | 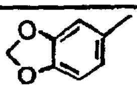 | 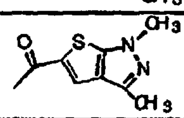 | MH+ = 650 |
| 161 | 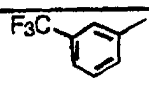 | 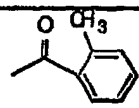 | HRMS<br>calc'd: 614.2664<br>found: 614.2664 |
| 162 | 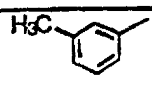 | 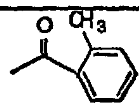 | HRMS<br>calc'd: 560.2947<br>found: 560.2955 |
| 163 | 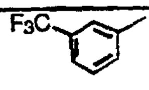 | 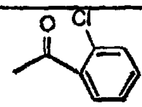 | Mass spec.<br>(FAB)M+1=635 |
| 164 | 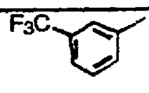 | 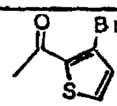 | Mass spec.<br>(FAB)M+1=687 |
| 165 | 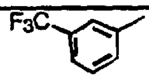 | 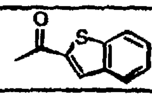 | Mass spec.<br>(FAB)M+1=656 |

| | | | |
|---|---|---|---|
| 166 | 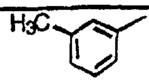 | 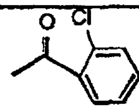 | Mass spec. (FAB)M+1=580 |
| 167 | 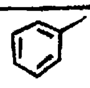 | 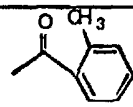 | HRMS calc'd: 560.2947 found: 560.2933 |
| 168 | 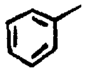 | 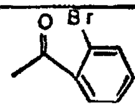 | HRMS calc'd: 610.1738 found: 610.1739 |
| 169 | 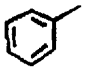 | 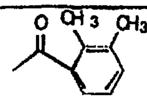 | HRMS calc'd: 546.2790 found: 546.2787 |
| 170 | 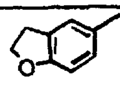 | 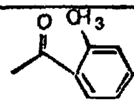 | HRMS calc'd: 588.2896 found: 588.2897 |
| 171 | 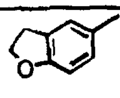 | 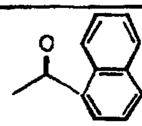 | HRMS calc'd: 624.2896 found: 624.2903 |
| 172 | 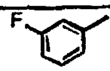 | 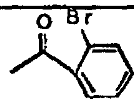 | HRMS calc'd: 628.1645 found: 628.1646 |
| 173 | 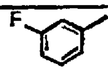 | 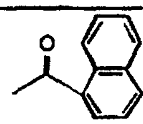 | HRMS calc'd: 600.2696 found: 600.2694 |
| 174 | 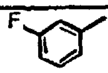 | 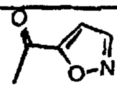 | HRMS calc'd: 541.2285 found: 541.2289 |
| 175 | 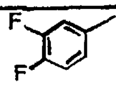 | 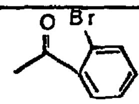 | HRMS calc'd: 646.1551 found: 646.1150 |
| 176 | 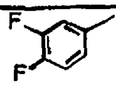 | 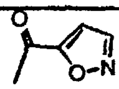 | HRMS calc'd: 559.2191 found: 559.2202 |
| 177 | 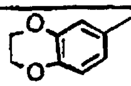 | 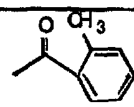 | HRMS calc'd: 604.2845 found: 604.2840 |
| 178 | 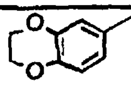 | 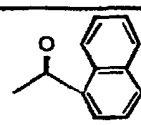 | HRMS calc'd: 640.2845 found: 640.2840 |
| 179 | 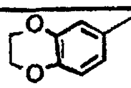 | 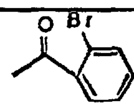 | HRMS calc'd: 668.1794 found: 668.1785 |
| 180 | 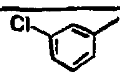 | 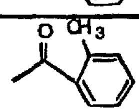 | HRMS calc'd: 580.2401 found: 580.2401 |
| 181 | 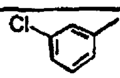 | 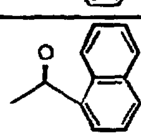 | HRMS calc'd: 616.2401 found: 616.2395 |

| | | | |
|---|---|---|---|
| 182 | 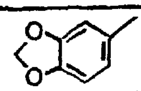 | 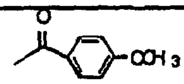 | FAB(MH+) = 606 |
| 183 | 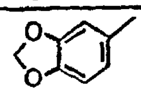 | 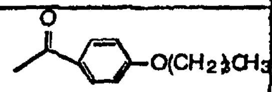 | FAB(MH+) = 648 |
| 184 | 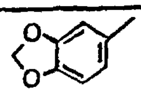 | 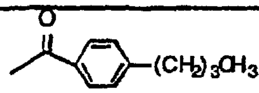 | FAB(MH+) = 632 |
| 185 | 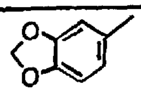 | 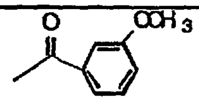 | FAB(MH+) = 606 |
| 186 | 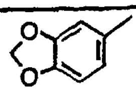 | 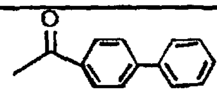 | FAB(MH+) = 652 |
| 187 | 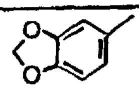 | 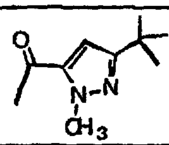 | FAB(MH+) = 636 |
| 188 | 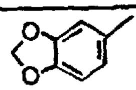 | 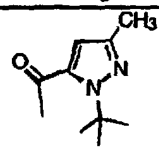 | FAB(MH+) = 636 |
| 189 | 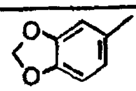 | 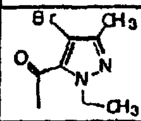 | FAB(MH+) = 686 |
| 190 | 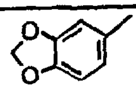 | 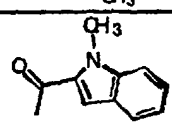 | m.p. = 200-202°C (dec) |
| 191 | 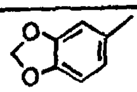 | 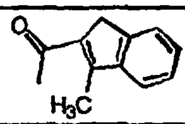 | m.p. = 201-203°C (dec) |
| 192 | 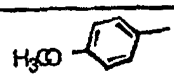 | 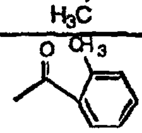 | m.p. = 215-216°C (dec) |
| 193 | 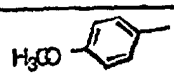 | 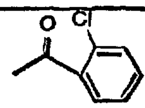 | m.p. = 210-201°C (dec) |
| 194 | 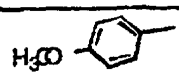 | 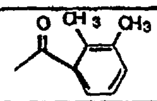 | m.p. = 219-220°C (dec) |
| 195 | 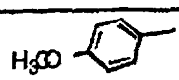 | 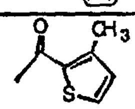 | FAB(MH+) = 582 |
| 196 | 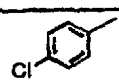 | 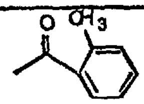 | m.p. = 204-206°C (dec) |

| | | | |
|---|---|---|---|
| 197 | 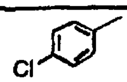 | 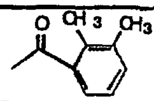 | m.p. = 198-200°C (dec) |
| 198 | 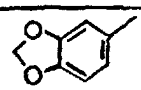 | 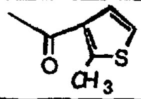 | MH+ = 596 |
| 199 | 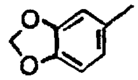 | 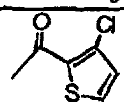 | MH+ = 610 |
| 200 | 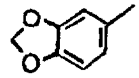 | 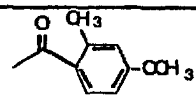 | MH+ = 620 |
| 201 | 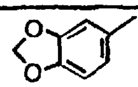 | 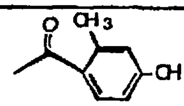 | MH+ = 606 |
| 202 | 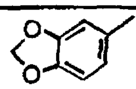 | 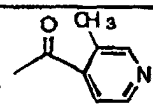 | MH+ = 541 |
| 203 | 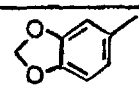 | 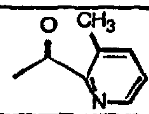 | MH+ = 591 |
| 204 | 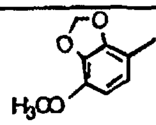 | 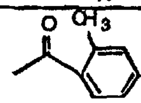 | MH+ = 620 |
| 205 | 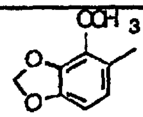 | 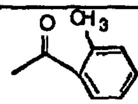 | MH+ = 620 |
| 206 | 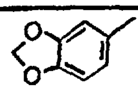 | 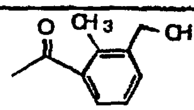 | MH+ = 620 |
| 207 | 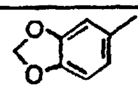 | 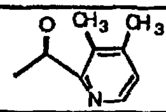 | MH+ = 605 |
| 208 | 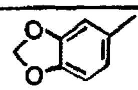 | 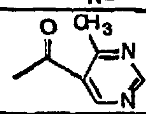 | MH+ = 592 |
| 209 | 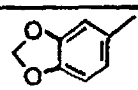 | 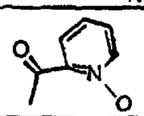 | MH+ = 520 |
| 211 | 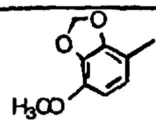 | 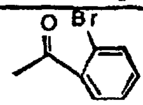 | MH+ = 684 |
| 212 | 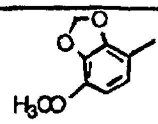 | 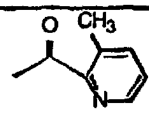 | MH+ = 621 |

| | | | |
|---|---|---|---|
| 213 | 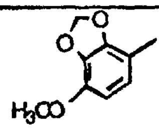 | 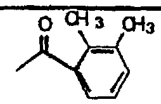 | MH+ = 634 |
| 214 | 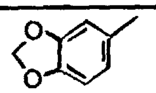 | 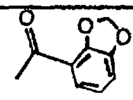 | m.p.= decomp >225°C |
| 215 | 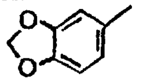 | 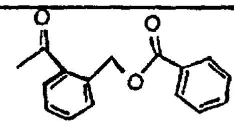 | m.p.= decomp 184°C |
| 216 | 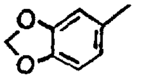 | 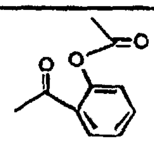 | m.p.= decomp above 189°C |
| 217 | 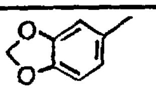 | 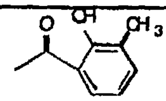 | m.p.= 172-176°C |

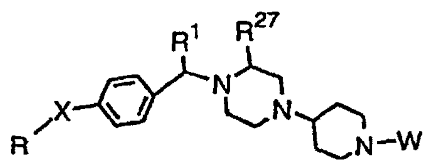

| No. | R | W | $R^1$ | $R^{27}$ | X | Physical Data |
|---|---|---|---|---|---|---|
| 218 | 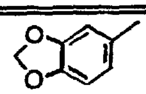 | 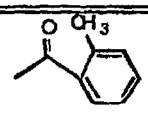 | H | H | -SO- | MS (electrospray): 546 (M+1), 487 (isomer A) |
| 219 | 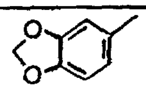 | 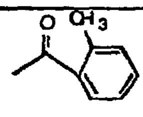 | H | H | -SO- | MS (electrospray): 546 (M+1), 463 (isomer B) |
| 220 | 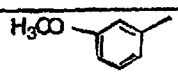 | 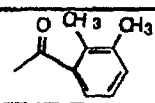 | H | H | $-SO_2-$ | MS (FAB): 562 (M+1), 302 |
| 221 | 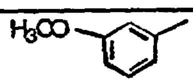 | 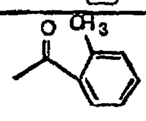 | H | H | $-SO_2-$ | MS (FAB): 548 (M+1), 461 |
| 222 | 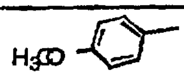 | 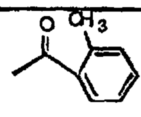 | H | H | $-SO_2-$ | MS (electrospray): 548.2 (M+1), 465 |
| 223 | 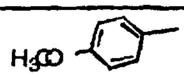 | 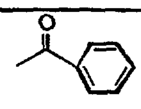 | H | H | $-SO_2-$ | MS (electrospray): 534.2 (M+1), 472 |
| 224 | 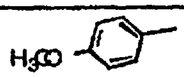 | 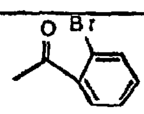 | H | H | $-SO_2-$ | MS (electrospray): 613, 615 (M+1), 472 |

| | | | | | | |
|---|---|---|---|---|---|---|
| 225 | 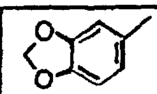 | 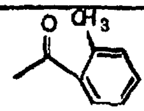 | H | H | $-SO_2-$ | m.p.= 230-246°C |
| 226 | 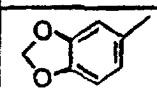 | 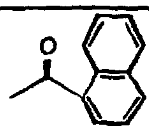 | H | H | $-SO_2-$ | m.p.= 220°C (dec) |
| 227 | 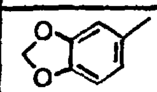 | 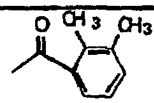 | H | H | $-SO_2-$ | m.p.= decomp above 245°C |
| 228 | 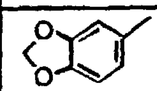 | 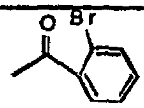 | H | H | $-SO_2-$ | m.p.= 169-171°C |
| 229 | 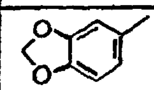 | 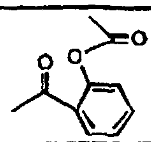 | H | H | $-SO_2-$ | m.p.= decomp above 235°C |
| 230 | 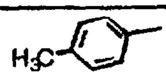 | 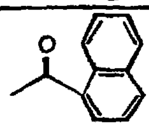 | H | H | $-SO_2-$ | m.p.= decomp above 202°C |
| 231 | 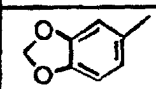 | 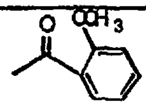 | H | H | $-SO_2-$ | m.p.= decomp above 208°C |
| 232 | 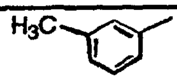 | 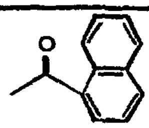 | H | H | $-SO_2-$ | m.p.= decomp above 195°C |
| 233 | 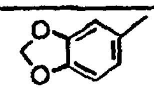 | 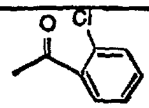 | H | H | $-SO_2-$ | m.p.= decomp above 235°C |
| 234 | 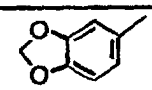 | 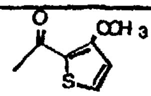 | H | H | $-SO_2-$ | m.p.= decomp above 217°C |
| 235 | 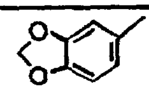 | 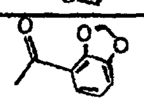 | H | H | $-SO_2-$ | m.p.= decomp above 185°C |
| 236 | 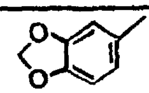 | 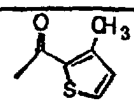 | H | H | $-SO_2-$ | m.p.= decomp above 207°C |
| 237 | 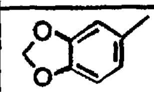 | 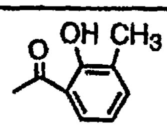 | H | H | $-SO_2-$ | m.p.= 164-166°C |
| 238 | 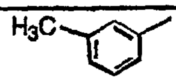 | 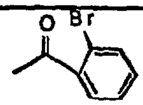 | H | H | $-SO_2-$ | m.p.= decomp above 250°C |
| 239 | 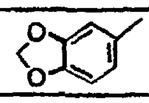 | 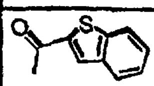 | H | H | $-SO_2-$ | m.p.= decomp above 239°C |

| | | | | | | |
|---|---|---|---|---|---|---|
| 240 | 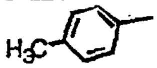 | 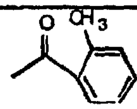 | H | H | $-SO_2-$ | m.p.= 204°C |
| 241 | 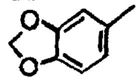 | 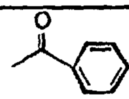 | H | H | $-SO_2-$ | m.p.= decomp above 232°C |
| 242 | 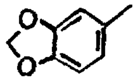 | 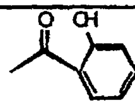 | H | H | $-SO_2-$ | m.p.= decomp above 205°C |
| 243 | 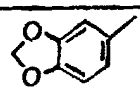 | 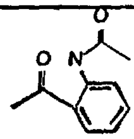 | H | H | $-SO_2-$ | m.p.= decomp above 180°C |
| 244 | 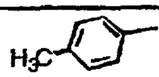 | 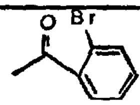 | H | H | $-SO_2-$ | m.p.= decomp above 200°C |
| 245 | 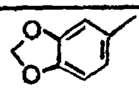 | 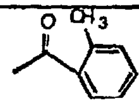 | $CH_3$ | H | $-SO_2-$ | MH+ = 576 |
| 246 | 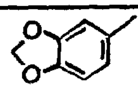 | 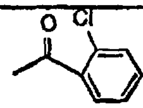 | $CH_3$ | H | $-SO_2-$ | MH+ = 596 |
| 247 | 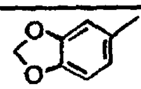 | 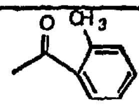 | H | $CH_3$ | $-SO_2-$ | m.p.= 174-177°C |
| 248 | 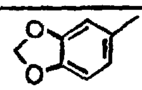 | 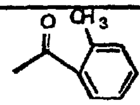 | $CH_3$ | H | 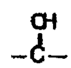 | MH+ = 542 |
| 249 | 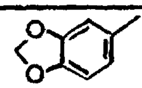 | 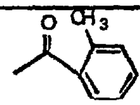 | H | H | 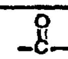 | FAB(MH+) = 526 |
| 250 | 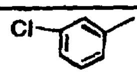 | 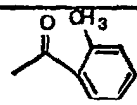 | $CH_3$ | $CH_3$ | 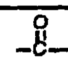 | HRMS calc'd: 544.2731 found: 544.2724 |
| 251 | 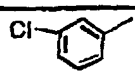 | 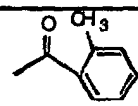 | $CH_3$ | $CH_3$ | 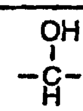 | Mass. Spec. (FAB)M+1 = 548 |
| 252 | 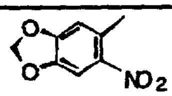 | 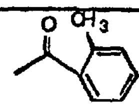 | $CH_3$ | $CH_3$ | 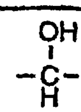 | Mass. Spec. (FAB)M+1 = 601 |
| 253 | 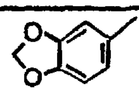 | 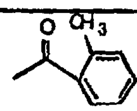 | $CH_3$ | H | $-CH_2-$ | MH+ = 526 |
| 254 | 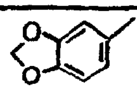 | 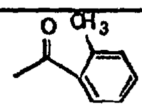 | $CH_3$ | $CH_3$ | $-CH_2-$ | HRMS calc'd: 554.3383 found: 554.3366 |

| | | | | | | |
|---|---|---|---|---|---|---|
| 255 | 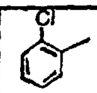 | 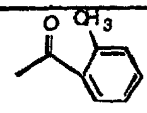 | $CH_3$ | $CH_3$ | $-CH_2-$ | HRMS calc'd: 530.2938 found: 530.2943 |
| 256 | 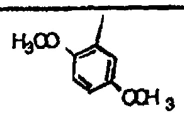 | 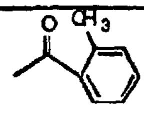 | $CH_3$ | $CH_3$ | $-CH_2-$ | HRMS calc'd: 556.3539 found: 556.3547 |
| 257 | 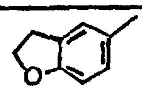 | 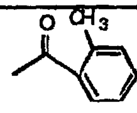 | $CH_3$ | $CH_3$ | $-CH_2-$ | Mass. Spec. (FAB)M+1 = 538 |
| 258 | 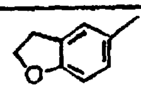 | 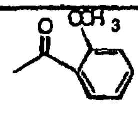 | $CH_3$ | $CH_3$ | $-CH_2-$ | HRMS calc'd: 554.3383 found: 554.3366 |
| 259 | 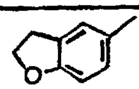 | 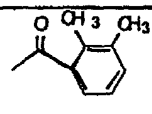 | $CH_3$ | $CH_3$ | $-CH_2-$ | HRMS calc'd: 552.3590 found: 552.3602 |
| 260 | 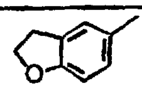 | 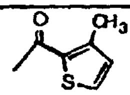 | $CH_3$ | $CH_3$ | $-CH_2-$ | HRMS calc'd: 544.2998 found: 544.2987 |
| 261 | 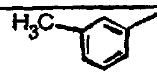 | 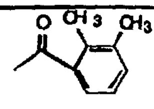 | $CH_3$ | $CH_3$ | $-CH_2-$ | HRMS calc'd: 524.3641 found: 524.3647 |
| 262 | 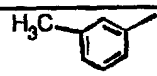 | 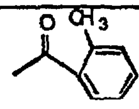 | $CH_3$ | $CH_3$ | $-CH_2-$ | HRMS calc'd: 510.3484 found: 510.3476 |
| 263 | 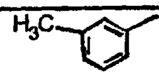 | 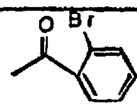 | $CH_3$ | $CH_3$ | $-CH_2-$ | HRMS calc'd: 574.2433 found: 574.2414 |
| 264 | 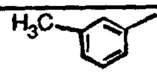 | 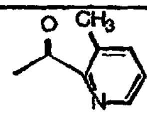 | $CH_3$ | $CH_3$ | $-CH_2-$ | HRMS calc'd: 511.3437 found: 511.3448 |
| 265 | 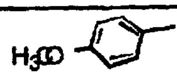 | 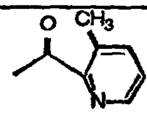 | $CH_3$ | $CH_3$ | $-CH_2-$ | HRMS calc'd: 527.3386 found: 527.3386 |
| 266 | 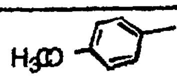 | 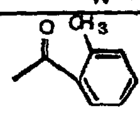 | $CH_3$ | $CH_3$ | $-CH_2-$ | HRMS calc'd: 526.3434 found: 526.3442 |
| 267 | 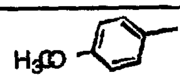 | 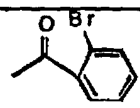 | $CH_3$ | $CH_3$ | $-CH_2-$ | HRMS calc'd: 590.2382 found: 590.2391 |
| 268 | 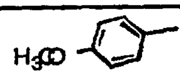 | 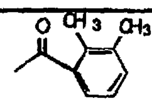 | $CH_3$ | $CH_3$ | $-CH_2-$ | HRMS calc'd: 540.3590 found: 540.3611 |
| 269 | 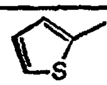 | 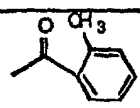 | $CH_3$ | $CH_3$ | $-CH_2-$ | HRMS calc'd: 502.2892 found: 502.2898 |

| | | | | | | |
|---|---|---|---|---|---|---|
| 270 | 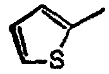 | 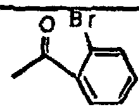 | $CH_3$ | $CH_3$ | $-CH_2-$ | HRMS calc'd: 566.1841 found: 566.1839 |
| 271 | 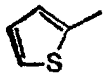 | 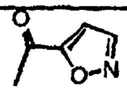 | $CH_3$ | $CH_3$ | $-CH_2-$ | HRMS calc'd: 479.2481 found: 479.2477 |
| 272 | 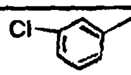 | 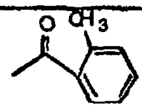 | $CH_3$ | $CH_3$ | $-CH_2-$ | HRMS calc'd: 530.2938 found: 530.2954 |
| 273 | 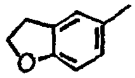 | 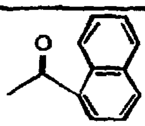 | $CH_3$ | $CH_3$ | $-CH_2-$ | HRMS calc'd: 574.3434 found: 574.3436 |
| 274 | 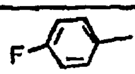 | 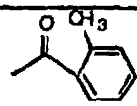 | $CH_3$ | $CH_3$ | $-CH_2-$ | HRMS calc'd: 514.3234 found: 514.3233 |
| 275 | 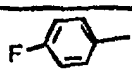 | 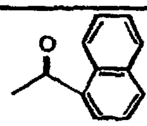 | $CH_3$ | $CH_3$ | $-CH_2-$ | HRMS calc'd: 550.3234 found: 550.3218 |
| 276 | 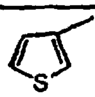 | 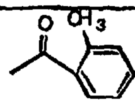 | $CH_3$ | $CH_3$ | $-CH_2-$ | HRMS calc'd: 502.2892 found: 502.2893 |
| 277 | 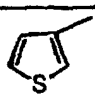 | 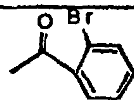 | $CH_3$ | $CH_3$ | $-CH_2-$ | HRMS calc'd: 479.2481 found: 479.2469 |
| 278 | 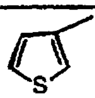 | 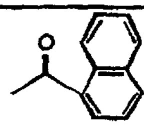 | $CH_3$ | $CH_3$ | $-CH_2-$ | HRMS calc'd: 538.2892 found: 538.2884 |
| 279 | 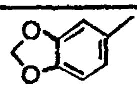 | 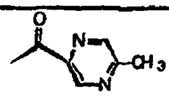 | $CH_3$ | $CH_3$ | $-CH_2-$ | FAB(MH+) = 542 |
| 280 | 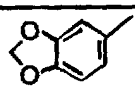 | 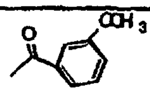 | $CH_3$ | $CH_3$ | $-CH_2-$ | FAB(MH+) = 556 |
| 281 | 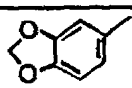 | 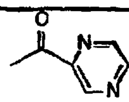 | $CH_3$ | $CH_3$ | $-CH_2-$ | FAB(MH+) = 528 |
| 282 | 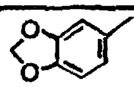 | 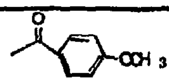 | $CH_3$ | $CH_3$ | $-CH_2-$ | FAB(MH+) = 556 |
| 283 | 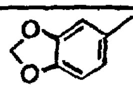 | 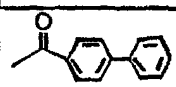 | $CH_3$ | $CH_3$ | $-CH_2-$ | FAB(MH+) = 602 |
| 284 | 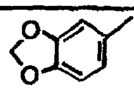 | 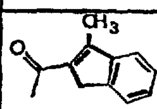 | $CH_3$ | $CH_3$ | $-CH_2-$ | FAB(MH+) = 578 |

| | | | | | | |
|---|---|---|---|---|---|---|
| 285 | 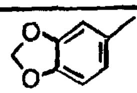 | 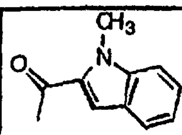 | $CH_3$ | $CH_3$ | $-CH_2-$ | FAB(MH+) = 579 |
| 286 | 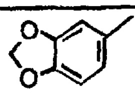 | 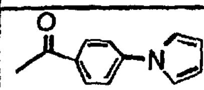 | $CH_3$ | $CH_3$ | $-CH_2-$ | FAB(MH+) = 591 |
| 287 | 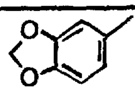 | 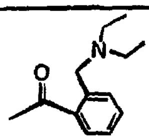 | $CH_3$ | $CH_3$ | $-CH_2-$ | FAB(MH+) = 611 |
| 288 | 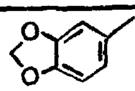 | 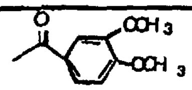 | $CH_3$ | $CH_3$ | $-CH_2-$ | m.p.= 215-216°C (dec) |
| 289 | 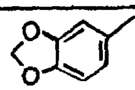 | 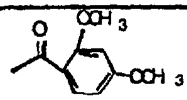 | $CH_3$ | $CH_3$ | $-CH_2-$ | m.p.= 178-179°C (dec) |
| 290 | 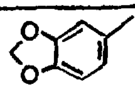 | 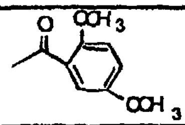 | $CH_3$ | $CH_3$ | $-CH_2-$ | m.p.= 209-211°C (dec) |
| 291 | 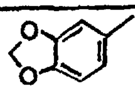 | 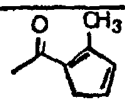 | $CH_3$ | $CH_3$ | $-CH_2-$ | MH+ = 530 |
| 292 | 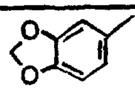 | 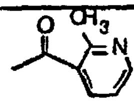 | $CH_3$ | $CH_3$ | $-CH_2-$ | MH+ = 541 |
| 293 | 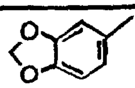 | 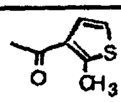 | $CH_3$ | $CH_3$ | $-CH_2-$ | MH+ = 546 |
| 294 | 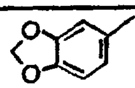 | 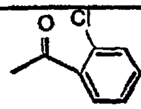 | $CH_3$ | $CH_3$ | $-CH_2-$ | MH+ = 561 |
| 295 | 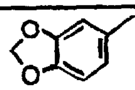 | 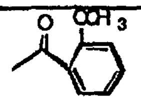 | $CH_3$ | $CH_3$ | $-CH_2-$ | MH+ = 556 |
| 296 | 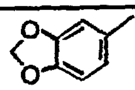 | 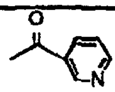 | $CH_3$ | $CH_3$ | $-CH_2-$ | MH+ = 527 |
| 297 | 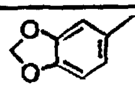 | 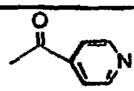 | $CH_3$ | $CH_3$ | $-CH_2-$ | MH+ = 527 |
| 298 | 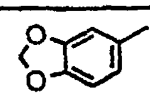 | 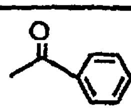 | $CH_3$ | $CH_3$ | $-CH_2-$ | MH+ = 526 |

| | | | | | | |
|---|---|---|---|---|---|---|
| 299 | 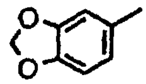 | 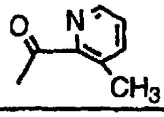 | $CH_3$ | $CH_3$ | $-CH_2-$ | MH+ = 541 |
| 301 | 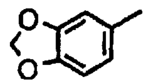 | 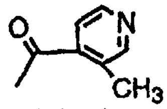 | $CH_3$ | $CH_3$ | $-CH_2-$ | MH+ = 541 |
| 303 | 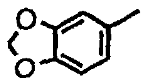 | 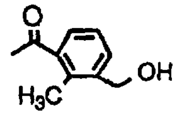 | $CH_3$ | $CH_3$ | $-CH_2-$ | MH+ = 570 |
| 304 | 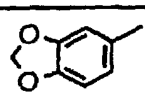 | 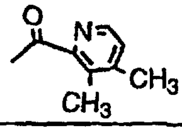 | $CH_3$ | $CH_3$ | $-CH_2-$ | MH+ = 555 |
| 305 | 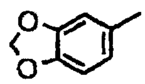 | 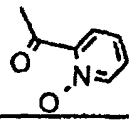 | $CH_3$ | $CH_3$ | $-CH_2-$ | MH+ = 543 |
| 306 | 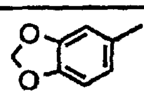 | 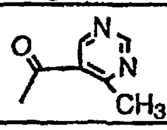 | $CH_3$ | $CH_3$ | $-CH_2-$ | MH+ = 542 |
| 307 | 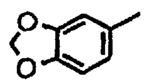 | 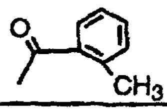 | H | H | $-CH_2-$ | m.p.= 216-223°C |
| 308 | 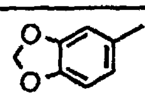 | 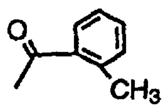 | H | $CH_3$ $CH_3$ | $-CH_2-$ | m.p.= decomp. above 145°C |
| 309 | 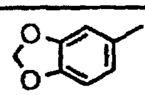 | 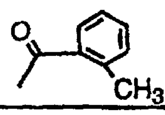 | H | $CH_3$ | $-CH_2-$ | m.p.= decomp. above110°C |
| 309 A | 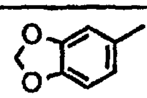 | 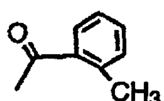 | H | $CH_3$ | $-CH_2-$ | m.p.= 228°C (dec) |
| 494 | 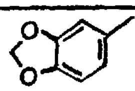 | 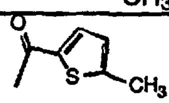 | $CH_3$ | $CH_3$ | $-CH_2-$ | FAB(MH+) = 546 |
| 495 | 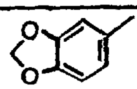 | 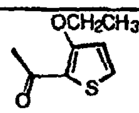 | $CH_3$ | $CH_3$ | $-CH_2-$ | FAB(MH+) = 576 |
| 496 | 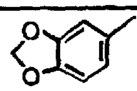 | 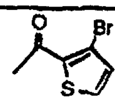 | $CH_3$ | $CH_3$ | $-CH_2-$ | m.p. = 195-201 °C |
| 497 | 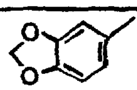 | 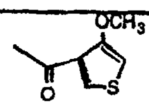 | $CH_3$ | $CH_3$ | $-CH_2-$ | m.p. = 202-204°C |

| | | | | | | |
|---|---|---|---|---|---|---|
| 498 | 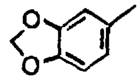 | 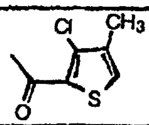 | 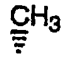 | 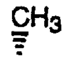 | $-CH_2-$ | m.p. = 202-204°C |
| 499 | 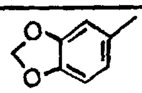 | 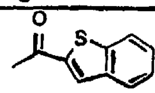 | 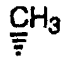 | 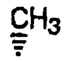 | $-CH_2-$ | m.p. = 228-232°C |
| 500 | 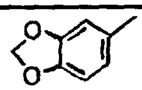 | 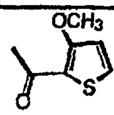 | 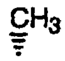 | 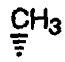 | $-CH_2-$ | MH+ = 562 |
| 501 | 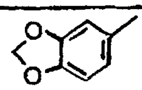 | 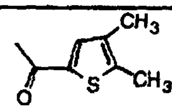 | 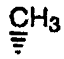 | 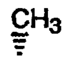 | $-CH_2-$ | MH+ = 560 |
| 521 | 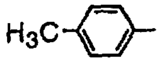 | 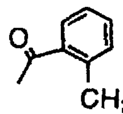 | H | 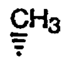 | -O- | m.p. = 197-199°C |
| 522 | 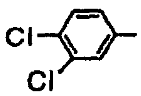 | 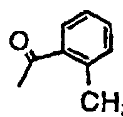 | H | 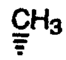 | -O- | m.p. = 175-177°C |
| 523 | 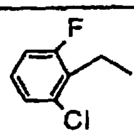 | 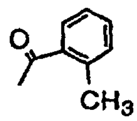 | H | 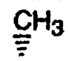 | -O- | m.p. = 195-197°C |
| 524 | 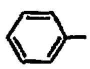 | 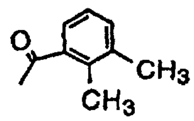 | H | 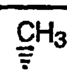 | -O- | m.p. = 60-62°C |
| 525 | 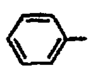 | 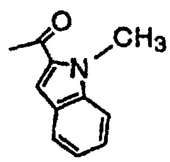 | H | 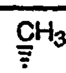 | -O- | m.p. = 196-200°C (isomer A) |
| 526 | 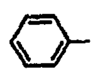 | 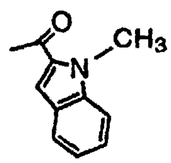 | H | 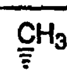 | -O- | FAB(MH+) = 523 (isomer B) |
| 527 | $H_3CO-$ | 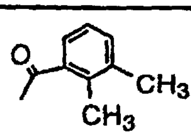 | H | H | 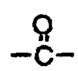 | HRMS (FAB) calc'd ($MH^+$): 450.2757 Found: 450.2752 m.p. = 245-260°C |
| 559 | 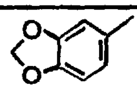 | 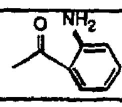 | H | H | $-SO_2-$ | m.p.= decomp above 115°C |

| | | | | | | |
|---|---|---|---|---|---|---|
| 560 | 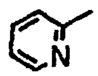 | 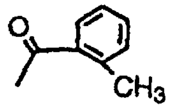 | $CH_3$ | $CH_3$ | OH<br>-C-<br>H | m.p.= 161-166°C |
| 561 | 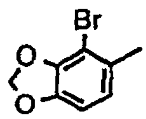 | 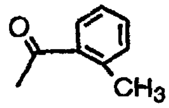 | $CH_3$ | $CH_3$ | OH<br>-C-<br>H | Mass. Spec.<br>(FAB)M+1 = 635 |
| 562 | 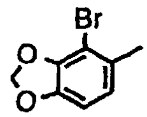 | 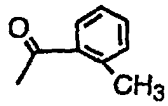 | $CH_3$ | $CH_3$ | $-CH_2-$ | HRMS<br>calc'd: 618.2331<br>found: 618.2316 |
| 592 | 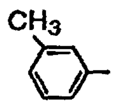 | 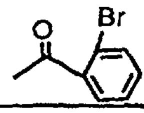 | $-CF_3$ | H | $-SO_2-$ | HRMS<br>calc'd: 664.1456<br>found: 664.1452 |
| 593 | 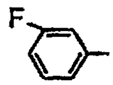 | 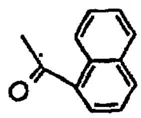 | $CH_3$ | $CH_3$ | OH<br>-C-<br>H | HRMS<br>calc'd: 566.3183<br>found: 566.3180 |

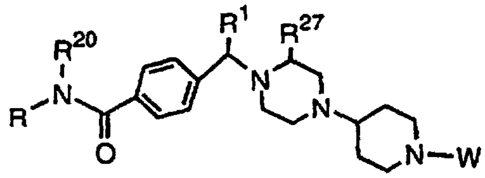

| No. | R | W | $R^1$ | $R^{27}$ | $R^{20}$ | Physical Data |
|---|---|---|---|---|---|---|
| 310 | 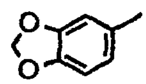 | 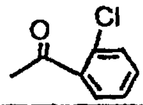 | 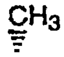 | 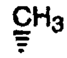 | $-CH_3$ | MH+ = 603 |
| 311 | 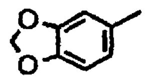 | 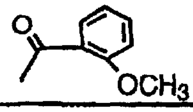 | 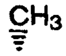 | 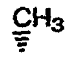 | $-CH_3$ | MH+ = 599 |
| 312 | 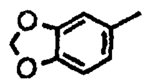 | 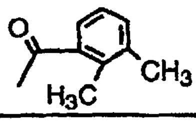 | 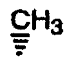 | 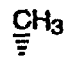 | $-CH_3$ | MH+ = 597 |
| 313 | 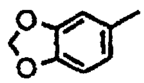 | 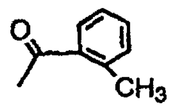 | 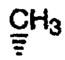 | 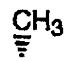 | 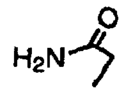 | MH+ = 626 |
| 314 | 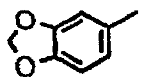 | 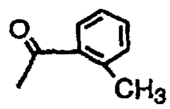 | 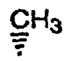 | 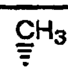 | 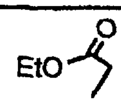 | MH+ = 655 |
| 315 | 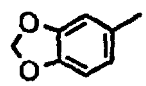 | 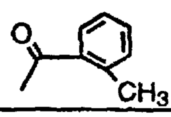 | 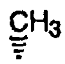 | 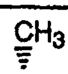 | n-Pr | MH+ = 611 |

| | | | | | | |
|---|---|---|---|---|---|---|
| 316 | 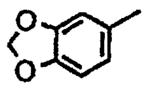 | 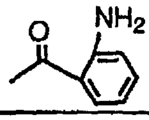 | 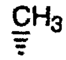 | 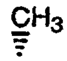 | -$CH_3$ | MH+ = 584 |
| 317 | 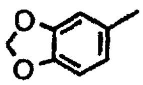 | 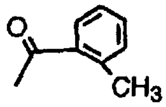 | H | H | -$CH_3$ | HRMS calc'd: 555.2971 found: 555.2983 m.p.=235-240°C |
| 318 | 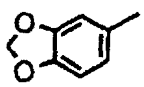 | 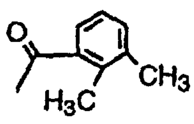 | H | H | -$CH_3$ | HRMS calc'd: 569.3128 found: 569.3132 m.p.=170-175°C |
| 319 | 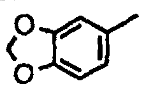 | 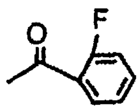 | H | H | -$CH_3$ | HRMS calc'd: 559.2721 found: 559.2732 m.p.=192-198°C |
| 320 | 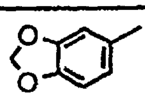 | 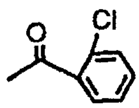 | H | H | -$CH_3$ | HRMS calc'd: 575.2425 found: 575.2418 m.p.=205-210°C |
| 321 | 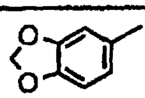 | 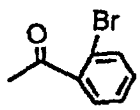 | H | H | -$CH_3$ | HRMS calc'd: 619.20 found: 619.1911 m.p.=170-180°C |
| 322 | 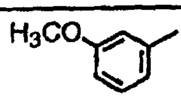 | 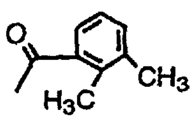 | H | H | -$CH_3$ | HRMS calc'd: 555.3355 found: 555.3355 m.p.=175-185°C |
| 323 | 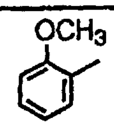 | 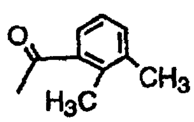 | H | H | -$CH_3$ | HRMS calc'd: 555.3335 found: 555.3330 m.p.=180-190°C |
| 324 | 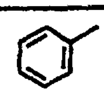 | 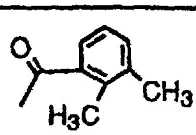 | H | H | -$CH_3$ | HRMS calc'd: 525.3230 found: 525.3224 m.p.=205-210°C |
| 325 | 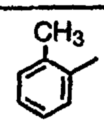 | 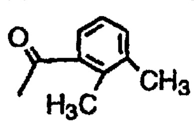 | H | H | -$CH_3$ | HRMS calc'd: 539.3386 found: 539.3397 m.p.=228-237°C |
| 326 | 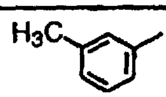 | 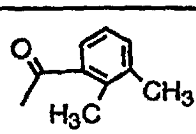 | H | H | -$CH_3$ | HRMS calc'd: 539.3386 found: 539.3396 m.p.=234-239°C |

| | | | | | | |
|---|---|---|---|---|---|---|
| 327 | 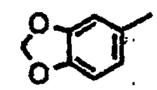 | 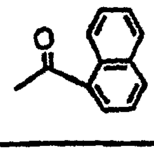 | H | H | $-CH_3$ | HRMS calc'd: 591.2971 found: 591.2966 m.p.=195-200°C |
| 329 | 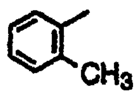 | 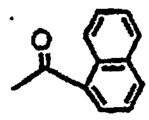 | H | H | $-CH_3$ | m.p. - 185-190°C (dec) |
| 330 | 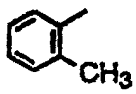 | 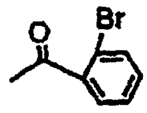 | H | H | $-CH_3$ | m.p. - 240-248°C (dec) |
| 331 | 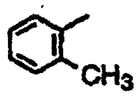 | 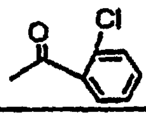 | H | H | $-CH_3$ | m.p. - 235-240°C (dec) |
| 332 | 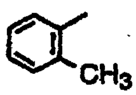 | 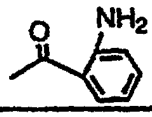 | H | H | $-CH_3$ | m.p. - 185-195°C (dec) |
| 333 | 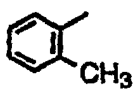 | 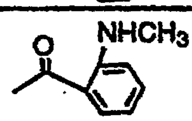 | H | H | $-CH_3$ | m.p. - 175-195°C (dec) |
| 334 | 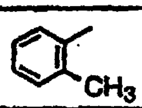 | 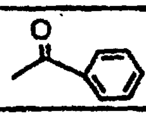 | H | H | $-CH_3$ | m.p. - 230-235°C (dec) |
| 335 | 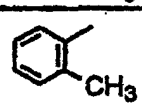 | 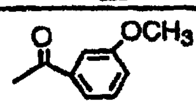 | H | H | $-CH_3$ | m.p. - 245-255°C (dec) |
| 336 | 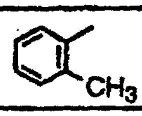 | 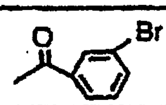 | H | H | $-CH_3$ | m.p. - 250-255°C (dec) |

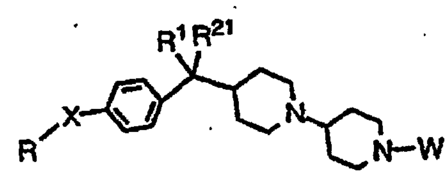

| No. | R | W | $R^1$, $R^{21}$ | X | Physical Data |
|---|---|---|---|---|---|
| 337 | 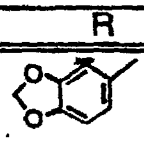 | 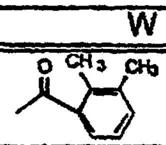 | 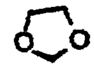 | $-CH_2-$ | LRMS: calc'd: 582 found: 583 |
| 338 | 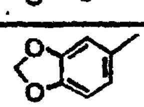 | 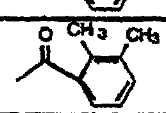 | =O | $-CH_2-$ | LRMS: calc'd: 538 found: 539 |
| 339 | 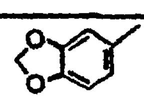 | 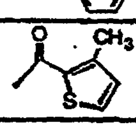 | 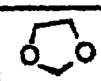 | $-CH_2-$ | LRMS: calc'd: 574 found: 575 |

| | | | | | |
|---|---|---|---|---|---|
| 340 | 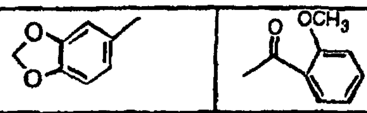 | | 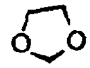 | $-CH_2-$ | LRMS: calc'd: 584 found: 585 |
| 341 | 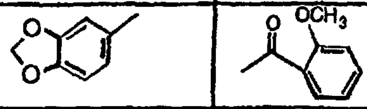 | | =O | $-CH_2-$ | LRMS: calc'd: 540 found: 541 |
| 342 | 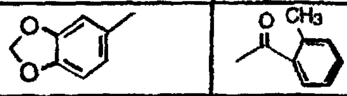 | | H, OH | $-CH_2-$ | LRMS: calc'd: 526 found: 527 |
| 436 | 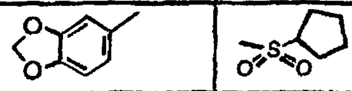 | | 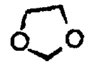 | $-SO_2-$ | LRMS: calc'd: 632 found: 633 |
| 437 | 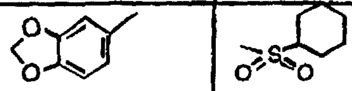 | | 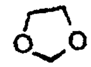 | $-SO_2-$ | LRMS: calc'd: 646 found: 647 |
| 438 | 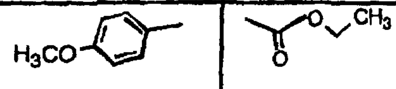 | | 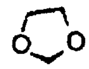 | $-SO_2-$ | LRMS: calc'd: 558 found (M+H): 559 |
| 439 | 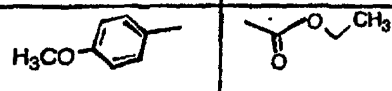 | | 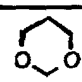 | $-SO_2-$ | LRMS: calc'd: 572 found (M+H): 573 |
| 440 | 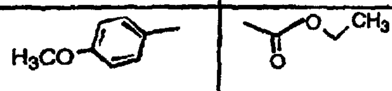 | | 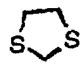 | $-SO_2-$ | LRMS (FAB): calc'd (M+H): 591 found: 591 |
| 441 | 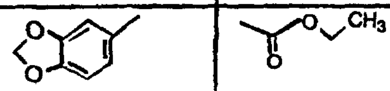 | | 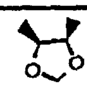 | $-SO_2-$ | LRMS (FAB): calc'd: 600 found (M+H): 601 |
| 442 | 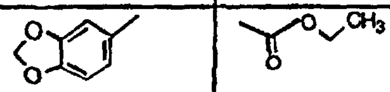 | | 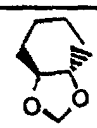 | $-SO_2-$ | LRMS (FAB): calc'd: 626 found (M+H): 627 |
| 443 | 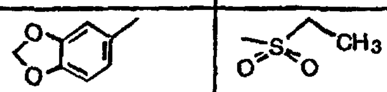 | | 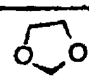 | $-CH_2-$ | LRMS: calc'd: 592 found (M+H): 593 |
| 444 | 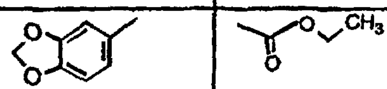 | | 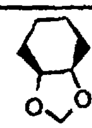 | $-SO_2-$ | LRMS: calc'd: 626 found (M+H): 627 |
| 445 | 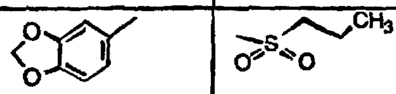 | | 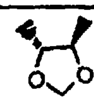 | $-SO_2-$ | LRMS: calc'd: 634 found (M+H): 635 |
| 446 | 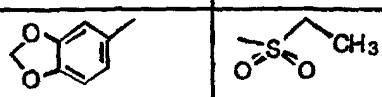 | | 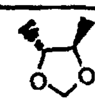 | $-SO_2-$ | LRMS: calc'd: 620 found (M+H): 621 |
| 447 | 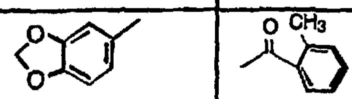 | | 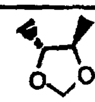 | $-SO_2-$ | LRMS: calc'd: 646 found (M+H): 647 |
| 448 | 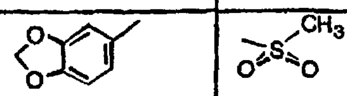 | | 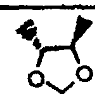 | $-SO_2-$ | LRMS: calc'd: 606 found (M+H): 607 |
| 449 | 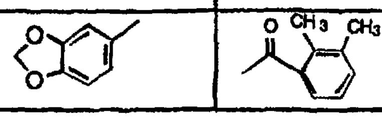 | | 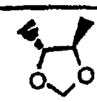 | $-SO_2-$ | LRMS: calc'd: 660 found (M+H): 661 |

| | | | | | |
|---|---|---|---|---|---|
| 450 | 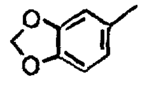 | 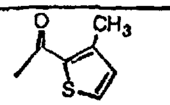 | 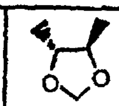 | $-SO_2-$ | LRMS:<br>calc'd: 652<br>found (M+H): 653 |
| 451 | 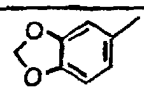 | 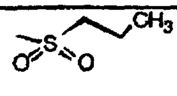 | 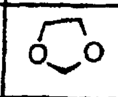 | -SO- | LRMS:<br>calc'd: 592<br>found (M+H): 593 |
| 452 | 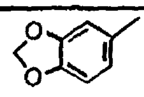 | 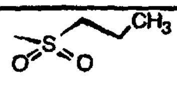 | 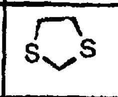 | -SO- | LRMS:<br>calc'd: 622<br>found (M+H): 623 |
| 453 | 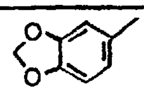 | 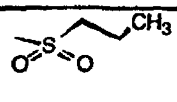 | 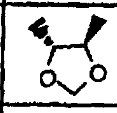 | -SO- | LRMS:<br>calc'd: 618<br>found (M+H): 619 |
| 454 | 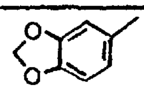 | 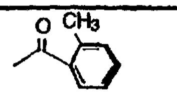 | 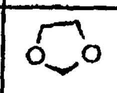 | -SO- | LRMS:<br>calc'd: 602<br>found (M+H): 603 |
| 455 | 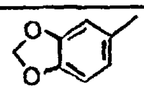 | 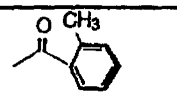 | 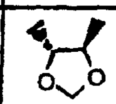 | -SO- | LRMS:<br>calc'd: 630<br>found (M+H): 631 |
| 456 | 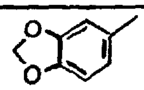 | 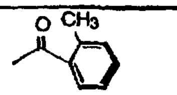 | 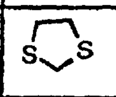 | $-SO_2-$ | LRMS:<br>calc'd: 650<br>found (M+H): 651 |
| 457 | 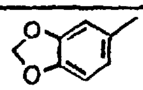 | 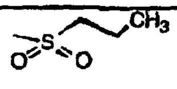 | | $-SO_2-$ | LRMS:<br>calc'd: 638<br>found (M+H): 639 |
| 458 | 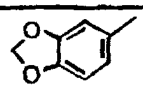 | 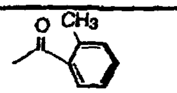 | | -SO- | LRMS:<br>calc'd: 634<br>found (M+H): 635 |
| 459 | 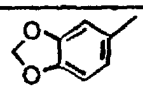 | 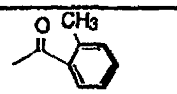 | | -S- | LRMS:<br>calc'd: 586<br>found (M+H): 587 |
| 460 | 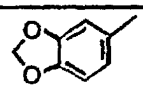 | 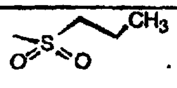 | | -S- | LRMS:<br>calc'd: 574<br>found (M+H): 575 |
| 461 | 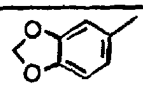 | 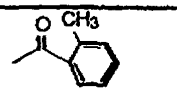 | | -S- | LRMS:<br>calc'd: 614<br>found (M+H): 615 |
| 462 | 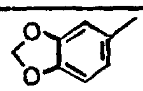 | 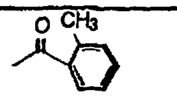 | | -S- | LRMS:<br>calc'd: 618<br>found (M+H): 619 |
| 463 | 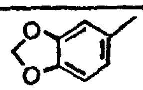 | 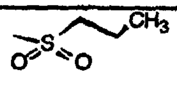 | | -S- | LRMS:<br>calc'd: 606<br>found (M+H): 607 |
| 464 | 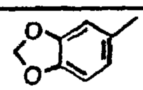 | 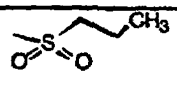 | | -S- | LRMS:<br>calc'd: 602<br>found (M+H): 603 |
| 465 | 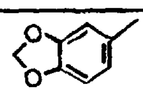 | 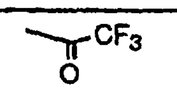 | | $-SO_2-$ | LRMS:<br>calc'd: 596<br>found (M+H): 597 |
| 466 | 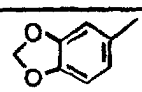 | H | | $-SO_2-$ | LRMS:<br>calc'd: 500<br>found (M+H): 501 |

| | | | | | |
|---|---|---|---|---|---|
| 467 | 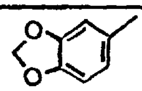 | 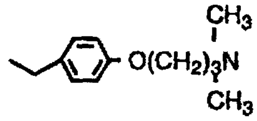 | 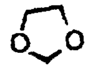 | $-SO_2-$ | LRMS: calc'd: 691 found (M+H): 692 |
| 468 | 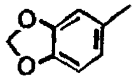 | 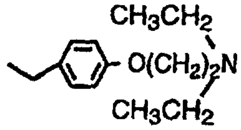 | 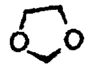 | $-SO_2-$ | LRMS: calc'd: 705 found (M+H): 706 |
| 469 | 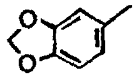 | 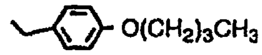 | 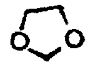 | $-SO_2-$ | LRMS: calc'd: 662 found (M+H):663 |
| 470 | 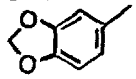 | 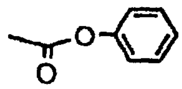 | 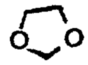 | $-SO_2-$ | LRMS: calc'd: 620 found (M+H):621 |
| 471 | 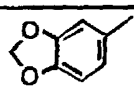 | 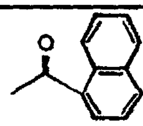 | 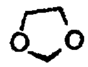 | $-SO_2-$ | LRMS: calc'd: 654 found (M+H): 655 |
| 472 | 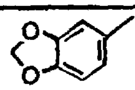 | 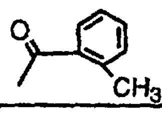 | 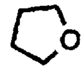 | -S- | LRMS: calc'd: 584 found (M+H): 585 |
| 473 | 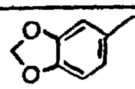 | 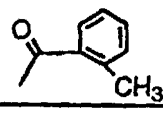 | 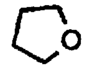 | -SO- | LRMS: calc'd: 600 found (M+H): 601 |
| 474 | 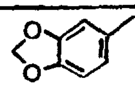 | 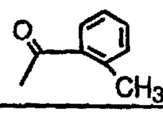 | 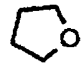 | $-SO_2-$ | LRMS: calc'd: 616 found (M+H): 617 |
| 475 | 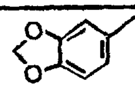 | 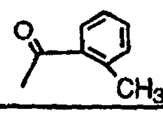 | OH, $(CH_2)_3OH$ | -SO- | LRMS: calc'd: 618 found (M+H): 619 |
| 476 | 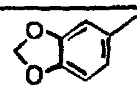 | 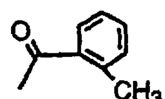 | OH, $(CH_2)_3OH$ | $-SO_2-$ | LRMS: calc'd: 634 found (M+H): 635 |
| 477 | 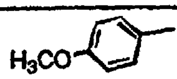 | 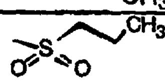 | 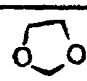 | $-SO_2-$ | LRMS: calc'd: 592 found (M+H): 593 |
| 478 | 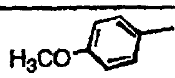 | 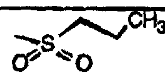 | 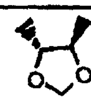 | $-SO_2-$ | LRMS: calc'd: 620 found (M+H): 621 |
| 479 | 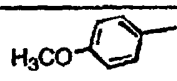 | 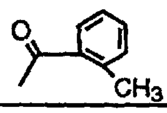 | 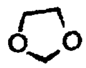 | $-SO_2-$ | LRMS: calc'd: 604 found (M+H): 605 |
| 480 | 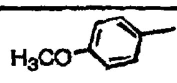 | 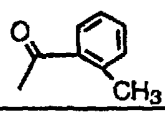 | 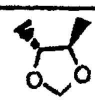 | $-SO_2-$ | LRMS: calc'd: 632 found (M+H): 633 |

| | | | | | |
|---|---|---|---|---|---|
| 481 | 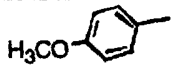 | 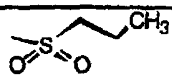 | 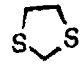 | $-SO_2-$ | LRMS: calc'd: 625 found (M+H): 625 |
| 482 | 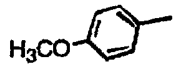 | 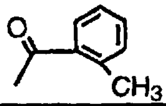 | 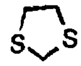 | $-SO_2-$ | LRMS: calc'd: 636 found (M+H): 637 |
| 483 | 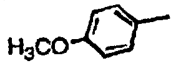 | 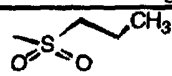 | 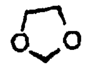 | -SO- | LRMS: calc'd: 576 found (M+H): 577 |
| 484 | 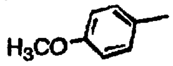 | 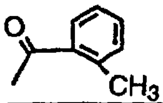 | 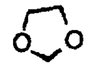 | -SO- | LRMS: calc'd: 588 found (M+H): 589 |
| 488 | 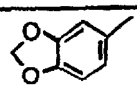 | 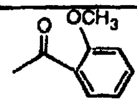 | =O | $-SO_2-$ | LRMS: calc's: 590 found: 591 |
| 489 | 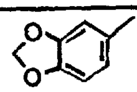 | 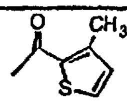 | =O | $-SO_2-$ | LRMS: calc's: 580 found: 581 |
| 490 | 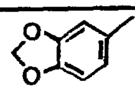 | 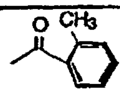 | H, OH | $-SO_2-$ | LRMS: calc's: 576 found: 577 |
| 491 | 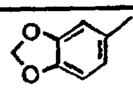 | 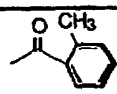 | CH3, OH | $-SO_2-$ | LRMS: calc's: 590 found: 591 |
| 492 | 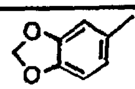 | 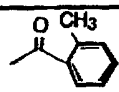 | 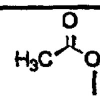 | $-SO_2-$ | LRMS: calc's: 618 found: 619 |
| 493 | 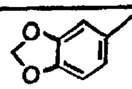 | 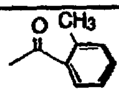 | =O | -S- | LRMS: calc's: 542 found (M+H): 543 |
| 502 | 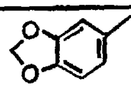 | 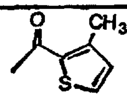 | H, H | $-SO_2-$ | LRMS: calc's: 566 found: 567 |
| 503 | 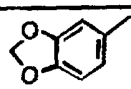 | 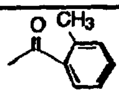 | H, H | $-SO_2-$ | LRMS: calc's: 576 found: 577 |
| 504 | 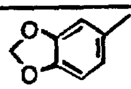 | 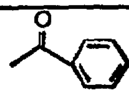 | H, H | $-SO_2-$ | LRMS: calc's: 546 found: 547 |
| 505 | 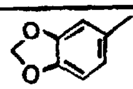 | 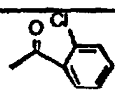 | H, H | $-SO_2-$ | LRMS: calc's: 581 found: 581 |
| 506 | 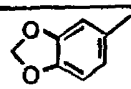 | 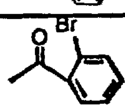 | H, H | $-SO_2-$ | LRMS: calc's: 625 found: 626 |
| 507 | 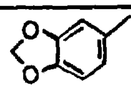 | 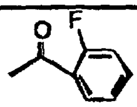 | H, H | $-SO_2-$ | LRMS: calc's: 564 found: 565 |
| 508 | 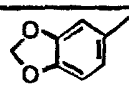 | 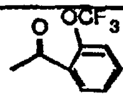 | H, H | $-SO_2-$ | LRMS: calc's: 630 found: 631 |

| | | | | | |
|---|---|---|---|---|---|
| 509 | 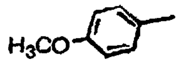 | 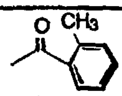 | H, H | $-SO_2-$ | LRMS:<br>calc's: 546<br>found: 547 |
| 510 | 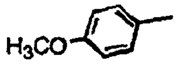 | 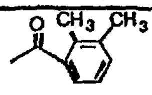 | H, H | $-SO_2-$ | LRMS:<br>calc's: 560<br>found: 561 |
| 511 | 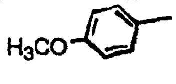 | 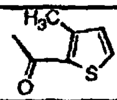 | H, H | $-SO_2-$ | LRMS:<br>calc's: 552<br>found: 553 |
| 512 | 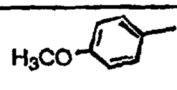 | 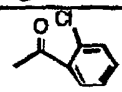 | H, H | $-SO_2-$ | LRMS:<br>calc's: 567<br>found: 567 |
| 513 | 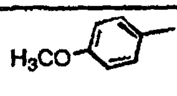 | 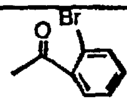 | H, H | $-SO_2-$ | LRMS:<br>calc's:611.6<br>found: 613 |
| 514 | 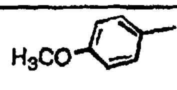 | 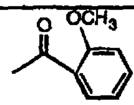 | H, H | $-SO_2-$ | LRMS:<br>calc's:562<br>found: 563 |
| 515 | 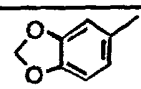 | 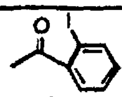 | H, H | $-SO_2-$ | LRMS:<br>calc's:672<br>found:673 |
| 516 | 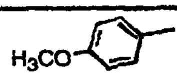 | 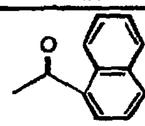 | H, H | $-SO_2-$ | LRMS:<br>calc's:582<br>found:583 |
| 517 | 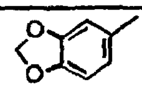 | 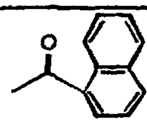 | H, H | $-SO_2-$ | LRMS:<br>calc's:596<br>found:597 |
| 518 | 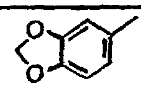 | 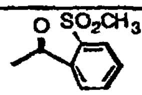 | H, H | $-SO_2-$ | LRMS:<br>calc's:624<br>found:625 |
| 519 | 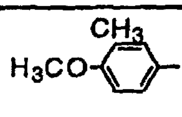 | 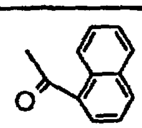 | H, H | $-SO_2-$ | LRMS:<br>calc's:596<br>found<br>$(M+H^+)$:597 |
| 563 | $(CH_3)_2CH_2-$ | 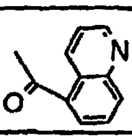 | H, H | -S- | HRMS:<br>calc'd (M+1):<br>488.2736<br>found: 488.2728 |
| 564 | $(CH_3)_2CH_2-$ | 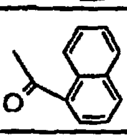 | H, H | $-SO_2-$ | HRMS:<br>calc'd (M+1):<br>519.2681<br>found: 519.2685 |
| 565 | $(CH_3)_2CH_2-$ | 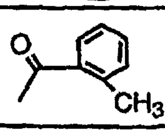 | H, H | $-SO_2-$ | HRMS:<br>calc'd (M+1):<br>483.2681<br>found: 483.2688 |
| 566 | $(CH_3)_2CH_2-$ | 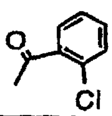 | H, H | $-SO_2-$ | HRMS:<br>calc'd (M+1):<br>503.2135<br>found: 503.2144 |

| | | | | | |
|---|---|---|---|---|---|
| 567 | $(CH_3)_2CH_2$- | 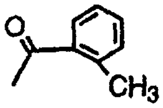 | H, H | -S- | HRMS: calc'd (M+1): 451.2873 found: 451.2870 |
| 568 | $(CH_3)_2CH_2$- | 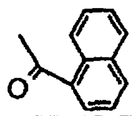 | H, H | $-CH_2-$ | HRMS: calc'd (M+1): 469.3219 found: 469.3219 |
| 569 | $(CH_3)_2CH_2$- | 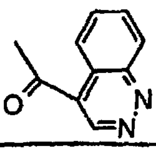 | H, H | -S- | HRMS: calc'd (M+1): 489.2688 found: 489.2687 |
| 570 | $(CH_3)_2CH_2$- | 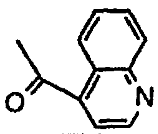 | H, H | -S- | HRMS: calc'd (M+1): 488.2736 found: 488.2739 |
| 571 | 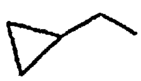 | 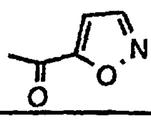 | H, H | -O- | HRMS: calc'd (M+1): 424.2600 found: 424.2608 |
| 572 | $-CH_3$ | 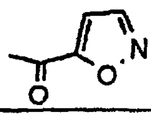 | H, H | -O- | HRMS: calc'd (M+1): 384.2287 found: 384.2294 |
| 573 | $(CH_3)_2CH_2$- | 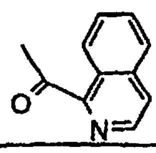 | H, H | -S- | HRMS: calc'd (M+1): 488.2736 found: 488.2751 |
| 574 | $(CH_3)_2CH_2$- | 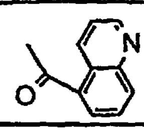 | H, H | -O- | HRMS: calc'd (M+1): 472.2964 found: 472.2964 |
| 575 | $(CH_3)_2CH_2$- | 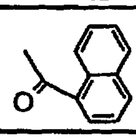 | H, H | -SO- | HRMS: calc'd (M+1): 503.2732 found: 503.2723 |
| 576 | $CH_3CH_2SO_2$- | 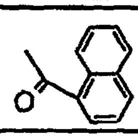 | H, H | -NH- | LRMS: calc'd: 519 found ($M+H^+$): 520 |
| 577 | $-CH_3$ | 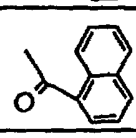 | H, H | -NH- | LRMS: calc'd: 441 found ($M+H^+$): 442 |
| 578 | 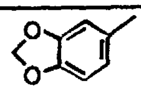 | 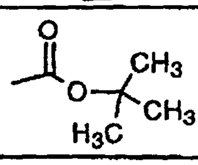 | 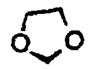 | =O | LRMS: calc'd: 564 found: 565 |

| | | | | | |
|---|---|---|---|---|---|
| 579 | 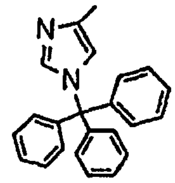 | 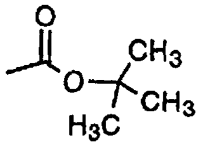 | 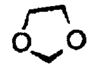 | 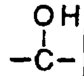 | LRMS: calc'd: 754 found: 755 |
| 580 | 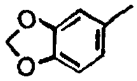 | 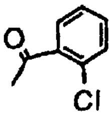 | 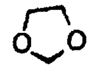 | =O | LRMS: calc'd: 603 found: 603 |
| 581 | 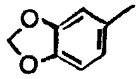 | $-SO_2(CH_2)_2CH_3$ | 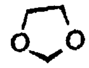 | =O | LRMS: calc'd: 570 found: 571 |
| 582 | 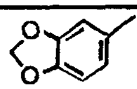 | 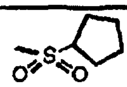 | 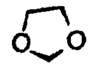 | =O | LRMS: calc'd: 596 found: 597 |
| 583 | 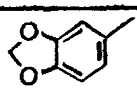 | 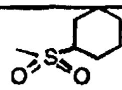 | 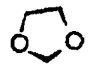 | =O | LRMS: calc'd: 610 found: 611 |
| 584 | 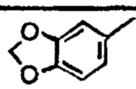 | 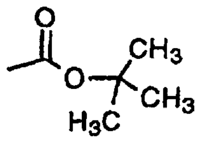 | 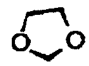 | 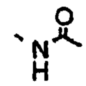 | LRMS: calc'd: 579 found: 580 |
| 585 | 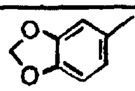 | 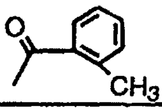 | H, OH | =O | LRMS: calc'd: 528 found: 529 |
| 586 | 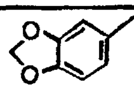 | 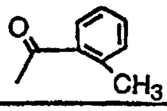 | H, H | =O | LRMS: calc'd: 512 found: 513 |
| 590 | 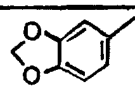 | 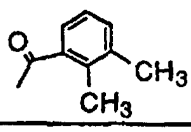 | H, H | $-SO_2-$ | LRMS: calc'd: 574 found: 575 |
| 591 | 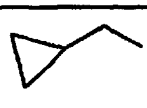 | 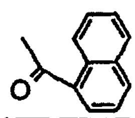 | H, H | -O- | HRMS: calc'd (M+1): 483.3012 found: 483.3008 |

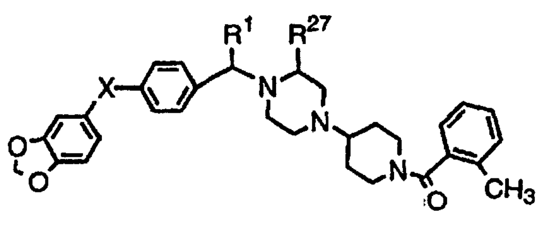

| No. | X | $R^1$ | $R^{27}$ | Physical Data |
|---|---|---|---|---|
| 533 | 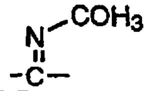 | H | H | MS (fFAB): 555.4 (M+1), 391,307 |

| | | | | |
|---|---|---|---|---|
| 534 | 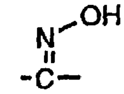 | $CH_3$ | $CH_3$ | MS (fFAB): 569.4 (M+1), 553,302 |
| 535 | 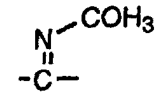 | $CH_3$ | $CH_3$ | MS (fFAB): 583.4 (M+1), 302, 282 |
| 536 | 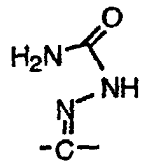 | $CH_3$ | $CH_3$ | MS (fFAB): 611 (M+1), 554<br>(isomer A) |
| 537 | 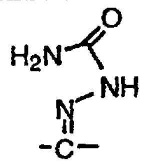 | $CH_3$ | $CH_3$ | MS (fFAB): 611 (M+1), 325<br>(isomer B) |
| 538 | 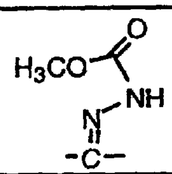 | $CH_3$ | $CH_3$ | MS (fFAB): 626.4 (M+1), 554 |
| 539 | 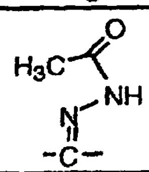 | $CH_3$ | $CH_3$ | MS (fFAB): 610.3 (M+1), 460<br>(isomer A) |
| 540 | 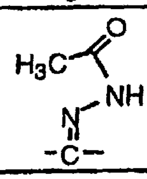 | $CH_3$ | $CH_3$ | MS (fFAB): 610.2 (M+1), 554, 309<br>(isomer B) |
| 541 | 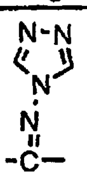 | $CH_3$ | $CH_3$ | MS (electrospray): 620 (M+1), 551,433 (isomer A) |
| 542 | 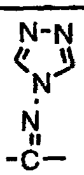 | $CH_3$ | $CH_3$ | MS (electrospray): 620 (M+1), 551,433 (isomer B) |
| 543 | 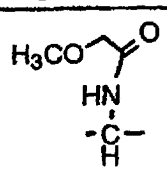 | H | H | MS (fFAB): 599.4 (M+1), 510, 277 |

| | | | | |
|---|---|---|---|---|
| 544 | 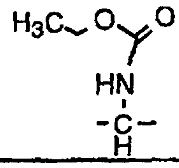 | 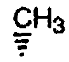 | 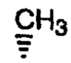 | MS (fFAB): 627.4 (M+1), 326, 302 |
| 545 | 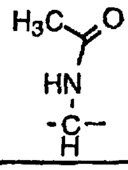 | 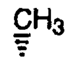 | 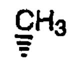 | MS (fFAB): 597.5 (M+1), 302 |
| 546 | 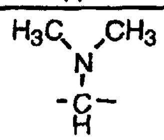 | H | H | MS (electrospray): 555 (M+1), 510,392 |
| 547 | 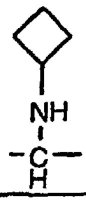 | H | H | MS (fFAB): 581.4 (M+1), 510 |
| 548 | 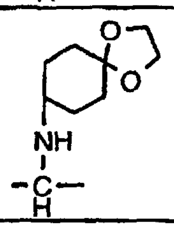 | H | H | MS (fFAB): 667.4 (M+1), 510 |
| 549 | 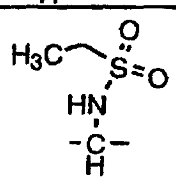 | H | H | MS (electrospray): 619.2 (M+1), 510 |
| 550 | 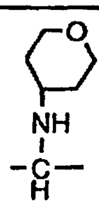 | H | H | FAB(MH+) = 611 |
| 551 | 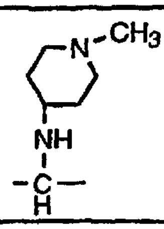 | H | H | MS (electrospray): 624.2 (M+1), 510 |
| 552 | 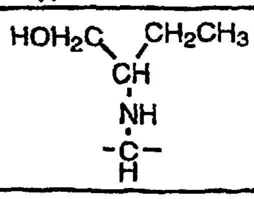 | H | H | MS (electrospray): 599.2 (M+1), 510 |

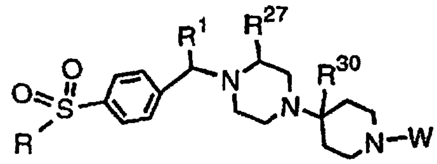

| No. | R | W | $R^1$ | $R^{27}$ | $R^{30}$ | Physical Data |
|---|---|---|---|---|---|---|
| 343 | 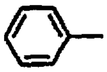 | 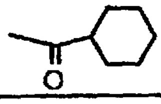 | H | H | $-CH_3$ | MS (electrospray): 524.1 (M+1), 373 |
| 344 | 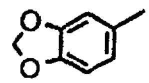 | 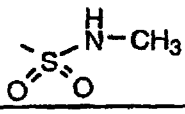 | $CH_3$ | $CH_3$ | $-CH_3$ | MH+ = 579 |
| 345 | 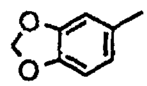 | 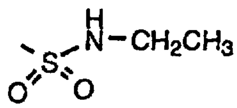 | $CH_3$ | $CH_3$ | $-CH_3$ | MH+ = 593 |
| 346 | 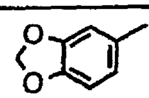 | 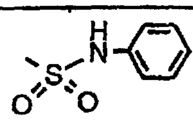 | $CH_3$ | $CH_3$ | $-CH_3$ | MH+ = 641 |
| 347 | 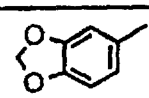 | 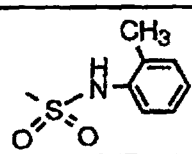 | $CH_3$ | $CH_3$ | $-CH_3$ | MH+ = 655 |
| 348 | 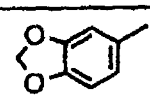 | 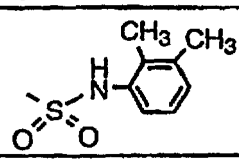 | $CH_3$ | $CH_3$ | $-CH_3$ | MH+ = 669 |
| 349 | 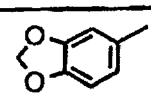 | 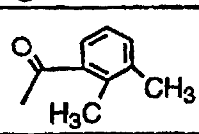 | $CH_3$ | $CH_3$ | $-CH_3$ | FAB (MH+) = 618 |
| 350 | 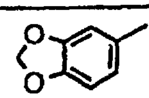 | 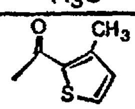 | $CH_3$ | $CH_3$ | $-CH_3$ | FAB (MH+) = 610 |
| 351 | 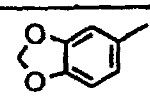 | 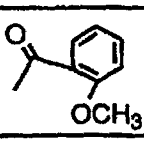 | $CH_3$ | $CH_3$ | $-CH_3$ | FAB (MH+) = 620 |
| 352 | 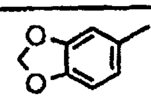 | 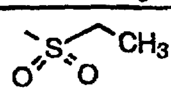 | $CH_3$ | $CH_3$ | $-CH_3$ | FAB (MH+) = 578 |
| 353 | 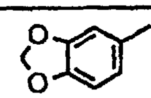 | 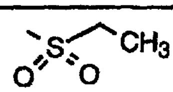 | $CH_3$ | $CH_3$ | 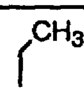 | FAB (MH+) = 592 |
| 354 | 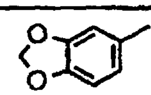 | 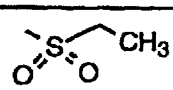 | $CH_3$ | $CH_3$ | 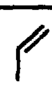 | FAB (MH+) = 590 |

| | | | | | | |
|---|---|---|---|---|---|---|
| 355 | 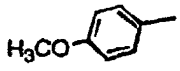 | 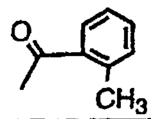 | | | $-CH_3$ | m.p. = 233-235°C (dec) |
| 356 | 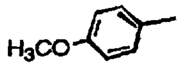 | 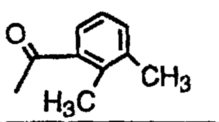 | | | $-CH_3$ | m.p. = 237-239°C (dec) |
| 357 | 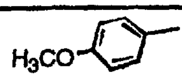 | 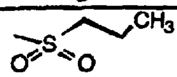 | | | $-CH_3$ | m.p. = 228-229°C (dec) |
| 358 | 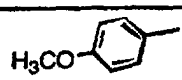 | 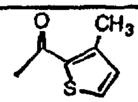 | | | $-CH_3$ | m.p. = 231-233°C (dec) |
| 359 | 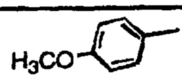 | 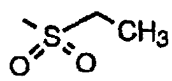 | | | $-CH_3$ | FAB(MH+) = 564 |
| 360 | 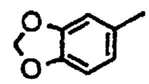 | 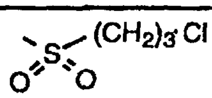 | | | $-CH_3$ | m.p. = 225-226°C (dec) |
| 361 | 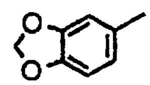 | 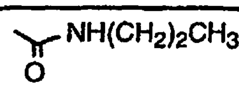 | | | $-CH_3$ | FAB(MH+) = 571 |
| 362 | 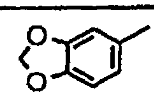 | 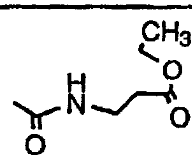 | | | $-CH_3$ | FAB(MH+) = 629 |
| 363 | 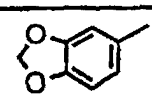 | 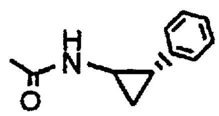 | | | $-CH_3$ | FAB(MH+) = 645 |
| 364 | 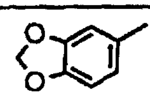 | 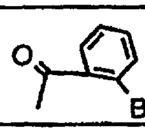 | | | $-CH_3$ | FAB(MH+) = 670,668 |
| 365 | 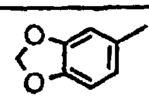 | 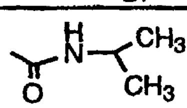 | | | $-CH_3$ | FAB(MH+) = 571 |
| 366 | 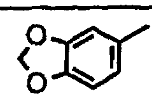 | 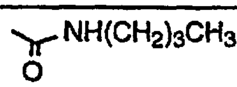 | | | $-CH_3$ | FAB(MH+) = 585 |
| 367 | 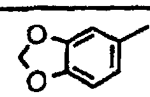 | 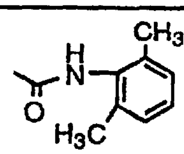 | | | $-CH_3$ | FAB(MH+) = 632 |

| | | | | | | |
|---|---|---|---|---|---|---|
| 368 | 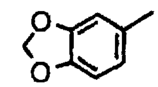 | 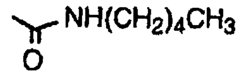 | | | -$CH_3$ | FAB(MH+) = 613 |
| 369 | 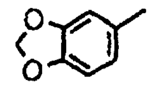 | 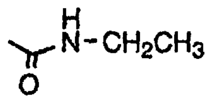 | | | -$CH_3$ | m.p. = 207-208°C (dec) |
| 370 | 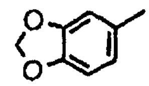 | 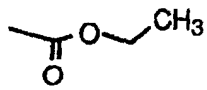 | | | -$CH_3$ | m.p. = 229-220°C (dec) |
| 371 | 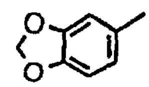 | 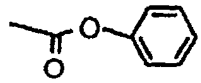 | | | -$CH_3$ | m.p. = 223-224°C (dec) |
| 372 | 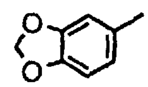 | 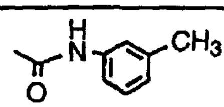 | | | -$CH_3$ | FAB(MH+) = 619 |
| 373 | 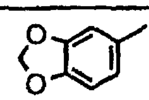 | 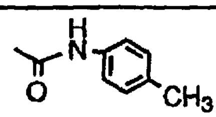 | | | -$CH_3$ | FAB(MH+) = 619 |
| 374 | 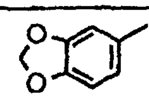 | 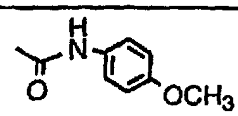 | | | -$CH_3$ | FAB(MH+) = 635 |
| 375 | 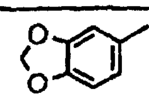 | 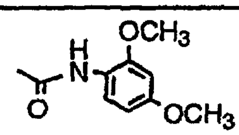 | | | -$CH_3$ | FAB(MH+) = 665 |
| 376 | 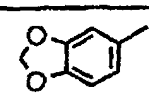 | 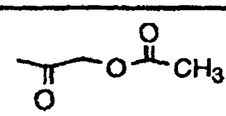 | | | -$CH_3$ | FAB(MH+) = 586 |
| 377 | 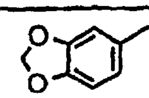 | 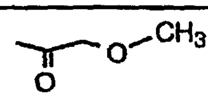 | | | -$CH_3$ | FAB(MH+) = 558 |
| 378 | 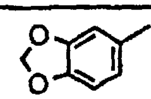 | 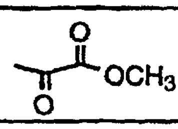 | | | -$CH_3$ | FAB(MH+) = 572 |
| 379 | 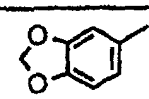 | 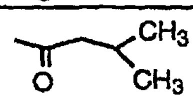 | | | -$CH_3$ | FAB(MH+) = 570 |
| 380 | 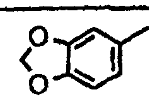 | 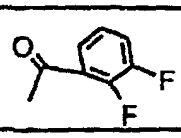 | | | -$CH_3$ | FAB(MH+) = 626 |
| 381 | 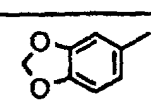 | 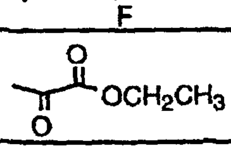 | | | -$CH_3$ | FAB(MH+) = 586 |

| | | | | | | |
|---|---|---|---|---|---|---|
| 382 | 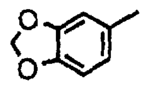 | 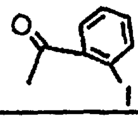 | $CH_3$ | $CH_3$ | $-CH_3$ | FAB(MH+) = 716 |
| 383 | 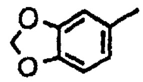 | 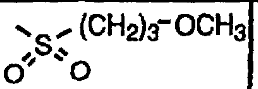 | $CH_3$ | $CH_3$ | $-CH_3$ | m.p. = 230-231°C (dec) |
| 384 | 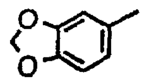 | 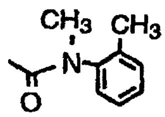 | $CH_3$ | $CH_3$ | $-CH_3$ | m.p. = 244-245°C (dec) |
| 385 | 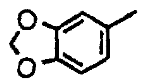 | 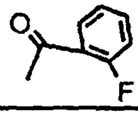 | $CH_3$ | $CH_3$ | $-CH_3$ | FAB(MH+) = 608 |
| 386 | 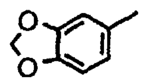 | 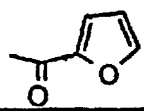 | $CH_3$ | $CH_3$ | $-CH_3$ | FAB(MH+) = 580 |
| 387 | 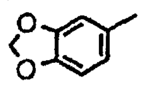 | 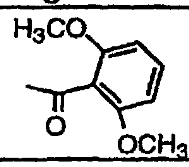 | $CH_3$ | $CH_3$ | $-CH_3$ | FAB(MH+) = 650 |
| 388 | 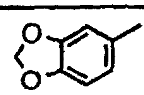 | 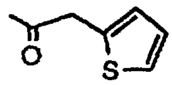 | $CH_3$ | $CH_3$ | $-CH_3$ | FAB(MH+) = 610 |
| 389 | 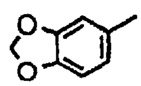 | 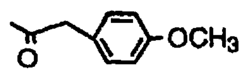 | $CH_3$ | $CH_3$ | $-CH_3$ | FAB(MH+) = 633 |
| 390 | 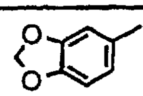 | 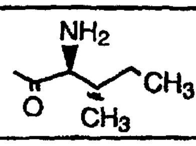 | $CH_3$ | $CH_3$ | $-CH_3$ | FAB(MH+) = 599 |
| 391 | 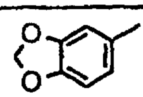 | 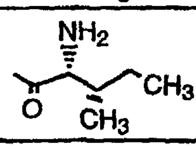 | $CH_3$ | $CH_3$ | $-CH_3$ | FAB(MH+) = 599 |
| 392 | 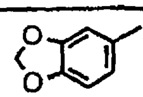 | 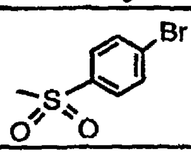 | $CH_3$ | $CH_3$ | $-CH_3$ | m.p. = 230-231°C (dec) |
| 393 | 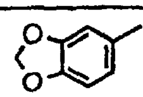 | 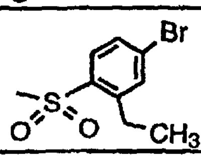 | $CH_3$ | $CH_3$ | $-CH_3$ | m.p. = 225-226°C (dec) |
| 394 | 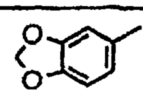 | 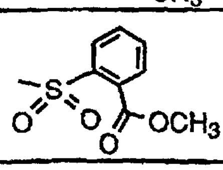 | $CH_3$ | $CH_3$ | $-CH_3$ | m.p. = 208-209°C (dec) |

| | | | | | | |
|---|---|---|---|---|---|---|
| 395 | 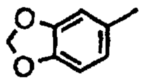 | 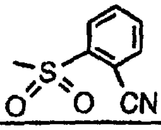 | $CH_3$ | $CH_3$ | $-CH_3$ | m.p. = 218-219°C (dec) |
| 396 | 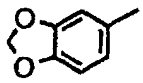 | 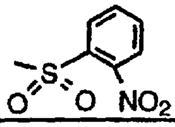 | $CH_3$ | $CH_3$ | $-CH_3$ | m.p. = 218-219°C (dec) |
| 397 | 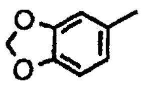 | 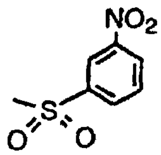 | $CH_3$ | $CH_3$ | $-CH_3$ | m.p. = 229-230°C (dec) |
| 398 | 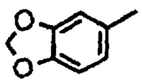 | 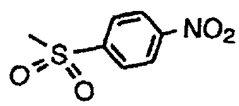 | $CH_3$ | $CH_3$ | $-CH_3$ | m.p. = 229-230°C (dec) |
| 399 | 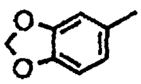 | 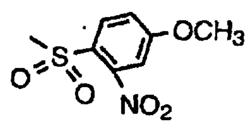 | $CH_3$ | $CH_3$ | $-CH_3$ | m.p. = 215-216°C (dec) |
| 400 | 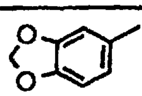 | 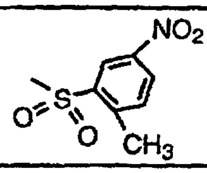 | $CH_3$ | $CH_3$ | $-CH_3$ | m.p. = 220-221°C (dec) |
| 401 | 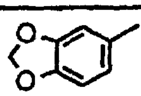 | 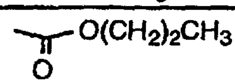 | $CH_3$ | $CH_3$ | $-CH_3$ | m.p. = 224-225°C (dec) |
| 402 | 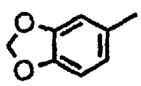 | 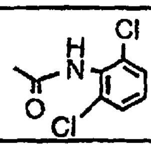 | $CH_3$ | $CH_3$ | $-CH_3$ | FAB(MH+) = 673 |
| 403 | 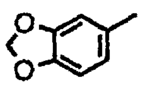 | 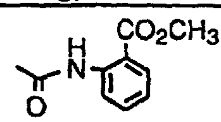 | $CH_3$ | $CH_3$ | $-CH_3$ | FAB(MH+) = 663 |
| 404 | 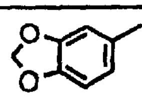 | 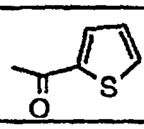 | $CH_3$ | $CH_3$ | $-CH_3$ | FAB(MH+) = 596 |
| 405 | 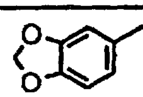 | 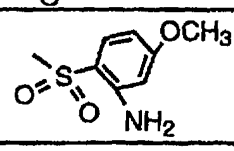 | $CH_3$ | $CH_3$ | $-CH_3$ | FAB(MH+) = 671 |
| 406 | 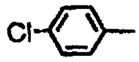 | 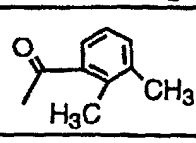 | $CH_3$ | $CH_3$ | $-CH_3$ | FAB(MH+) = 608 |

| | | | | | | |
|---|---|---|---|---|---|---|
| 407 | 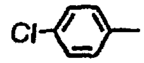 | 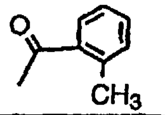 | | | $-CH_3$ | FAB(MH+) = 594 |
| 408 | 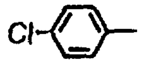 | 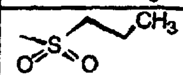 | | | $-CH_3$ | FAB(MH+) = 582 |
| 409 | 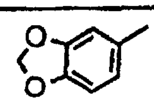 | 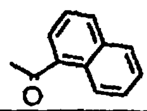 | | | $-CH_3$ | FAB(MH+) = 640 |
| 410 | 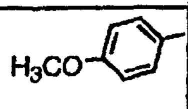 | 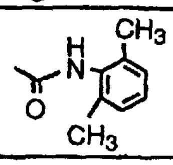 | | | $-CH_3$ | FAB(MH+) = 619 |
| 411 | 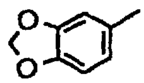 | 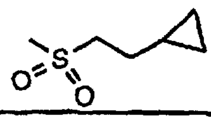 | | | $-CH_3$ | LRMS: calc'd:617 found ($M+H^+$): 618 |
| 412 | 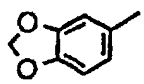 | 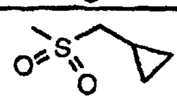 | | | $-CH_3$ | LRMS: calc'd:603 found ($M+H^+$): 604 |
| 413 | 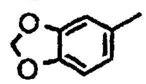 | 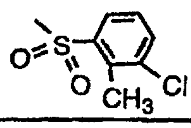 | | | $-CH_3$ | LRMS: calc'd:673 found ($M+H^+$): 674 |
| 414 | 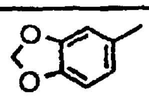 | 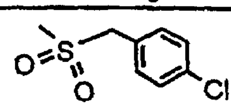 | | | $-CH_3$ | LRMS: calc'd:673 found ($M+H^+$): 674 |
| 415 | 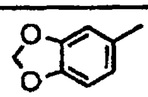 | 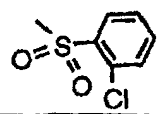 | | | $-CH_3$ | LRMS: calc'd:659 found ($M+H^+$): 660 |
| 416 | 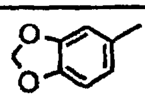 | 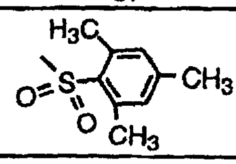 | | | $-CH_3$ | LRMS: calc'd:667 found ($M+H^+$): 668 |
| 417 | 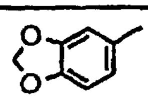 | 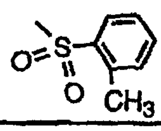 | | | $-CH_3$ | LRMS: calc'd:639 found ($M+H^+$): 640 |
| 418 | 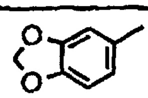 | $-SO_2(CH_2)_2CF_3$ | | | $-CH_3$ | LRMS: calc'd:645 found ($M+H^+$): 646 |
| 419 | 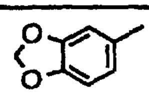 | 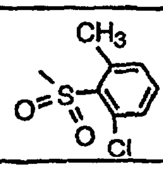 | | | $-CH_3$ | LRMS: calc'd:673 found ($M+H^+$): 674 |

| | | | | | | |
|---|---|---|---|---|---|---|
| 420 | 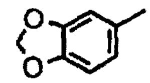 | 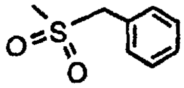 | 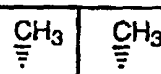 | | $-CH_3$ | LRMS: calc'd:639 found (M+H+): 640 |
| 421 | 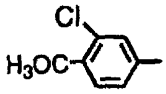 | $-SO_2(CH_2)_2CH_3$ | 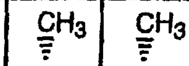 | | $-CH_3$ | LRMS: calc'd:611 found (M+H+): 612 |
| 422 | 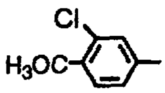 | $-SO_2CH_2CH_3$ | 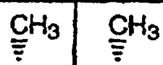 | | $-CH_3$ | LRMS: calc'd:597 found (M+H+): 598 |
| 423 | 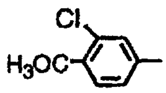 | 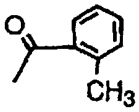 | 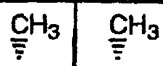 | | $-CH_3$ | LRMS: calc'd:623 found (M+H+): 624 |
| 424 | 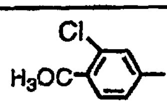 | 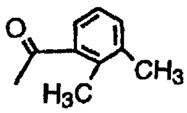 | 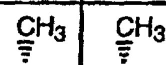 | | $-CH_3$ | LRMS: calc'd:637 found (M+H+): 637 |
| 425 | 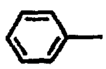 | $-SO_2(CH_2)_2CH_3$ | 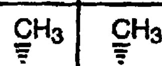 | | $-CH_3$ | LRMS: calc'd:547 found (M+H+): 548 |
| 426 | 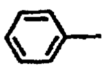 | $-SO_2CH_2CH_3$ | 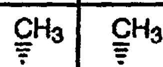 | | $-CH_3$ | LRMS: calc'd:533 found (M+H+): 534 |
| 427 | 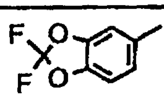 | $-SO_2(CH_2)_2CH_3$ | 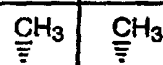 | | $-CH_3$ | LRMS: calc'd:627 found (M+H+): 628 |
| 428 | 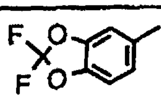 | 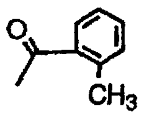 | 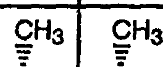 | | $-CH_3$ | LRMS: calc'd:639 found (M+H+): 640 |
| 429 | 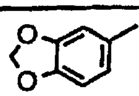 | 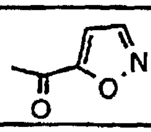 | 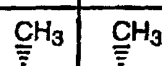 | | $-CH_3$ | LRMS: calc'd:580 found (M+H+): 581 |
| 430 | 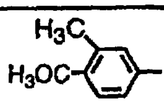 | 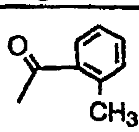 | 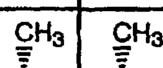 | | $-CH_3$ | LRMS: calc'd:603 found (M+H+): 604 |
| 431 | 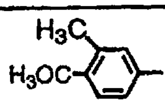 | 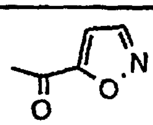 | 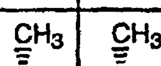 | | $-CH_3$ | LRMS: calc'd:580 found (M+H+): 581 |
| 432 | 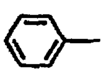 | 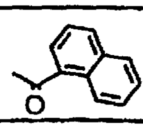 | 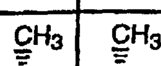 | | $-CH_3$ | LRMS: calc'd:595 found (M+H+): 596 |

| | | | | | | |
|---|---|---|---|---|---|---|
| 433 | 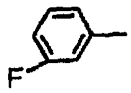 | 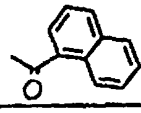 | $CH_3$ | $CH_3$ | $-CH_3$ | LRMS: calc'd:613 found (M+H+): 614 |
| 434 | 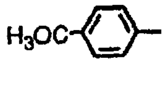 | 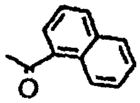 | $CH_3$ | $CH_3$ | $-CH_3$ | LRMS: calc'd:626 found (M+H+): 627 |
| 435 | 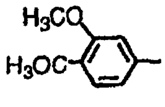 | 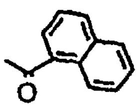 | $CH_3$ | $CH_3$ | $-CH_3$ | LRMS: calc'd:655 found (M+H+): 656 |
| 528 | 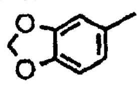 | 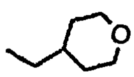 | $CH_3$ | $CH_3$ | $-CH_3$ | m.p. = 212-214°C (dec) |
| 529 | 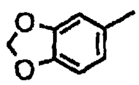 | $-CH_2CH_2CH_3$ | $CH_3$ | $CH_3$ | $-CH_3$ | m.p. = 215-217°C (dec) |
| 530 | 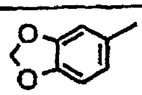 | 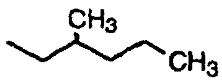 | $CH_3$ | $CH_3$ | $-CH_3$ | m.p. = 210-211°C (dec) |
| 531 | 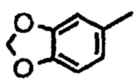 | 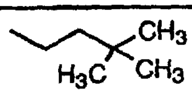 | $CH_3$ | $CH_3$ | $-CH_3$ | m.p. = 223-225°C (dec) |
| 532 | 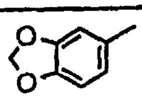 | 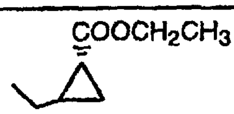 | $CH_3$ | $CH_3$ | $-CH_3$ | m.p. = 209-211°C (dec) |
| 563 | 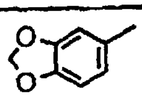 | 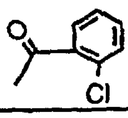 | H | H | 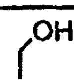 | HRMS calc'd: 612.1935 found: 612.1952 m.p.=187-197°C |
| 564 | 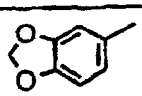 | 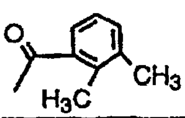 | H | H | 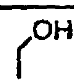 | HRMS calc'd: 606.2638 found: 606.2638 m.p.=185-195°C |
| 565 | 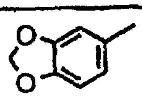 | 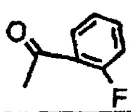 | H | H | 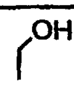 | m.p. = 178-188°C |
| 566 | 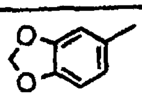 | 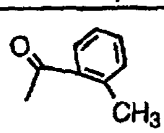 | H | H | 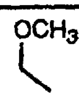 | HRMS calc'd: 606.2638 found: 606.2633 m.p.=175-183°C |
| 567 | 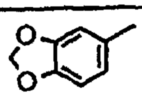 | 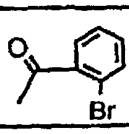 | H | H | 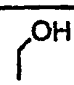 | HRMS calc'd: 656.1430 found: 656.1438 m.p.=185-192°C |
| 568 | 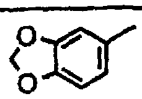 | 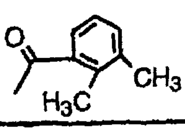 | $CH_3$ | $CH_3$ | 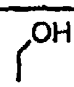 | HRMS calc'd: 634.2951 found: 634.2968 m.p.=185-195°C |

| | | | | | | |
|---|---|---|---|---|---|---|
| 569 | 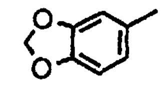 | $-SO_2(CH_2)_2CH_3$ | 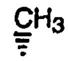 | 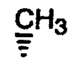 | 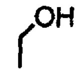 | HRMS calc'd: 608.2464 found: 608.2477 m.p.=205-215°C |
| 570 | 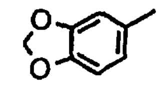 | $-SO_2CH_2CH_3$ | 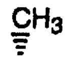 | 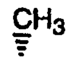 | 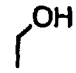 | HRMS calc'd: 594.2308 found: 594.2311 m.p.=175-185°C |
| 571 | 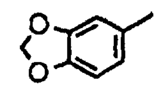 | 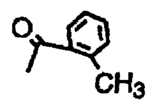 | 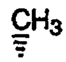 | 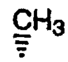 | 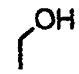 | HRMS calc'd: 620.2794 found: 620.2805 m.p.=185-190°C |

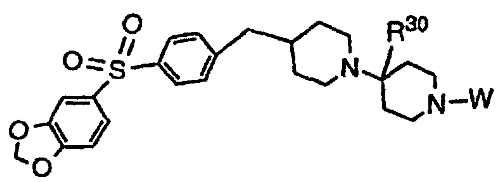

| No. | W | $R^{30}$ | Physical Data |
|---|---|---|---|
| 553 | $-SO_2(CH_2)_2CH_3$ | -CN | LRMS: calc'd: 573;found $(M+1)^+$: 574 |
| 554 | 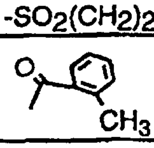 | $-CH_3$ | LRMS: calc'd: 574 found $(M+1)^+$: 575 |
| 555 | 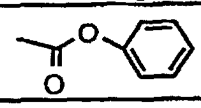 | $-CH_3$ | LRMS: calc'd: 576 found $(M+1)^+$: 577 |
| 556 | $-SO_2CH_2CH_3$ | $-CH_3$ | LRMS: calc'd: 548; found $(M+1)^+$: 549 |
| 557 | $-SO_2CH_2CF_3$ | $-CH_3$ | LRMS: calc'd: 602; found $(M+1)^+$: 603 |
| 558 | 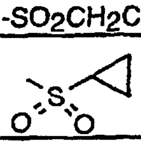 | $-CH_3$ | LRMS: calc'd: 560 found $(M+1)^+$: 561 |

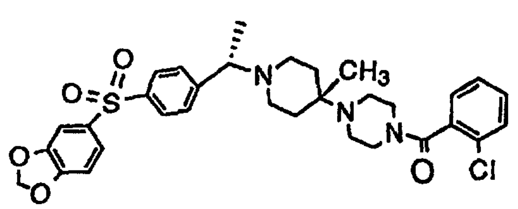

**588**

MH+ = 610

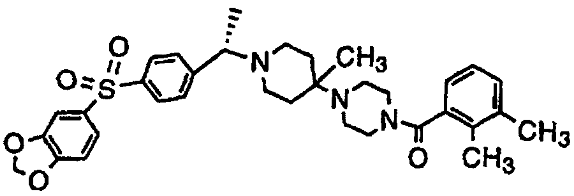

**589** MH+ = 604

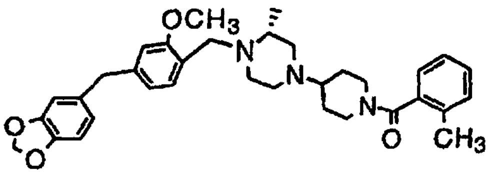

**594** MH+ = 596

## Claims

**1.** A compound having the structural formula

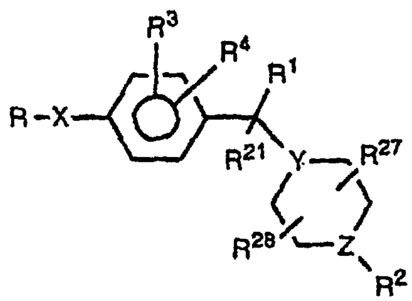

including all isomers and pharmaceutically acceptable salts, esters, and solvates thereof,

wherein one of Y and Z is N and the other is N, CH, or C-alkyl;

X is -O-. -S-, -SO-, $-SO_2-$, $-NR^6-$, -CO-, $-CH_2-$, -CS-, $-C(OR^5)_2-$, $-C(SR^5)_2-$, $-CONR^{20}-$, $-C(alkyl)_2-$, -C(H)(alkyl)-, $-NR^{20}-SO_2-$, $-SO_2-NR^{20}-$, $-NR^{20}CO-$, -O-CO-NH-, -NH-CO-O-,

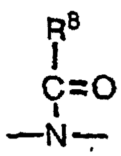 , 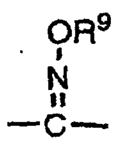 , 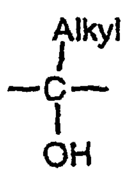 , 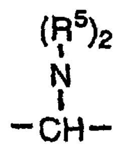 , 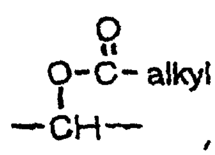 ,

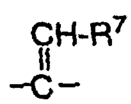 , 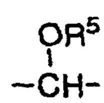 , CH = CH− , —C ≡ C— , 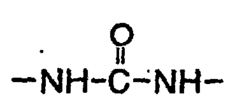 ,

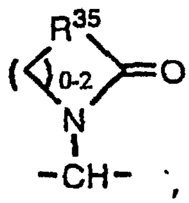 , 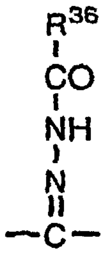 , 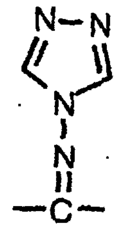 , 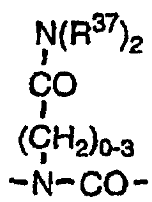 , 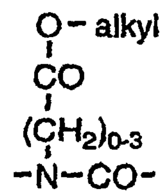 or 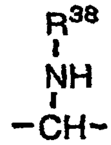 ;

R is

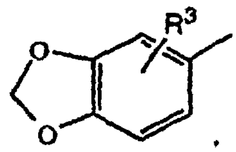 , 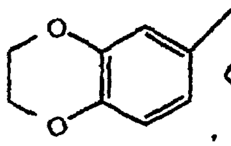 , 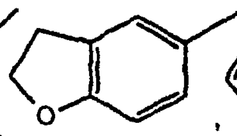 , 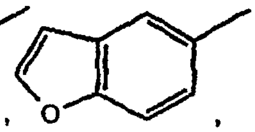 ,

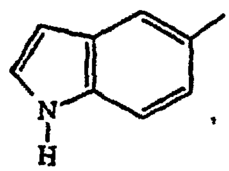 , 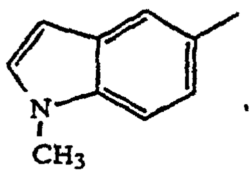 , 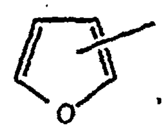 , 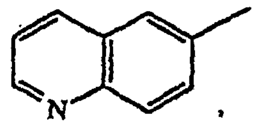 ,

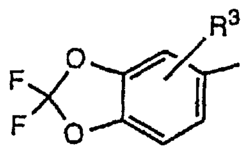 , 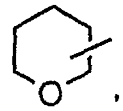 , 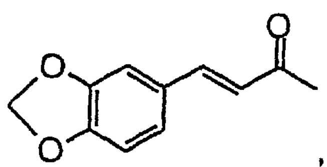 ,

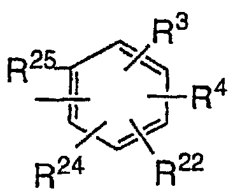 , 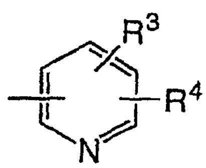 , 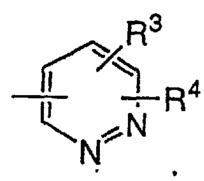 , 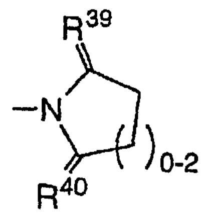 ,

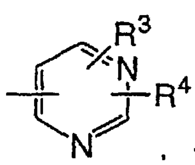 , 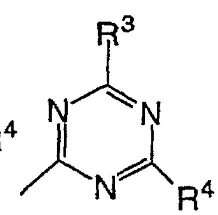 , 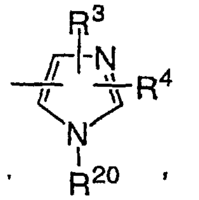 , 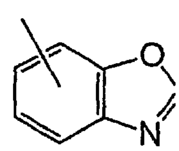

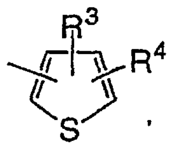, 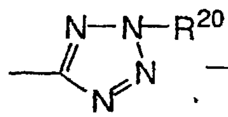, 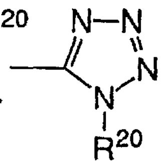, 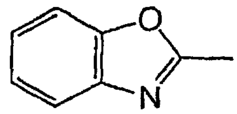,

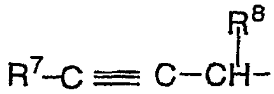, 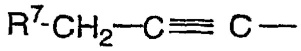, 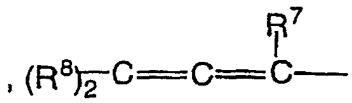,

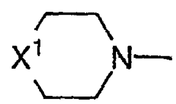

(wherein $X^1$ is $-CH_2-$, -O-, or $-NR^7-$),

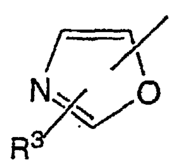, 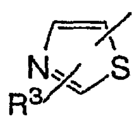, 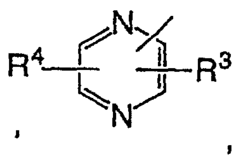, 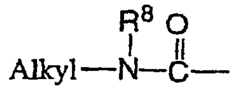,

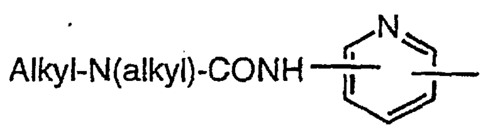,

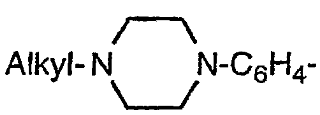, 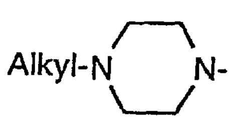, 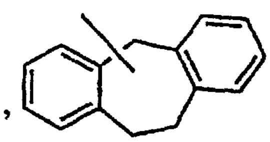,

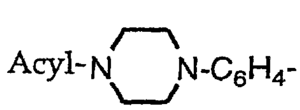, 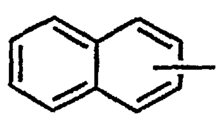,

hydrogen, acyl, alkyl, alkoxy, alkenyl, cycloalkyl, cycloalkyl substituted with one or two groups selected from the group consisting of alkyl and carbonyl, cycloalkenyl, bicycloalkyl, arylalkenyl, benzyl, benzyl substituted with up to three independently selected $R^3$ groups, cycloalkylalkyl, polyhaloacyl, benzyloxyalkyl, hydroxy$C_2$-$C_{20}$alkyl, alkenylcarbonyl, alkylarylsulfonyl, alkoxycarbonylaminoacyl, alkylsulfonyl, or arylsulfonyl, additionally, when X is $-CH_2-$, R may also be -OH; in further addition, when X is not N, R may also be hydroxymethyl, in further addition, R and X may combine to form the group Prot-$(NOAA)_r$-NH- wherein r is an integer of 1 to 4, Prot is a nitrogen protecting group and when r is 1, NOAH is a naturally occuring amino acid or an enantiomer thereof, or when r is 2 to 4, each NOAA is a peptide of an independently selected naturally occuring amino acid or an enantiomer thereof;

$R^1$ and $R^{21}$ are independently selected from the group consisting of H, alkyl, alkenyl, cycloalkyl, cycloalkenyl, bicycloalkyl, alkynyl, cyano, aminoalkyl, alkoxycarbonyl, aminocarbonyl, hydroxyamidino, alkoxycarbonylalkyl, phenyl alkyl, alkylcarbonlyoxyalkyl,

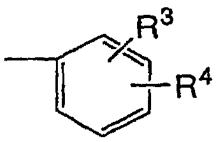 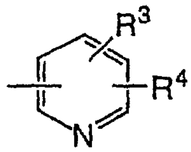, 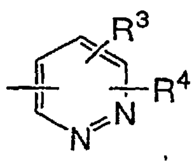, 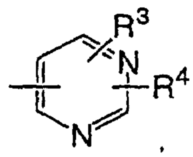, 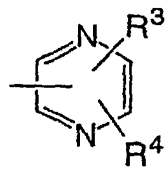,

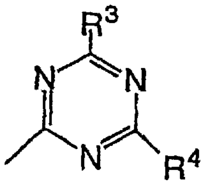, 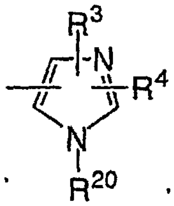, 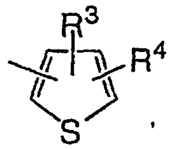, 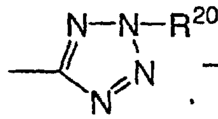, 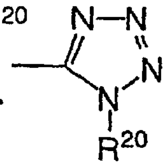,

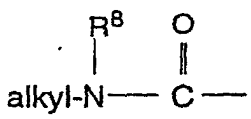, 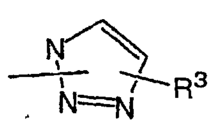, 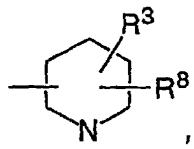,

H, —OH, (provided $R^1$ and $R^{21}$ are both not —OH and Y is not N), formyl, —CO alkyl, -COacyl, —COaryl, and hydroxyalkyl; additionally $R^1$ and $R^{21}$ together may form the group $=CH_2$, $=N\text{-}OR^5$, =N-CN, $=N\text{-}N(R^5)_2$, =CH-alkyl, alkylene,

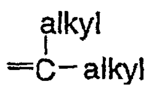

or $=C(halo)_2$; in further addition, $R^1$ and $R^{21}$ together with the carbon atom to which they are attached may form the group

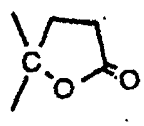 or 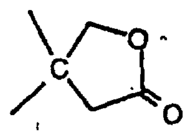

or $R^1$ and $R^{21}$ together with the carbon atom to which they are attached may form a saturated heterocyclic ring containing 3 to 7 carbon atoms, one or more of which may be optionally substituted by alkyl, and one or two groups independently selected from S, O, and $N\text{-}R^{20}$;

$R^2$ is

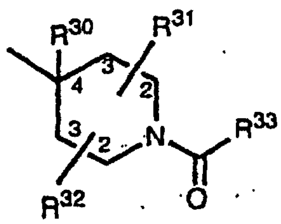 or 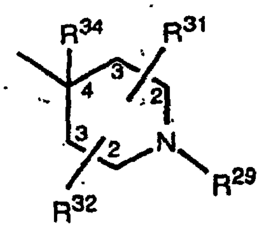 ;

$R^3$, $R^4$, $R^{22}$, $R^{24}$, and $R^{25}$ are independently selected from the group consisting of alkyl, H, halo, alkoxy, benzyloxy, benzyloxy substituted by nitro or aminoalkyl, haloalkyl, polyhaloalkyl, nitro, cyano, sulfonyl, hydroxy, amino, alkylamino, formyl, alkylthio, polyhaloalkoxy, acytoxy, trialkylsilyl, alkylsulfonyl, arylsulfonyl, acyl, alkoxy-carbonyl alkylsulfinyl; $-OCONH_2$, -OCONH-alkyl, alkylaminoalkyl. dialkylaminoalkyl, -COOH, $-CON(R^{20})_2$, $-OCON(alkyl)_2$, -NHCOO-alkyl, -NHCO-alkyl, phenyl, hydroxyalkyl, or morpholino;

each $R^5$ and $R^6$ is independently selected from the group consisting of H and alkyl, provided that when X is

$C(OR^5)_2$ or $C(SR^5)_2$, both $R^5$ groups cannot be H, and in addition, when X is $C(OR^5)_2$ or $C(SR^5)_2$, the two $R^5$ groups in X may be joined to form $-(CR^{20}{}_2)_p-$ wherein p is an integer of 2 to 4;

$R^7$ is independently selected from the group consisting of H, alkyl, arylalkyl, cycloalkyl, aryl and aryl substituted with $R^3$ and $R^4$ as defined herein;

each $R^8$ is independently selected from the group consisting of H, hydroxyalkyl or alkyl, or two $R^8$ groups may be joined to form an alkylene group;

$R^9$ is H, alkyl, aralkyl, or acyl;

$R^{20}$ is H, aryl or alkyl;

$R^{27}$ and $R^{28}$ are independently selected from the group consisting of H, alkyl, hydroxyalkyl, arylalkyl, aminoalkyl, haloalkyl, thioalkyl, alkylthioalkyl, carboxyalkyl, imidazolyalkyl, and indolyalkyl; or $R^{27}$ and $R^{28}$ may combine to form an alkylene group;

$R^{29}$ is H, alkyl, -CO-alkyl, -CO-cycloalkyl, alkoxycarbonyl, aminocarbonyl, aryloxycarbonyl, alkylaminocarbonyl, dialkylaminocarbonyl, alkylsulfonyl, arysulfonyl or $-SO_2-NH-R^{20}$;

$R^{30}$ is H, alkyl, aryl, cycloalkyl, hydroxyalkyl, aminoalkyl, $-COOR^{20}$, $-CON(R^{20})_2$ or cyano;

$R^{31}$ and $R^{32}$ are the same as $R^{30}$ and in addition, two $R^{30}$, $R^{31}$ and $R^{32}$ groups may form the group $-(CH_2)_r-$ (wherein r is 1 to 6), in further addition, $R^{31}$ and $R^{32}$ can also be hydroxy, $-N(R^{20})_2$, -O-acyl, $-N(R^{20})$acyl, $-OCOOR^{20}$, or $-OCON(R^{20})_2$;

$R^{33}$ is aryl or heteroaryl, with the proviso that when $R^{33}$ is heteroaryl, the CO-$R^{33}$ bond is to a carbon atom in the $R^{33}$ group;

$R^{34}$ is alkyl, cycloalkyl or aryl and in addition $R^{34}$ may also be H when $R^1$ and $R^{21}$ together with the carbon atom to which they are attached form a saturated heterocyclic ring containing 3 to 7 carbon atoms and two groups independently selected from S, O, and N-$R^{20}$;

$R^{35}$ is $-CH_2-$, $-NR^{20}-$ or -O-;

$R^{36}$ is $-NH_2$, alkyl or alkoxy;

$R^{37}$ is independently selected from the group consisting of H and alkyl;

$R^{38}$ is $-CO-(CH_2)_{0-5}-OR^5$, $-SO_2-$(alkyl), or

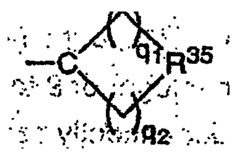

wherein $q_1$ and $q_2$ are independently 1-5, provided that the sum of $q_1$ and $q_2$ is 2-5; and

$R^{39}$ and $R^{40}$ are independently selected from the group consisting of =O and (H,H);

wherein:

"alkyl" represents a straight or branched saturated hydrocarbon chain having 1 to 20 carbon atoms, preferably 1 to 8 carbon atoms;

"alkenyl" represents a straight or branched hydrocarbon chain of from 2 to 15 carbon atoms, more preferably 2 to 12 carbon atoms, having at least one carbon-to-carbon double bond;

"alkynyl" represents a straight or branched hydrocarbon chain of from 2 to 10 carbons atoms, more preferably 2 to 8 carbon atoms. having at least one carbon-to-carbon triple bond;

"bicycloalkyl" represents a carbocyclic ring having 3 to 12 carbon atoms;

"cycloalkyl" represents a saturated carbocyclic ring having 3 to 12 carbons atoms;

"cycloalkenyl" represents a carbocyclic ring having from 5 to 8 carbon atoms and at least one carbon-to-carbon double bond in the ring;

"acyl" represents a radical of a carboxylic acid having the formula alkyl-CO-, aryl-CO-, aralkyl-CO-, cycloalkyl-CO-, alkylcycloalkyl-CO-, and heteroaryl-CO-;

"halo" represents fluoro, chloro, bromo or iodo;

"aryl" represents phenyl or naphthyl optionally substituted with 1 to 5 $R^3$ groups; and

"heteroaryl" represents a cyclic group of 5 or 6 atoms, or a bicyclic group of 9 or 10 atoms, at least one of which is carbon and having at least one O, S, or N atom interrupting a carbocyclic ring having a sufficient number of pi electrons to provide aromatic character, wherein the carbon atoms may optionally be substituted by $R^3$ groups; the nitrogen atoms may optionally be substituted by $-R^{20}$ or $-COR^{20}$ groups, with preferred heteroatomatic groups being selected from pyridyl, furanyl, benzofuranyl, thienyl, benzothienyl, thiazolyl, thiadiazolyl, imidazolyl, pyrimidinyl, pyrazinyl, pyridazinyl, 1,3,5-triazinyl, and indolyl;

"polyhalo" represents substituted of at least 2 halo atoms to the group modified by the term "polyhalo";

**2.** A compound of claim 1 wherein Y and Z are N.

**3.** A compound of claim 1 wherein Y is CH and Z is N.

**4.** A compound of any of claims 1, 2 or 3 wherein R is

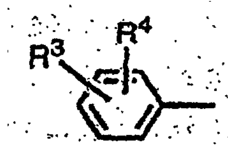, 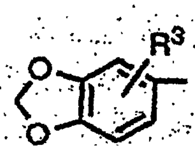, or 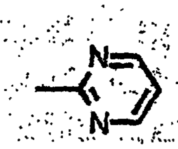

and X is O, SO, $SO_2$, $CH_2$, CH(alkyl), C(alkyl)$_2$, CH(OH), or N($R^{20}$)CO.

**5.** A compound of any of claims 1, 2, 3, 4 or 5 wherein $R^3$ and $R^4$ are H; either $R^1$ is H, cycloalkyl or alkyl and $R^{21}$ is H or $R^1$ and $R^{21}$ together form =O or

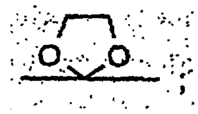;

and wherein at least one of $R^{27}$ and $R^{28}$ is alkyl.

**6.** A compound of any of claims 1, 2, 3, 4 or 5 wherein $R^2$ is

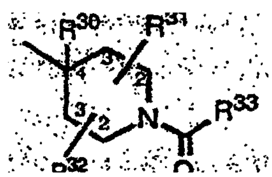

and $R^{30}$ is H or $CH_3$: $R^{31}$ and $R^{32}$ are H: and $R^{33}$ is ortho-substituted aryl or heteroaryl.

**7.** A compound of claim 6 wherein $R^{33}$ is

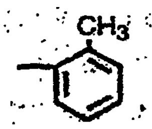, 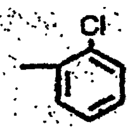, 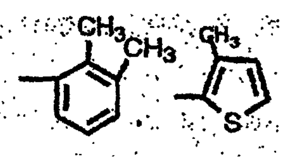, 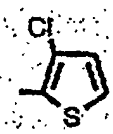 or 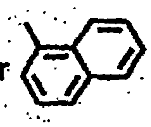.

**8.** A compound of any one of claims 1 to 5 wherein $R^2$ is

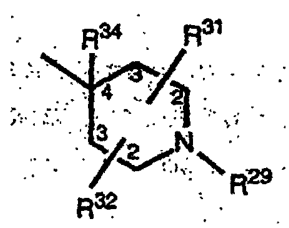

and $R^{34}$ is methyl and $R^{31}$ and $R^{32}$ are H.

**9.** A compound according to Claim 1 selected from the group consisting of

| | |
|---|---|
| 17 | 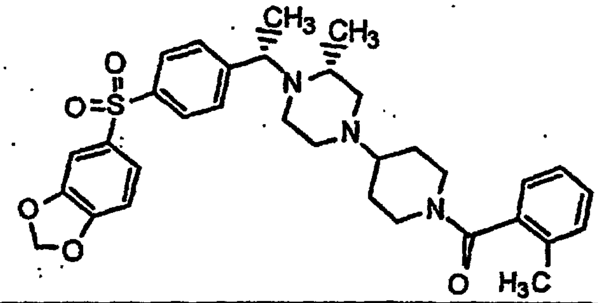 |
| 18 | 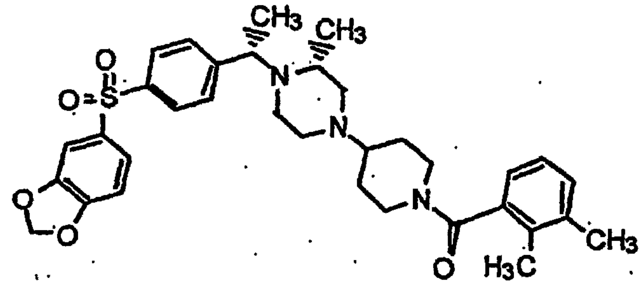 |
| 25 | 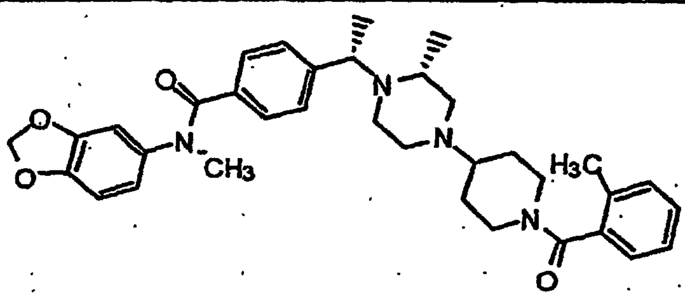 |
| 30 | 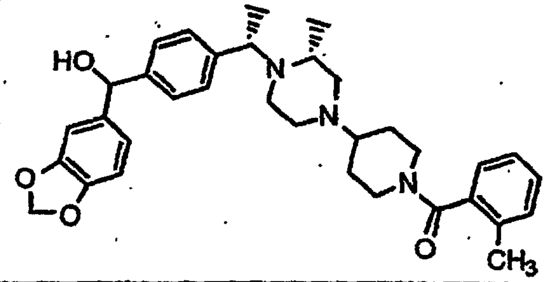 |
| 31 | 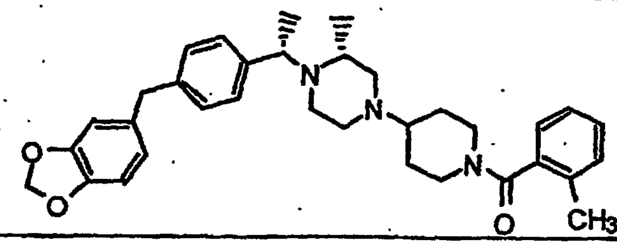 |

| | |
|---|---|
| 32 | 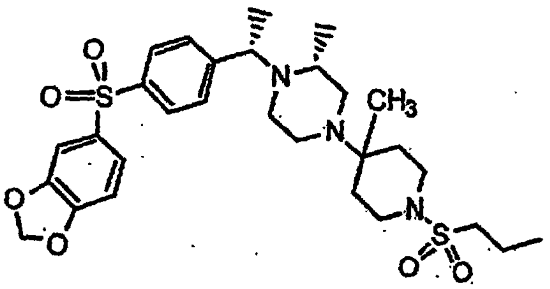 |
| 34 | 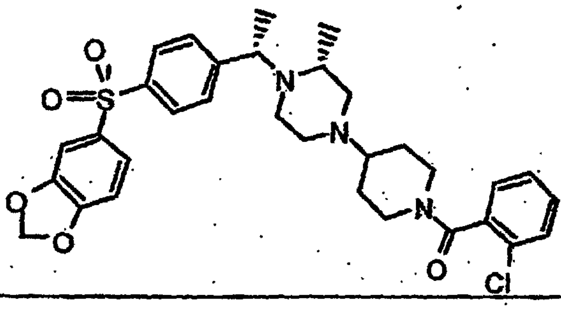 |
| 35 | 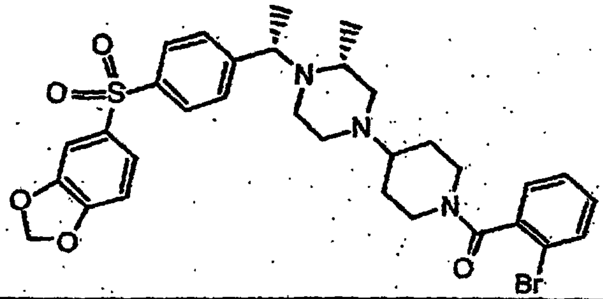 |
| 36 | 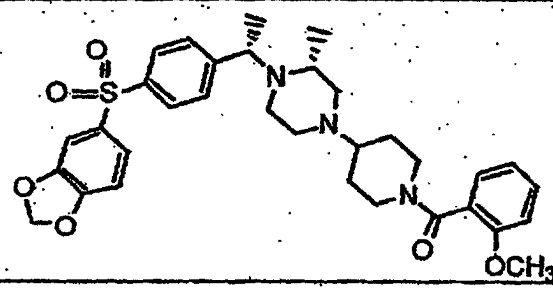 |
| 37 | 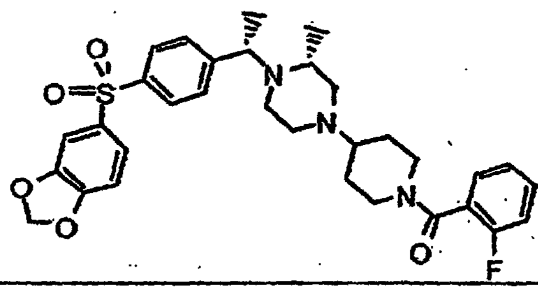 |

| | |
|---|---|
| 41 | 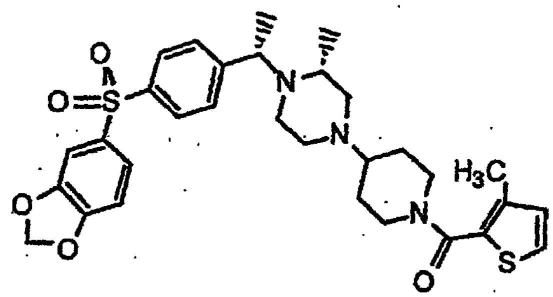 |
| 43 | 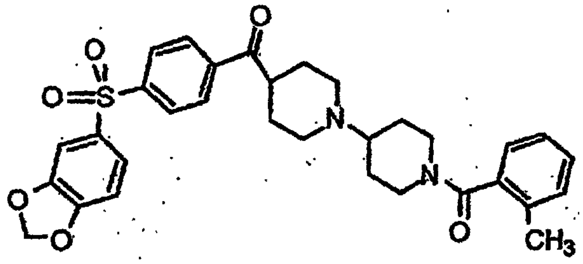 |
| 44 | 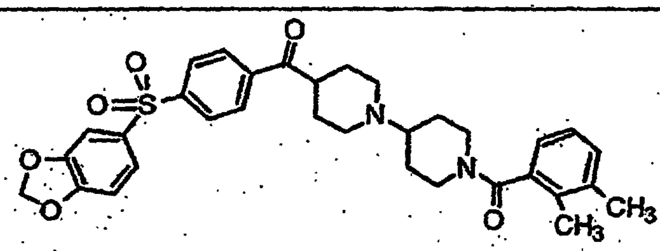 |
| 49 | 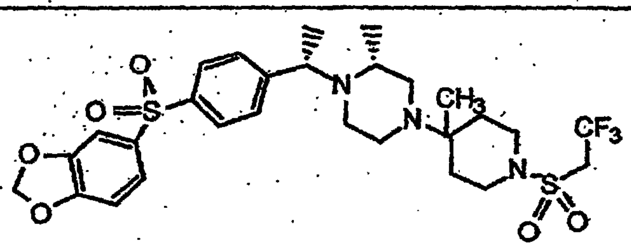 |
| 53 | 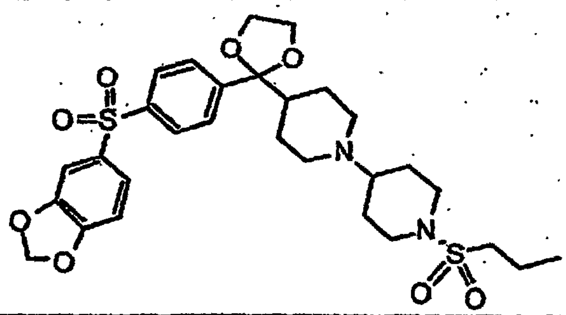 |
| 54 | 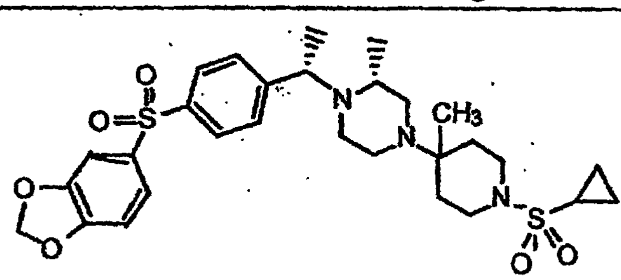 |

| | |
|---|---|
| 56 | 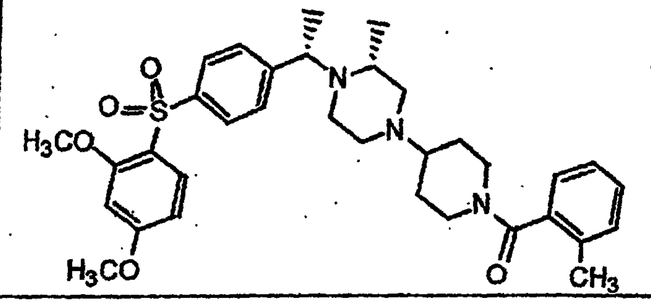 |
| 57 | 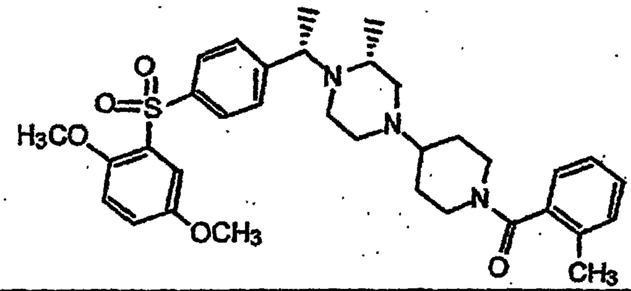 |
| 58 | 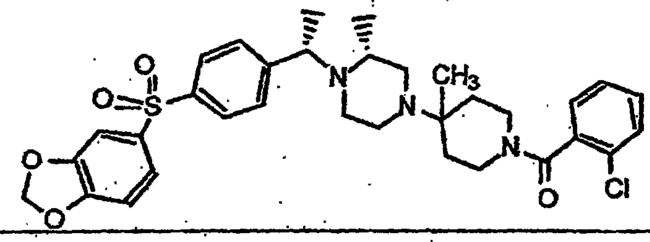 |
| 59 | 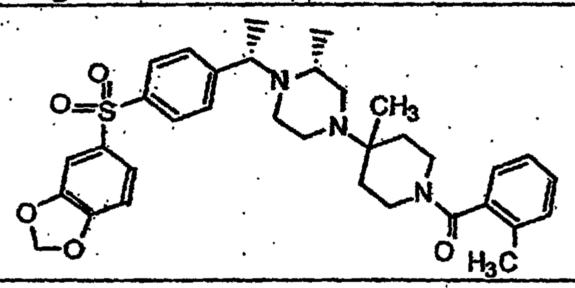 |
| 80 | 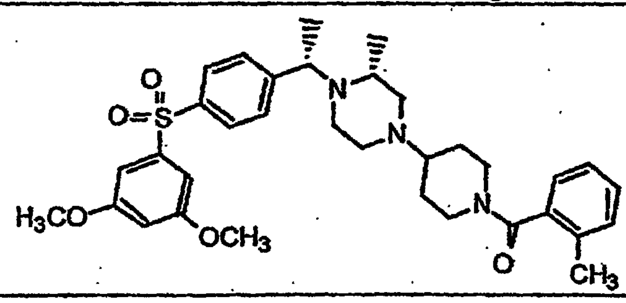 |
| 82 | 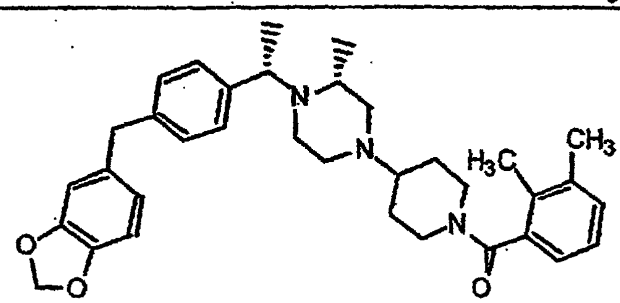 |

| | |
|---|---|
| 84 | 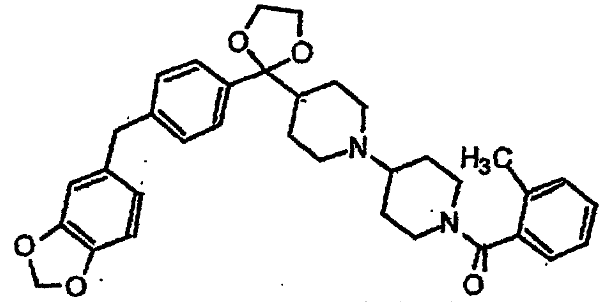 |
| 85 | 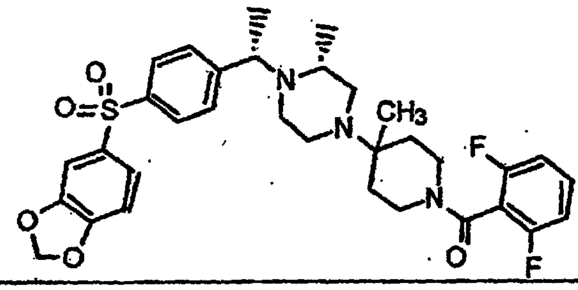 |
| 94 | 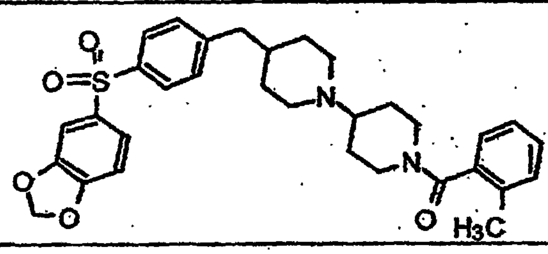 |
| 98 | 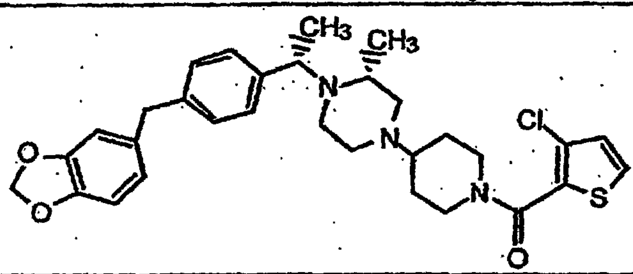 |
| 100 | 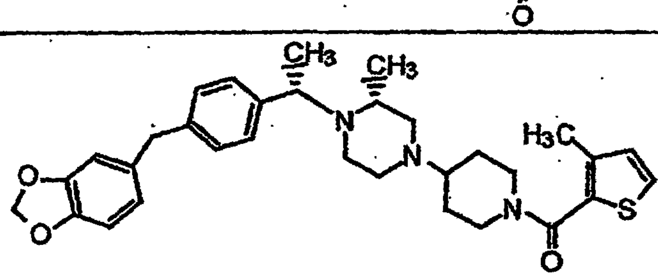 |
| 108 | 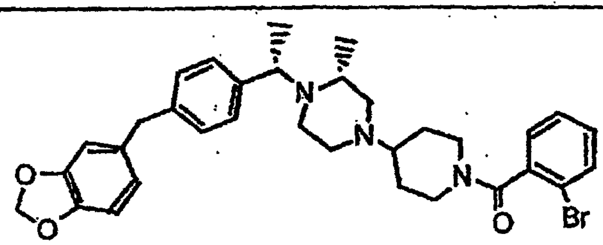 |

or a compound selected from the group consisting of compounds represented by the structural formulas

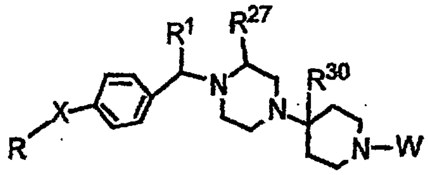

wherein R, X, $R^1$, $R^{27}$, $R^{30}$ and W are as defined in the following table:

| No. | R | X | W | $R^1$ | $R^{27}$ | $R^{30}$ |
|---|---|---|---|---|---|---|
| 121 | 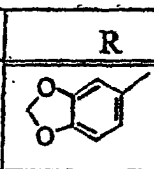 | 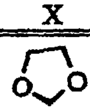 | 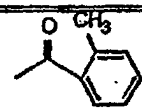 | H | H | H |
| 127 | 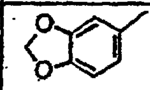 | $-SO_2-$ | 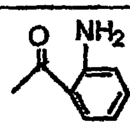 | $CH_3$ | $CH_3$ | H |
| 151 | 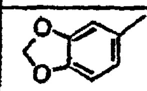 | $-SO_2-$ | 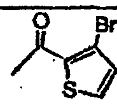 | $CH_3$ | $CH_3$ | H |
| 152 | 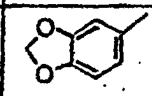 | $-SO_2-$ | 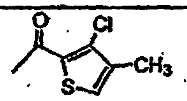 | $CH_3$ | $CH_3$ | H |
| 154 | 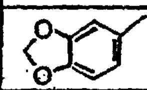 | $-SO_2-$ | 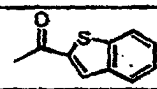 | $CH_3$ | $CH_3$ | H |
| 155 | 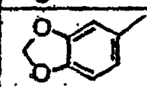 | $-SO_2-$ | 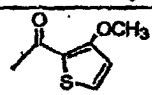 | $CH_3$ | $CH_3$ | H |
| 162 | 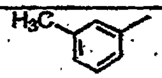 | $-SO_2-$ | 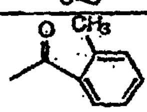 | $CH_3$ | $CH_3$ | H |
| 166 | 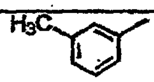 | $-SO_2-$ | 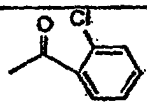 | $CH_3$ | $CH_3$ | H |
| 178 | 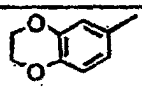 | $-SO_2-$ | 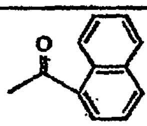 | $CH_3$ | $CH_3$ | H |
| 179 | 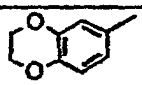 | $-SO_2-$ | 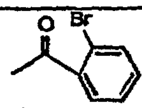 | $CH_3$ | $CH_3$ | H |
| 181 | 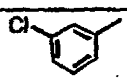 | $-SO_2-$ | 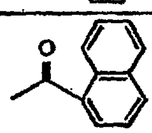 | $CH_3$ | $CH_3$ | H |
| 185 | 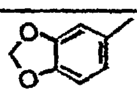 | $-SO_2-$ | 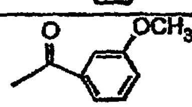 | $CH_3$ | $CH_3$ | H |
| 190 | 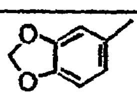 | $-SO_2-$ | 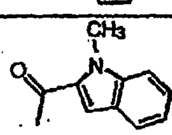 | $CH_3$ | $CH_3$ | H |

| | | | | | | |
|---|---|---|---|---|---|---|
| 191 | 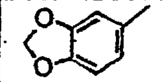 | $-SO_2-$ | 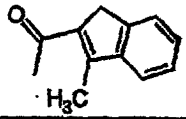 | $CH_3$ | $CH_3$ | H |
| 194 | 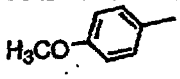 | $-SO_2-$ | 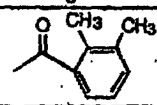 | $CH_3$ | $CH_3$ | H |
| 199 | 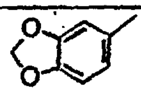 | $-SO_2-$ | 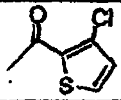 | $CH_3$ | $CH_3$ | H |
| 214 | 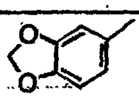 | $-SO_2-$ | 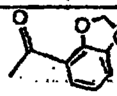 | $CH_3$ | $CH_3$ | H |
| 215 | 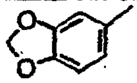 | $-SO_2-$ | 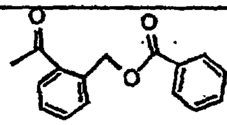 | $CH_3$ | $CH_3$ | H |
| 216 | 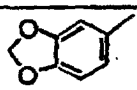 | $-SO_2-$ | 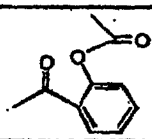 | $CH_3$ | $CH_3$ | H |
| 225 | 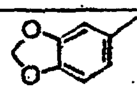 | $-SO_2-$ | 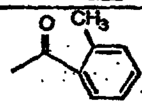 | H | H | H |
| 247 | 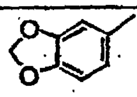 | $-SO_2-$ | 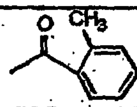 | H | $CH_3$ | H |
| 253 | 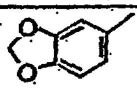 | $-CH_2-$ | 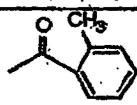 | $CH_3$ | H | H |
| 256 | 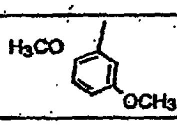 | $-CH_2-$ | 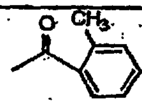 | $CH_3$ | $CH_3$ | H |
| 257 | 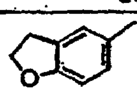 | $-CH_2-$ | 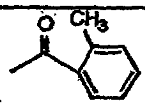 | $CH_3$ | $CH_3$ | H |
| 500 | 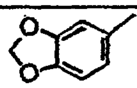 | $-CH_2-$ | 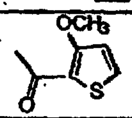 | $CH_3$ | $CH_3$ | H |
| 501 | 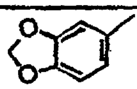 | $-CH_2-$ | 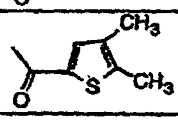 | $CH_3$ | $CH_3$ | H |
| 562 | 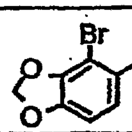 | $-CH_2-$ | 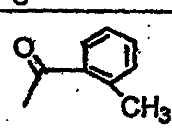 | $CH_3$ | $CH_3$ | H |
| 349 | 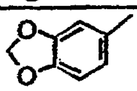 | $-SO_2-$ | 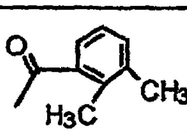 | $CH_3$ | $CH_3$ | $-CH_3$ |

| | | | | | | |
|---|---|---|---|---|---|---|
| 351 | 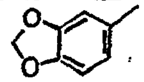 | -SO2- | 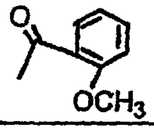 | 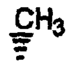 | 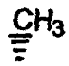 | $-CH_3$ |
| 367 | 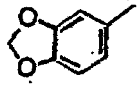 | -SO2- | 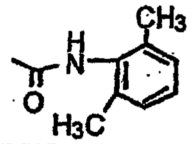 | 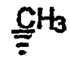 | 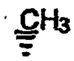 | $-CH_3$ |
| 409 | 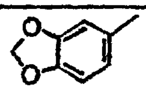 | -SO2- | 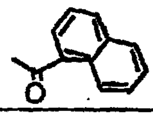 | 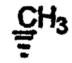 | 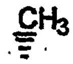 | $-CH_3$ |

and compounds represented by the structural formula:

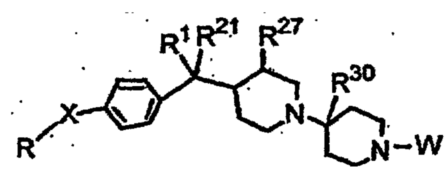

| No. | R | X | W | $R^1$, $R^{21}$ | $R^{30}$ |
|---|---|---|---|---|---|
| 126 | 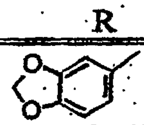 | $-SO_2-$ | $-SO_2-CH_2CH_2CH_3$ | H,H | $-CH_3$ |
| 137 | 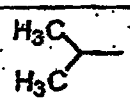 | -S- | 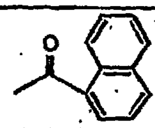 | H,H | H |
| 145 | 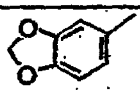 | $-CH_2-$ | 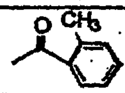 | H,H | H |
| 337 | 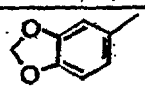 | $-CH_2-$ | 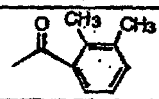 | 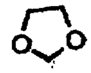 | H |
| 339 | 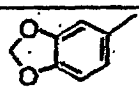 | $-CH_2-$ | 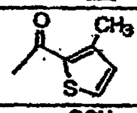 | 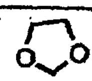 | H |
| 340 | 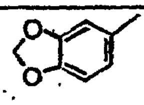 | $-CH_2-$ | 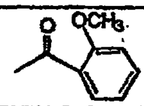 | 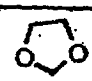 | H |
| 341 | 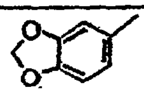 | $-CH_2-$ | 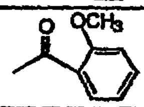 | =O | H |
| 459 | 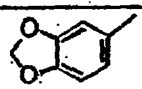 | -S- | 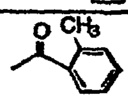 | 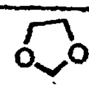 | H |
| 479 | 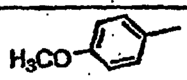 | $-SO_2-$ | 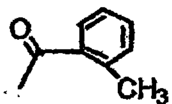 | 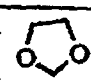 | H |
| 488 | 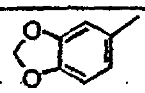 | $-SO_2-$ | 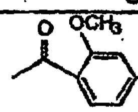 | =O | H |
| 489 | 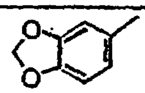 | $-SO_2-$ | 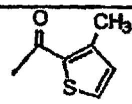 | =O | H |

| | | | | | |
|---|---|---|---|---|---|
| 490 | 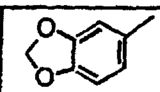 | -SO2- | 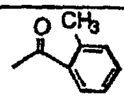 | H, OH | H |
| 502 | 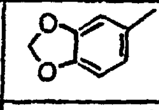 | -SO2- | 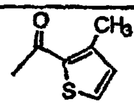 | H, H | H |
| 503 | 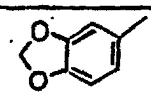 | -SO2- | 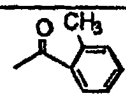 | H, H | H |
| 505 | 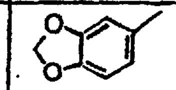 | -SO2- | 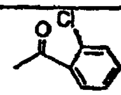 | H, H | H |
| 506 | 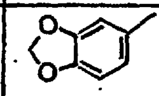 | -SO2- | 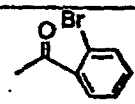 | H, H | H |
| 507 | 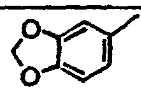 | -SO2- | 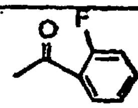 | H, H | H |
| 515 | 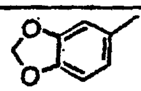 | -SO2- | 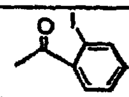 | H, H | H |
| 516 | 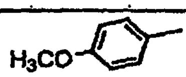 | -SO2- | 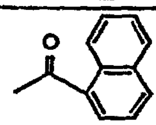 | H, H | H |
| 517 | 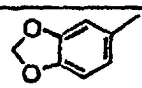 | -SO2- | 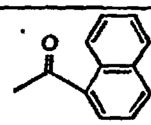 | H, H | H |
| 555 | 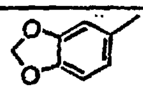 | -SO2- | 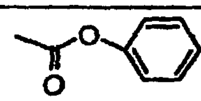 | H,H | -CH3 |

**10.** A compound having the structural formula:

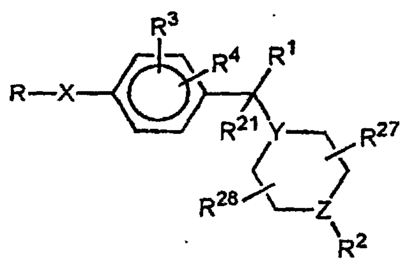

wherein R, $R^2$, $R^{27}$, $R^{28}$, X, Y and Z are as defined as in Claim 1, $R^3$ and $R^4$ are H, and either $R^1$ is cycloalkyl or alkyl, and $R^{21}$ is H, or $R^1$ and $R^{21}$ together form =O.

**11.** A compound according to Claim 10 having the structure:

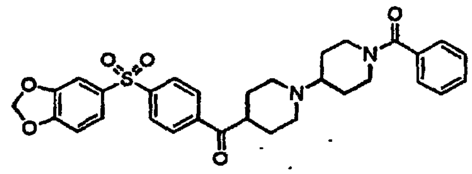

**42**

,

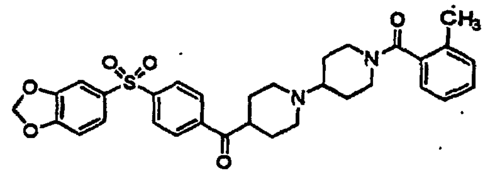

**43**

,

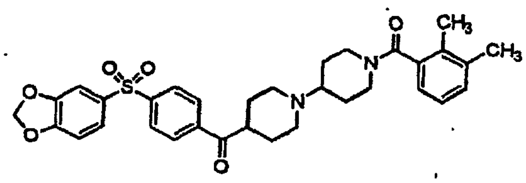

**44**

,

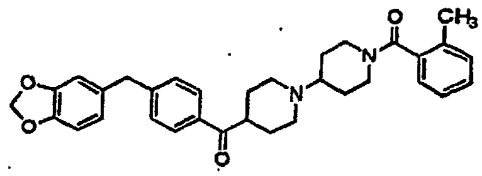

**83**

,

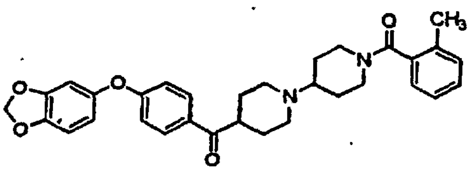

**142**

,

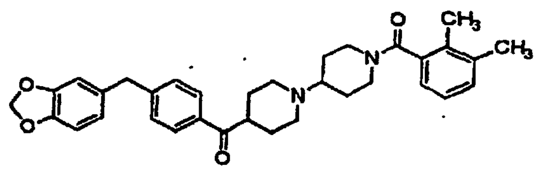

**338**

,

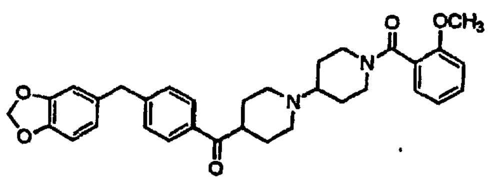

**341**

,

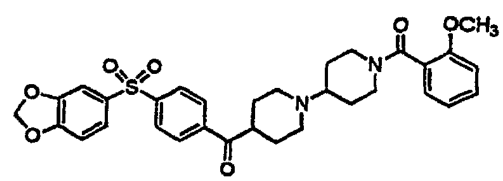

**488**

,

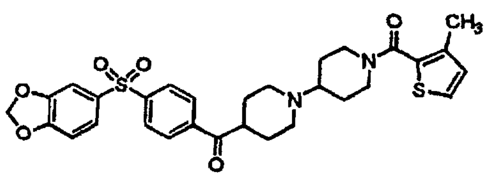

**489**

, or

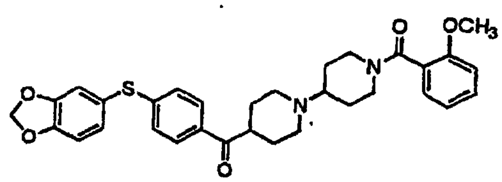

**493**

**12.** A pharmaceutical composition which comprises a compound as defined in claim 1 in combination with a pharmaceutically acceptable carrier.

**13.** A process for the preparation of a pharmaceutical composition as defined in claim 12 comprising admixing a compound as claimed in any of claims 1 to 9 with a pharmaceutically acceptable carrier.

**14.** The use of a compound of claim 1 for the preparation of a medicament for treating a cognitive or neurodegenerative disease.

**15.** A kit for treating a cognitive or neurodegenerative disease comprising in separate containers in a single package pharmaceutical compounds for use in combination, in one container a compound in accordance with claim 1 and in a separate container an acetylcholinesterase inhibitor, said compound and inhibitor each being in a pharmaceutically acceptable carrier and their combined quantities being an effective amount.

**16.** Use of a compound according to Claim 1 in the manufacture of a medicament for the treatment of a cognitive or neurodegenerative disease said medicament comprising an effective amount of a compound of claim 1, alone or in combination with an acetylcholinesterase inhibitor.

**Patentansprüche**

**1.** Verbindung mit der Strukturformel

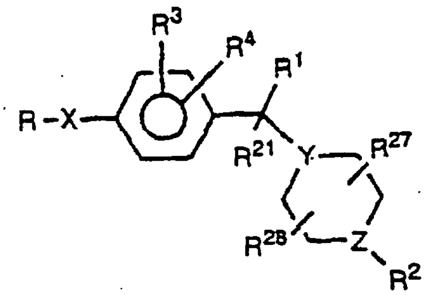

einschließlich aller Isomere und pharmazeutisch annehmbarer Salze, Ester und Solvate derselben,
wobei eines von Y und Z N ist und das andere N, CH oder C-Alkyl ist:

X ist -O-, -S-, -SO-, $-SO_2-$, $-NR^6-$, -CO-, $-CH_2-$, -CS-, $-C(OR^5)_2-$, $-C(SR^5)_2-$, $-CONR^{20}-$, $-C(Alkyl)_2-$, -C(H) (Alkyl)-, $-NR^{20}-SO_2-$, $-SO_2-NR^{20}-$, $-NR^{20}CO-$, -O-CO-NH-, -NH-CO-O-,

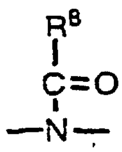, 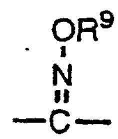, 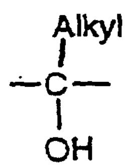, 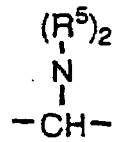, 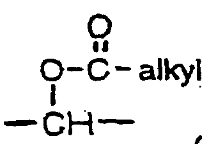,

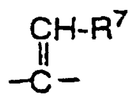, 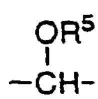, 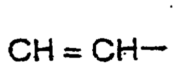, 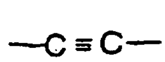, 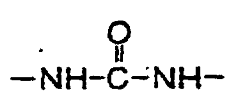,

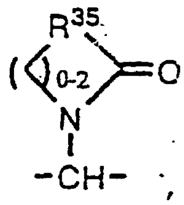,

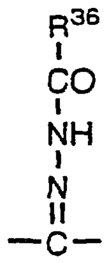, 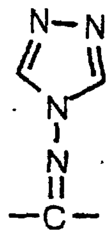, 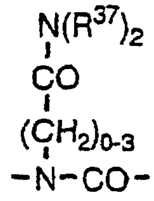, 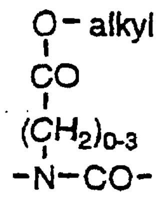 oder 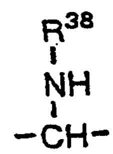;

R ist

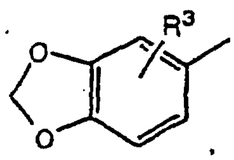, 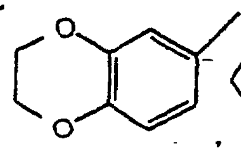, 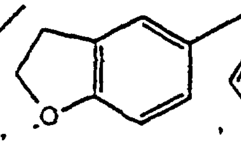, 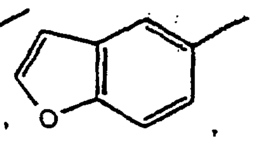,

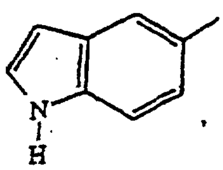, 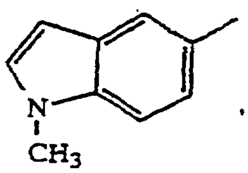, 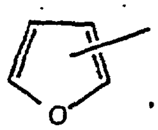, 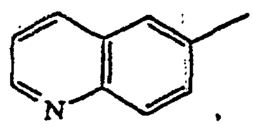,

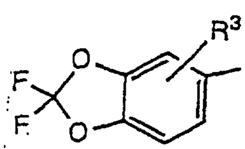, 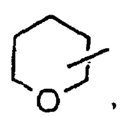, 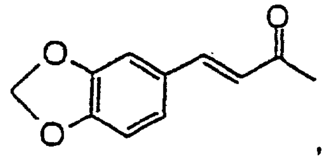,

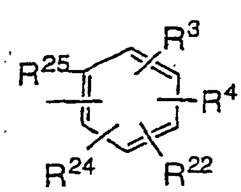, 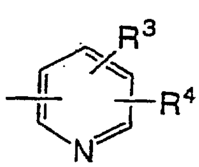, 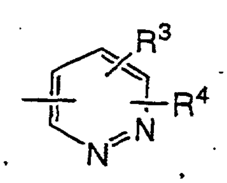, 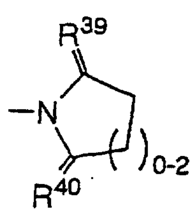,

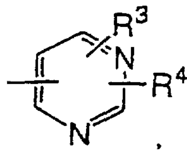 , 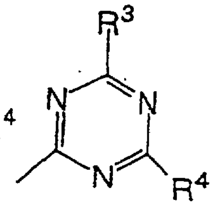 , 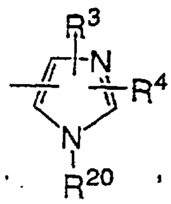 , 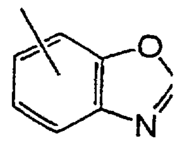

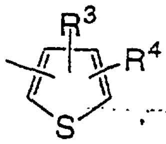 , 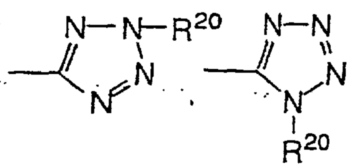 , 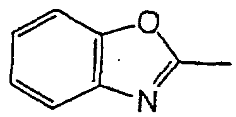 ,

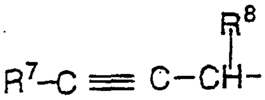 , 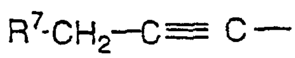 , 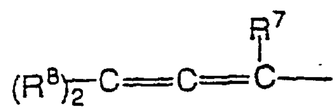 ,

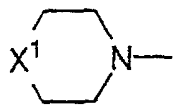

(wobei $X^1$ $-CH_2-$, -O- oder $-NH^7-$ ist),

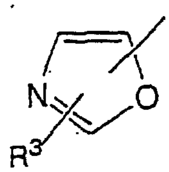 , 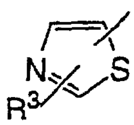 , 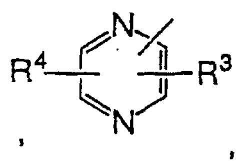 , 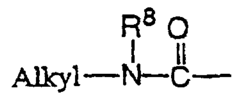 ,

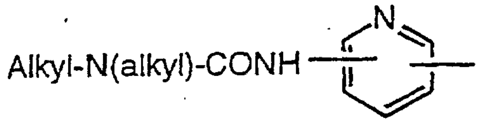

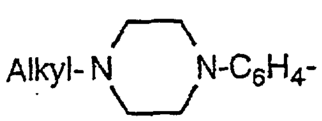 , 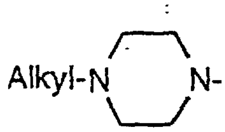 , 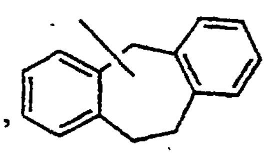 ,

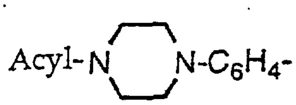 , 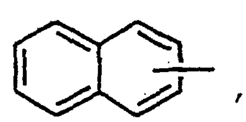 ,

Wasserstoff, Acyl, Alkyl, Alkoxy, Alkenyl, Cycloalkyl, Cycloalkyl, das mit einer oder zwei Gruppen ausgewählt

aus der Gruppe bestehend aus Alkyl und Carbonyl substituiert ist, Cycloalkenyl, Bicycloalkyl, Arylalkenyl, Benzyl, Benzyl, das mit bis zu drei unabhängig ausgewählten $R^3$-Gruppen substituiert ist, Cycloalkylalkyl, Polyhalogenacyl, Benzyloxyalkyl, Hydroxy-$C_2$-$C_{20}$-alkyl, Alkenylcarbonyl, Alkylarylsulfonyl, Alkoxycarbonylaminoacyl, Alkylsulfonyl oder Arylsulfonyl, wobei zusätzlich, wenn X -$CH_2$- ist, R auch -OH sein kann, wobei des Weiteren, wenn X nicht N ist, R auch Hydroxymethyl sein kann, wobei ferner zusätzlich R und X unter Bildung der Gruppe Prot-$(NOAA)_r$-NH- kombinieren können, wobei r eine ganze Zahl von 1 bis 4 ist, Prot eine Stickstoffschutzgruppe ist, und wenn r 1 ist, NOAA eine natürlich vorkommende Aminosäure oder ein Enantiomer davon ist, oder wenn r 2 bis 4 ist, jedes NOAA ein Peptid einer unabhängig ausgewählten, natürlich vorkommenden Aminosäure oder ein Enantiomer davon sein kann;

$R^1$ und $R^{21}$ unabhängig ausgewählt sind aus der Gruppe bestehend aus H, Alkyl, Alkenyl, Cycloalkyl, Cycloalkenyl, Bicycloalkyl, Alkinyl, Cyano, Aminoalkyl, Alkoxycarbonyl, Aminocarbonyl, Hydroxyamidino, Alkoxycarbonylalkyl, Phenylalkyl, Alkylcarbonyloxyalkyl,

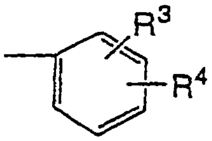, 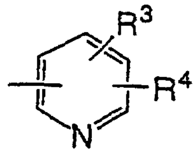, 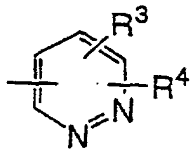, 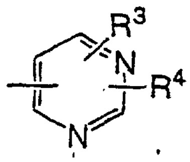, 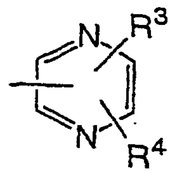,

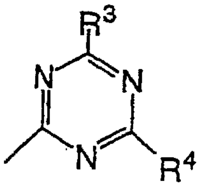, 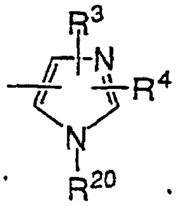, 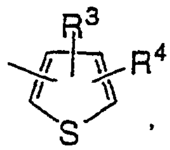, 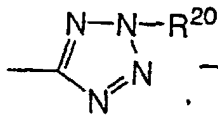, 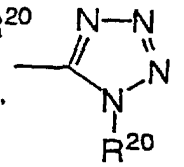,

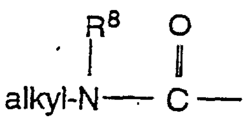, 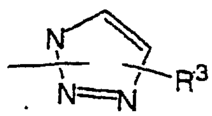, 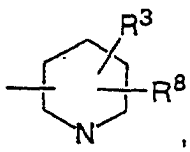,

H, -OH (mit der Maßgabe, dass nicht beide $R^1$ und $R^{21}$ -OH sind und Y nicht N ist), Formyl, -CO-Alkyl, -CoAcyl, -CoAryl und Hydroxyalkyl; außerdem können $R^1$ und $R^{21}$ zusammen die Gruppe =$CH_2$, =N-$OR^5$, =N-CN, =N-N$(R^5)_2$, =CH-alkyl, Alkylen, =C(alkyl)(alkyl) oder =C$(Halogen)_2$ bilden; des Weiteren können $R^1$ und $R^{21}$ zusammen mit dem Kohlenstoffatöm, an das sie gebunden sind, die Gruppe

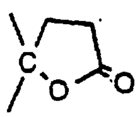 oder 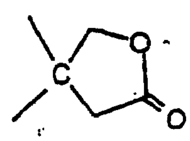

bilden, oder

$R^1$ und $R^{21}$ können zusammen mit dem Kohlenstoffatom, an das sie gebunden sind, einen gesättigten heterocyclischen Ring bilden, der 3 bis 7 Kohlenstoffatome enthält, von denen ein oder mehrere gegebenenfalls durch Alkyl substituiert sein können, und eine oder zwei Gruppen unabhängig ausgewählt aus S, O und N-$R^{20}$ sind;

$R^2$

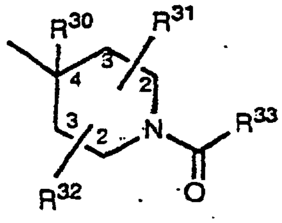

oder

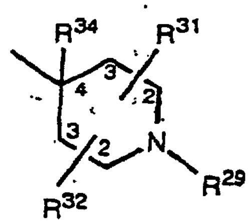

;

ist;
$R^3$, $R^4$, $R^{22}$, $R^{24}$ und $R^{25}$ unabhängig ausgewählt sind aus der Gruppe bestehend aus Alkyl, H, Halogen, Benzyloxy, Benzyloxy, das durch Nitro oder Aminoalkyl substituiert ist, Halogenalkyl, Polyhalogenalkyl, Nitro, Cyano, Sulfonyl, Hydroxy, Amino, Alkylamino, Formyl, Alkylthio, Polyhalogenalkoxy, Acyloxy, Trialkylsilyl, Alkylsulfonyl, Arylsulfonyl, Acyl, Alkoxycarbonyl, Alkylsulfinyl, $-OCONH_2$, -OCONH-Alkyl, Alkylaminoalkyl, Dialkylaminoalkyl, -COOH, $-CON(R^{20})_2$, $-OCON(Alkyl)_2$, -NHCOO-Alkyl, -NHCO-Alkyl, Phenyl, Hydroxyalkyl oder Morpholino; jedes $R^5$ und $R^6$ unabhängig ausgewählt ist aus der Gruppe bestehend aus H und Alkyl mit der Maßgabe, dass, wenn X $C(OR^5)_2$ oder $C(SR^5)_2$ ist, nicht beide $R^5$-Gruppen H sein können und zusätzlich, wenn X $C(OR^5)_2$ oder $C(SR^5)_2$ ist, die beiden $R^5$-Gruppen in X unter Bildung von $-(CR^{20}{}_2)_p-$ verbunden sein können, wobei p eine ganze Zahl von 2 bis 4 ist;
$R^7$ unabhängig ausgewählt ist aus der Gruppe bestehend aus H, Alkyl, Arylalkyl, Cycloalkyl, Aryl und Aryl, das mit $R^3$ und $R^4$ wie hier definiert substituiert ist;
jedes $R^8$ unabhängig ausgewählt ist aus der Gruppe bestehend aus H, Hydroxyalkyl oder Alkyl, oder zwei $R^8$-Gruppen unter Bildung einer Alkylengruppe verbunden sein können;
$R^9$ H, Alkyl, Aralkyl oder Acyl ist;
$R^{20}$ H, Aryl oder Alkyl ist;
$R^{27}$ und $R^{28}$ unabhängig ausgewählt sind aus der Gruppe bestehend aus H, Alkyl, Hydroxyalkyl, Arylalkyl, Aminoalkyl, Halogenalkyl, Thioalkyl, Alkylthioalkyl, Carboxyalkyl, Imidazolylalkyl und Indolylalkyl; oder $R^{27}$ und $R^{28}$ unter Bildung einer Alkylengruppe kombinieren können;
$R^{29}$ H, Alkyl, -CO-Alkyl, -CO-Cycloalkyl, Alkoxycarbonyl, Aminocarbonyl, Aryloxycarbonyl, Alkylaminocarbonyl, Dialkylaminocarbonyl, Alkylsulfonyl, Arylsulfonyl oder $-SO_2-NH-R^{20}-$ ist;
$R^{30}$ H, Alkyl, Aryl, Cycloalkyl, Hydroxyalkyl, Aminoalkyl, $-COOR^{20}$, $-CON(R^{20})_2$ oder Cyano ist;
$R^{31}$ und $R^{32}$ die gleichen wie $R^{30}$ sind und außerdem zwei $R^{30}$-, $R^{31}$- und $R^{32}$-Gruppen die Gruppe $-(CH_2)_r-$ bilden können (in der r 1 bis 6 ist), wobei ferner $R^{31}$ und $R^{32}$ außerdem Hydroxy, $-N(R^{20})_2$, -O-Acyl, $-N(R^{20})$ Acyl, $-OCOOR^{20}$ oder $-OCON(R^{20})_2$ sein können;
$R^{33}$ Aryl oder Heteroaryl ist mit der Maßgabe, dass, wenn $R^{33}$ Heteroaryl ist, die $CO-R^{33}$-Bindung zu einem Kohlenstoffatom in der $R^{33}$-Gruppe führt;
$R^{34}$ Alkyl, Cycloalkyl oder Aryl ist und außerdem $R^{34}$ auch H sein kann, wenn $R^1$ und $R^{21}$ zusammen mit dem Kohlenstoffatom, an das sie gebunden sind, einen gesättigten heterocyclischen Ring bilden, der 3 bis 7 Kohlenstoffatome enthält und zwei Gruppen enthält, die unabhängig ausgewählt sind aus S, O und $N-R^{20}$;
$R^{35}$ $-CH_2-$, $-NR^{20}-$ oder -O- ist;
$R^{36}$ $-NH_2$, Alkyl oder Alkoxy ist;
$R^{37}$ unabhängig ausgewählt ist aus der Gruppe bestehend aus H und Alkyl;
$R^{38}$ -CO- $(CH_2)_{0-5}-OR^5$, $-SO_2-$ (Alkyl) oder

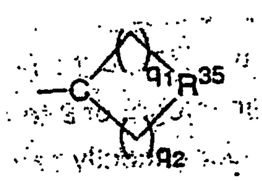

ist, worin $q_1$ und $q_2$ unabhängig 1 bis 5 sind mit der Maßgabe, dass die Summe aus $q_1$ und $q_2$ 2 bis 5 ist; und
$R^{39}$ und $R^{40}$ unabhängig ausgewählt sind aus der Gruppe bestehend aus =O und (H,H);

wobei
"Alkyl" eine geradkettige oder verzweigte gesättigte Kohlenwasserstoffkette mit 1 bis 20 Kohlenstoffatomen, vorzugsweise 1 bis 8 Kohlenstoffatomen wiedergibt;
"Alkenyl" eine geradkettige oder verzweigte Kohlenwasserstoffkette mit 2 bis 15, vorzugsweise 2 bis 12 Kohlenstoffatomen mit mindestens einer Kohlenstoff-Kohlenstoff-Doppelbindung wiedergibt;
"Alkinyl" eine geradkettige oder verzweigte Kohlenwasserstoffkette mit 2 bis 10, vorzugsweise 2 bis 8 Kohlenstoffatomen mit mindestens einer Kohlenstoff-Kohlenstoff-Dreifachbindung wiedergibt;
"Bicycloalkyl" einen carbocyclischen Ring mit 3 bis 12 Kohlenstoffatomen wiedergibt;
"Cycloalkyl" einen gesättigten carbocyclischen Ring mit 3 bis 12 Kohlenstoffatomen wiedergibt;
"Cycloalkenyl" einen carbocyclischen Ring mit 5 bis 8 Kohlenstoffatomen und mindestens einer Kohlenstoff-Kohlenstoff-Doppelbindung im Ring wiedergibt;
"Acyl" einen Rest einer Carbonsäure mit der Formel Alkyl-CO-, Aryl-CO-, Aralkyl-CO-, Cycloalkyl-CO-, Alkylcycloalkyl-CO- und Heteroaryl-CO- wiedergibt;
"Halogen" Fluor, Chlor, Brom oder Iod wiedergibt;
"Aryl" Phenyl oder Naphthyl wiedergibt, das gegebenenfalls mit 1 bis 5 $R^3$-Gruppen substituiert sind; und
"Heteroaryl" eine cyclische Gruppe mit 5 oder 6 Atomen oder eine bicyclische Gruppe mit 9 oder 10 Atomen wiedergibt, von denen mindestens eines Kohlenstoff ist, und welche mindestens ein O-, S- oder N-Atom aufweist, welches einen carbocyclischen Ring unterbricht, der eine ausreichende Zahl von $\pi$-Elektronen hat, um aromatischen Charakter zu liefern, wobei die Kohlenstoffatome gegebenenfalls durch $R^3$-Gruppen substituiert sein können; die Stickstoffatome gegebenenfalls mit $-R^{20}$ oder $-COR^{20}$-Gruppen substituiert sein können, wobei bevorzugte heteroaromatische Gruppen ausgewählt sind aus Pyridyl, Furanyl, Benzofuranyl, Thienyl, Benzothienyl, Thiazolyl, Thiadiazolyl, Imidazolyl, Pyrimidinyl, Pyrazinyl, Pyridazinyl, 1,3,5-Triazinyl und Indolyl;
"Polyhalogen" das Substituieren von mindestens 2 Halogenatomen bei der Gruppe wiedergibt, die durch den Begriff
"Polyhalogen" modifiziert worden ist.

**2.** Verbindung nach Anspruch 1, bei der Y und Z N sind.

**3.** Verbindung nach Anspruch 1, bei der Y CH und Z N ist.

**4.** Verbindung nach einem der Ansprüche 1, 2 oder 3, bei der R

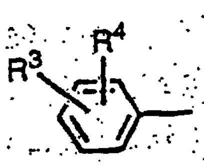 , 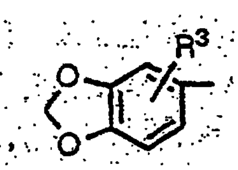 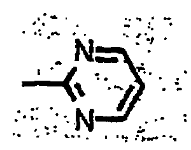

oder

und X O, SO, $SO_2$, $CH_2$, CH(Alkyl), $C(Alkyl)_2$, CH(OH) oder $N(R^{20})CO$ ist.

**5.** Verbindung nach einem der Ansprüche 1, 2, 3, 4 oder 5, bei der $R^3$ und $R^4$ H sind. $R^1$ entweder H, Cycloalkyl oder Alkyl ist und $R^{21}$ H ist, oder $R^1$ und $R^{21}$ zusammen =O oder

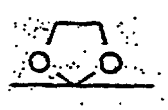

bilden; und wobei mindestens einer von $R^{27}$ und $R^{28}$ Alkyl ist.

**6.** Verbindung nach einem der Ansprüche 1, 2, 3, 4 oder 5, bei der $R^2$

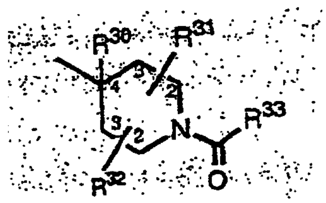

ist und $R^{30}$ H oder $CH_3$ ist, $R^{31}$ und $R^{32}$ H sind und $R^{33}$ ortho-substituiertes Aryl oder Heteroaryl ist.

**7.** Verbindung nach Anspruch 6, bei der $R^{33}$

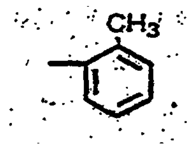, 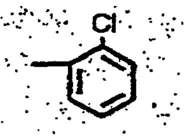, 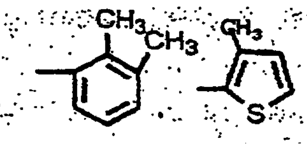, 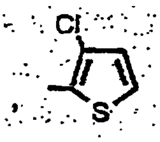 oder 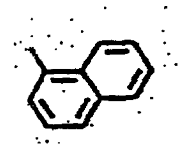

ist.

**8.** Verbindung nach einem der Ansprüche 1 bis 5, bei der $R^2$

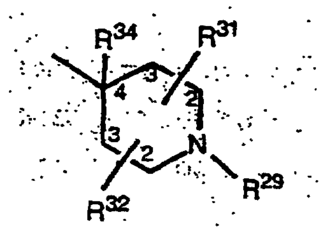

und $R^{34}$ Methyl ist und $R^{31}$ und $R^{32}$ H sind.

**9.** Verbindung nach Anspruch 1 ausgewählt aus der Gruppe bestehend aus:

| | |
|---|---|
| 17 | 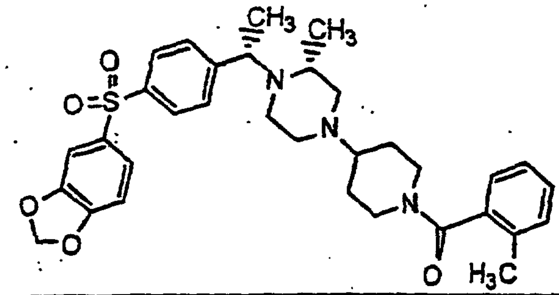 |
| 18 | 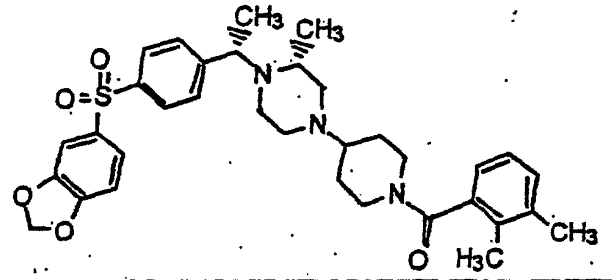 |
| 25 | 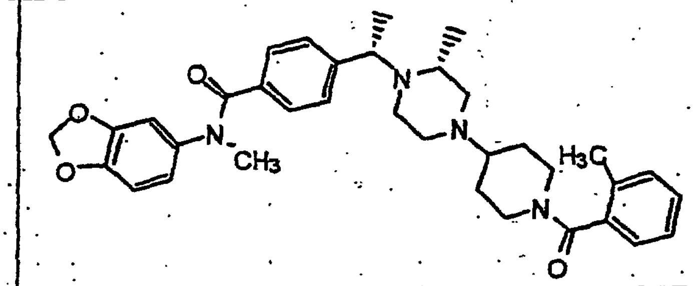 |
| 30 | 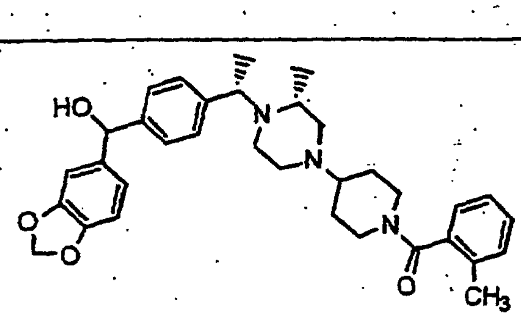 |
| 31 | 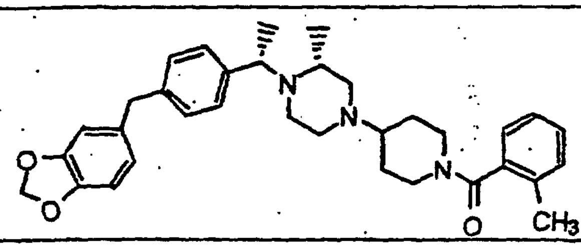 |
| 32 | 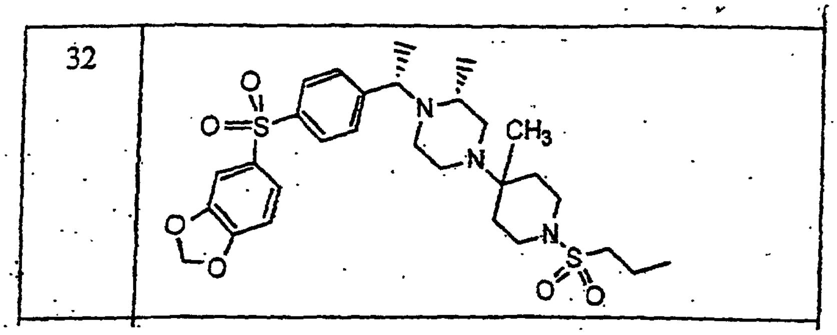 |

| | |
|---|---|
| 34 | 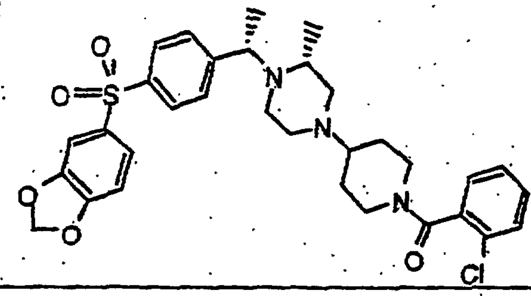 |
| 35 | 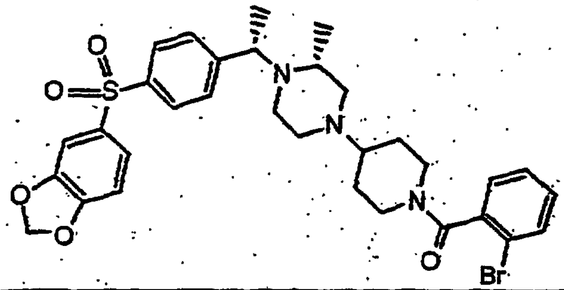 |
| 36 | 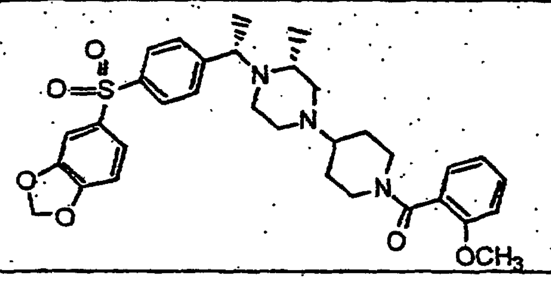 |
| 37 | 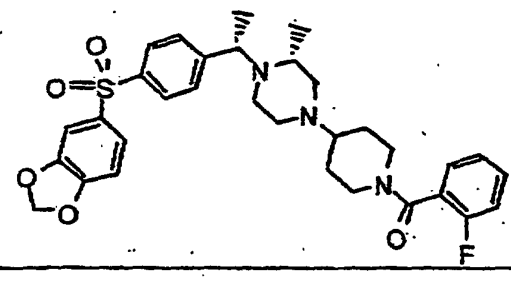 |

| | |
|---|---|
| 41 | 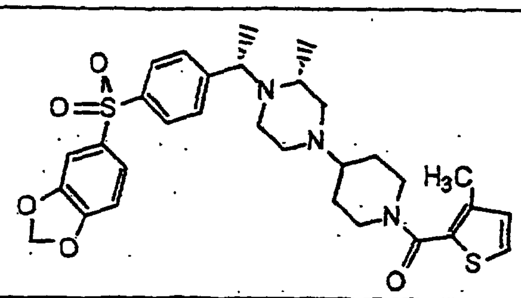 |
| 43 | 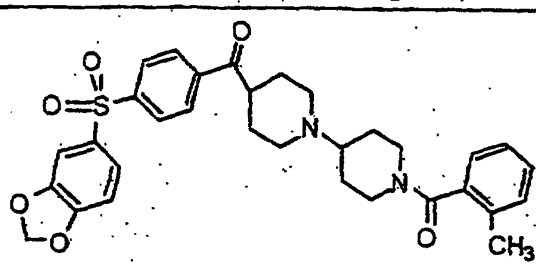 |

| | |
|---|---|
| 44 | 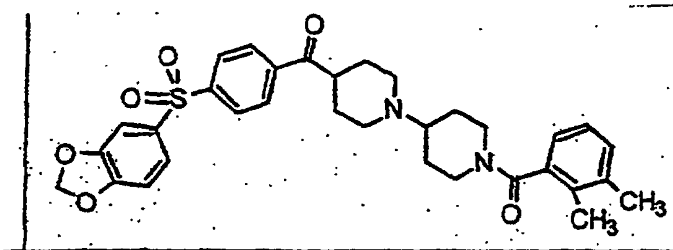 |
| 49 | 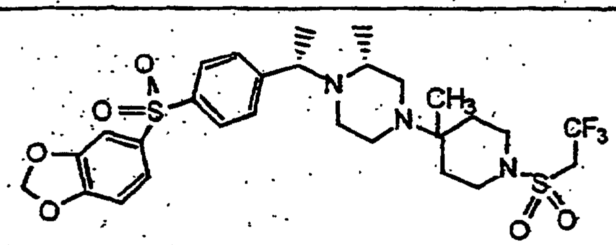 |
| 53 | 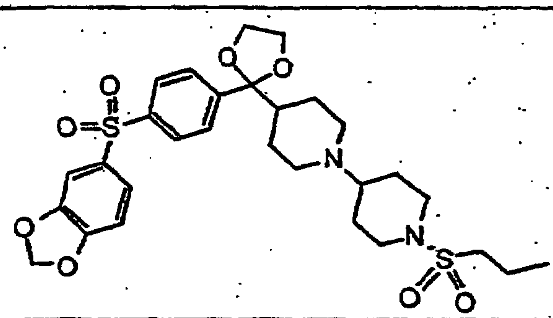 |
| 54 | 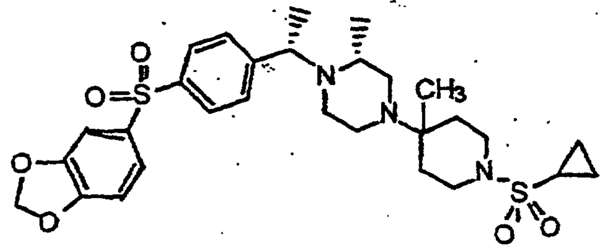 |
| 56 | 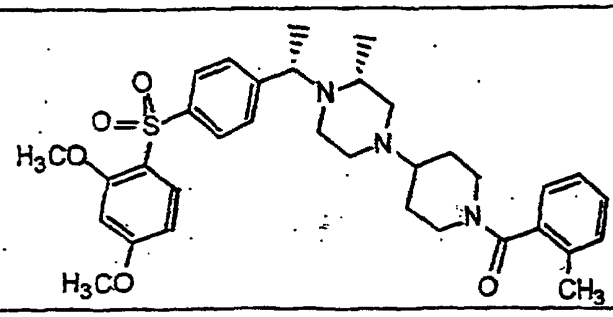 |
| 57 | 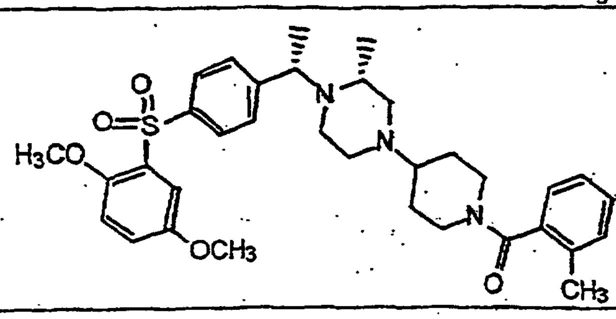 |

| | |
|---|---|
| 58 | 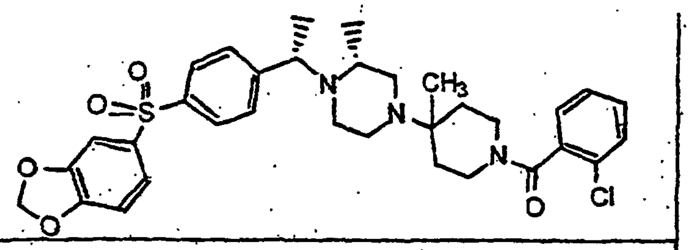 |
| 59 | 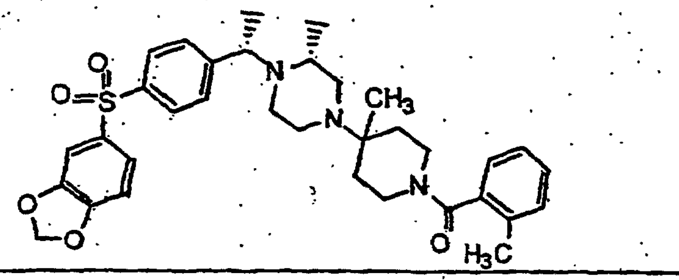 |
| 80 | 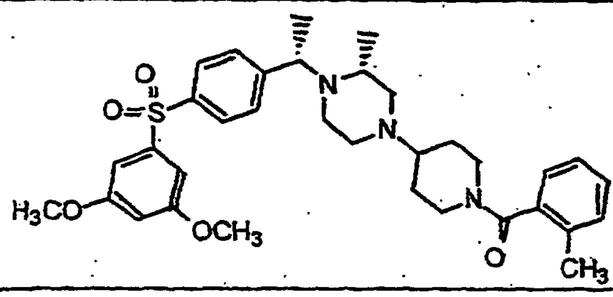 |
| 82 | 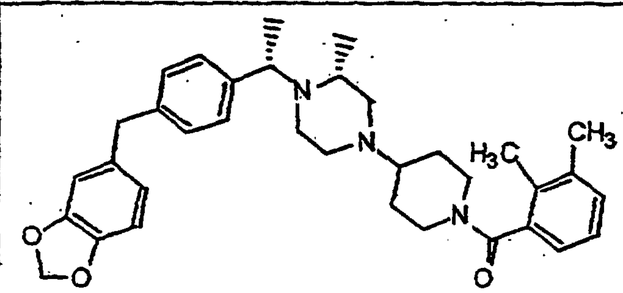 |
| 84 | 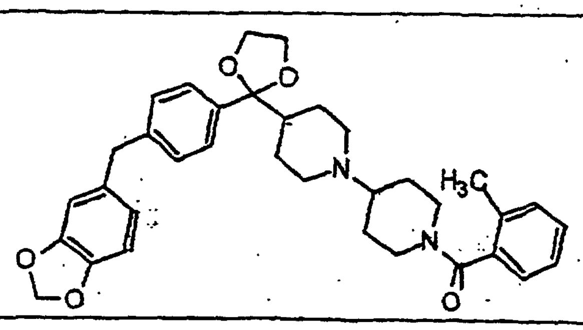 |
| 85 | 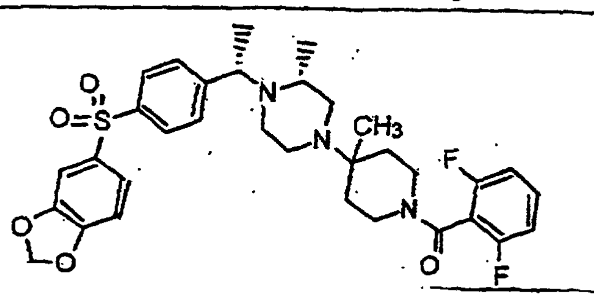 |

| | |
|---|---|
| 94 | 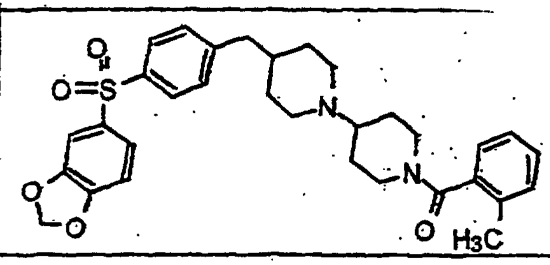 |
| 98 | 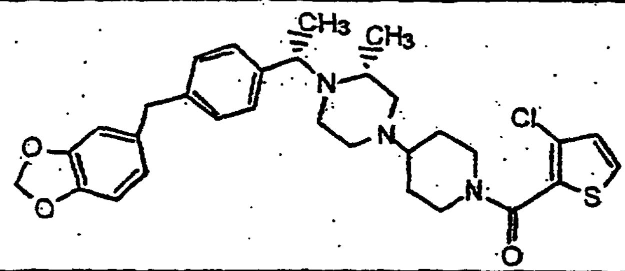 |
| 100 | 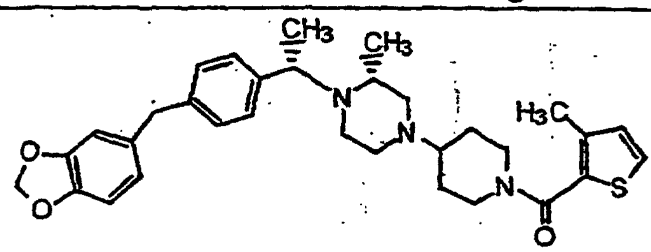 |
| 108 | 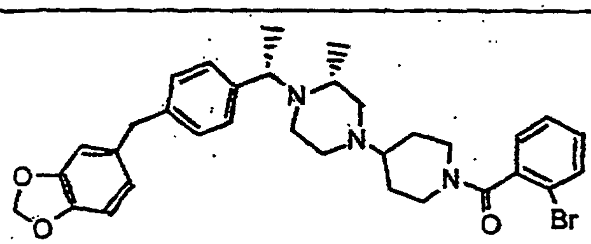 |

oder eine Verbindung ausgewählt aus der Gruppe bestehend aus Verbindungen, die durch die Strukturformeln

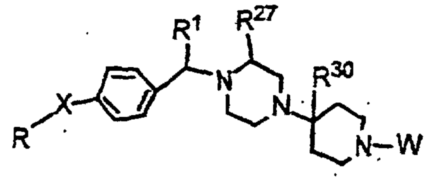

wiedergegeben werden, worin R, $R^1$, $R^{27}$, $R^{30}$ und W wie in der folgenden Tabelle definiert sind:

| No. | R | X | W | $R^1$ | $R^{27}$ | $R^{30}$ |
|---|---|---|---|---|---|---|
| 121 | 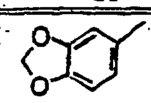 | 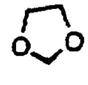 | 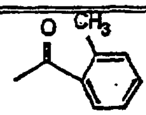 | H | H | H |
| 127 | 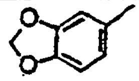 | $-SO_2-$ | 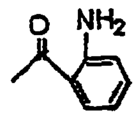 | $CH_3$ | $CH_3$ | H |
| 151 | 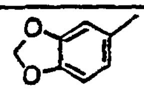 | $-SO_2-$ | 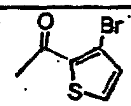 | $CH_3$ | $CH_3$ | H |
| 152 | 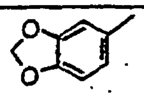 | $-SO_2-$ | 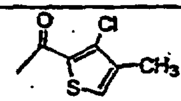 | $CH_3$ | $CH_3$ | H |
| 154 | 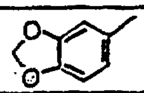 | $-SO_2-$ | 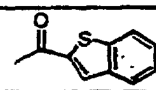 | $CH_3$ | $CH_3$ | H |
| 155 | 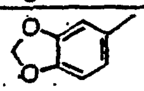 | $-SO_2-$ | 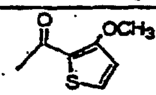 | $CH_3$ | $CH_3$ | H |
| 162 | 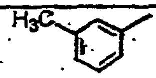 | $-SO_2-$ | 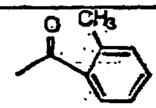 | $CH_3$ | $CH_3$ | H |
| 166 | 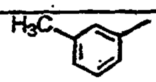 | $-SO_2-$ | 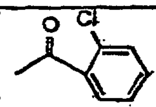 | $CH_3$ | $CH_3$ | H |
| 178 | 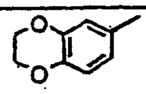 | $-SO_2-$ | 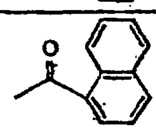 | $CH_3$ | $CH_3$ | H |
| 179 | 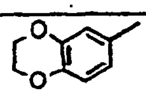 | $-SO_2-$ | 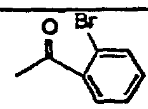 | $CH_3$ | $CH_3$ | H |
| 181 | 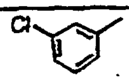 | $-SO_2-$ | 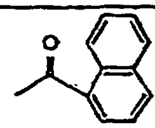 | $CH_3$ | $CH_3$ | H |
| 185 | 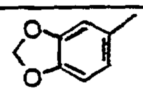 | $-SO_2-$ | 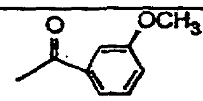 | $CH_3$ | $CH_3$ | H |
| 190 | 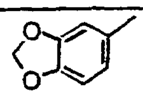 | $-SO_2-$ | 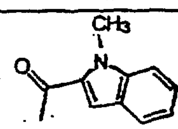 | $CH_3$ | $CH_3$ | H |

| | | | | | | |
|---|---|---|---|---|---|---|
| 191 | 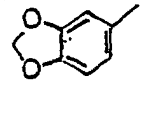 | $-SO_2-$ | 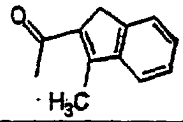 | $CH_3$ | $CH_3$ | H |
| 194 | 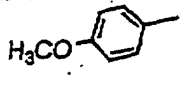 | $-SO_2-$ | 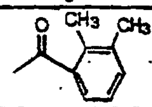 | $CH_3$ | $CH_3$ | H |
| 199 | 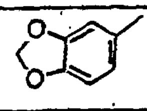 | $-SO_2-$ | 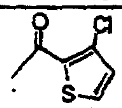 | $CH_3$ | $CH_3$ | H |
| 214 | 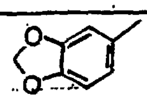 | $-SO_2-$ | 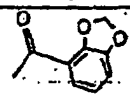 | $CH_3$ | $CH_3$ | H |
| 215 | 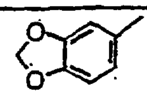 | $-SO_2-$ | 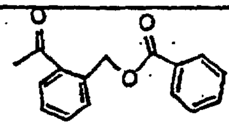 | $CH_3$ | $CH_3$ | H |
| 216 | 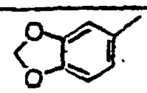 | $-SO_2-$ | 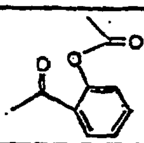 | $CH_3$ | $CH_3$ | H |
| 225 | 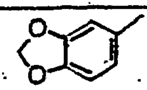 | $-SO_2-$ | 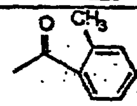 | H | H | H |
| 247 | 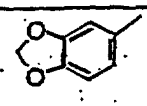 | $-SO_2-$ | 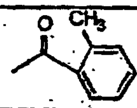 | H | $CH_3$ | H |
| 253 | 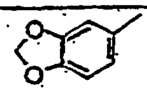 | $-CH_2-$ | 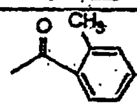 | $CH_3$ | H | H |
| 256 | 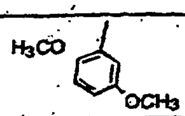 | $-CH_2-$ | 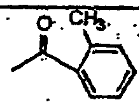 | $CH_3$ | $CH_3$ | H |
| 257 | 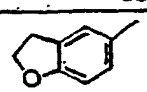 | $-CH_2-$ | 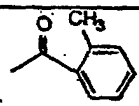 | $CH_3$ | $CH_3$ | H |
| 500 | 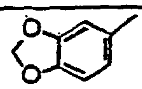 | $-CH_2-$ | 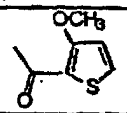 | $CH_3$ | $CH_3$ | H |
| 501 | 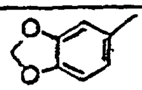 | $-CH_2-$ | 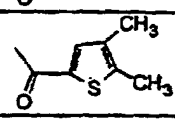 | $CH_3$ | $CH_3$ | H |
| 562 | 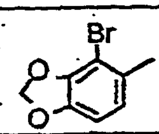 | $-CH_2-$ | 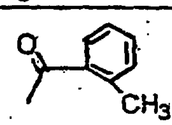 | $CH_3$ | $CH_3$ | H |
| 349 | 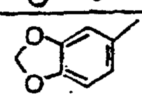 | $-SO_2-$ | 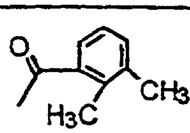 | $CH_3$ | $CH_3$ | $-CH_3$ |

| | | | | | | |
|---|---|---|---|---|---|---|
| 351 | 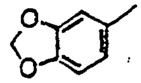 | $-SO_2-$ | 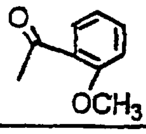 | 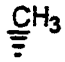 | 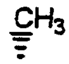 | $-CH_3$ |
| 367 | 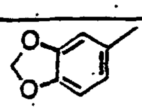 | $-SO_2-$ | 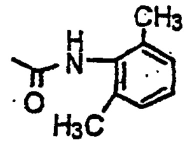 | 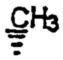 | 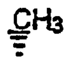 | $-CH_3$ |
| 409 | 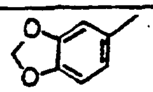 | $-SO_2-$ | 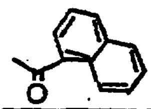 | 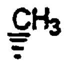 | 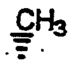 | $-CH_3$ |

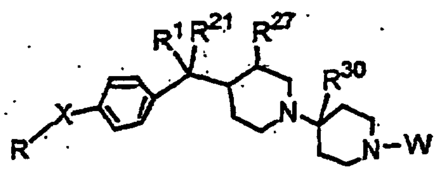

| No. | R | X | W | $R^1$, $R^{21}$ | $R^{30}$ |
|---|---|---|---|---|---|
| 126 | 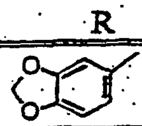 | $-SO_2-$ | $-SO_2-CH_2CH_2CH_3$ | H,H | $-CH_3$ |
| 137 | 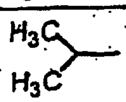 | -S- | 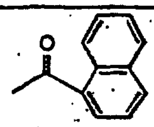 | H,H | H |
| 145 | 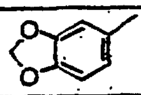 | $-CH_2-$ | 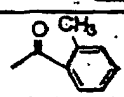 | H,H | H |
| 337 | 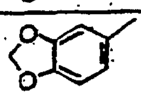 | $-CH_2-$ | 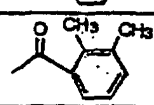 | 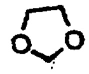 | H |
| 339 | 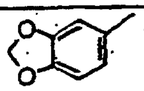 | $-CH_2-$ | 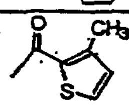 | 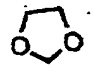 | H |
| 340 | 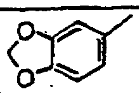 | $-CH_2-$ | 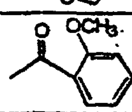 | 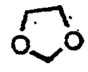 | H |
| 341 | 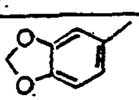 | $-CH_2-$ | 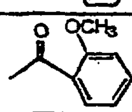 | =O | H |
| 459 | 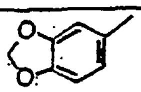 | -S- | 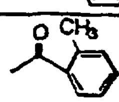 | 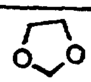 | H |
| 479 | 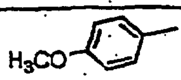 | $-SO_2-$ | 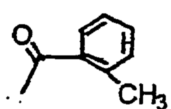 | 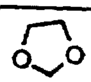 | H |

und Verbindungen mit der Strukturformel

| | | | | | |
|---|---|---|---|---|---|
| 488 | 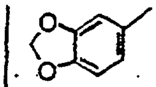 | $-SO_2-$ | 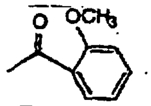 | =O | H |
| 489 | 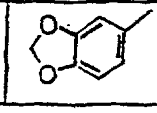 | $-SO_2-$ | 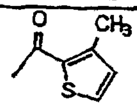 | =O | H |
| 490 | 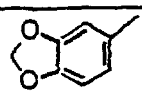 | $-SO_2-$ | 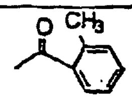 | H, OH | H |
| 502 | 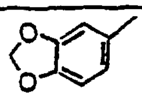 | $-SO_2-$ | 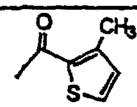 | H, H | H |
| 503 | 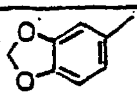 | $-SO_2-$ | 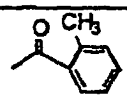 | H, H | H |
| 505 | 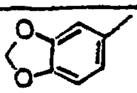 | $-SO_2-$ | 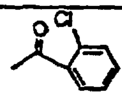 | H, H | H |
| 506 | 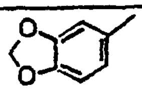 | $-SO_2-$ | 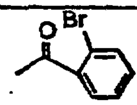 | H, H | H |
| 507 | 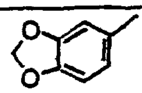 | $-SO_2-$ | 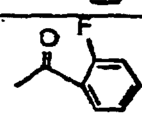 | H, H | H |
| 515 | 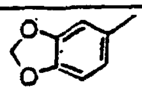 | $-SO_2-$ | 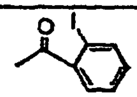 | H, H | H |
| 516 | 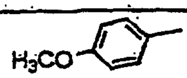 | $-SO_2-$ | 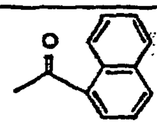 | H, H | H |
| 517 | 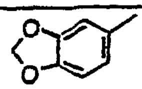 | $-SO_2-$ | 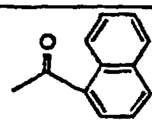 | H, H | H |
| 555 | 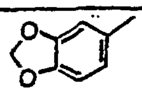 | $-SO_2-$ | 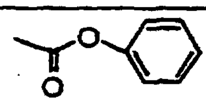 | H,H | $-CH_3$ |

**10.** Verbindung mit der Strukturformel:

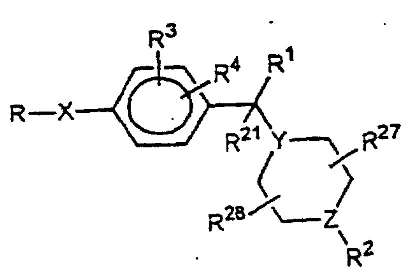

in der R, $R^2$, $R^{27}$, $R^{28}$, X, Y und Z wie in Anspruch 1 definiert sind, $R^3$ und $R^4$ H sind und entweder $R^1$ Cycloalkyl oder Alkyl ist und $R^{21}$ H ist, oder $R^1$ und $R^{21}$ zusammen =O bilden.

**11.** Verbindung nach Anspruch 10 mit der Struktur:

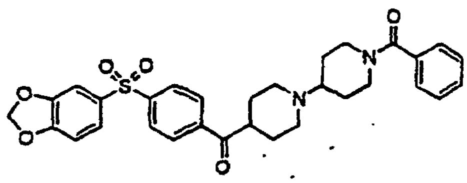

42

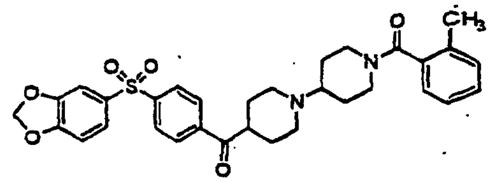

43

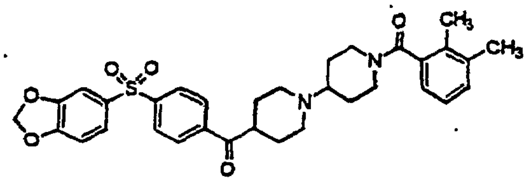

44

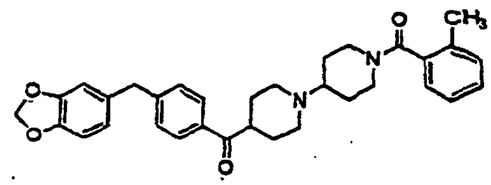

83

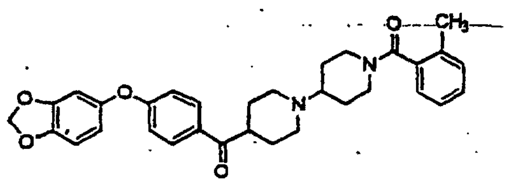

142

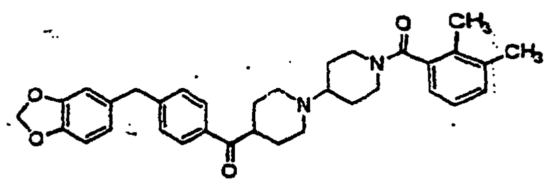

338

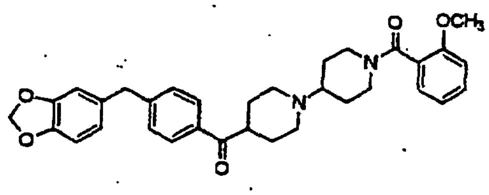

341

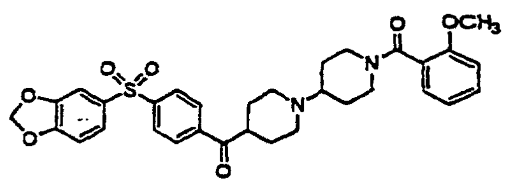

488

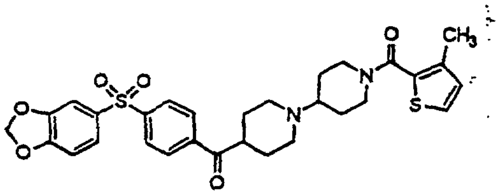

489

oder

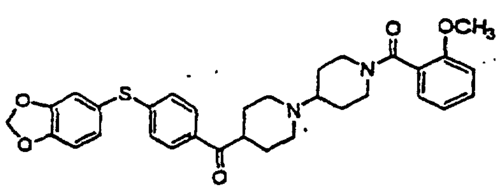

493

**12.** Pharmazeutische Zusammensetzung, die eine Verbindung wie in Anspruch 1 definiert in Kombination mit einem pharmazeutisch annehmbaren Träger enthält.

**13.** Verfahren zur Herstellung einer pharmazeutischen Zusammensetzung wie in Anspruch 12 definiert, bei dem eine Verbindung wie in einem der Ansprüche 1 bis 9 definiert mit einem pharmazeutisch annehmbaren Träger gemischt wird.

**14.** Verwendung einer Verbindung gemäß Anspruch 1 zur Herstellung eines Medikaments zur Behandlung einer kognitiven oder neurodegenerativen Erkrankung.

**15.** Kit zur Behandlung einer kognitiven oder neurodegenerativen Erkrankung, der in separaten Behältern in einer einzigen Packung Verbindungen zur Verwendung in Kombination enthält, in einem Behälter eine Verbindung gemäß Anspruch 1 und in einem separaten Behälter einen Acetylcholinesterase-Inhibitor, wobei die Verbindung und der Inhibitor jeweils in einem pharmazeutisch annehmbaren Träger vorliegen und ihre kombinierten Mengen eine wirksame Menge sind.

**16.** Verwendung einer Verbindung gemäß Anspruch 1 zur Herstellung eines Medikaments zur Behandlung einer kogriitiven oder neurodegenerativen Erkrankung, wobei das Medikament eine wirksame Menge einer Verbindung gemäß Anspruch 1 allein oder in Kombination mit einem Acetylcholinesterase-Inhibitor enthält.

**Revendications**

**1.** Composé de formule développée

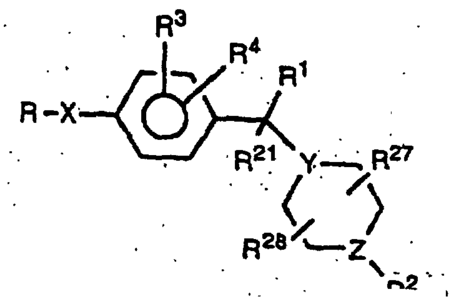

et tous les isomères de ce composé et tous les sels, esters et formes solvatées, pharmaceutiquement acceptables, de ce composé,

formule dans laquelle l'un de Y et Z représente N, et l'autre représente N, CH, ou C-alkyl,

X représente

-O-, -S-, -SO-, $-SO_2-$, $-NR^6-$, -CO-, $-CH_2-$, -CS-, $-C(OR^5)_2-$, $-C(SR^5)_2-$, $-CONR^{20}-$, $-C(alkyl)_2-$, -C(H)(alkyl)-, $-NR^{20}-SO_2-$, $-SO_2-NR^{20}-$, $-NR^{20}CO-$, -O-CO-NH-, -NH-CO-O-,

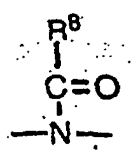 , 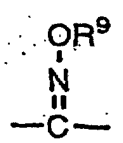 , 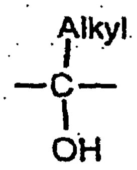 , 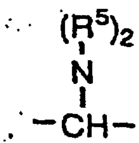 , 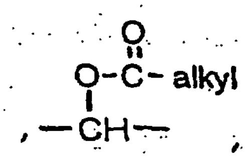 ,

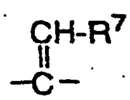 , 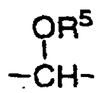 , CH = CH— , —C ≡ C— , 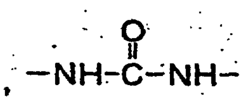 ,

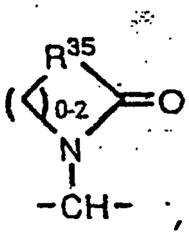 , 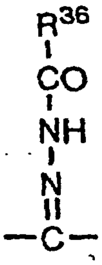 , 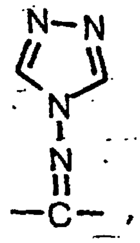 , 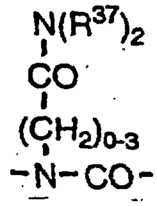 , 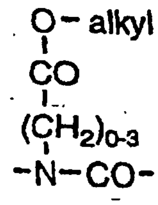 ou 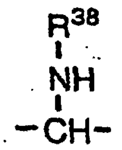 ;

R représente

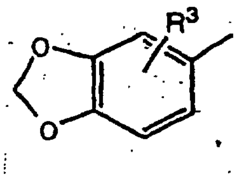 , 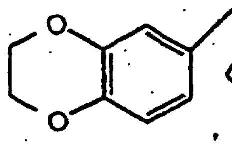 , 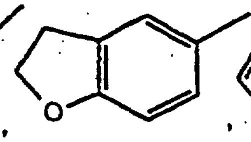 , 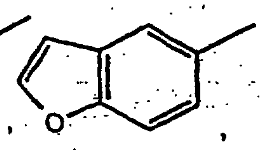 ,

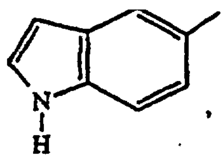 , 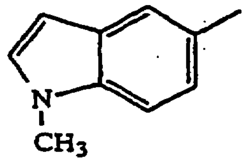 , 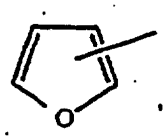 , 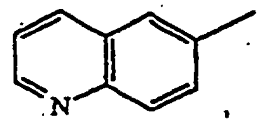 ,

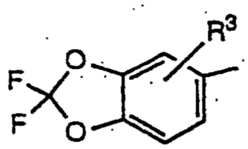 , 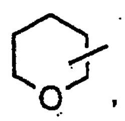 , 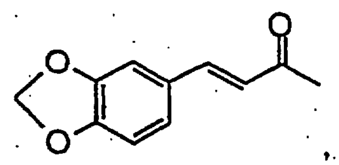 ,

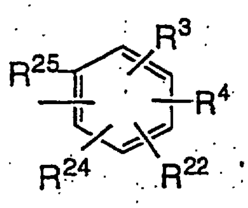 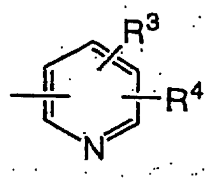 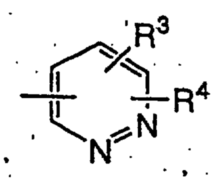 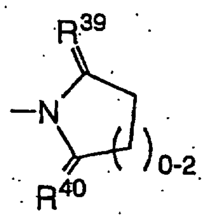 ,

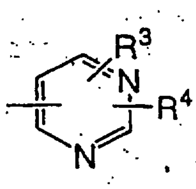 , 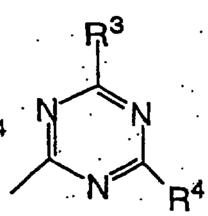 , 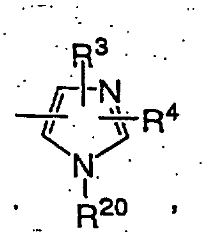 , 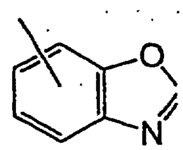

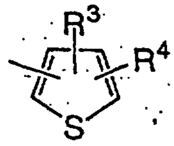 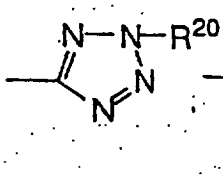 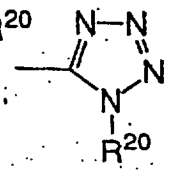 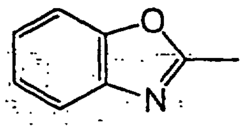 ,

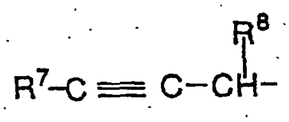 , $R^7-CH_2-C\equiv C-$ , 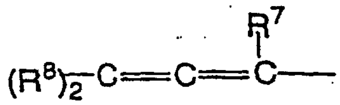

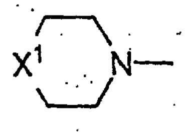

(où $X^1$ est $CH_2$-, -O-, ou -$NR^7$-),

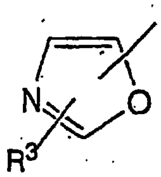 , 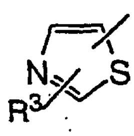 , 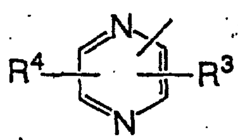 , 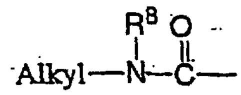 ,

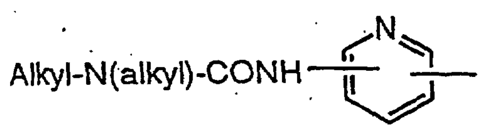 ,

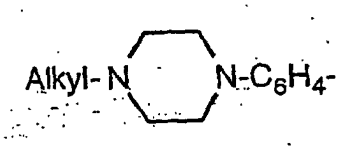 , 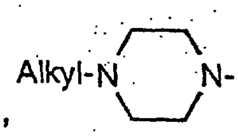 , 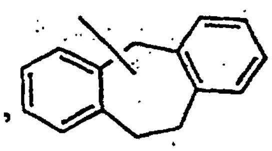 ,

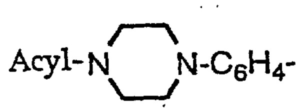 , 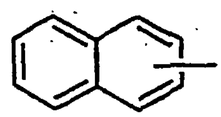

un atome d'hydrogène, un groupe acyle, un groupe alkyle, un groupe alcoxy, un groupe alcényle, un groupe cycloalkyle, un groupe cycloalkyle substitué par un ou deux groupes pris parmi les groupes alkyle et carbonyle, un groupe cycloalcényle, un groupe bicycloalkyle, un groupe arylalcényle, un groupe benzyle, un groupe benzyle substitué par jusqu'à trois groupes $R^3$ choisis indépendamment, un groupe cycloalkylalkyle, un groupe polyhalogénoacyle, un groupe benzyloxyalkyle, un groupe hydroxyalkyle en $C_2$-$C_{20}$, un groupe alcénylcarbonyle, un groupe alkylarylsulfonyle, un groupe alcoxycarbonylaminoacyle, un groupe alkylsulfonyle, ou un groupe arylsulfonyle, et en plus, lorsque X représente -$CH_2$-, R peut aussi être -OH, et en plus, lorsque X n'est pas N, R peut aussi être un groupe hydroxyméthyle, et en plus, R et X peuvent se combiner pour former le groupe Prot-$(NOAA)_r$-NH- où r

désigne un nombre entier de 1 à 4, Prot désigne un groupe protecteur de l'azote, et, lorsque r est égal à 1, NOAA désigne un amino-acide naturel ou un énantiomère de cet amino-acide, ou, lorsque r est égal à 2 à 4, chaque NOAA représente un peptide d'un amino-acide naturel, choisi indépendamment, ou d'un énantiomère d'un tel amino-acide,

$R^1$ et $R^{21}$ représentent chacun, indépendamment, un atome d'hydrogène, ou un groupe alkyle, alcényle, cycloalkyle, cycloalcényle, bicycloalkyle, alcynyle, cyano, aminoalkyle, alcoxycarbonyle, aminocarbonyle, hydroxyamidino, alcoxycarbonylalkyle, phénylalkyle, alkylcarbonyloxyalkyle,

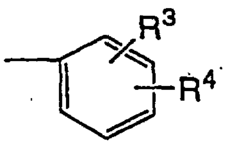 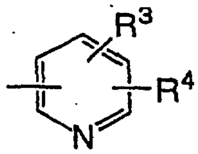 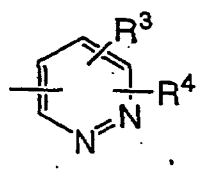 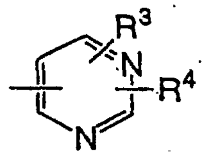 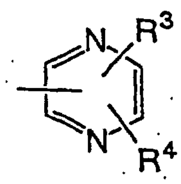 ,

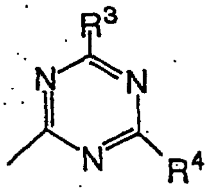 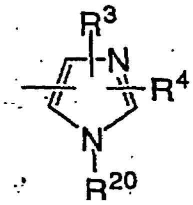 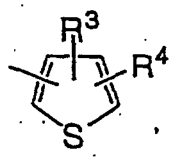 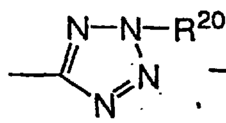 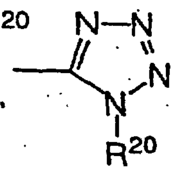

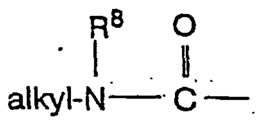 , 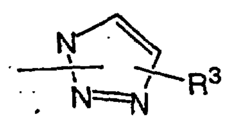 , 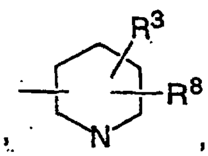 ,

-OH (étant entendu que $R^1$ et $R^{21}$ ne sont pas tous les deux -OH et que Y n'est pas N), formyle, -CO-alkyl, -CO-acyl, -CO-aryl ou hydroxyalkyle, et en plus $R^1$ et $R^{21}$ peuvent former ensemble le groupe $=CH_2$, $=N-OR^5$, =N-CN, $=N-N(R^5)_2$, =CH-alkyl, alkylène,

alkyl
=C-alkyl

ou =C(halogène)2 ; et en plus $R^1$ et $R^{21}$ peuvent former ensemble, avec l'atome de carbone auquel ils sont liés, le groupe

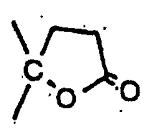 ou 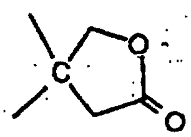

ou bien $R^1$ et $R^{21}$ peuvent former ensemble, avec l'atome de carbone auquel ils sont liés, un noyau hétérocyclique saturé contenant 3 à 7 atomes de carbone dont un ou plusieurs peuvent éventuellement être substitués par un groupe alkyle, et un ou deux groupes choisis indépendamment parmi S, O, et $N-R^{20}$,

$R^2$ représente

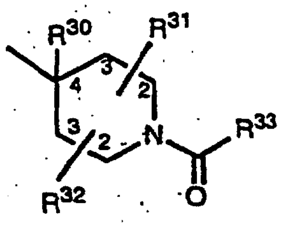 ou 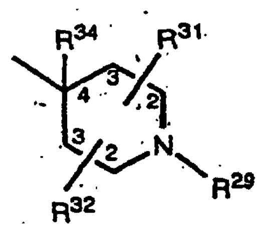

$R^3$, $R^4$, $R^{22}$, $R^{24}$ et $R^{25}$ représentent chacun, indépendamment, un atome d'hydrogène ou d'halogène, ou un groupe alkyle, alcoxy, benzyloxy, benzyloxy substitué par un groupe nitro ou aminoalkyle, halogénoalkyle, polyhalogénoalkyle, nitro, cyano, sulfonyle, hydroxyle, amino, alkylamino, formyle, alkylthio, polyhalogénoalcoxy, acyloxy, trialkylsilyle, alkylsulfonyle, arylsulfonyle, acyle, alcoxycarbonyle, alkylsulfinyle, $-OCONH_2$, -OCONH-alkyl, alkylaminoalkyle, dialkylaminoalkyle, -COOH, $-CON(R^{20})_2$, $-OCON(alkyl)_2$, -NHCOO-alkyl, -NHCO-alkyl, phényle, hydroxyalkyle, ou morpholino,

$R^5$ et $R^6$ représentent chacun, indépendamment, un atome d'hydrogène ou un groupe alkyle, étant entendu que lorsque X est $C(OR^5)_2$ ou $C(SR^5)_2$, les deux groupes $R^5$ ne peuvent pas être H, et en plus, lorsque X est $C(OR^5)_2$ ou $C(SR^5)_2$, les deux groupes $R^5$ de X peuvent être unis pour former $-(CR^{20}{}_2)_P-$ où p désigne un nombre entier de 2 à 4,

$R^7$ représente un atome d'hydrogène, un groupe alkyle, un groupe arylalkyle, un groupe cycloalkyle, un groupe aryle ou un groupe aryle substitué par $R^3$ et $R^4$, tels qu'ils sont définis ici,

chaque $R^8$ représente, indépendamment, un atome d'hydrogène ou un groupe hydroxyalkyle ou alkyle, ou bien deux groupes $R^8$ peuvent être réunis pour former un groupe alkylène,

$R^9$ représente un atome d'hydrogène ou un groupe alkyle, aralkyle ou acyle,

$R^{20}$ représente un atome d'hydrogène ou un groupe aryle ou alkyle,

$R^{27}$ et $R^{28}$ représentent chacun, indépendamment, un atome d'hydrogène ou un groupe alkyle, hydroxyalkyle, arylalkyle, aminoalkyle, halogénoalkyle, thioalkyle, alkylthioalkyle, carboxyalkyle, imidazolylalkyle, ou indolylalkyle, ou bien $R^{27}$ et $R^{28}$ peuvent se combiner pour former un groupe alkylène,

$R^{29}$ représente un atome d'hydrogène ou un groupe alkyle, -CO-alkyl, -CO-cycloalkyl, alcoxycarbonyle, aminocarbonyle, aryloxycarbonyle, alkylaminocarbonyle, dialkylaminocarbonyle, alkylsulfonyle, arylsulfonyle ou $-SO_2-NH-R^{20}$,

$R^{30}$ représente un atome d'hydrogène ou un groupe alkyle, aryle, cycloalkyle, hydroxyalkyle, aminoalkyle, $-COOR^{20}$, $-CON(R^{20})_2$ ou cyano,

$R^{31}$ et $R^{32}$ ont les mêmes significations que $R^{30}$, et en outre deux des groupes $R^{30}$, $R^{31}$ et $R^{32}$ peuvent former le groupe $-(CH_2)_r-$ (où r est égal à 1 à 6), et en plus $R^{31}$ et $R^{32}$ peuvent aussi être un groupe hydroxyle, $-N(R^{20})_2$, -O-acyl, $-N(R^{20})acyl$, $-OCOOR^{20}$, ou $-OCON(R^{20})_2$,

$R^{33}$ représente un groupe aryle ou hétéroaryle, étant entendu que lorsque $R^{33}$ représente un groupe hétéroaryle, la liaison $CO-R^{33}$ est sur un atome de carbone du groupe $R^{33}$,

$R^{34}$ représente un groupe alkyle, cycloalkyle ou aryle, et en outre $R^{34}$ peut également être H lorsque $R^1$ et $R^{21}$ forment ensemble, avec l'atome de carbone auquel ils sont liés, un noyau hétérocyclique saturé contenant 3 à 7 atomes de carbone et deux groupes choisis indépendamment parmi S, O, et $N-R^{20}$,

$R^{35}$ représente $-CH_2-$, $-NR^{20}-$ ou -O-,

$R^{36}$ représente un groupe $-NH_2$, alkyle ou alcoxy,

chaque $R^{37}$ représente, indépendamment, un atome d'hydrogène ou un groupe alkyle,

$R^{38}$ représente $-CO-(CH_2)_{0-5}-OR^5$, $-SO_2-(alkyl)$, ou

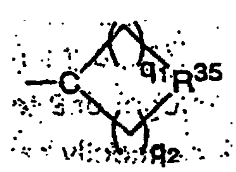

où $q_1$ et $q_2$ représentent chacun, indépendamment, un nombre de 1 à 5, étant entendu que la somme de $q_1$ et $q_2$ est égale à 2 à 5, et

$R^{39}$ et $R^{40}$ représentent chacun, indépendamment, =O ou (H,H), étant entendu que :

« alkyle » désigne une chaîne hydrocarbonée saturée, droite ou ramifiée, ayant 1 à 20 atomes de carbone,

de préférence 1 à 8 atomes de carbone,
« alcényle » désigne une chaîne hydrocarbonée, droite ou ramifiée, ayant 2 à 15 atomes de carbone, de préférence 2 à 12 atomes de carbone, et ayant au moins une double liaison entre deux atomes de carbone,
« alcynyle » désigne une chaîne hydrocarbonée, droite ou ramifiée, ayant 2 à 10 atomes de carbone, de préférence 2 à 8 atomes de carbone, et ayant au moins une triple liaison entre deux atomes de carbone,
« bicycloalkyle » désigne un noyau carbocyclique ayant 3 à 12 atomes de carbone,
« cycloalkyle » désigne un noyau carbocyclique saturé, ayant 3 à 12 atomes de carbone,
« cycloalcényle » désigne un noyau carbocyclique ayant 5 à 8 atomes de carbone et présentant au moins une double liaison entre deux atomes de carbone du cycle,
« acyle » désigne le radical d'un acide carboxylique, de formule alkyl-CO-, aryl-CO-, aralkyl-CO-, cycloalkyl-CO-, alkylcycloalkyl-CO, ou hétéroaryl-CO,
« halogène » désigne le fluor, le chlore, le brome ou l'iode, et « halogéno » désigne fluoro, chloro, bromo ou iodo,
« aryle » désigne phényle ou naphtyle, éventuellement substitué par 1 à 5 groupes $R^3$, et
« hétéroaryle » désigne un groupe cyclique de 5 ou 6 atomes, ou un groupe bicyclique de 9 ou 10 atomes, au moins l'un des atomes étant un atome de carbone et ledit groupe possédant au moins un atome d'oxygène, de soufre ou d'azote, interrompant un noyau carbocyclique et ayant un nombre suffisant d'électrons pi pour avoir le caractère aromatique, les atomes de carbone pouvant éventuellement être substitués par des groupes $R^3$, et les atomes d'azote pouvant éventuellement être substitués par des groupes $-R^{20}$ ou $-COR^{20}$, des groupes hétéroaromatiques préférés étant les groupes pyridyle, furanyle, benzofuranyle, thiényle, benzothiényle, thiazolyle, thiadiazolyle, imidazolyle, pyrimidinyle, pyrazinyle, pyridazinyle, 1,3,5-triazinyle et indolyle,
et « polyhalogéno » indique que le groupe modifié par le terme « polyhalogéno » est substitué par au moins deux atomes d'halogène.

**2.** Composé selon la revendication 1, pour lequel Y et Z représentent N.

**3.** Composé selon la revendication 1, pour lequel Y représente CH et Z représente N.

**4.** Composé selon l'une quelconque des revendications 1 à 3, pour lequel R représente

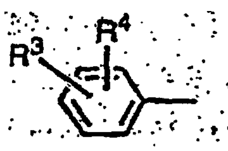
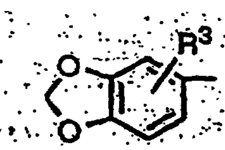
ou
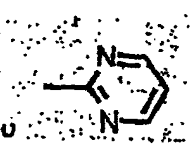

et X représente O, SO, $SO_2$, $CH_2$, CH(alkyl), $C(alkyl)_2$, CH(OH) ou $N(R^{20})CO$.

**5.** Composé selon l'une quelconque des revendications 1 à 4, pour lequel $R^3$ et $R^4$ représentent H, $R^1$ représente H ou un groupe cycloalkyle ou alkyle, et $R^{21}$ représente H, ou bien $R^1$ et $R^{21}$ forment ensemble =O ou

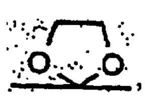

et au moins l'un de $R^{27}$ et $R^{28}$ représente un groupe alkyle.

**6.** Composé selon l'une quelconque des revendications 1 à 5, pour lequel $R^2$ représente

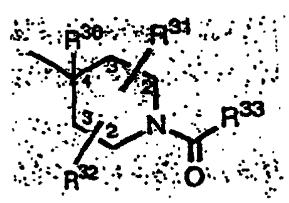

où $R^{30}$ représente H ou $CH_3$, $R^{31}$ et $R^{32}$ représentent H, et $R^{33}$ représente un groupe aryle ou hétéroaryle substitué en position ortho.

**7.** Composé selon la revendication 6, pour lequel $R^{33}$ représente

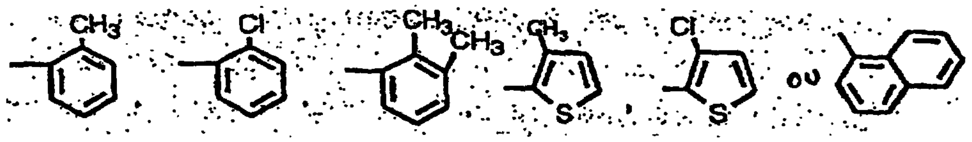
ou

**8.** Composé selon l'une quelconque des revendications 1 à 5, pour lequel $R^2$ représente

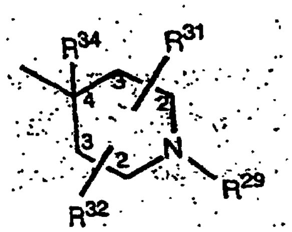

où $R^{34}$ représente un groupe méthyle et $R^{31}$ et $R^{32}$ représentent H.

**9.** Composé selon la revendication 1, qui est choisi parmi les composés suivants :

| | |
|---|---|
| 17 | 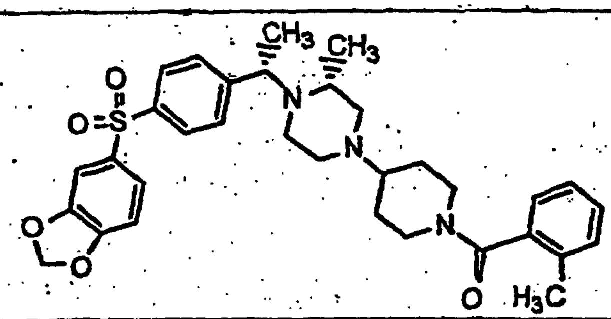 |
| 18 | 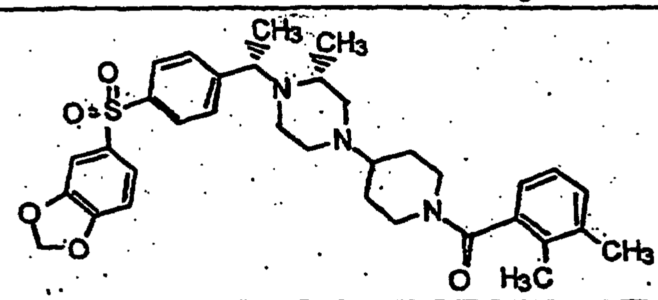 |
| 25 | 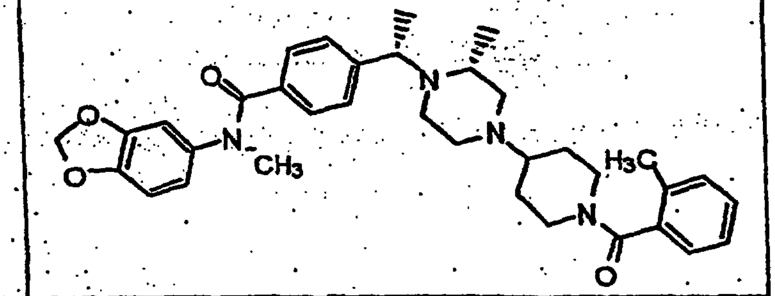 |
| 30 | 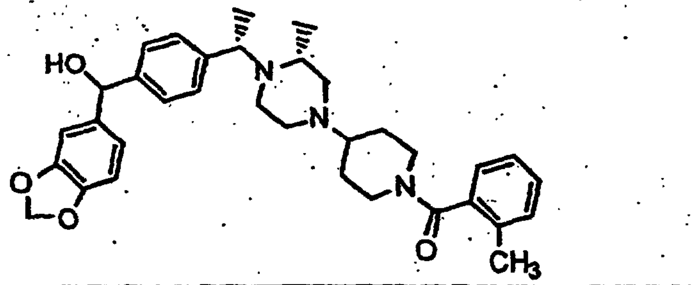 |
| 31 | 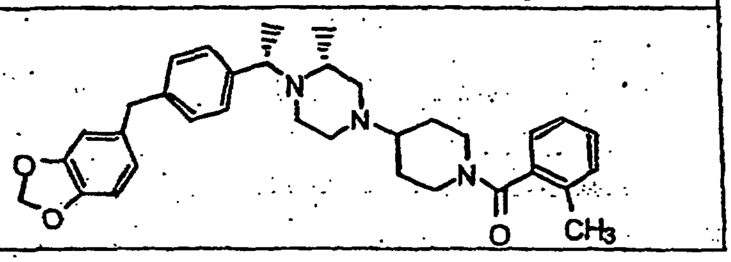 |

| | |
|---|---|
| 32 | 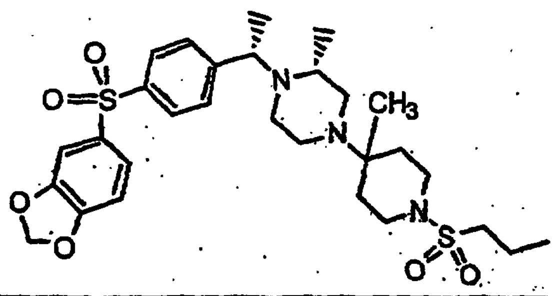 |
| 34 | 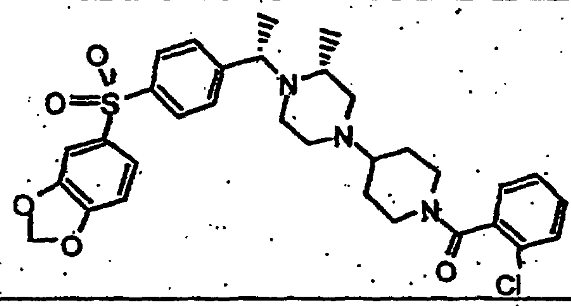 |
| 35 | 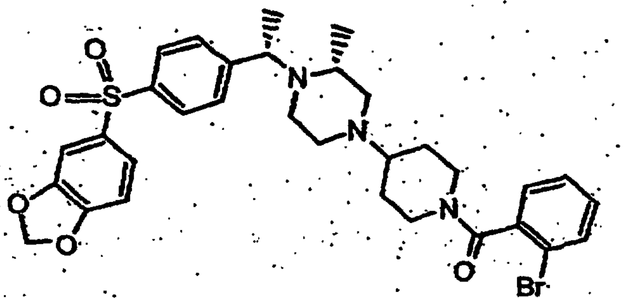 |
| 36 | 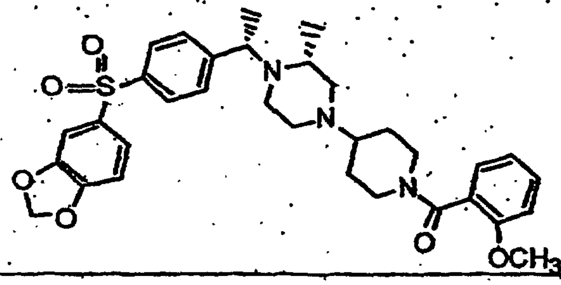 |
| 37 | 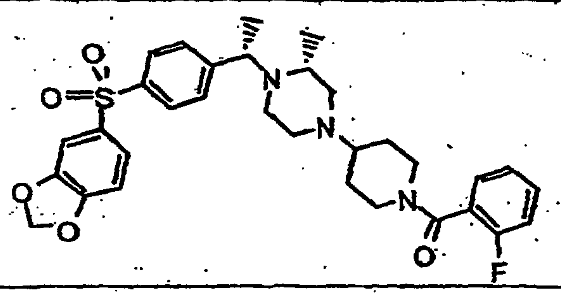 |

| | |
|---|---|
| 41 | 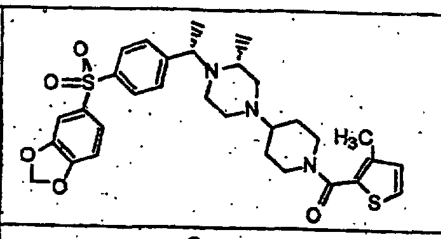 |
| 43 | 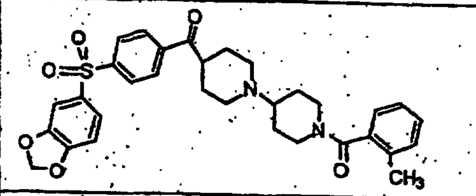 |
| 44 | 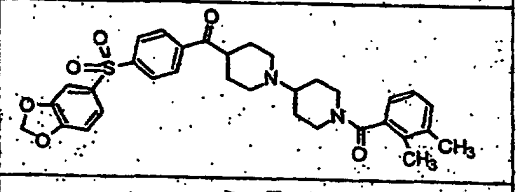 |
| 49 | 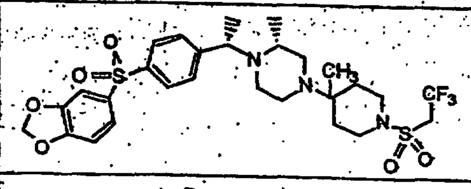 |
| 53 | 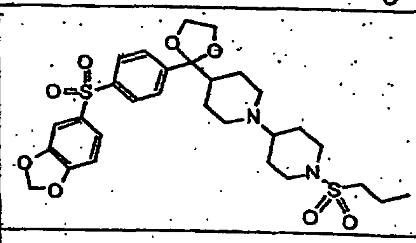 |
| 54 | 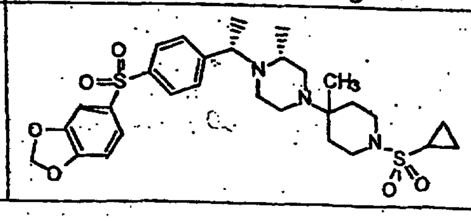 |

| | |
|---|---|
| 56 | 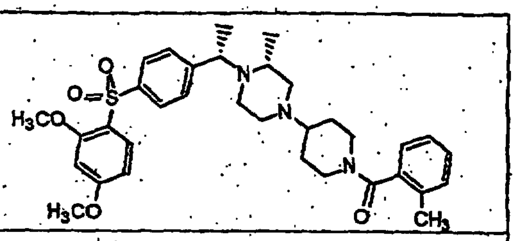 |
| 57 | 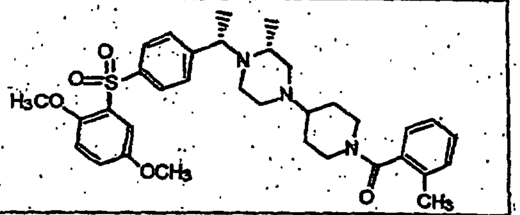 |
| 58 | 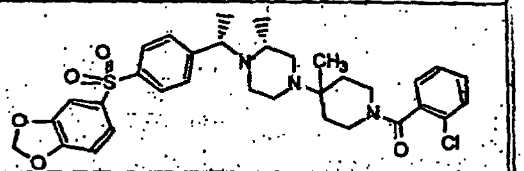 |
| 59 | 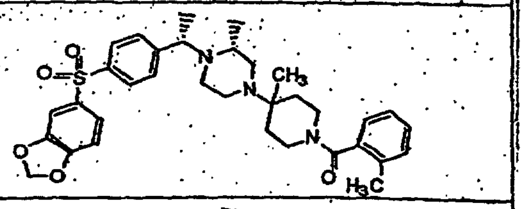 |
| 80 | 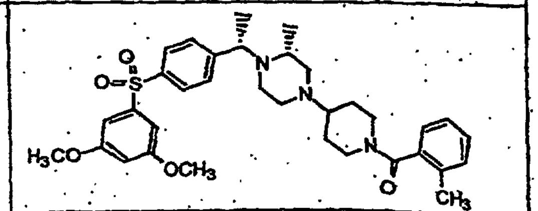 |
| 82 | 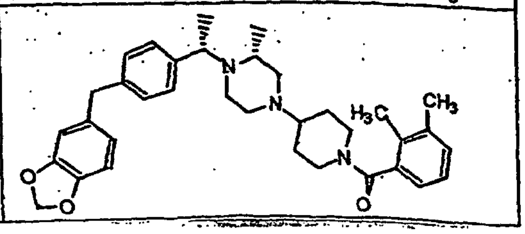 |

| | |
|---|---|
| 84 | 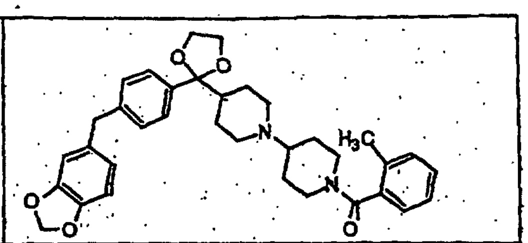 |
| 85 | 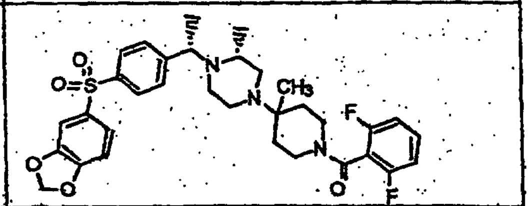 |
| 94 | 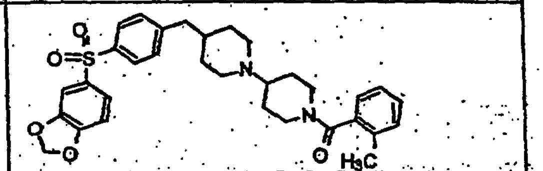 |
| 98 | 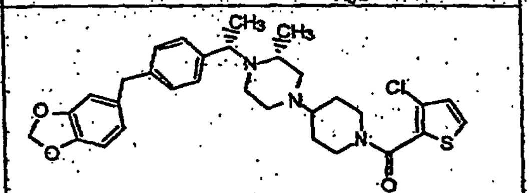 |
| 100 | 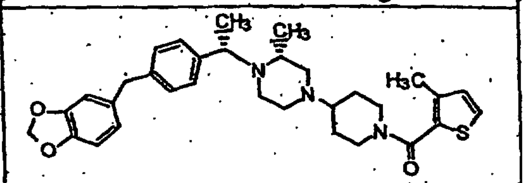 |
| 108 | 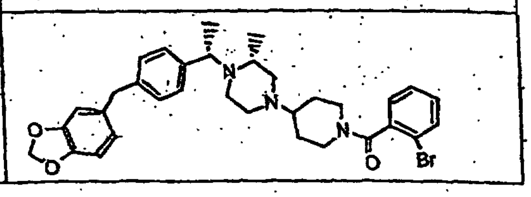 |

ou composé choisi parmi les composés représentés par la formule développée

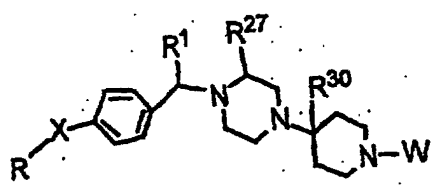

dans laquelle R, X, $R^1$, $R^{27}$, $R^{30}$ et W ont les significations indiquées dans le tableau suivant :

| No. | R | X | W | $R^1$ | $R^{27}$ | $R^{30}$ |
|---|---|---|---|---|---|---|
| 121 | 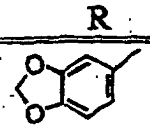 | 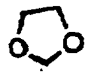 | 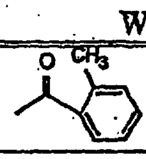 | H | H | H |
| 127 | 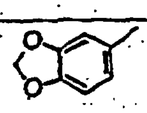 | -SO2- | 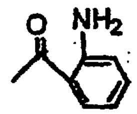 | CH3 | CH3 | H |
| 151 | 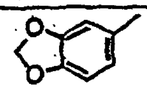 | -SO2- | 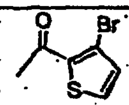 | CH3 | CH3 | H |
| 152 | 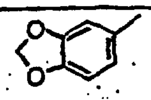 | -SO2- | 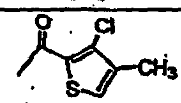 | CH3 | CH3 | H |
| 154 | 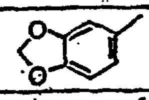 | -SO2- | 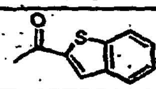 | CH3 | CH3 | H |
| 155 | 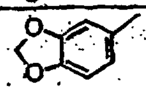 | -SO2- | 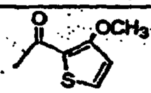 | CH3 | CH3 | H |
| 162 | 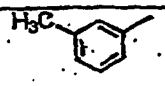 | -SO2- | 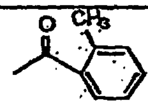 | CH3 | CH3 | H |
| 166 | 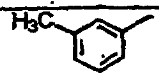 | -SO2- | 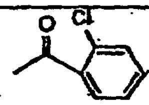 | CH3 | CH3 | H |
| 178 | 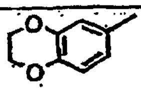 | -SO2- | 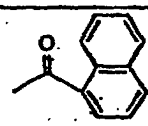 | CH3 | CH3 | H |
| 179 | 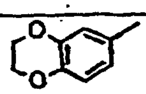 | -SO2- | 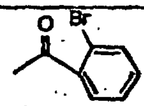 | CH3 | CH3 | H |
| 181 | 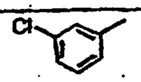 | -SO2- | 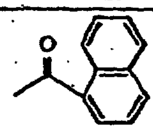 | CH3 | CH3 | H |
| 185 | 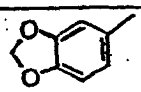 | -SO2- | 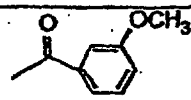 | CH3 | CH3 | H |
| 190 | 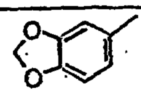 | -SO2- | 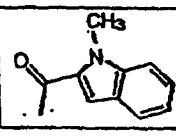 | CH3 | CH3 | H |

| | | | | | | |
|---|---|---|---|---|---|---|
| 191 | 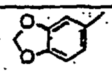 | -$SO_2$- | 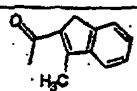 | $CH_3$ | $CH_3$ | H |
| 194 | 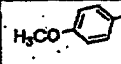 | -$SO_2$- | 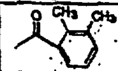 | $CH_3$ | $CH_3$ | H |
| 199 | 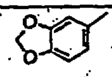 | -$SO_2$- | 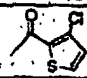 | $CH_3$ | $CH_3$ | H |
| 214 | 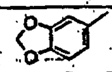 | -$SO_2$- | 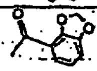 | $CH_3$ | $CH_3$ | H |
| 215 | 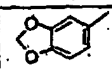 | -$SO_2$- | 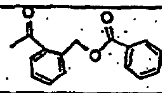 | $CH_3$ | $CH_3$ | H |
| 216 | 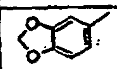 | -$SO_2$- | 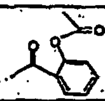 | $CH_3$ | $CH_3$ | H |
| 225 | 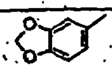 | -$SO_2$- | 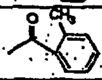 | H | H | H |
| 247 | 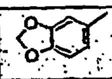 | -$SO_2$- | 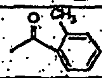 | H | $CH_3$ | H |
| 253 | 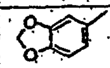 | -$CH_2$- | 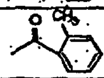 | $CH_3$ | H | H |
| 256 | 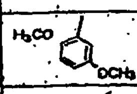 | -$CH_2$- | 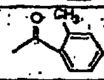 | $CH_3$ | $CH_3$ | H |
| 257 | 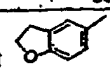 | -$CH_2$- | 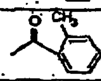 | $CH_3$ | $CH_3$ | H |
| 500 | 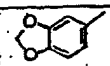 | -$CH_2$- | 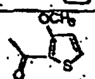 | $CH_3$ | $CH_3$ | H |
| 501 | 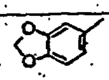 | -$CH_2$- | 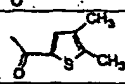 | $CH_3$ | $CH_3$ | H |
| 562 | 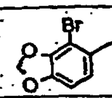 | -$CH_2$- | 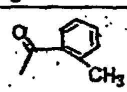 | $CH_3$ | $CH_3$ | H |
| 349 | 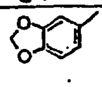 | -$SO_2$- | 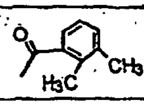 | $CH_3$ | $CH_3$ | -$CH_3$ |

| | | | | | | |
|---|---|---|---|---|---|---|
| 351 | 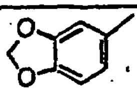 | -$SO_2$- | 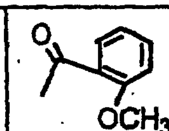 | $CH_3$ | $CH_3$ | -$CH_3$ |
| 367 | 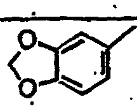 | -$SO_2$- | 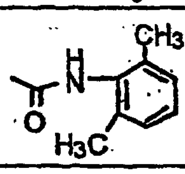 | $CH_3$ | $CH_3$ | -$CH_3$ |
| 409 | 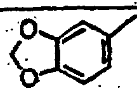 | -$SO_2$- | 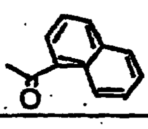 | $CH_3$ | $CH_3$ | -$CH_3$ |

et les composés représentés par la formule développée :

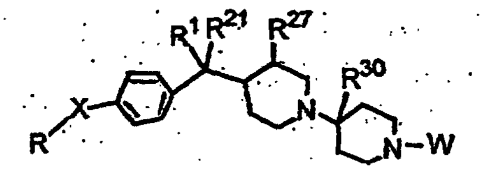

| No. | R | X | W | $R^1$, $R^{21}$ | $R^{30}$ |
|---|---|---|---|---|---|
| 126 | 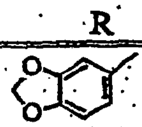 | $-SO_2-$ | $-SO_2-CH_2CH_2CH_3$ | H,H | $-CH_3$ |
| 137 | 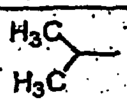 | -S- | 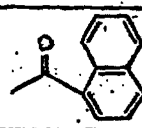 | H,H | H |
| 145 | 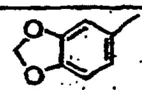 | $-CH_2-$ | 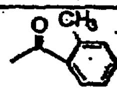 | H,H | H |
| 337 | 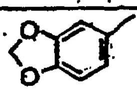 | $-CH_2-$ | 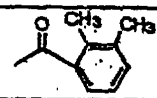 | 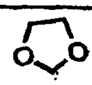 | H |
| 339 | 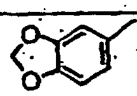 | $-CH_2-$ | 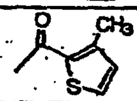 | 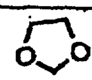 | H |
| 340 | 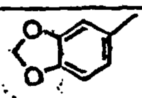 | $-CH_2-$ | 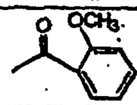 | 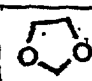 | H |
| 341 | 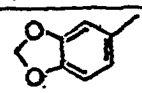 | $-CH_2-$ | 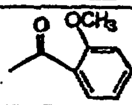 | =O | H |
| 459 | 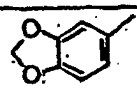 | -S- | 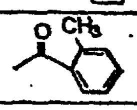 | 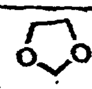 | H |
| 479 | 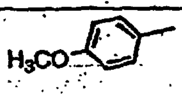 | $-SO_2-$ | 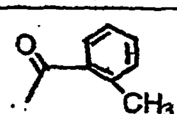 | 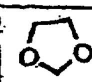 | H |
| 488 | 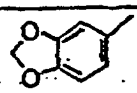 | $-SO_2-$ | 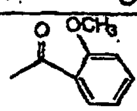 | =O | H |
| 489 | 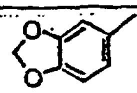 | $-SO_2-$ | 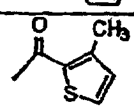 | =O | H |

| | | | | | |
|---|---|---|---|---|---|
| 490 | 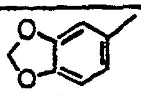 | $-SO_2-$ | 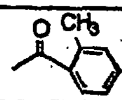 | H, OH | H |
| 502 | 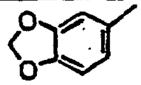 | $-SO_2-$ | 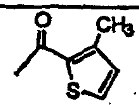 | H, H | H |
| 503 | 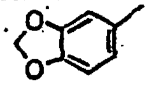 | $-SO_2-$ | 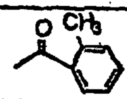 | H, H | H |
| 505 | 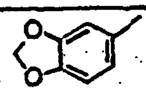 | $-SO_2-$ | 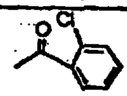 | H, H | H |
| 506 | 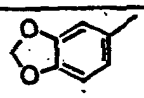 | $-SO_2-$ | 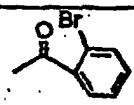 | H, H | H |
| 507 | 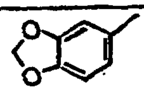 | $-SO_2-$ | 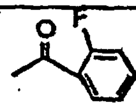 | H, H | H |
| 515 | 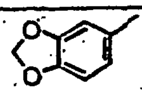 | $-SO_2-$ | 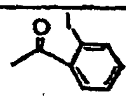 | H, H | H |
| 516 | 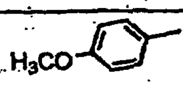 | $-SO_2-$ | 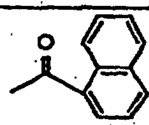 | H, H | H |
| 517 | 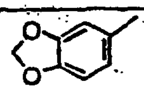 | $-SO_2-$ | 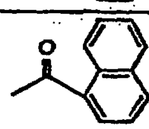 | H, H | H |
| 555 | 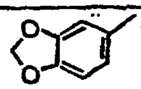 | $-SO_2-$ | 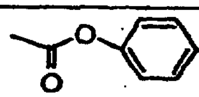 | H,H | $-CH_3$ |

**10.** Composé de formule développée :

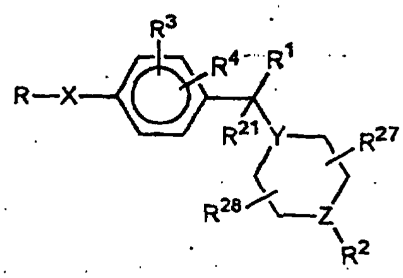

dans laquelle R, $R^2$, $R^{27}$, $R^{28}$, X, Y et Z sont tels que définis à la revendication 1, $R^3$ et $R^4$ représentent H, $R^1$ représente un groupe cycloalkyle ou alkyle et $R^{21}$ représente H, ou bien $R^1$ et $R^{21}$ forment ensemble =O.

**11.** Composé selon la revendication 10, qui a la structure :

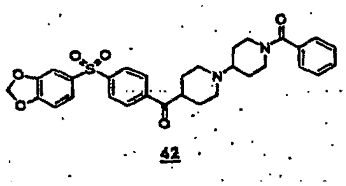

42

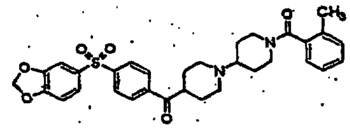

43

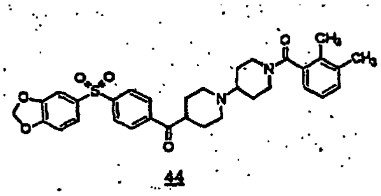

44

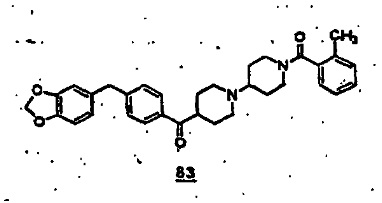

83

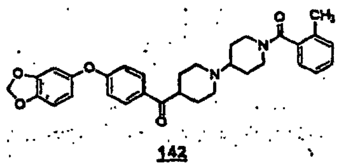

142

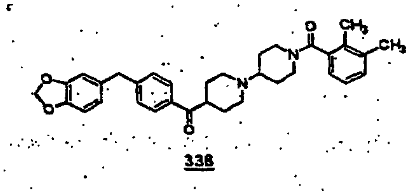

338

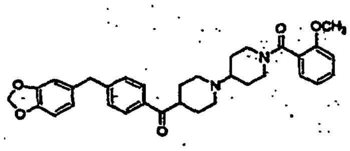

341

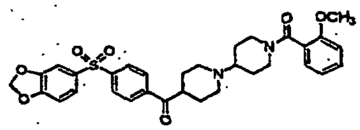

488

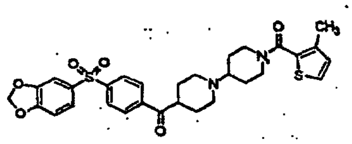

489

ou

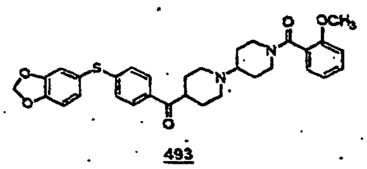

493

**12.** Composition pharmaceutique qui comprend un composé tel que défini dans la revendication 1, en combinaison avec un support pharmaceutiquement acceptable.

**13.** Procédé de préparation d'une composition pharmaceutique telle que définie dans la revendication 12, qui comprend le mélange d'un composé selon l'une quelconque des revendications 1 à 9, avec un support pharmaceutiquement acceptable.

**14.** Utilisation d'un composé selon la revendication 1, pour la préparation de médicaments destinés au traitement des troubles de la connaissance ou des maladies neurodégénératives.

**15.** Kit pour le traitement des troubles de la connaissance ou des maladies neurodégénératives, qui comprend dans des récipients séparés, dans un seul emballage, des composés pharmaceutiques à utiliser en combinaison, à savoir un composé selon la revendication 1, dans un premier récipient, et un inhibiteur d'acétylcholinestérase dans un récipient séparé, ledit composé et ledit inhibiteur étant chacun dans un support pharmaceutiquement acceptable et leurs quantités combinées constituant une dose efficace.

**16.** Utilisation d'un composé selon la revendication 1, dans la préparation d'un médicament, et destiné au traitement

des troubles de la connaissance ou des maladies neurodégénératives, ledit médicament comprenant une quantité efficace d'un composé selon la revendication 1, seul ou en combinaison avec un inhibiteur d'acétylcholinestérase.